# EUROPEAN PATENT APPLICATION

(11) **EP 2 161 320 A2**
(43) Date of publication of application: **10.03.2010**
(21) Application number: 09250919.9
(22) Date of filing: 30.03.2009
(51) Int. Cl.: C09K 11/06

(54) **Fluorene derivatives and organic electroluminescent device using the same**

(30) Priority: 04.09.2008 KR 20080087069
(71) Applicant: Gracel Display Inc., Seoul 133-833 (KR)
(72) Inventor: Kim, Young Gil, Anyang-si, Gyeonggi-do, 431-075 (KR); Cho, Young Jun, Seoul 136-060 (KR); Kwon, Hyuck Joo, Seoul 130-100 (KR); Kim, Bong Ok, Seoul 135-090 (KR); Kim, Sung Min, Seoul-city 157-886 (KR); Yoon, Seung Soo, Seoul 135-884 (KR); Kim, Chi Sik, Seoul 156-825 (KR); Eum, Sung Jin, Seoul (KR)
(74) Representative: Kent, Venetia Katherine

(57) **Abstract**

Provided are a novel organic electroluminescent compound and an organic electroluminescent device using the same as an electroluminescent material. The disclosed organic electroluminescent compound is represented by Chemical Formula 1: 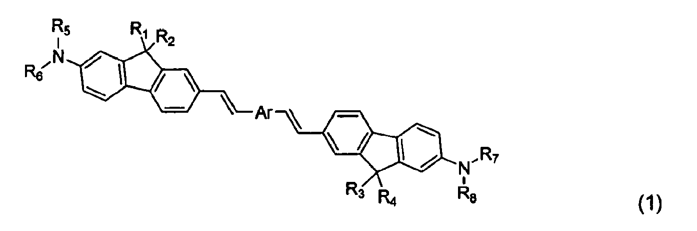 wherein
Ar represents (C6-C60)arylene, (C3-C60)heteroarylene containing one or more heteroatom(s) selected from N, O and S, 5- or 6-membered heterocycloalkylene containing one or more heteroatom(s) selected from N, O and S, (C3-C60)cycloalkylene, adamantylene, (C7-C60)bicycloalkylene, (C2-C60)alkenylene, (C2-C60)alkynylene, (C1-C60)alkylenethio, (C6-C60)alkyleneoxy, (C6-C60)aryleneoxy, (C6-C60)arylenethio or

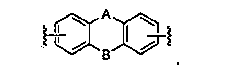

## Description

### CROSS-REFERENCE TO RELATED APPLICATION

This application claims priority to Korean Patent Application No. 10-2008-0087069, filed on September 4, 2008, and all the benefits accruing therefrom under 35 U.S.C. §119.

### BACKGROUND

### 1. Field

The present invention relates to a novel organic electroluminescent compound and an organic electroluminescent device using the same in an electroluminescent layer. More specifically, it relates to a novel organic electroluminescent compound used as a blue-emitting electroluminescent material and an organic electroluminescent device using the same as a dopant.

### 2. Description of the Related Art

Among display devices, electroluminescence (EL) devices are advantageous in that they provide wide view angle, superior contrast and fast response rate as self-emissive display devices. In 1987, Eastman Kodak first developed an organic EL device using low-molecular-weight aromatic diamine and aluminum complex as electroluminescent materials [Appl. Phys. Lett. 51, 913, 1987].

In an organic EL device, the most important factor affecting such quality as luminous efficiency, life property, etc. is the electroluminescent material. Several requirements of the electroluminescent material include high fluorescence quantum yield in solid state, high electron and hole mobility, resistance to decomposition during vacuum deposition, ability to form uniform and thin film, and good stability.

Organic electroluminescent materials may be roughly classified into high-molecular-weight materials and low-molecular-weight materials. The low-molecular-weight materials may be classified into metal complexes and metal-free pure organic electroluminescent materials, depending on molecular structure. Chelate complexes such as tris(8-quinolato)aluminum, coumarin derivatives, tetraphenylbutadiene derivatives, bisstyrylarylene derivatives, oxadiazole derivatives, or the like are known. It is reported that electroluminescence from the blue to the red region can be obtained using these materials, and the realization of full-color display devices is being expected.

For blue fluorescent materials, a lot of materials have been commercialized following Idemitsu Kosan's DPVBi (Compound a). Idemitsu Kosan's blue material system and Kodak's dinaphthylanthracene (Compound b) and tetra(t-butyl)perylene (Compound c) are known, but much more research and development are necessary. Until now, Idemitsu Kosan's distyryl compound system is known to have the best efficiency. It exhibits a power efficiency of 6 Im/W and operation life of 30,000 hours or more. However, its color purity is degraded with time, and operation life when applied to a full-color display is only thousands of hours. In general, blue electroluminescence is advantageous in luminous efficiency if the electroluminescence wavelength shifts a little toward the longer wavelength. But, it is not applicable to high-quality displays because pure blue color is not attained. Therefore, researches and developments to improve color purity, efficiency and thermal stability are highly required.

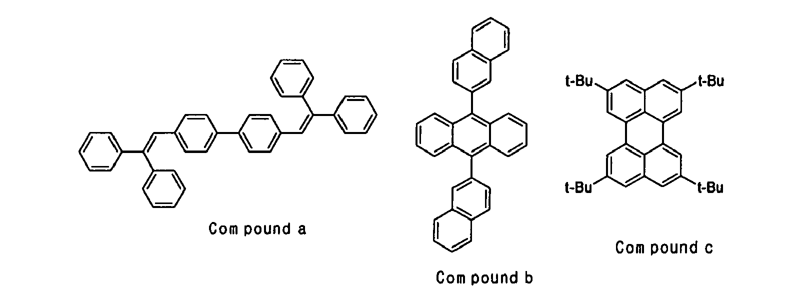

### SUMMARY

The present inventors have invented a novel electroluminescent compound to realize an electroluminescent device having superior luminous efficiency and remarkably improved operation life.

To solve the aforesaid problems, in one aspect, the present invention provides an organic electroluminescent compound having superior backbone structure, which has better luminous efficiency and operation life than existing dopant materials and adequate color coordinates. In another aspect, the present invention provides an organic electroluminescent device having high efficiency and long operation life, which comprises the organic electroluminescent compound as an electroluminescent material.

The present invention relates to an organic electroluminescent compound represented by Chemical Formula 1 and an organic electroluminescent device comprising the same. The organic electroluminescent compound according to the present invention has good luminous efficiency and excellent color purity and life property. Therefore, it can be used to manufacture OLED devices having very superior operation life.

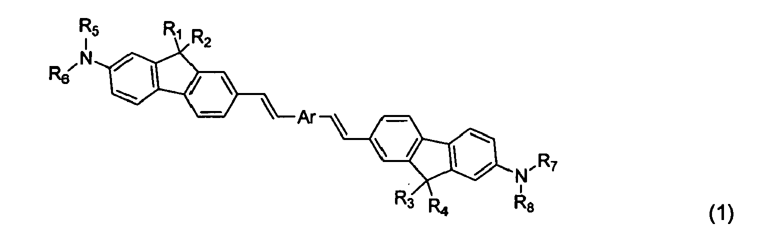

In Chemical Formula 1,
Ar represents (C6-C60)arylene, (C3-C60)heteroarylene, 5- or 6-membered heterocycloalkylene containing one or more heteroatom(s) selected from N, O and S, (C3-C60)cycloalkylene, adamantylene, (C7-C60)bicycloalkylene, (C2-C60)alkenylene, (C2-C60)alkynylene, (C1-C60)alkylenethio, (C1-C60)alkyleneoxy, (C6-C60)aryleneoxy, (C6-C60)arylenethio or 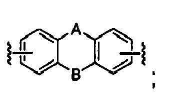
A and B independently represent a chemical bond, -(CR₁₁)(R₁₂)ₘ-, -N(R₁₃)-, -S-, -O-, -Si(R₁₄)(R₁₅)-, -P(R₁₆)-, -C(=O)-, -B(R₁₇)-, -In(R₁₈)-, -Se-, -Ge(R₁₉)(R₂₀)-, -Sn(R₂₁)(R₂₂)-, -Ga(R₂₃)-or -(R₂₄)C=C(R₂₅)-, except for the case where A and B are chemical bonds at the same time;
R₁ through R₈ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁ and R₂, R₃ and R₄, R₅ and R₆, and R₇ and R₈ may be linked via (C3-C60)alkylene or (C3-C60)alkenylene with or without a fused ring to form an alicyclic ring or a mono- or polycyclic aromatic ring;
R₁₁ through R₂₅ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁₁ and R₁₂, R₁₄ and R₁₅, R₁₉ and R₂₀, R₂₁ and R₂₂, and R₂₄ and R₂₅ may be linked via (C3-C60)alkylene or (C3-C60)alkenylene with or without a fused ring to form an alicyclic ring or a mono- or polycyclic aromatic ring;
the arylene, heteroarylene, heterocycloalkylene, cycloalkylene, adamantylene, bicycloalkylene, alkenylene, alkylenethio, alkyleneoxy, aryleneoxy or arylenethio of Ar, and the alkyl, aryl, heteroaryl, heterocycloalkyl, cycloalkyl, trialkylsilyl, dialkylarylsilyl, triarylsilyl, adamantyl, bicycloalkyl, alkenyl, aralkyl, alkyloxy, alkylthio, aryloxy, arylthio, alkylamino, arylamino, alkoxycarbonyl, alkylcarbonyl or arylcarbonyl of R₁ through R₈ and R₁₁ through R₂₅ may be further substituted by one or more substituent(s) selected from a group consisting of deuterium, halogen, (C1-C60)alkyl with or without halogen substituent(s), (C6-C60)aryl, (C3-C60)heteroaryl containing one or more heteroatom(s) selected from N, O and S with or without (C6-C60)aryl substituent(s), morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl; and
m represents an integer from 1 to 3.

### BRIEF DESCRIPTION OF THE DRAWINGS

The above and other aspects, features and advantages of the disclosed exemplary embodiments will be more apparent from the following detailed description taken in conjunction with the accompanying drawings in which:

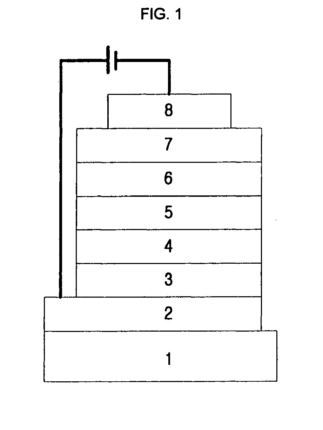

FIG. 1 is a cross-sectional view of an OLED device.

### DETAILED DESCRIPTION

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein. Rather, these exemplary embodiments are provided so that this disclosure will be thorough and complete, and will fully convey the scope of this disclosure to those skilled in the art. In the description, details of well-known features and techniques may be omitted to avoid unnecessarily obscuring the presented embodiments.

The terminology used herein is for the purpose of describing particular embodiments only and is not intended to be limiting of this disclosure. As used herein, the singular forms "a", "an" and "the" are intended to include the plural forms as well, unless the context clearly indicates otherwise. Furthermore, the use of the terms a, an, etc. does not denote a limitation of quantity, but rather denotes the presence of at least one of the referenced item. The use of the terms "first", "second", and the like does not imply any particular order, but they are included to identify individual elements. Moreover, the use of the terms first, second, etc. does not denote any order or importance, but rather the terms first, second, etc. are used to distinguish one element from another. It will be further understood that the terms "comprises" and/or "comprising", or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

In the present invention, "alkyl", "alkoxy" and other substituents including "alkyl" moiety may be either linear or branched.

In the present invention, "aryl" means an organic radical derived from an aromatic hydrocarbon by the removal of one hydrogen, and may include a 4- to 7-membered, particularly 5- or 6-membered, single ring or fused ring. Specific examples include phenyl, naphthyl, biphenyl, anthryl, indenyl, fluorenyl, phenanthryl, triphenylenyl, pyrenyl, perylenyl, chrysenyl, naphthacenyl, fluoranthenyl, etc., but not limited thereto.

In the present invention, "heteroaryl" means aryl group containing 1 to 4 heteroatom(s) selected from N, O and S as aromatic ring backbone atom(s), other remaining aromatic ring backbone atoms being carbon. It may be 5- or 6-membered monocyclic heteroaryl or polycyclic heteroaryl resulting from condensation with a benzene ring, and may be partially saturated. The heteroaryl includes a divalent aryl group wherein the heteroatom(s) in the ring may be oxidized or quaternized to form, for example, N-oxide or quaternary salt. Specific examples include monocyclic heteroaryl such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl, etc., polycyclic heteroaryl such as benzofuranyl, benzothiophenyl, isobenzofuranyl, benzoimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenanthridinyl, benzodioxolyl, etc., N-oxide thereof (e.g., pyridyl N-oxide, quinolyl N-oxide, etc.), quaternary salt thereof, etc., but not limited thereto.

And, in the present invention, the substituents including "(C1-C60)alkyl" moiety may have 1 to 60 carbon atoms, 1 to 20 carbon atoms, or 1 to 10 carbon atoms. The substituents including "(C6-C60)aryl" moiety may have 6 to 60 carbon atoms, 6 to 20 carbon atoms, or 6 to 12 carbon atoms. The substituents including "(C3-C60)heteroaryl" moiety may have 3 to 60 carbon atoms, 4 to 20 carbon atoms, or 4 to 12 carbon atoms. The substituents including "(C3-C60)cycloalkyl" moiety may have 3 to 60 carbon atoms, 3 to 20 carbon atoms, or 3 to 7 carbon atoms. The substituents including "(C2-C60)alkenyl or alkynyl" moiety may have 2 to 60 carbon atoms, 2 to 20 carbon atoms, or 2 to 10 carbon atoms.

Ar is selected from the following structures, but not limited thereto: 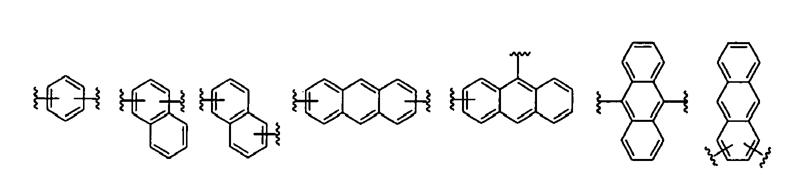 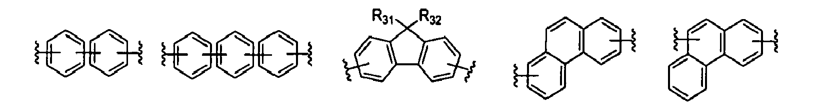 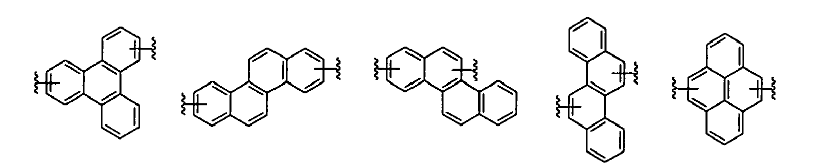 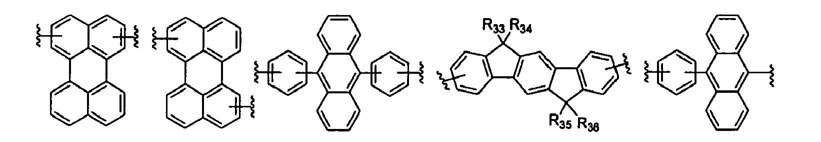 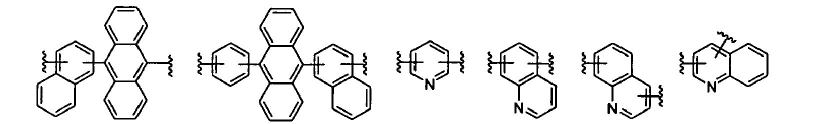 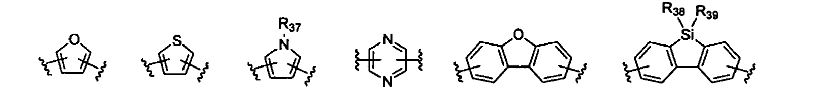 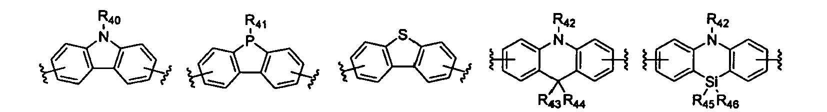 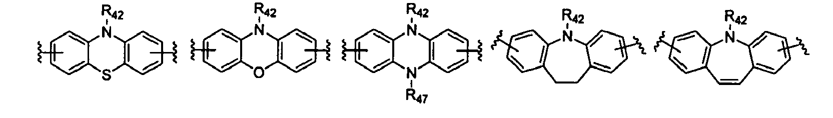 wherein
R₃₁ through R₄₂ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₃ and R₃₄, R₃₅ and R₃₆, R₃₈ and R₃₉, R₄₃ and R₄₄, and R₄₅ and R₄₆ may be linked via (C3-C60)alkylene or (C3-C60)alkenylene with or without a fused ring to form an alicyclic ring or a mono- or polycyclic aromatic ring; and
the alkyl, aryl, heteroaryl, heterocycloalkyl, cycloalkyl, trialkylsilyl, dialkylarylsilyl, triarylsilyl, adamantyl, bicycloalkyl, alkenyl, aralkyl, alkyloxy, alkylthio, aryloxy, arylthio, alkylamino, arylamino, alkoxycarbonyl, alkylcarbonyl or arylcarbonyl of R₃₁ through R₄₂ may be further substituted by one or more substituent(s) selected from a group consisting of deuterium, halogen, (C1-C60)alkyl with or without halogen substituent(s), (C6-C60)aryl, (C3-C60)heteroaryl containing one or more heteroatom(s) selected from N, O and S with or without (C6-C60)aryl substituent(s), morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl.

More specifically, Ar may be exemplified by the following structures, but not limited thereto: 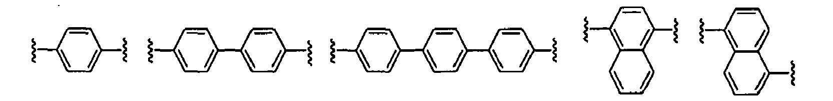 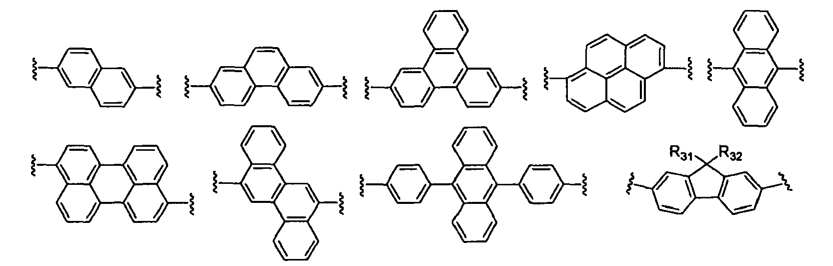 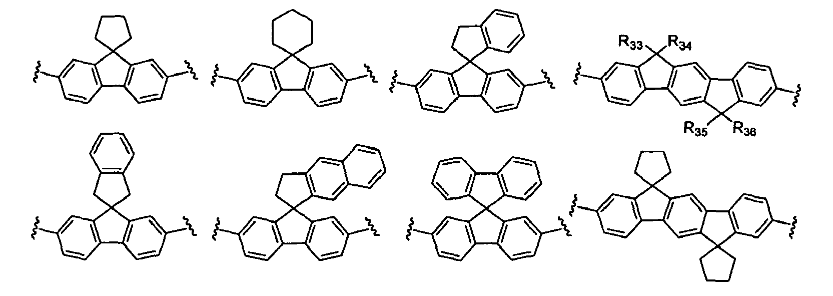 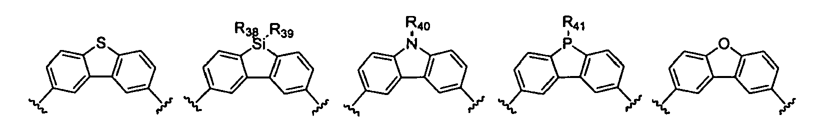 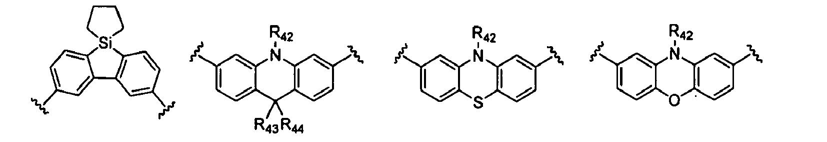 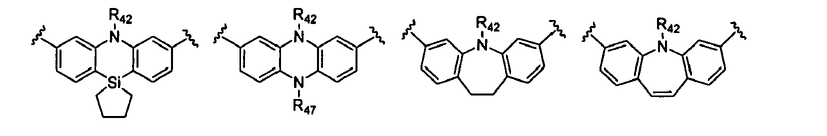 wherein
R₃₁, R₃₂, R₃₃, R₃₄, R₃₅, R₃₆, R₃₈, R₃₉, R₄₀, R₄₁, R₄₂, R₄₃, R₄₄ and R₄₇ independently represent hydrogen, deuterium, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, benzyl, trifluoromethyl, perfluoroethyl, trifluoroethyl, perfluoropropyl, perfluorobutyl, methoxy, ethoxy, n-propoxy, i-propoxy, n-butoxy, i-butoxy, t-butoxy, n-pentoxy, i-pentoxy, n-hexyloxy, n-heptoxy, trimethylsilyl, triethylsilyl, tripropylsilyl, tri(t-butyl)silyl, t-butyldimethylsilyl, dimethylphenylsilyl, triphenylsilyl, cyclopropyl, cyclobutyl, cyclopentyl, cyclohexyl, cycloheptyl, cyclooctyl, cyclononyl, cyclodecyl, phenyl, naphthyl, biphenyl, 9,9-dimethylfluorenyl, 9,9-diphenylfluorenyl, phenanthryl, anthryl, fluoranthenyl, triphenylenyl, pyrenyl, chrysenyl, naphthacenyl, perylenyl, morpholino, thiomorpholino, adamantyl, bicyclo[2.2.1]heptyl, bicyclo[2.2.2]octyl, bicyclo[3.2.1]octyl, bicyclo[5.2.0]nonyl, bicyclo[4.2.2]decyl, bicyclo[2.2.2]octyl, 4-pentylbicyclo[2.2.2]octyl, ethenyl, phenylethenyl, ethynyl, phenylethynyl, cyano, dimethylamino, diphenylamino, monomethylamino, monophenylamino, phenyloxy, phenylthio, methoxycarbonyl, ethoxycarbonyl, t-butoxycarbonyl, carboxyl, nitro, chloro, fluoro or hydroxyl.

More specifically, R₁ through R₄ are independently selected from hydrogen, deuterium, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, benzyl, trifluoromethyl, perfluoroethyl, trifluoroethyl, perfluoropropyl, perfluorobutyl, methoxy, ethoxy, n-propoxy, i-propoxy, n-butoxy, i-butoxy, t-butoxy, n-pentoxy, i-pentoxy, n-hexyloxy, n-heptoxy, trimethylsilyl, triethylsilyl, tripropylsilyl, tri(t-butyl)silyl, t-butyldimethylsilyl, dimethylphenylsilyl, triphenylsilyl, cyclopropyl, cyclobutyl, cyclopentyl, cyclohexyl, cycloheptyl, cyclooctyl, cyclononyl, cyclodecyl, phenyl, naphthyl, biphenyl, 9,9-dimethylfluorenyl, 9,9-diphenylfluorenyl, phenanthryl, anthryl, fluoranthenyl, triphenylenyl, pyrenyl, chrysenyl, naphthacenyl, perylenyl, morpholino, thiomorpholino, adamantyl, bicyclo[2.2.1]heptyl, bicyclo[2.2.2]octyl, bicyclo[3.2.1]octyl, bicyclo[5.2.0]nonyl, bicyclo[4.2.2]decyl, bicyclo[2.2.2]octyl, 4-pentylbicyclo[2.2.2]octyl, ethenyl, phenylethenyl, ethynyl, phenylethynyl, cyano, dimethylamino, diphenylamino, monomethylamino, monophenylamino, phenyloxy, phenylthio, methoxycarbonyl, ethoxycarbonyl, t-butoxycarbonyl, carboxyl, nitro, chloro, fluoro and hydroxyl; and
R₁ and R₂ or R₃ and R₄ may be linked via (C3-C60)alkylene or (C3-C60)alkenylene with or without a fused ring to form an alicyclic ring or a mono- or polycyclic aromatic ring.
the alicyclic ring or the mono- or polycyclic aromatic ring formed as R₁ and R₂ or R₃ and R₄ of 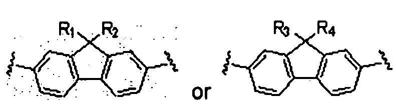 are linked via alkylene or alkenylene with or without a fused ring is selected from the following structures:

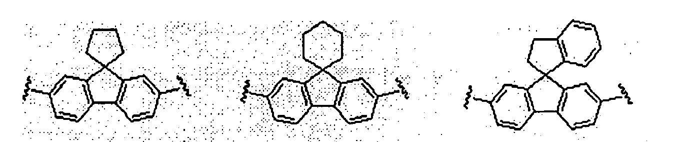

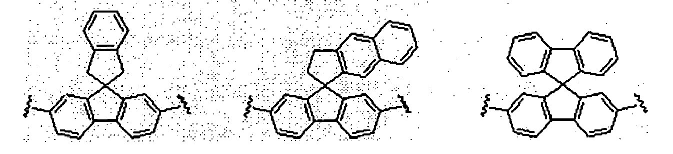

Specifically, R₅ through R₈ may be independently exemplified by the following structures, but not limited thereto:

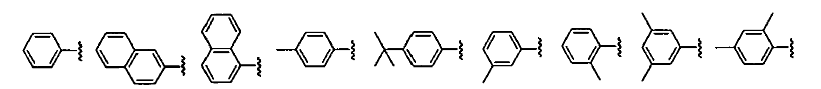

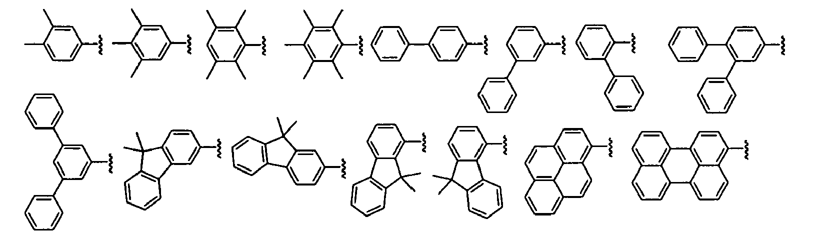

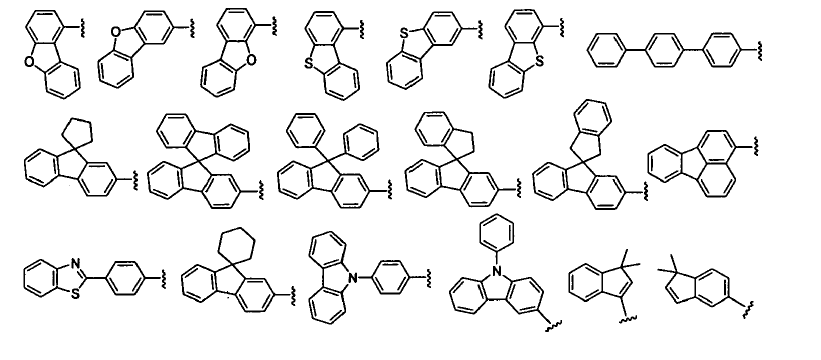

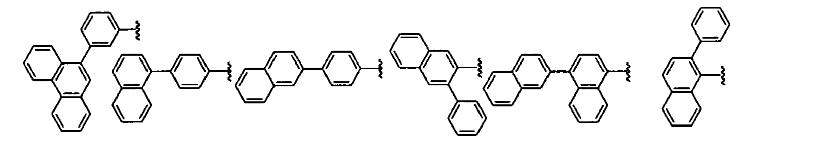

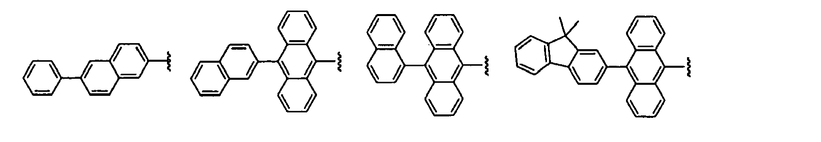

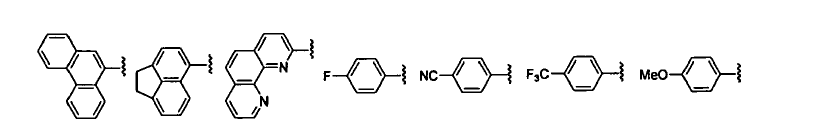

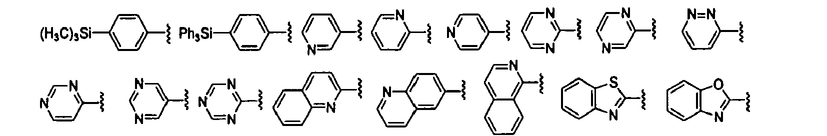

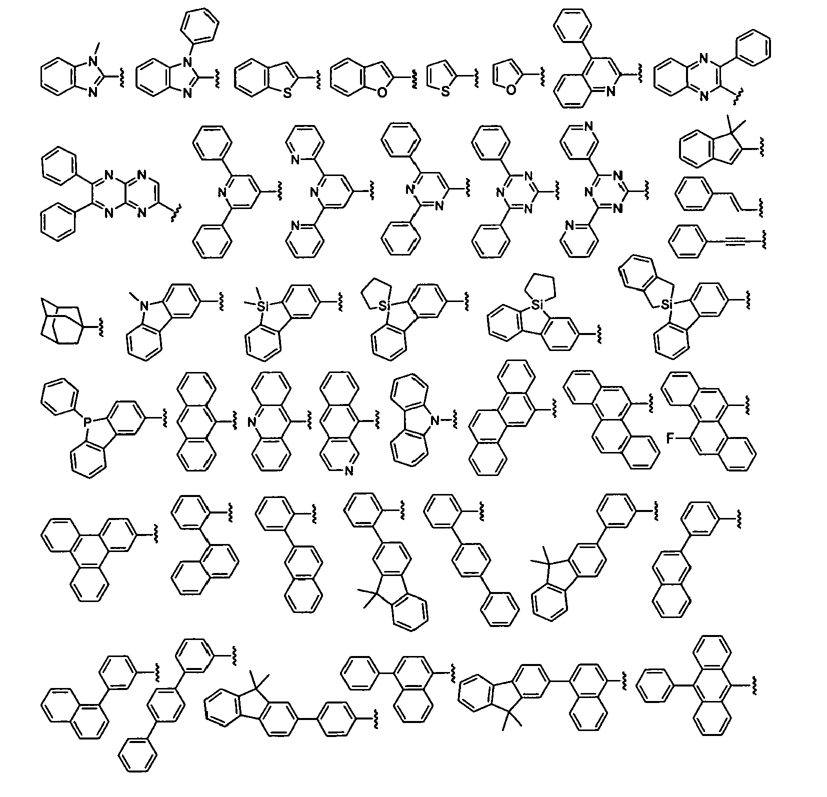

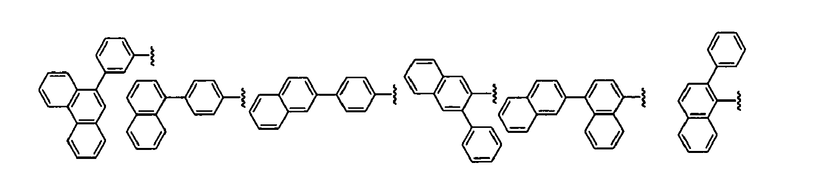

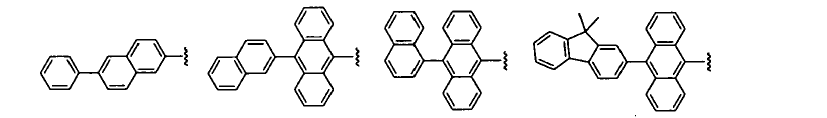

More specifically, the organic electroluminescent compound according to the present invention may be exemplified by the following compounds, but not limited thereto:

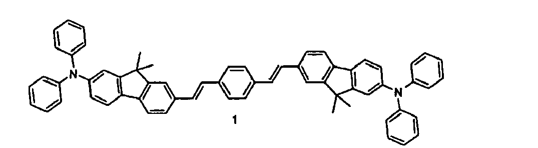

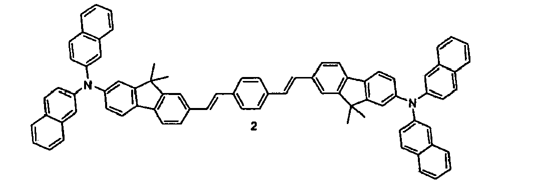

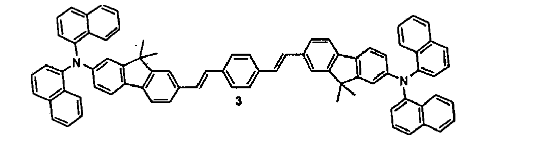

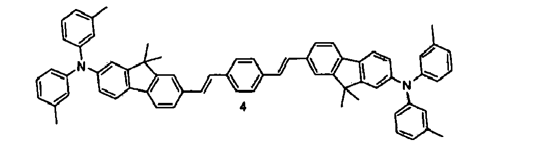

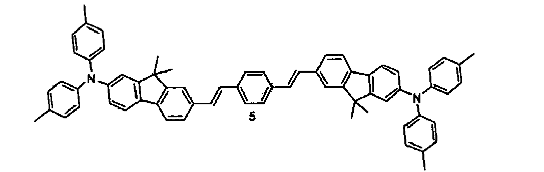

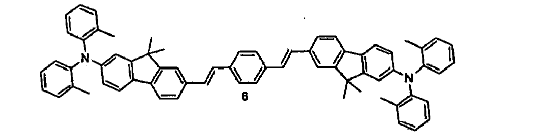

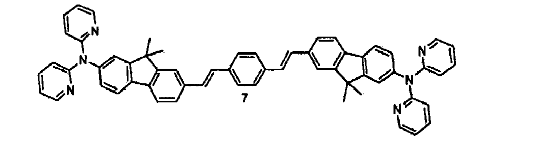

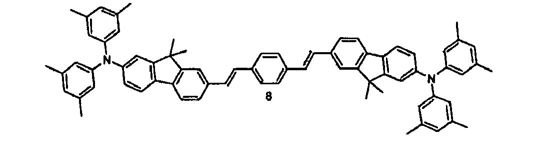

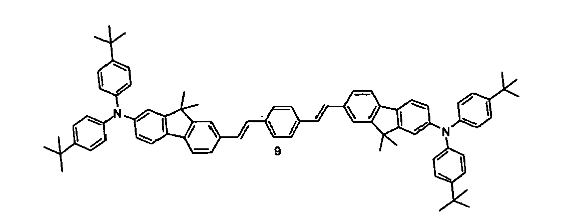

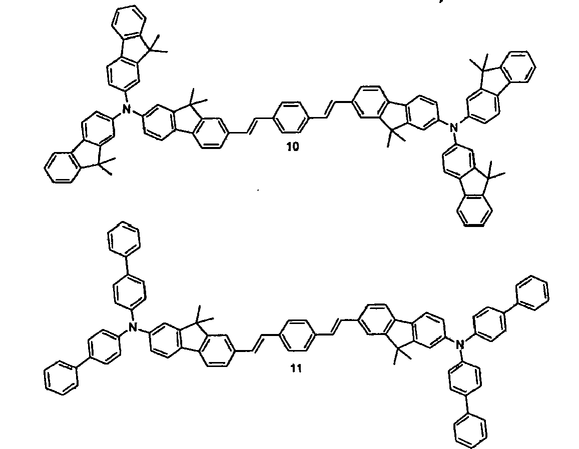

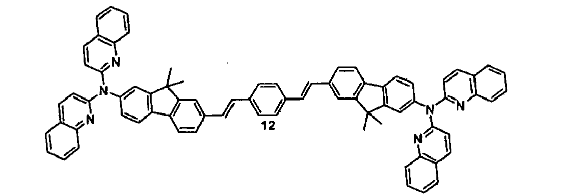

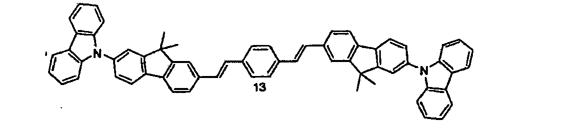

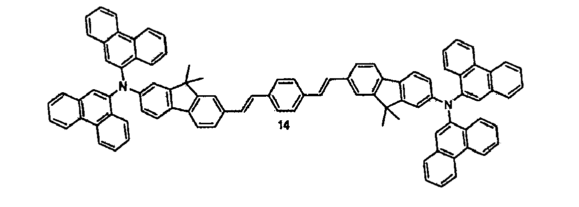

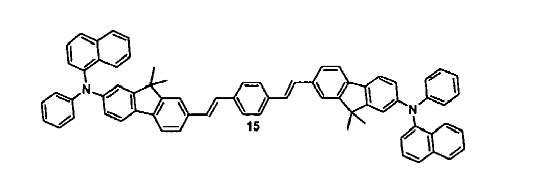

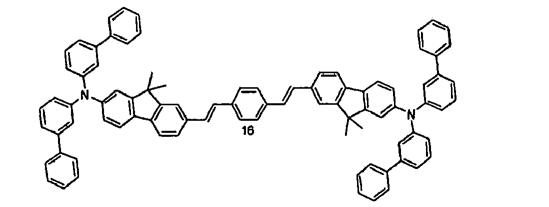

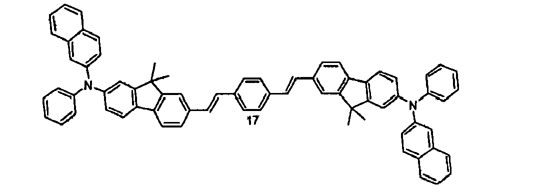

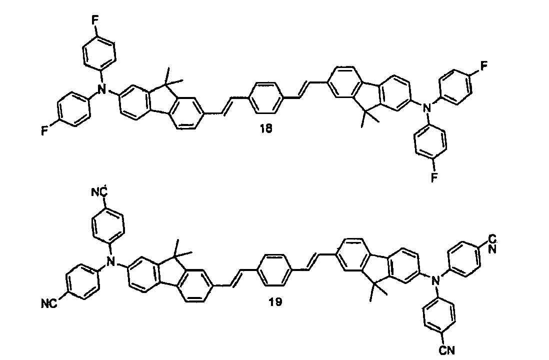

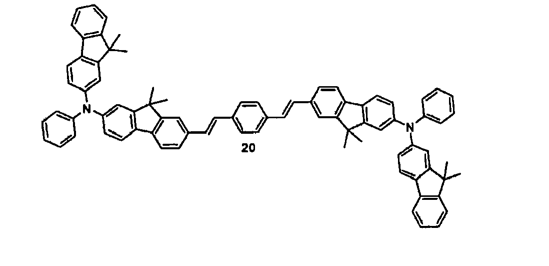

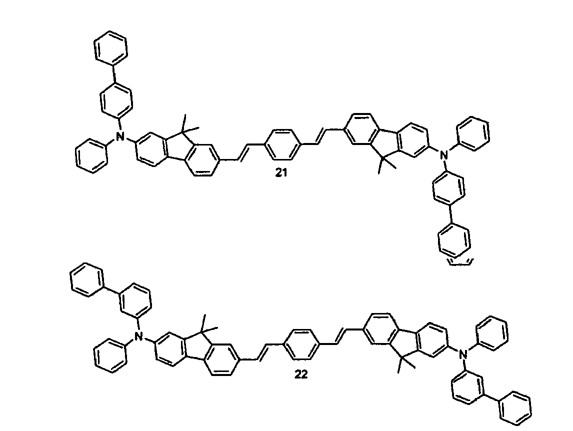

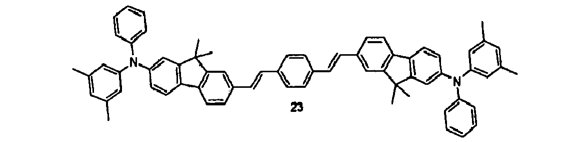

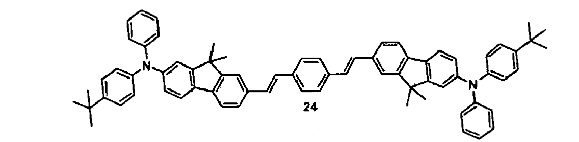

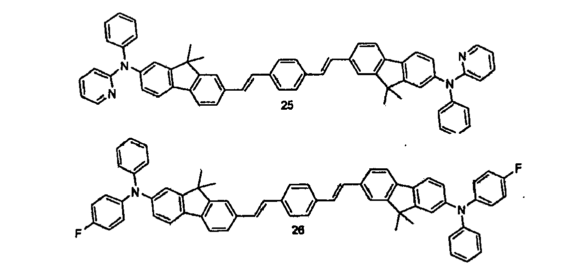

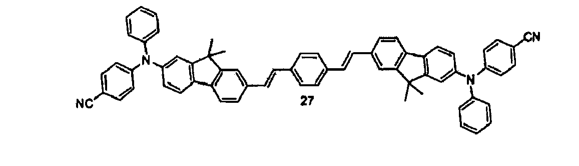

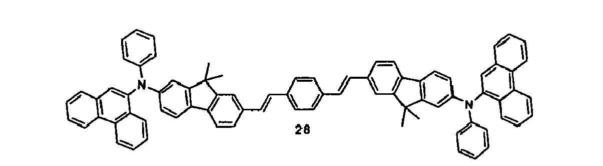

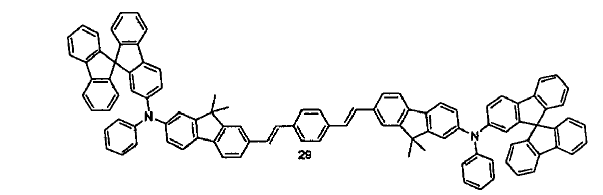

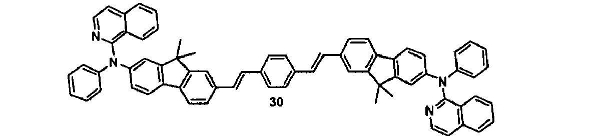

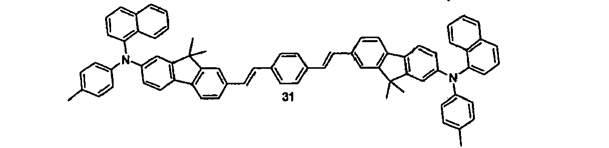

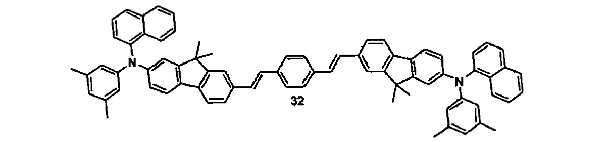

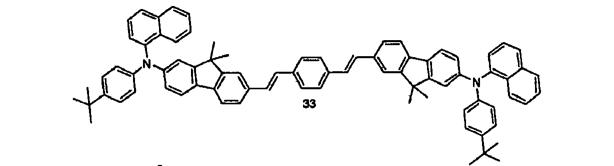

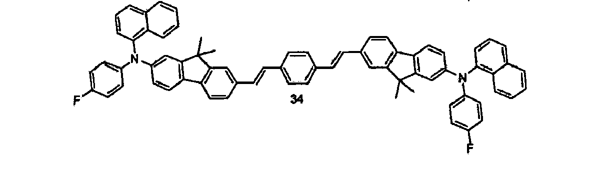

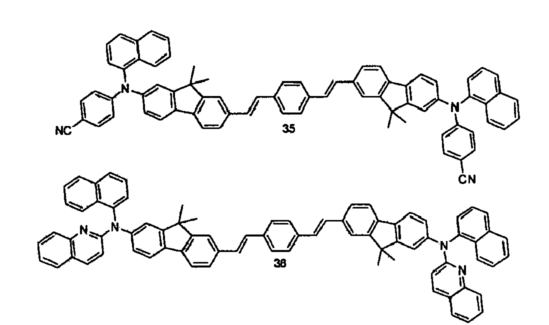

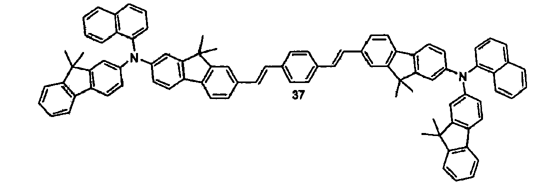

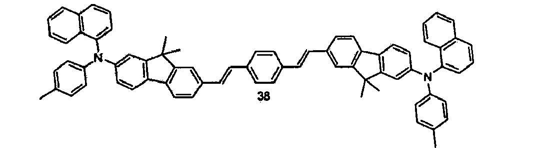

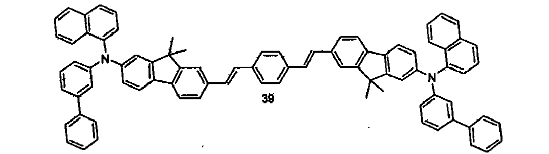

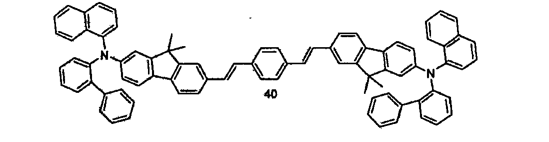

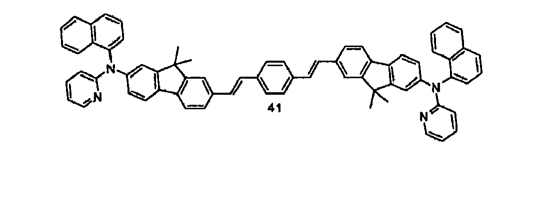

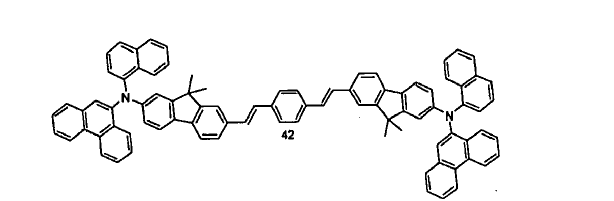

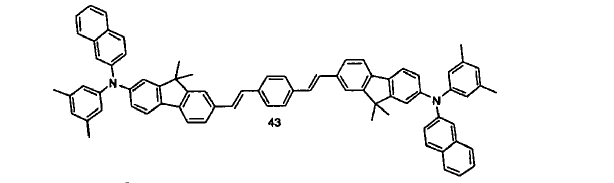

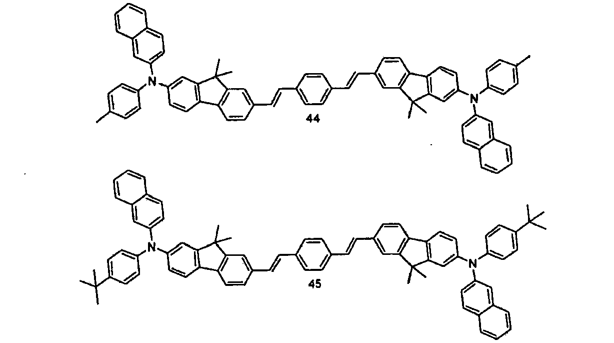

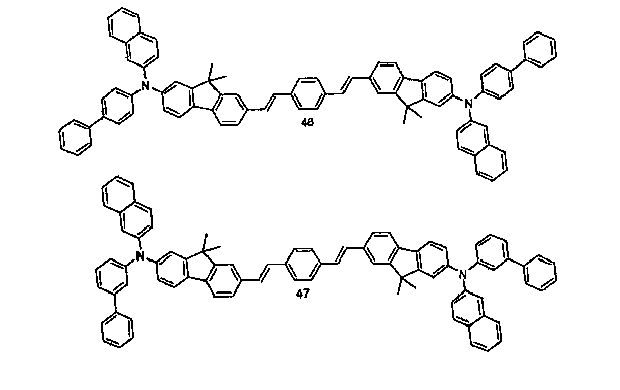

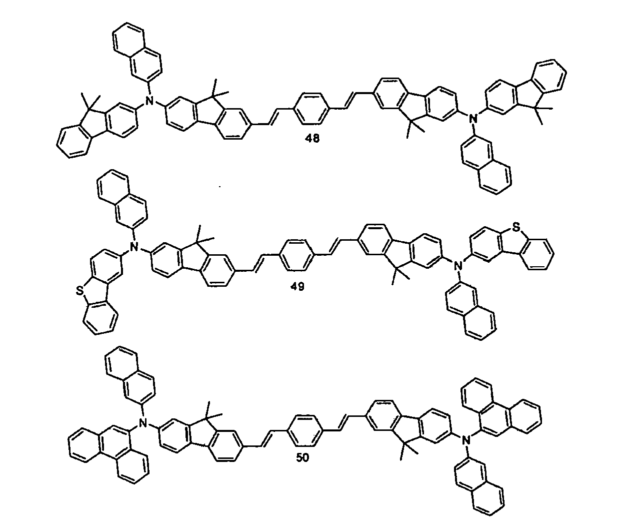

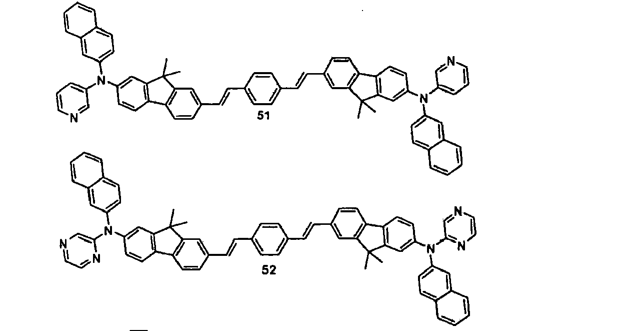

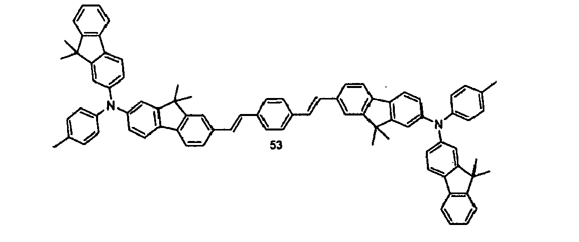

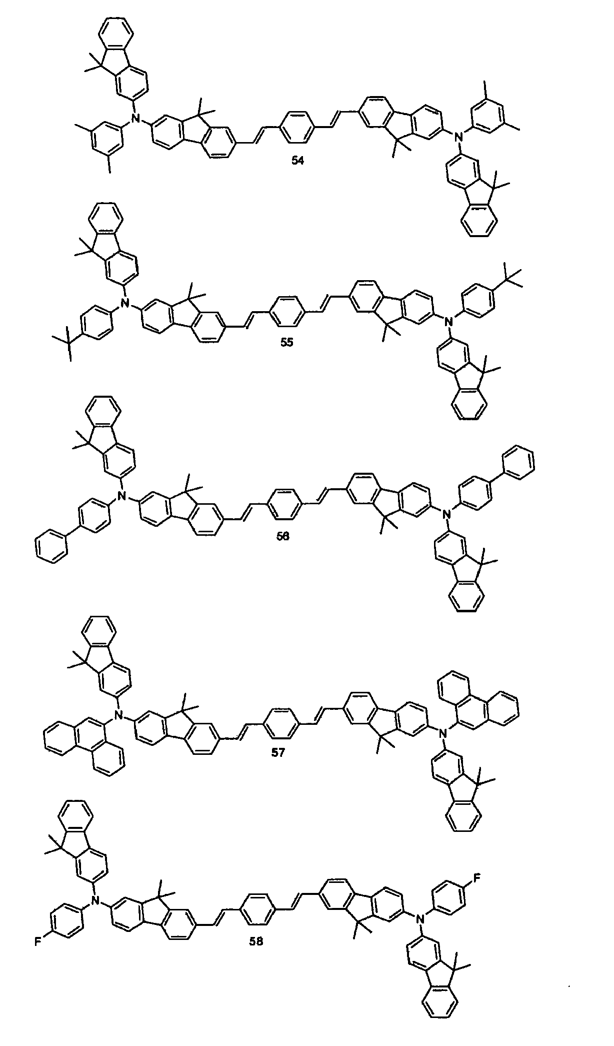

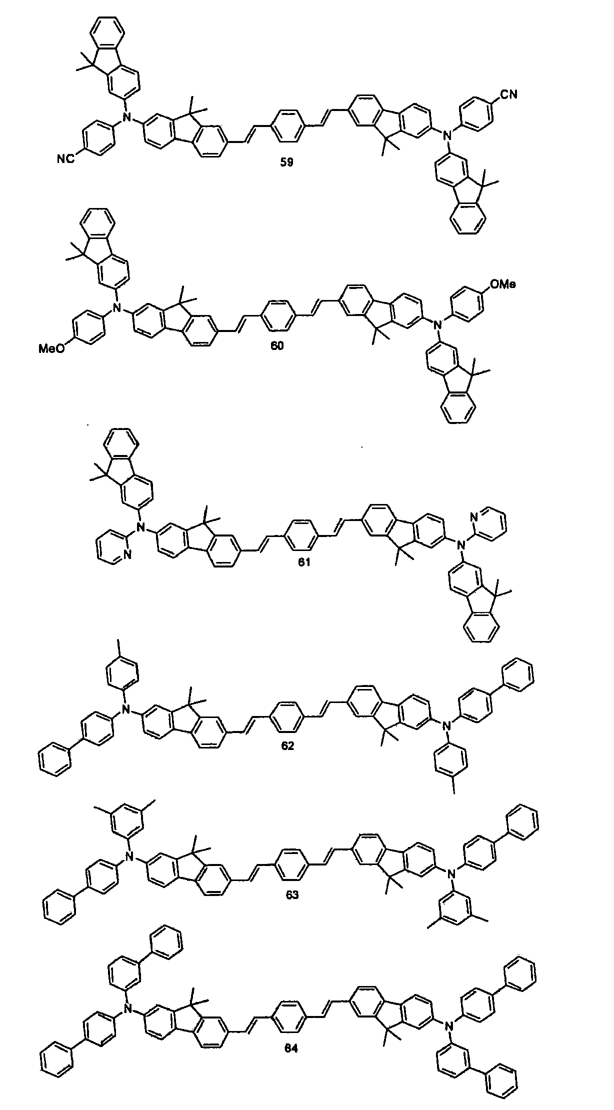

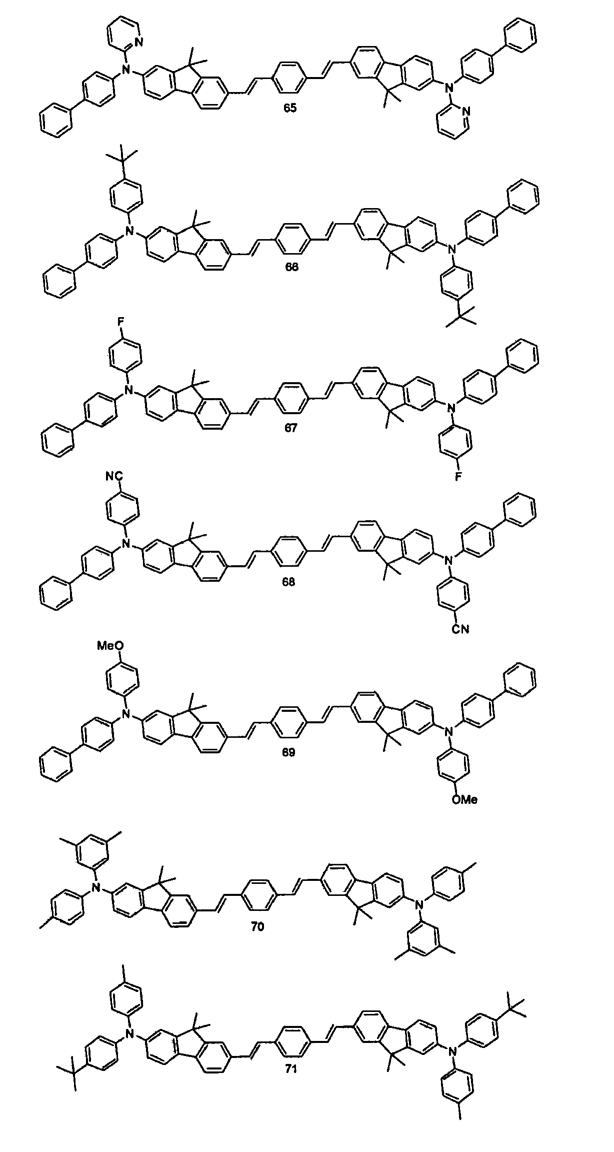

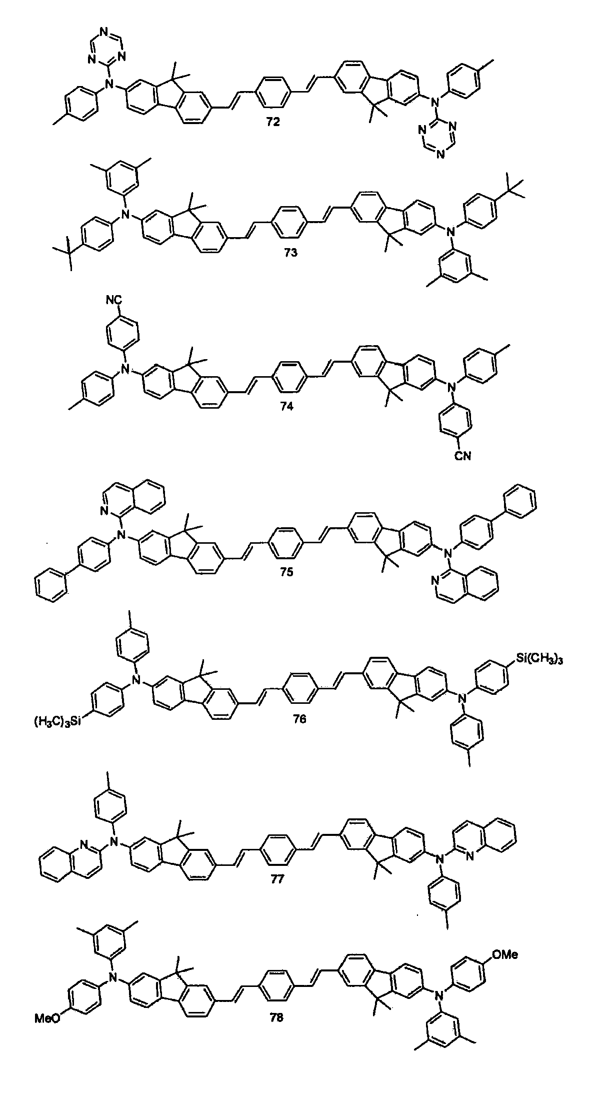

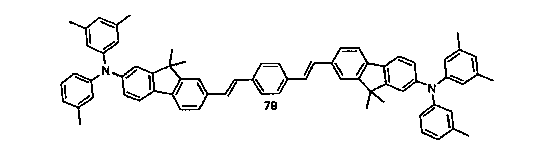

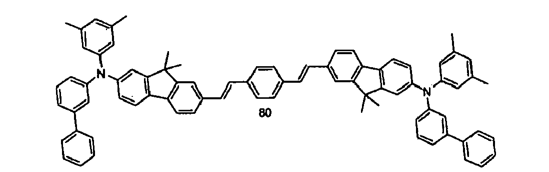

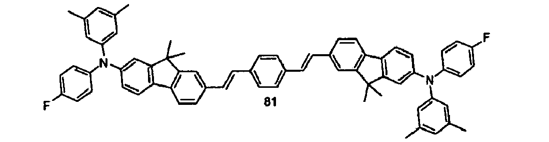

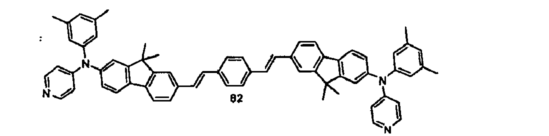

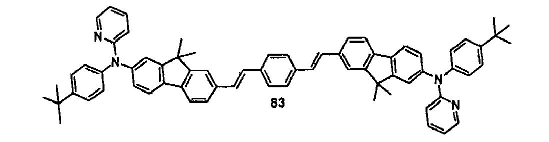

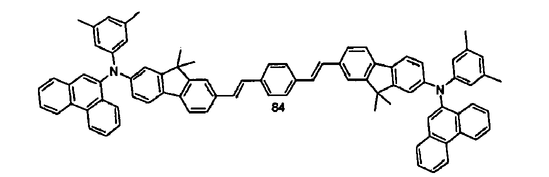

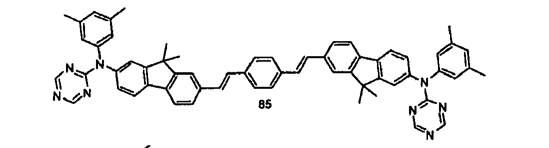

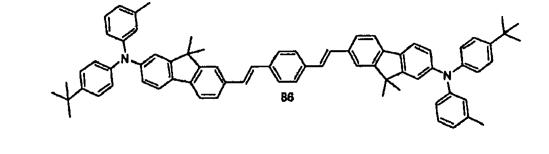

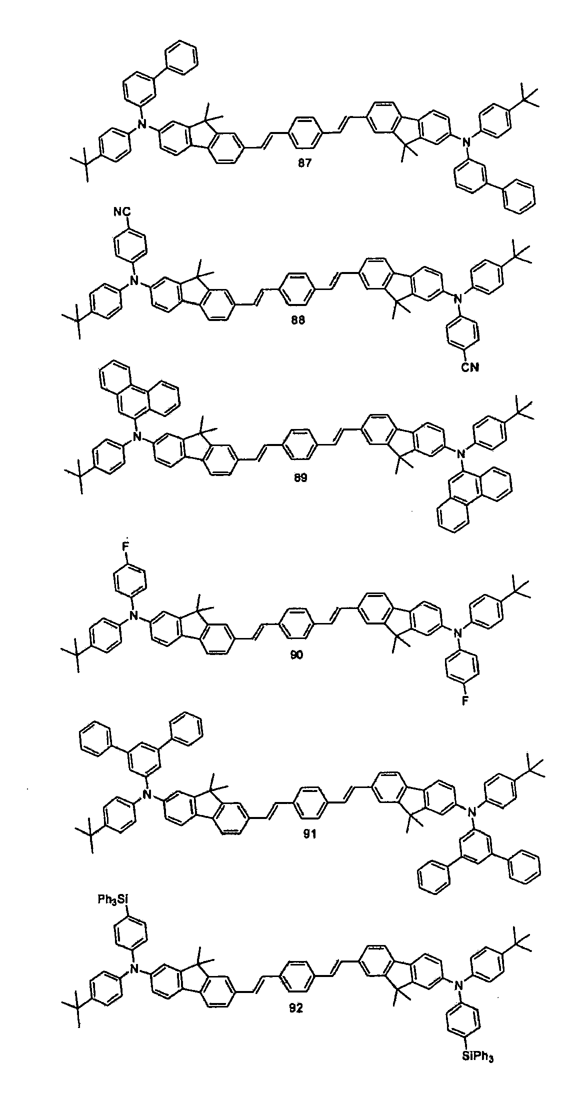

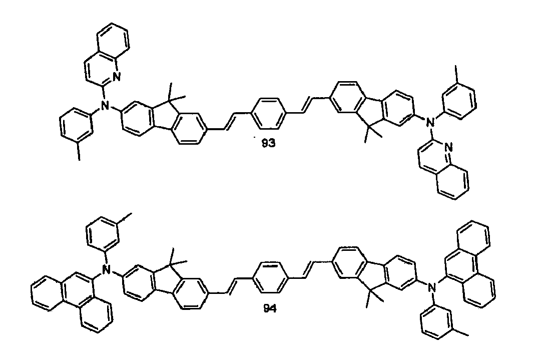

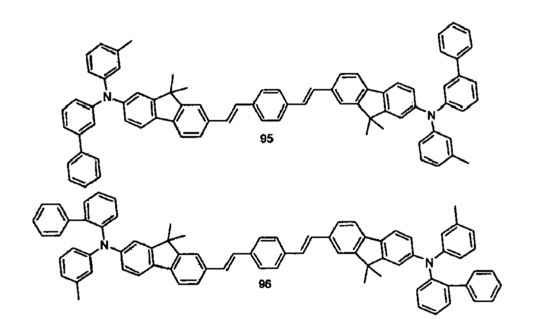

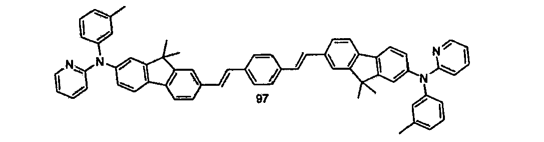

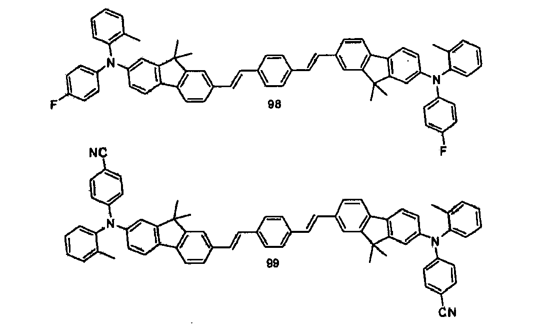

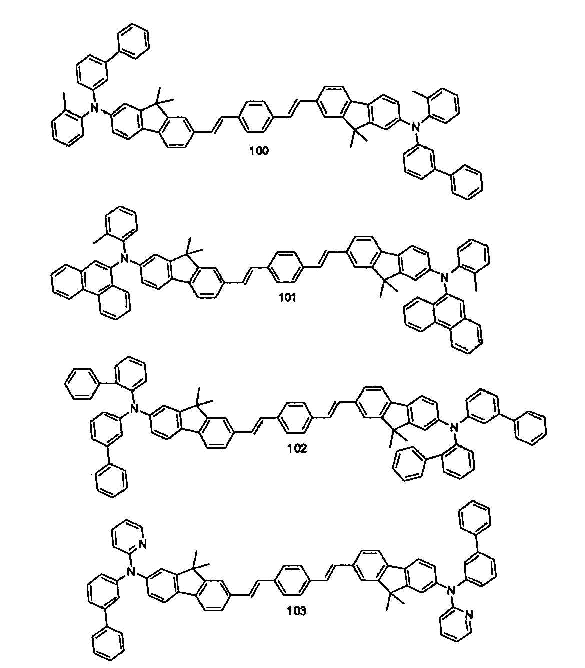

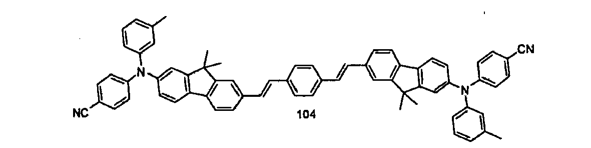

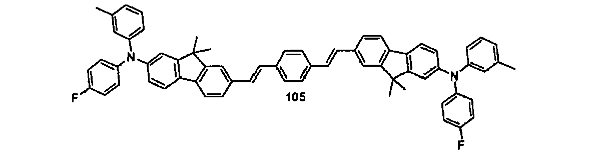

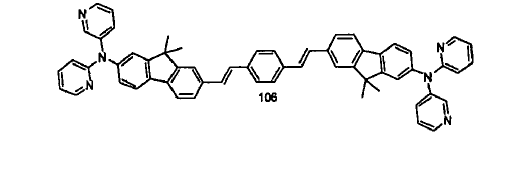

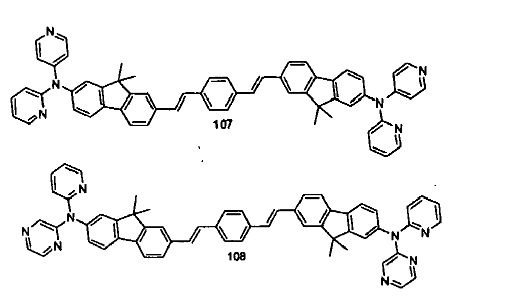

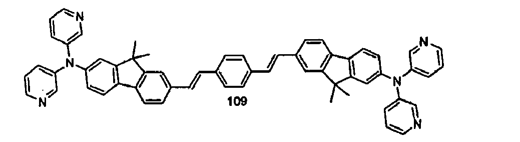

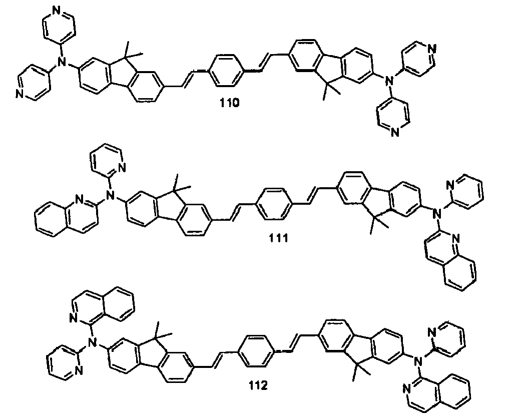

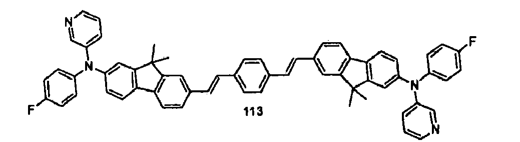

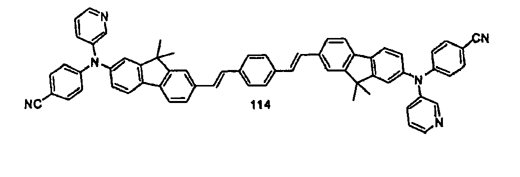

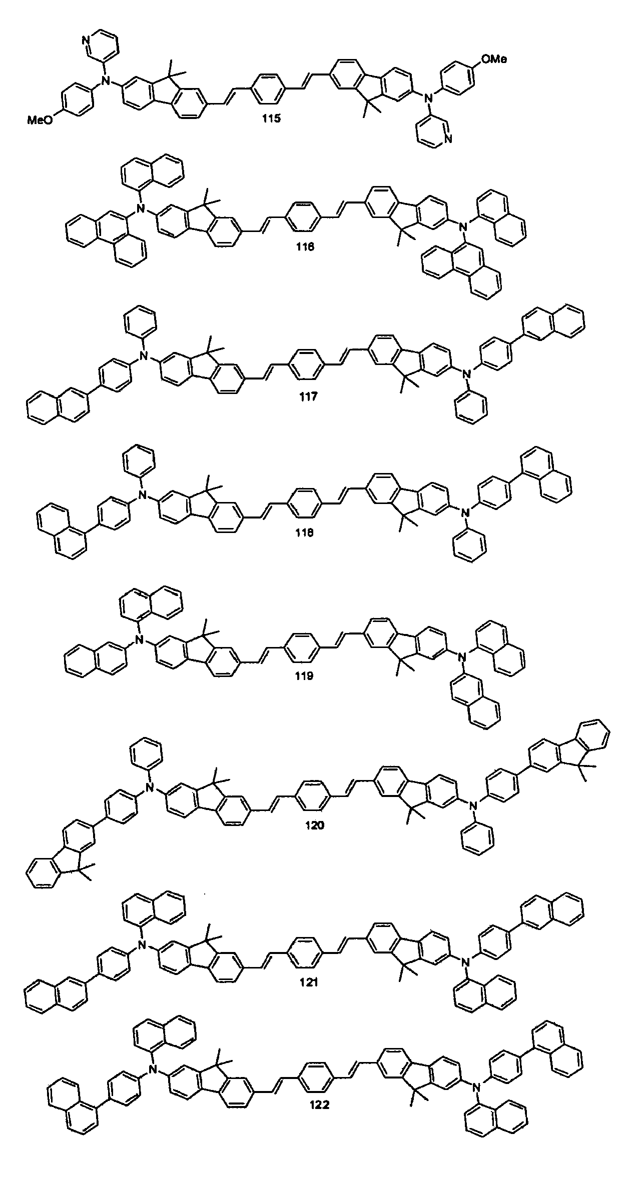

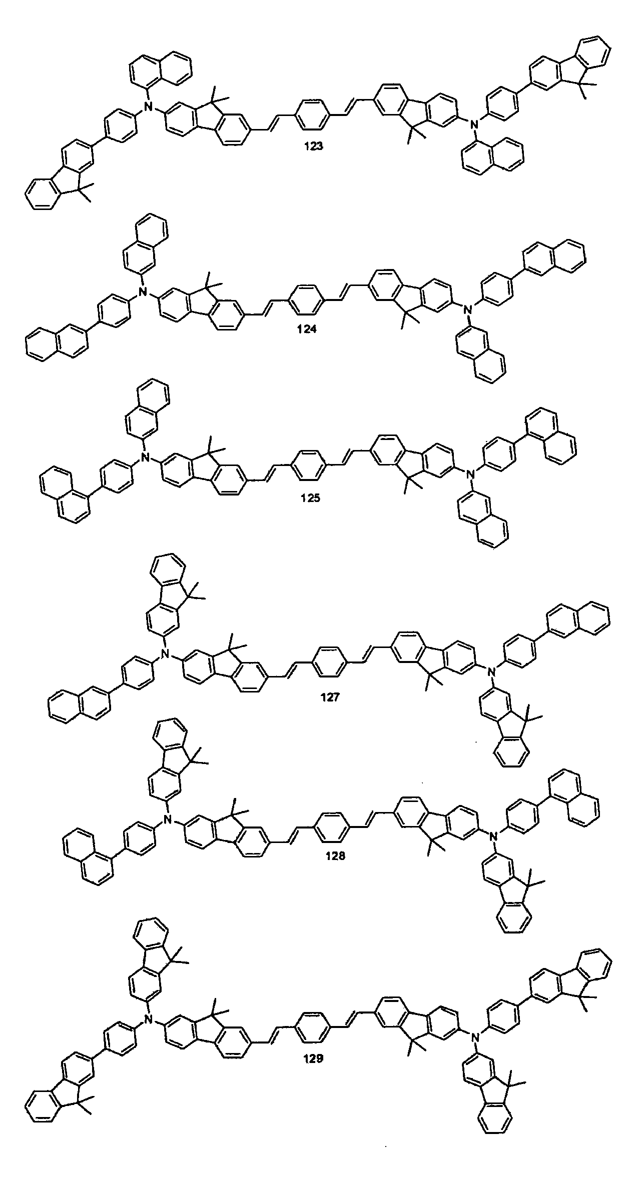

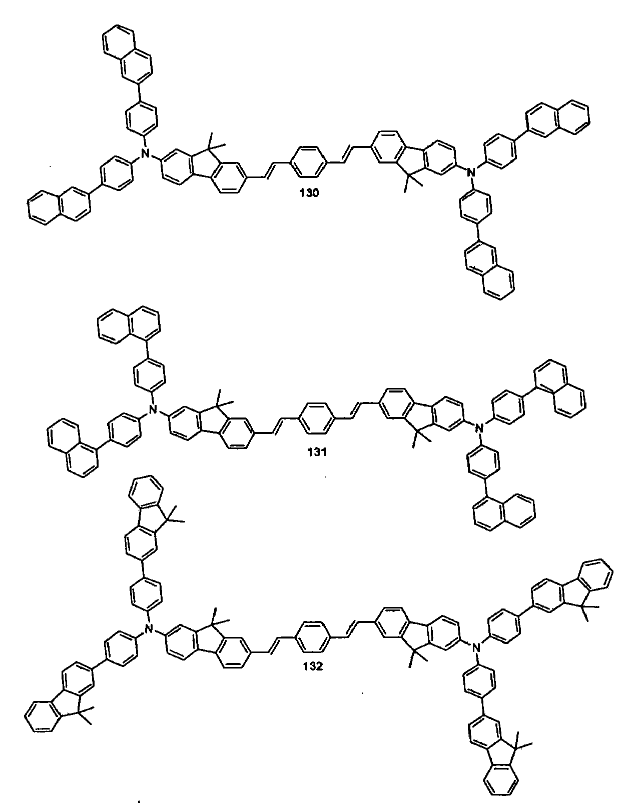

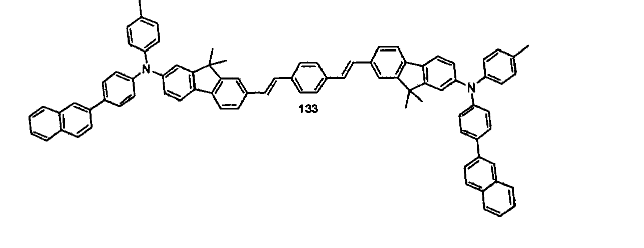

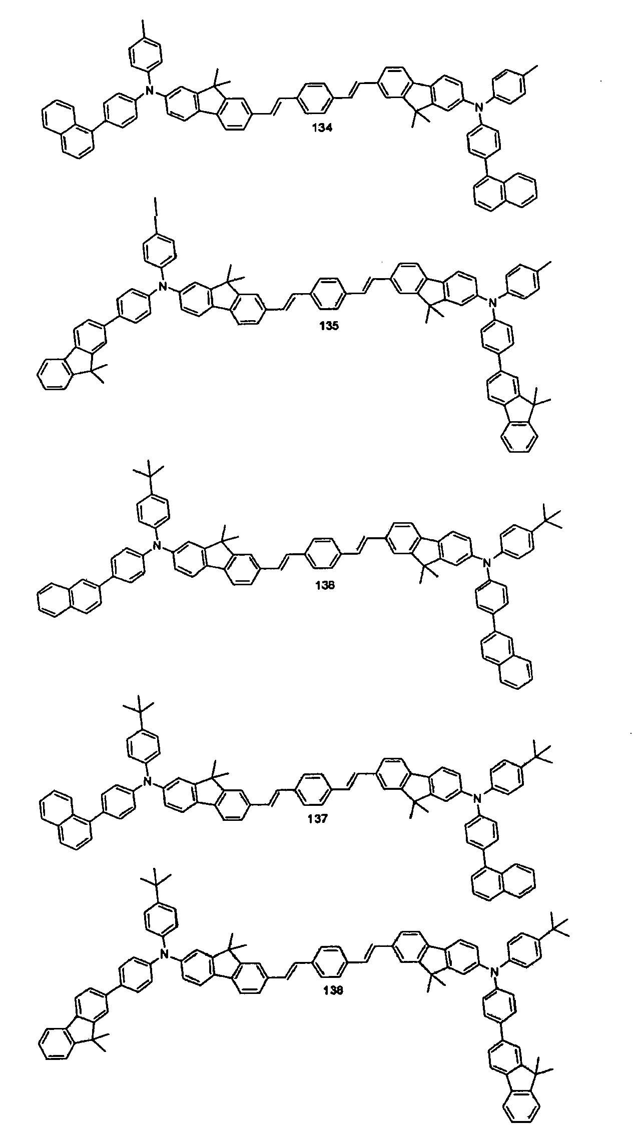

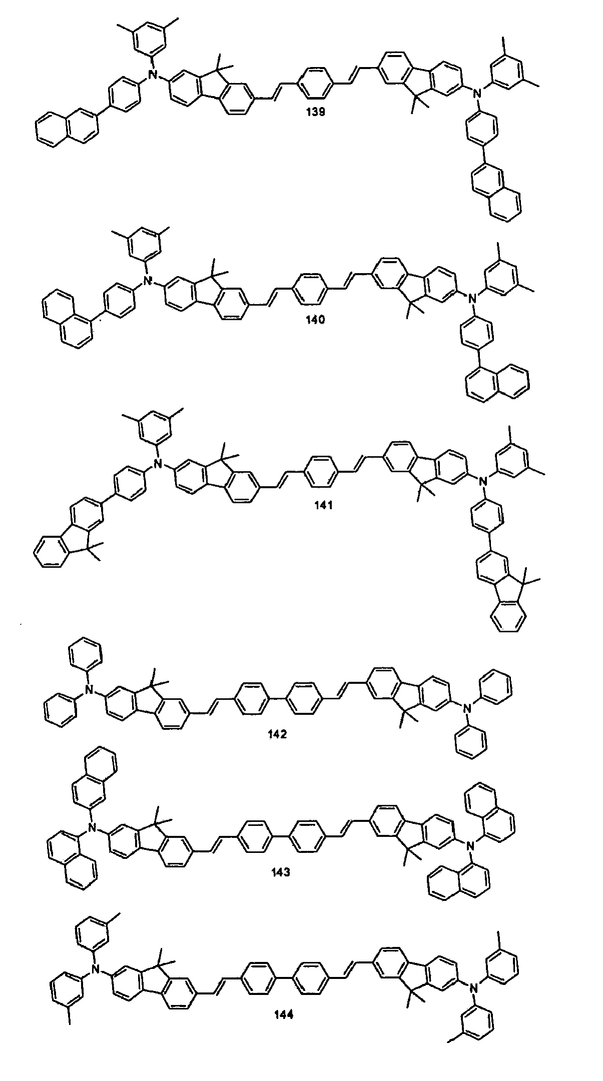

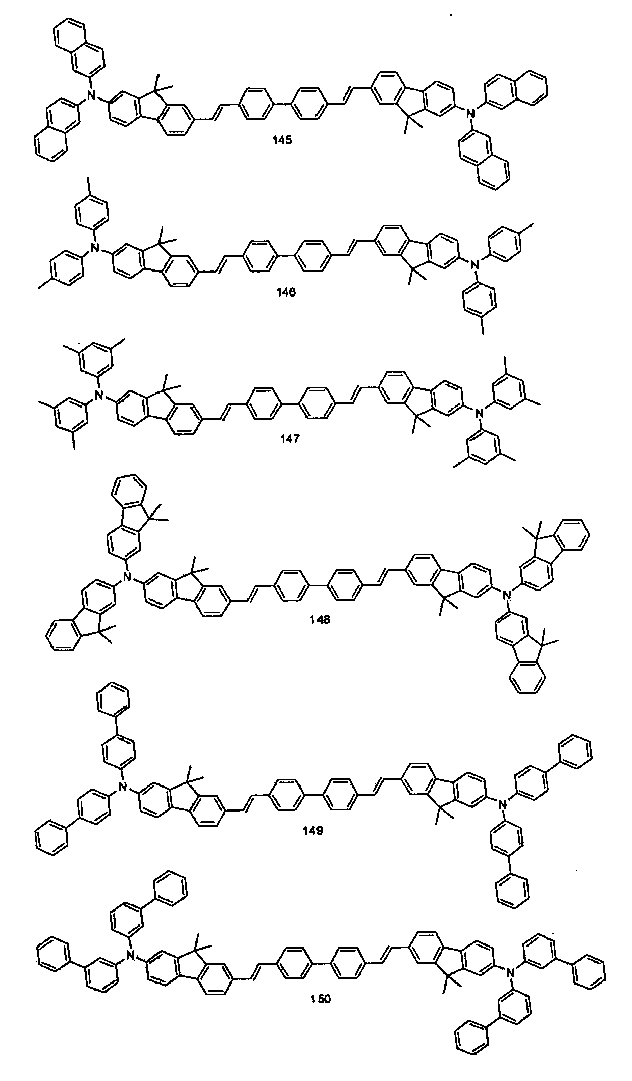

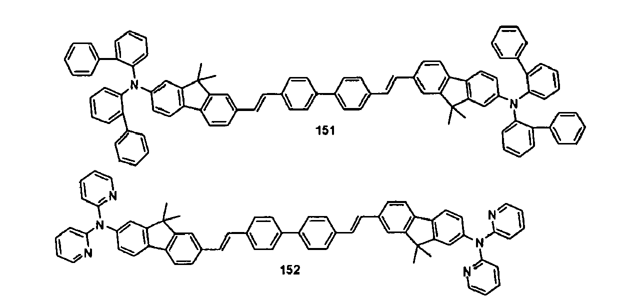

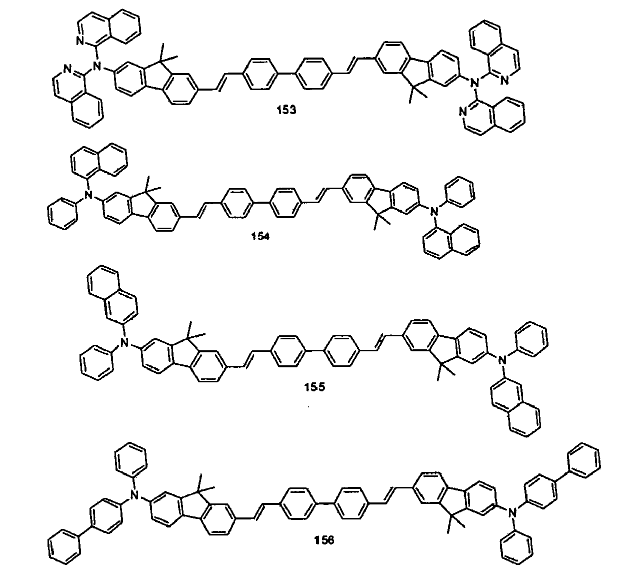

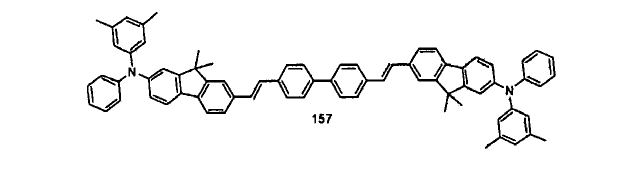

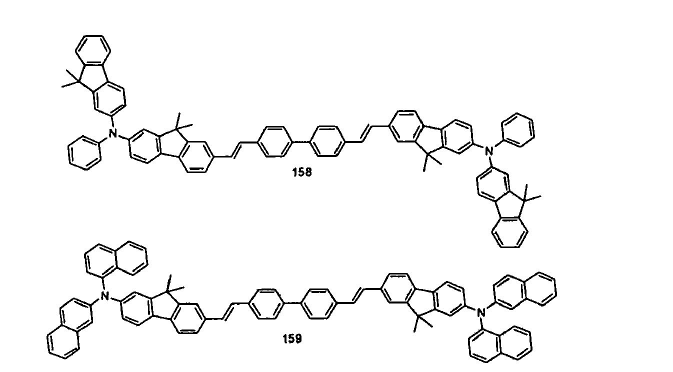

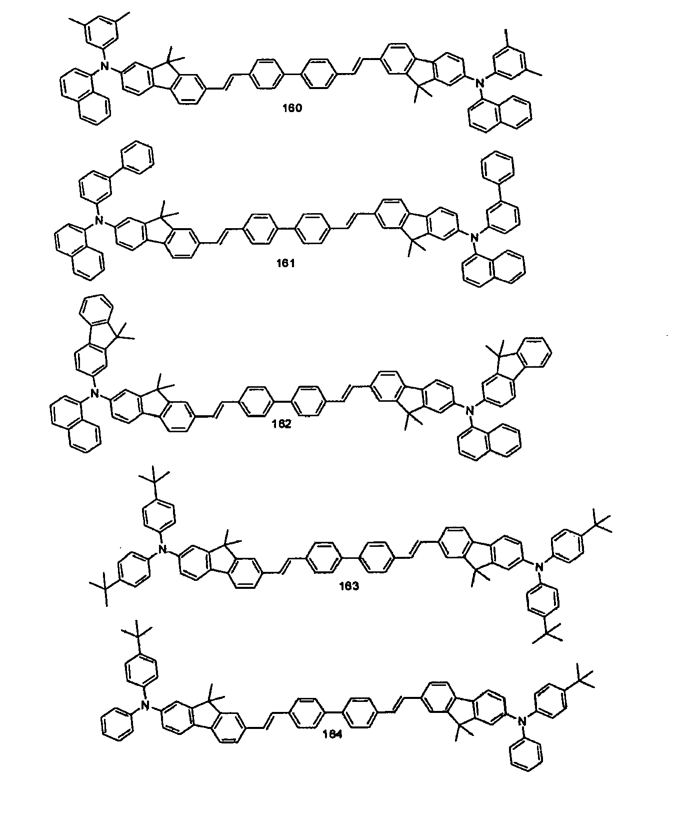

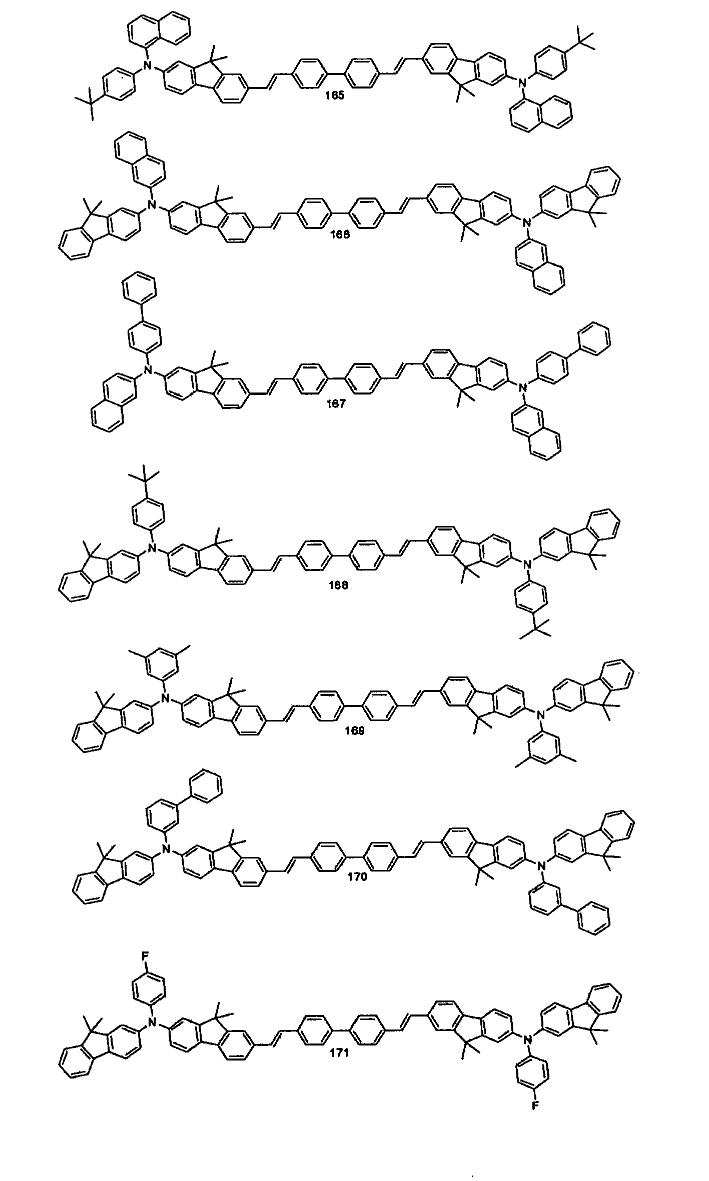

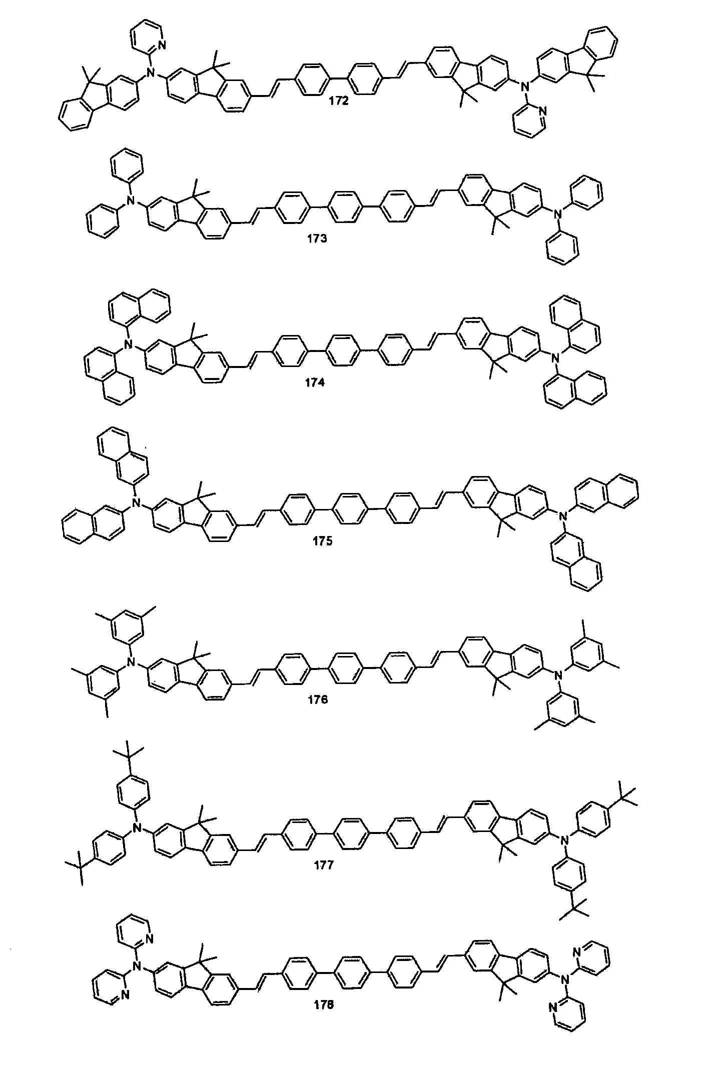

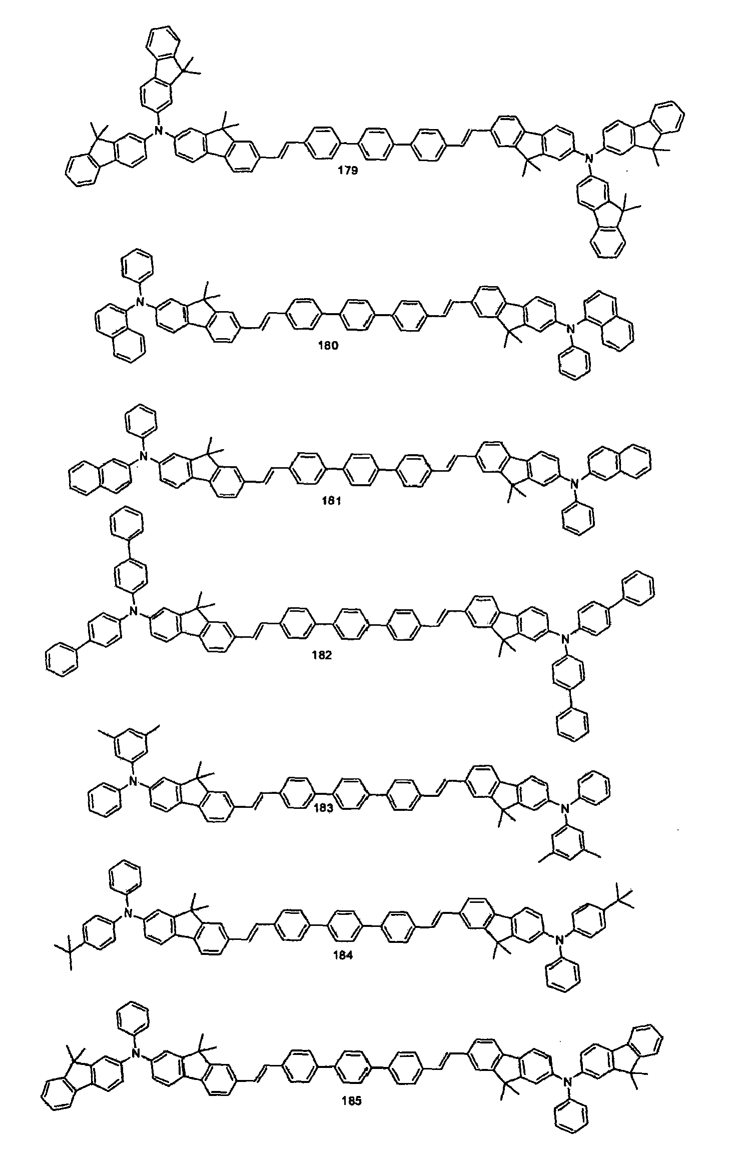

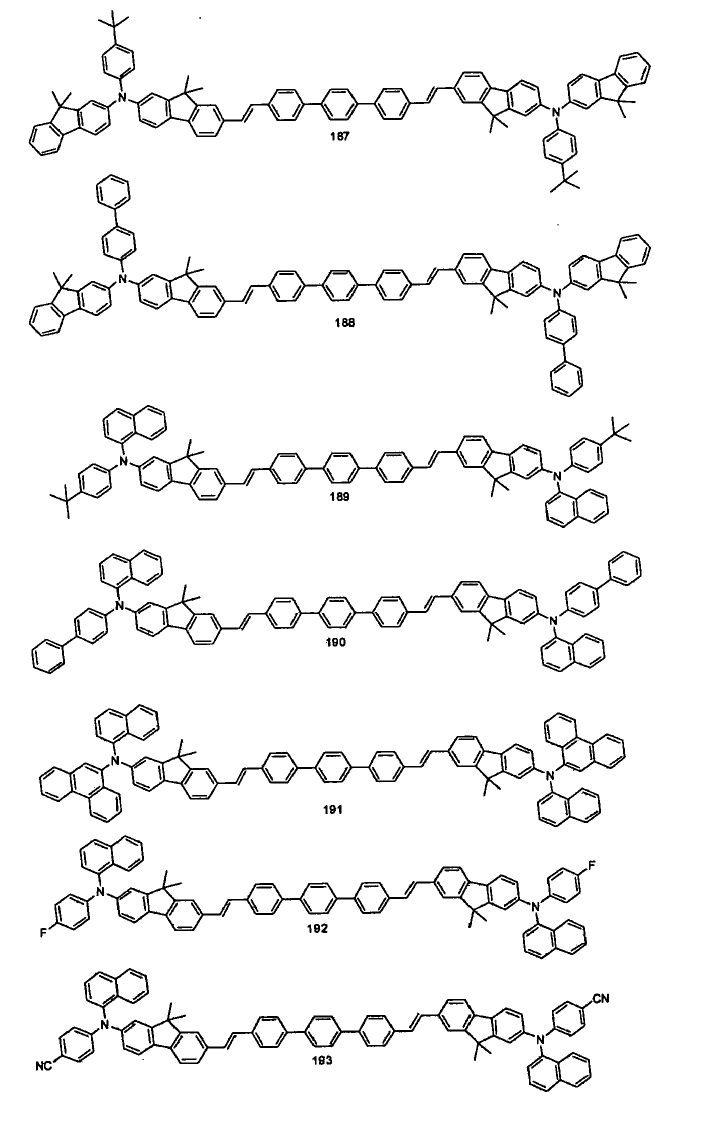

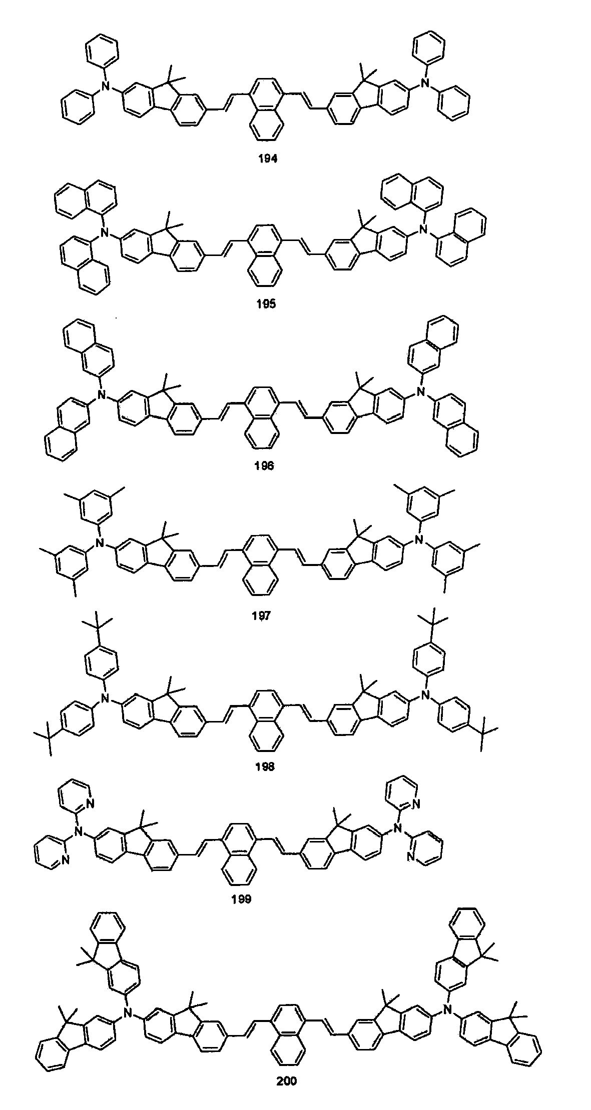

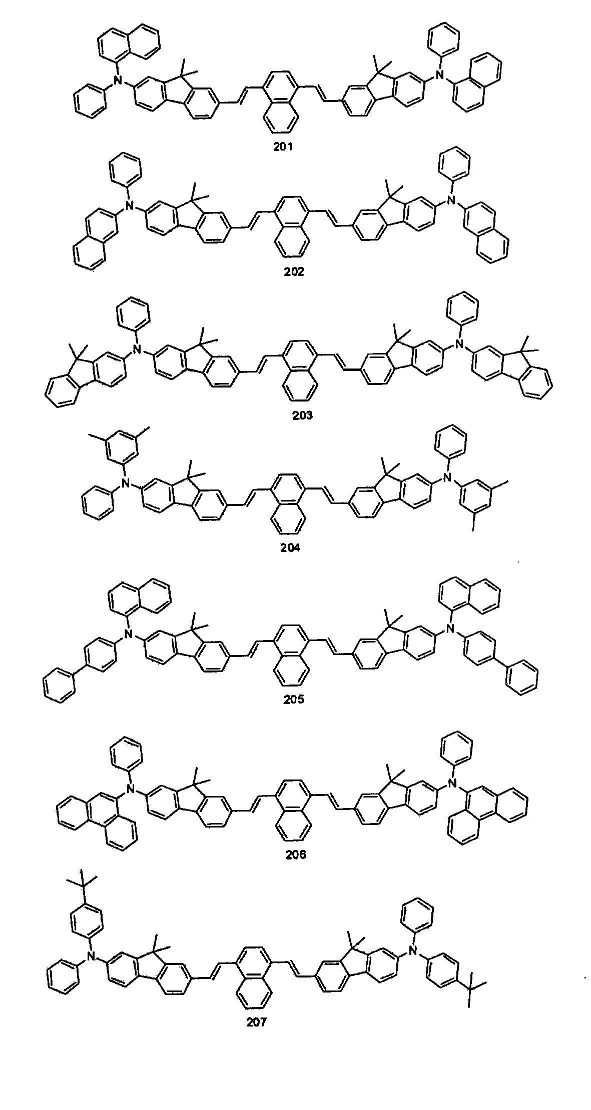

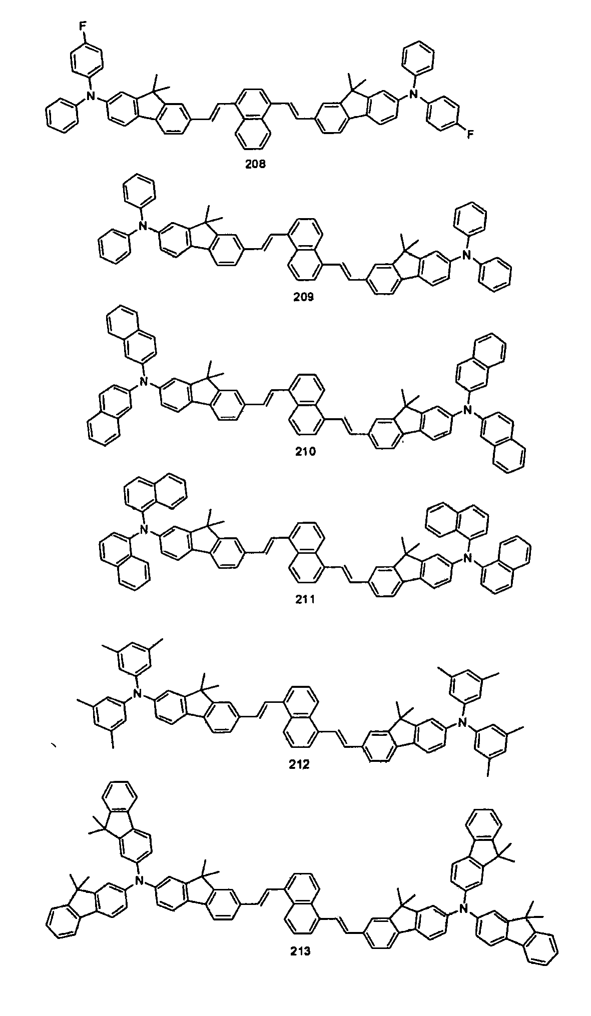

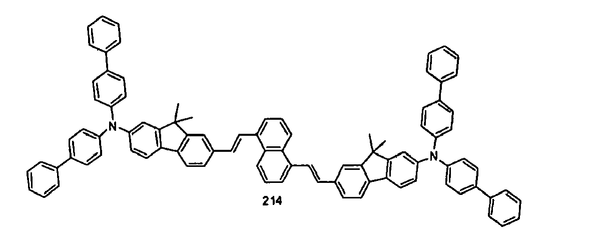

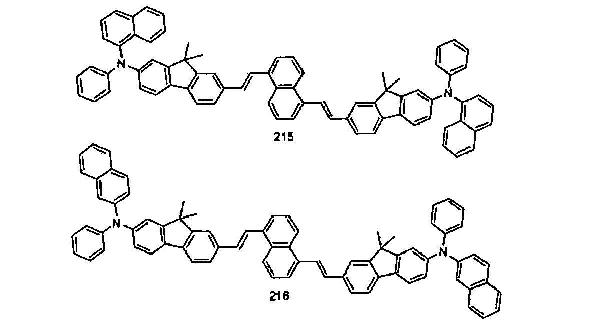

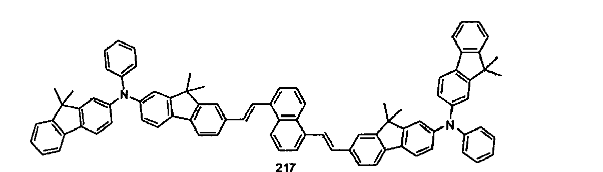

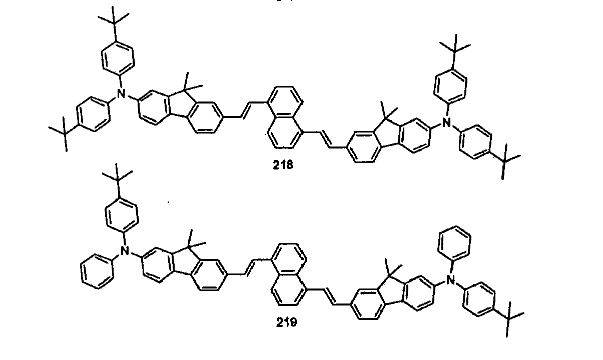

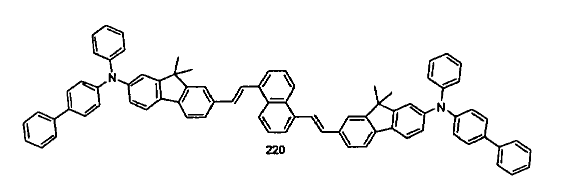

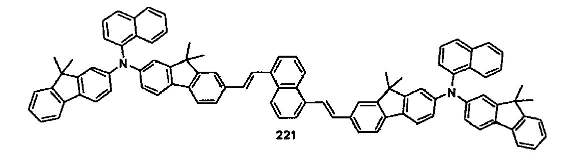

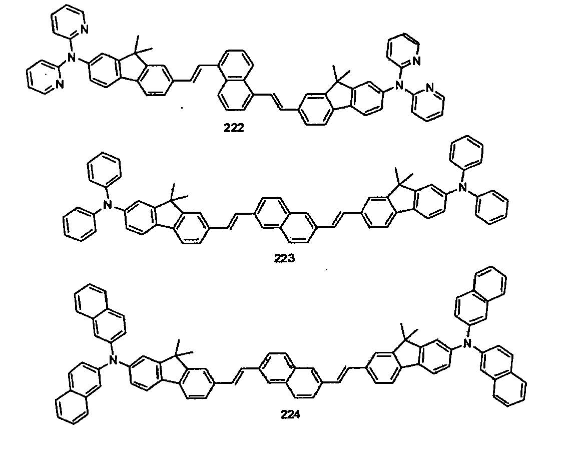

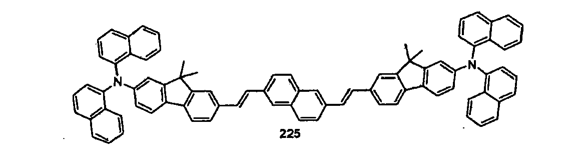

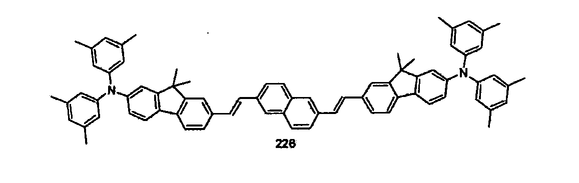

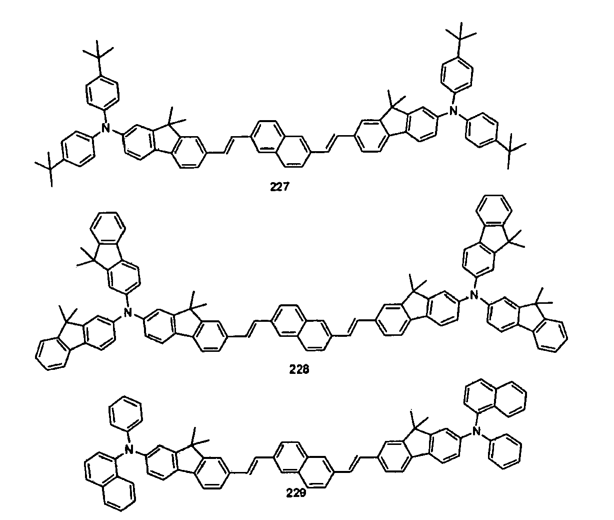

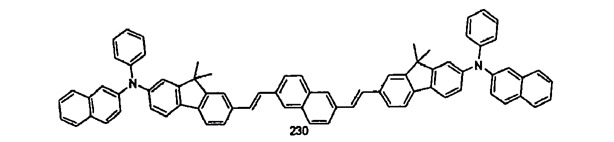

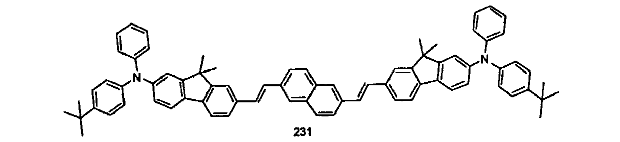

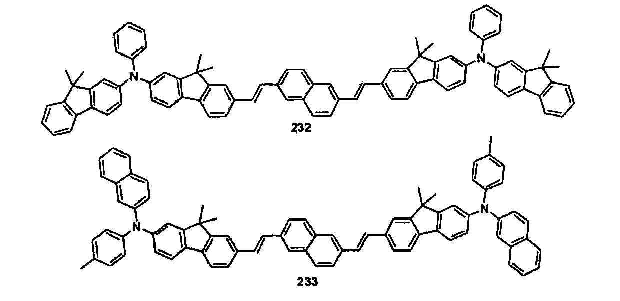

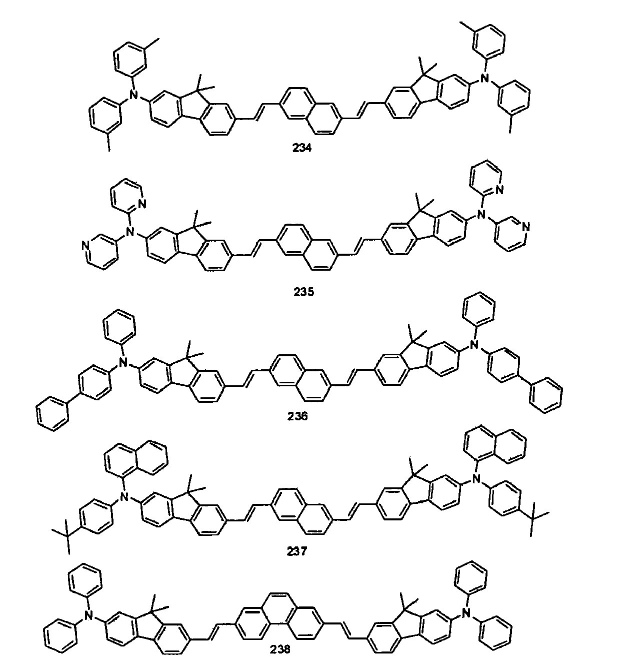

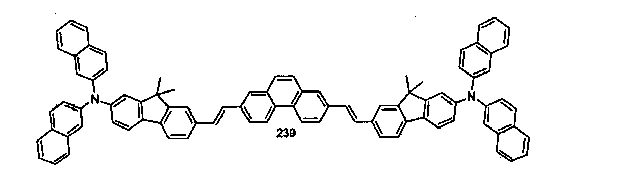

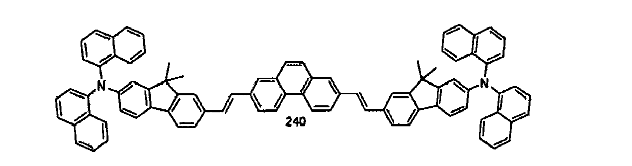

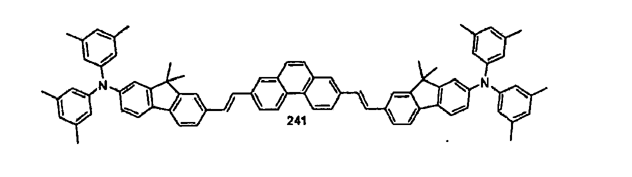

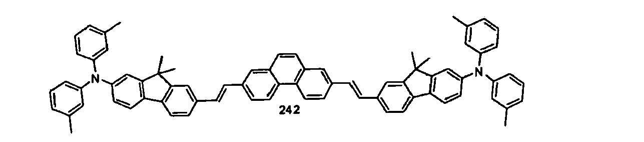

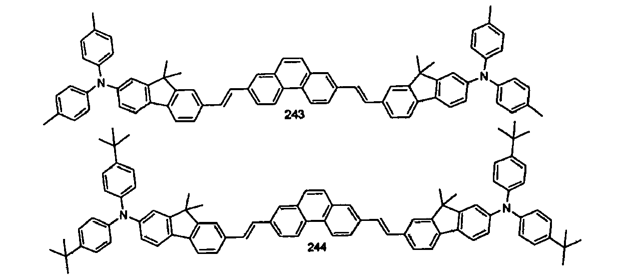

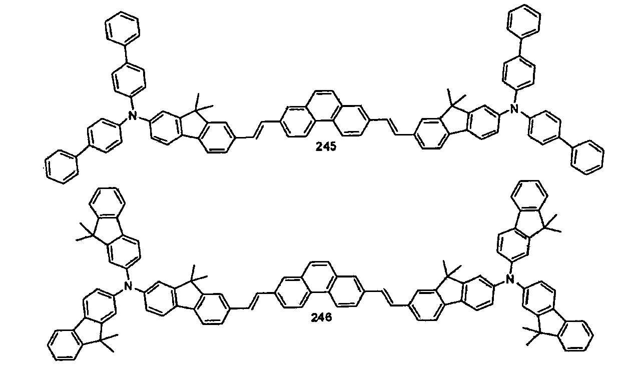

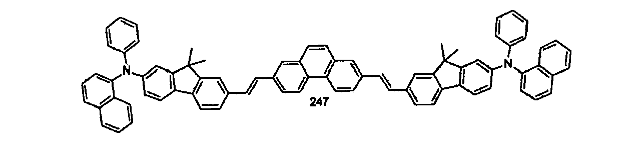

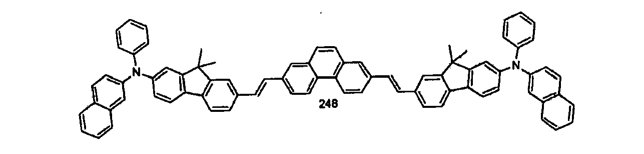

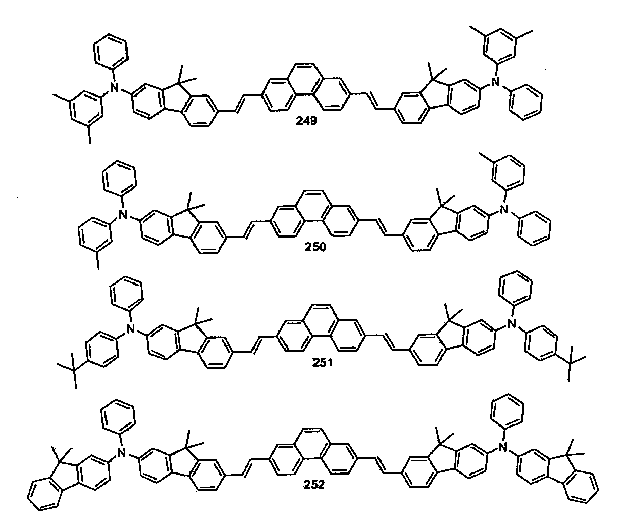

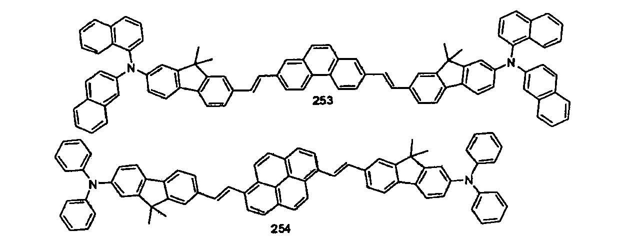

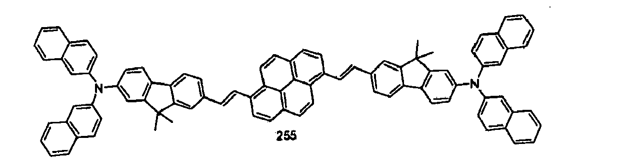

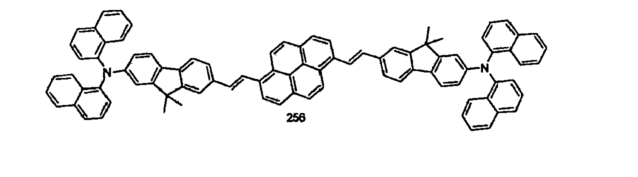

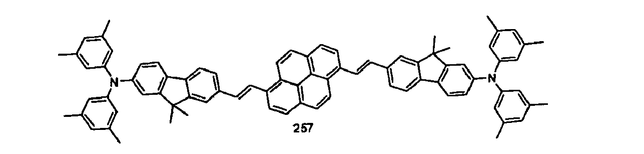

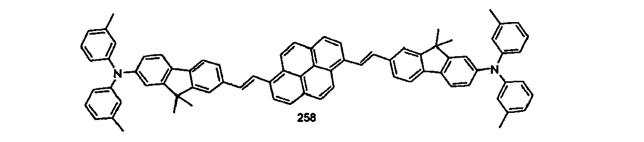

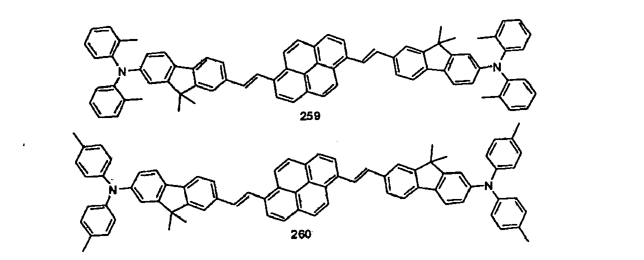

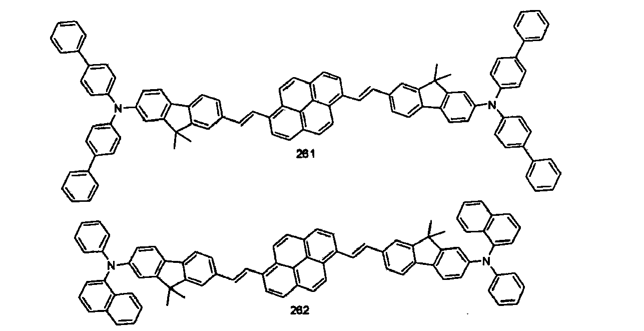

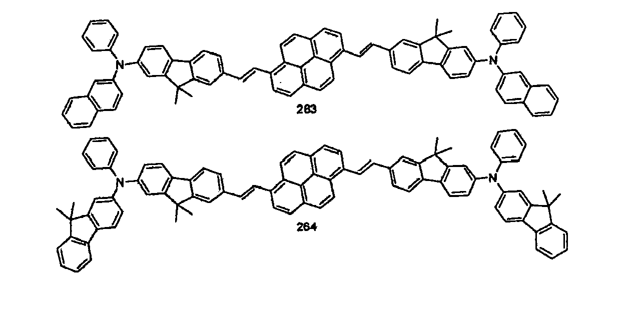

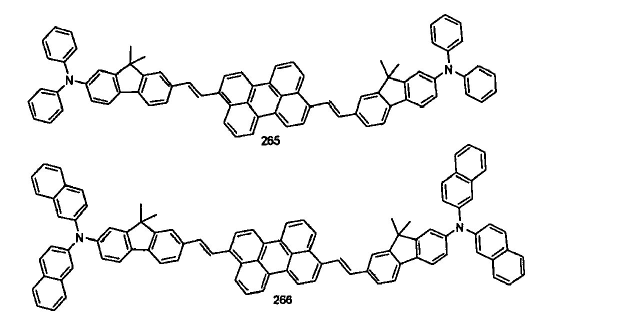

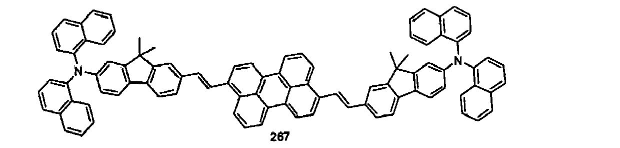

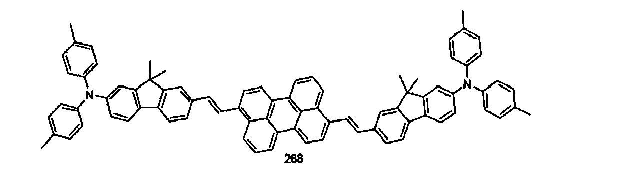

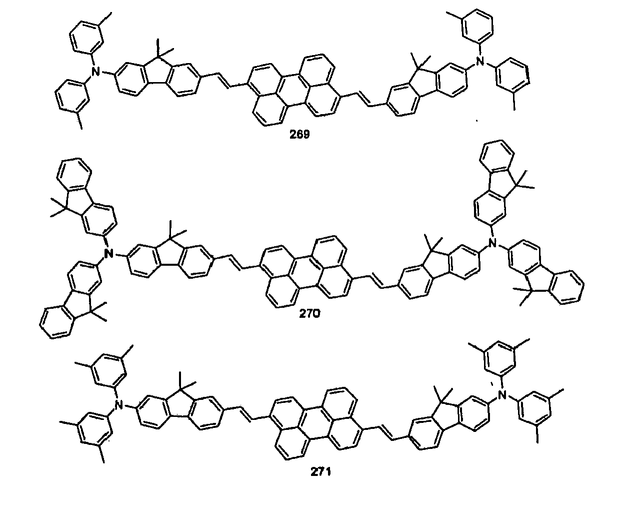

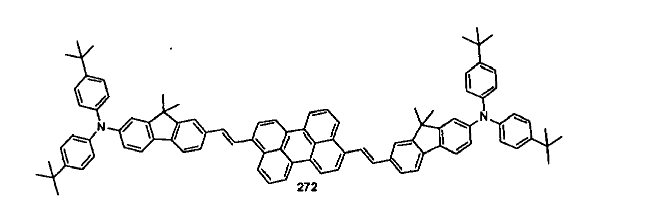

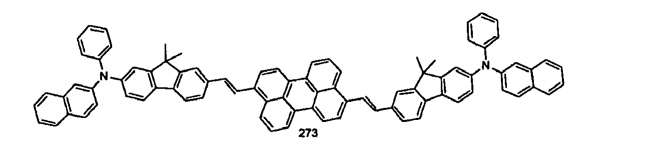

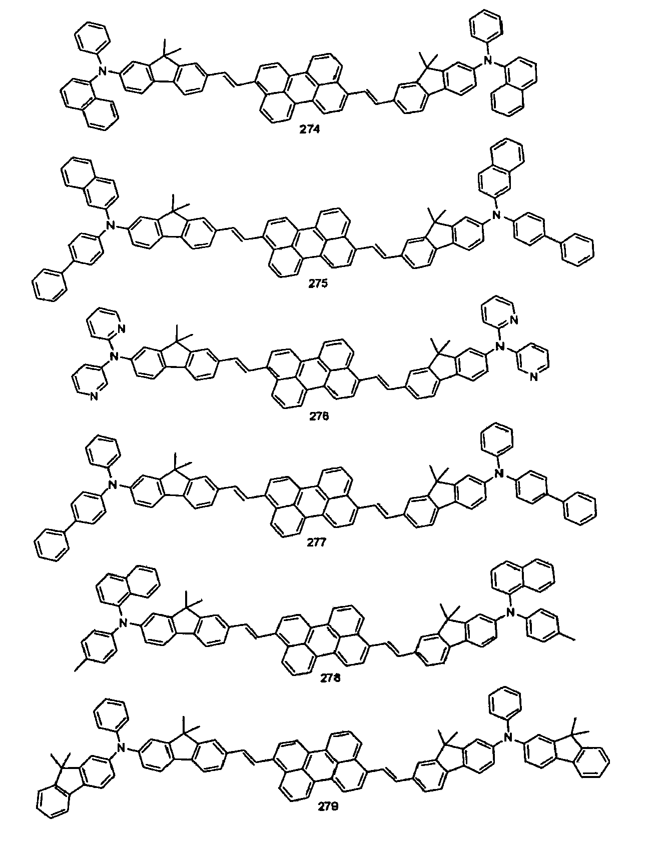

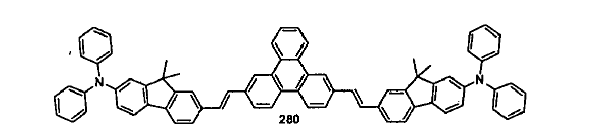

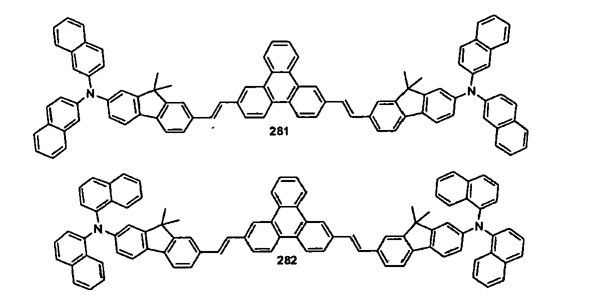

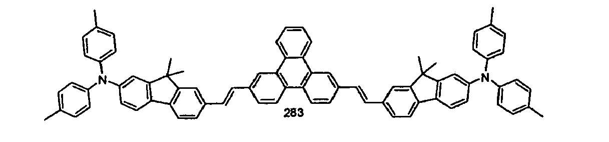

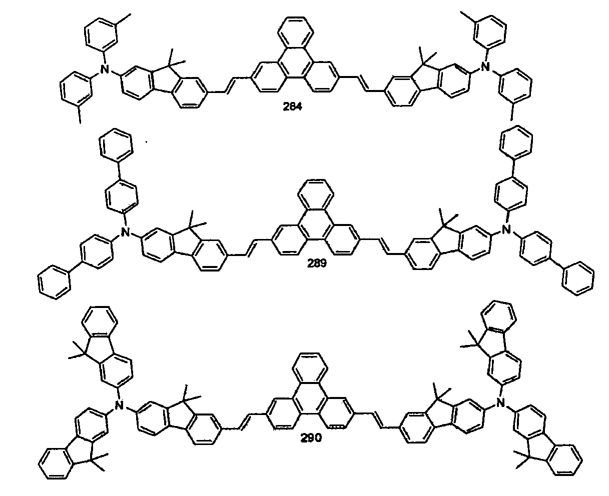

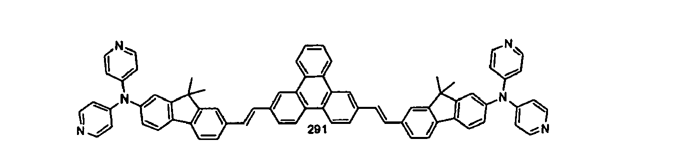

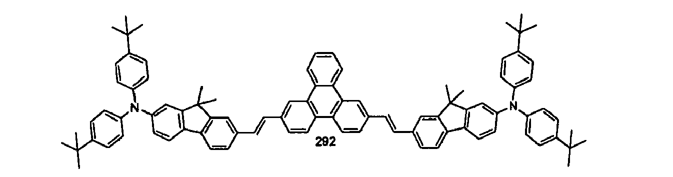

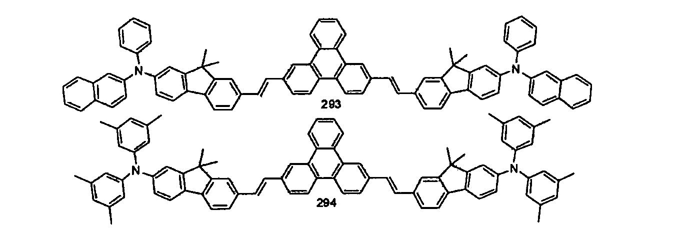

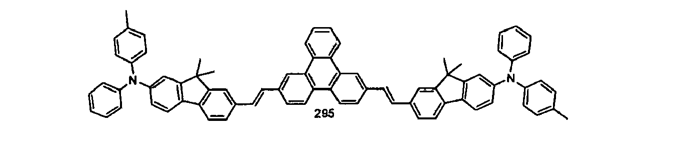

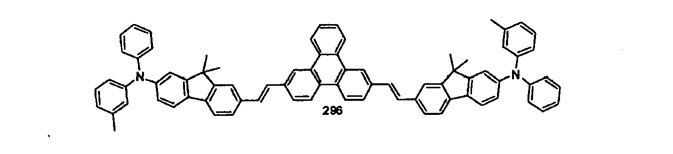

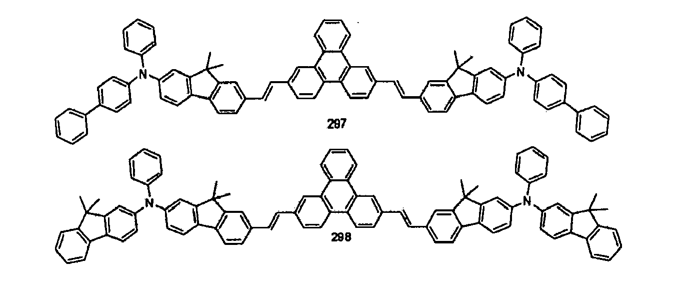

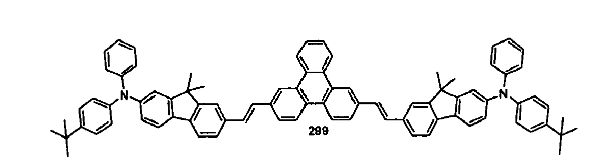

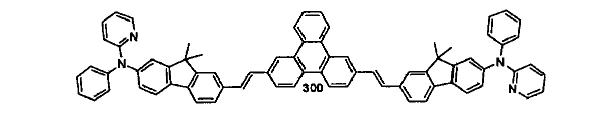

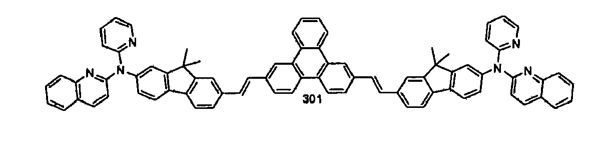

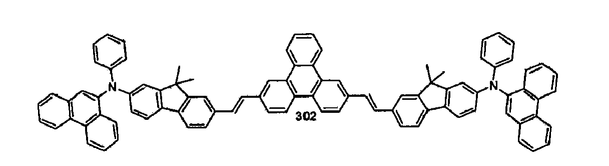

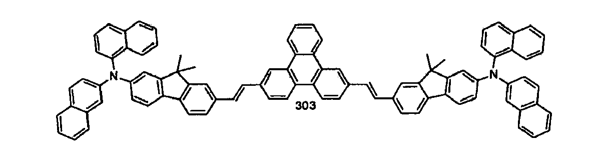

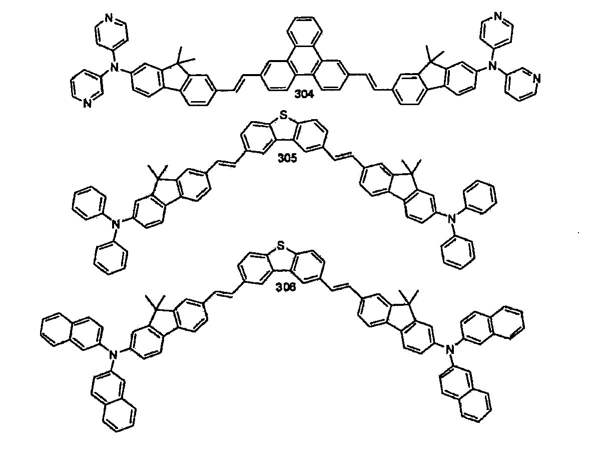

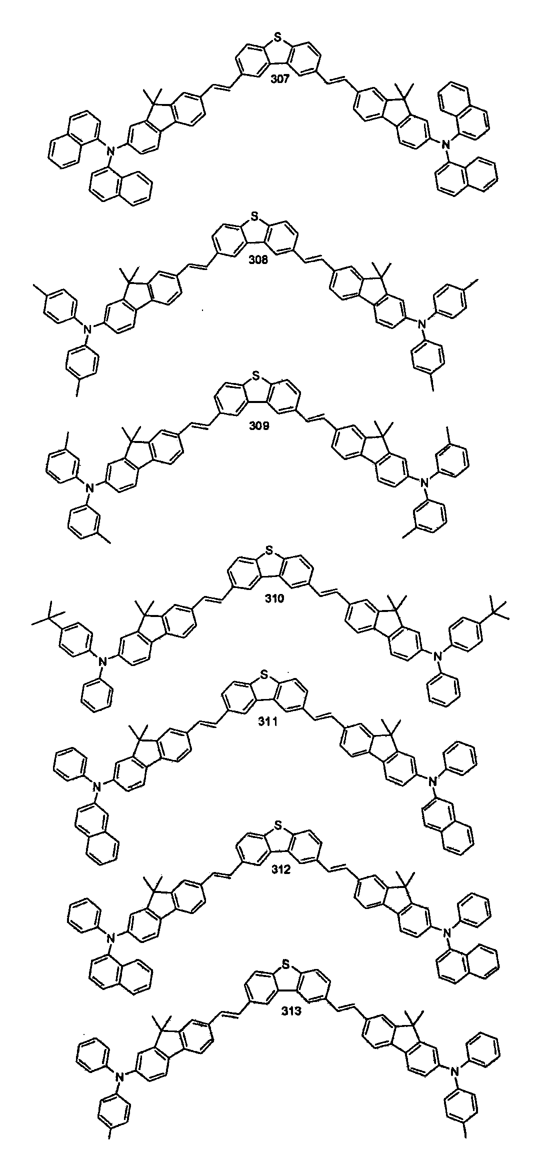

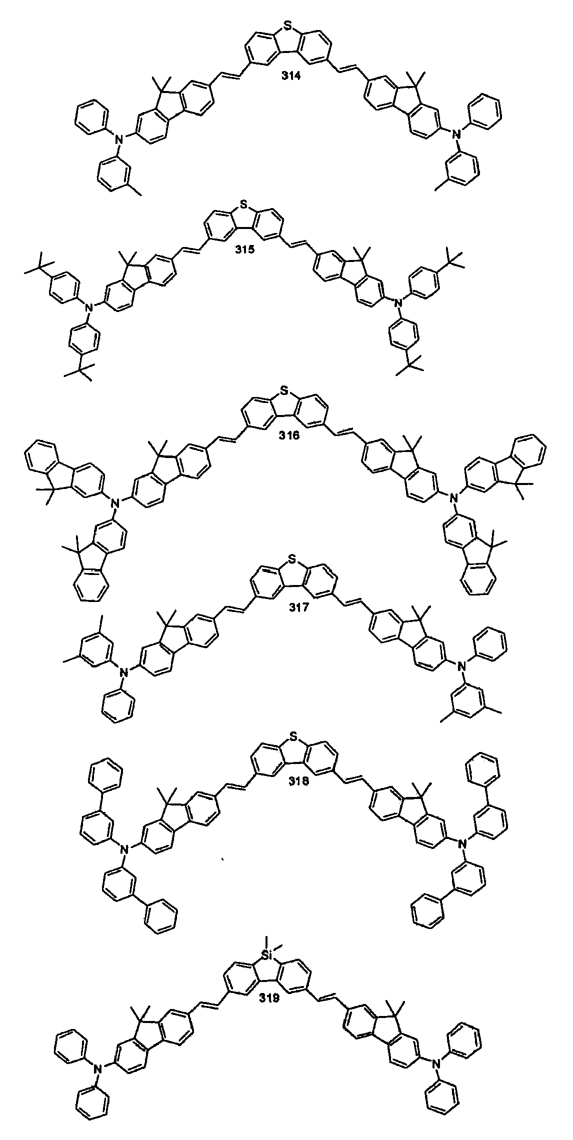

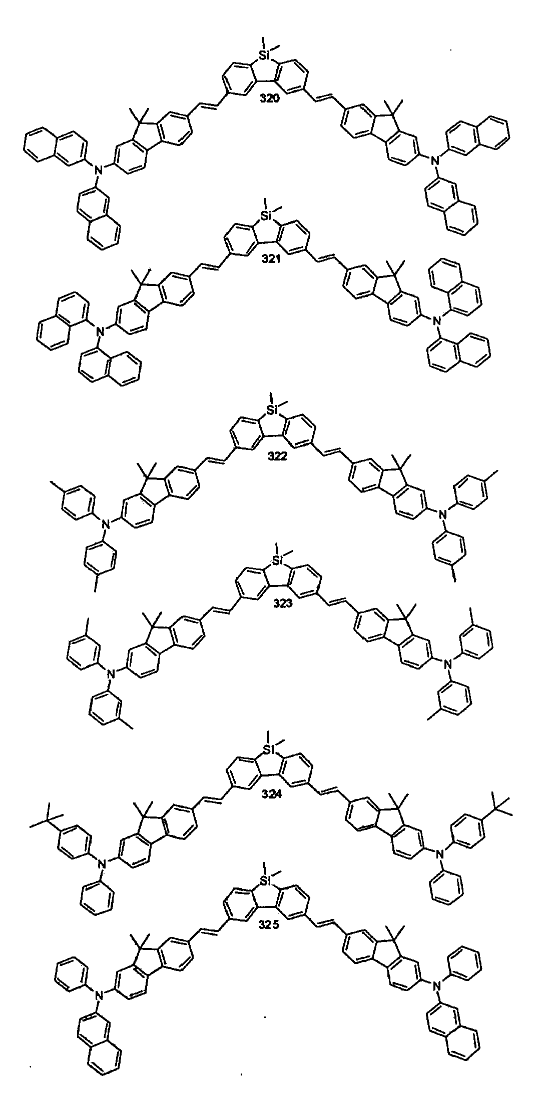

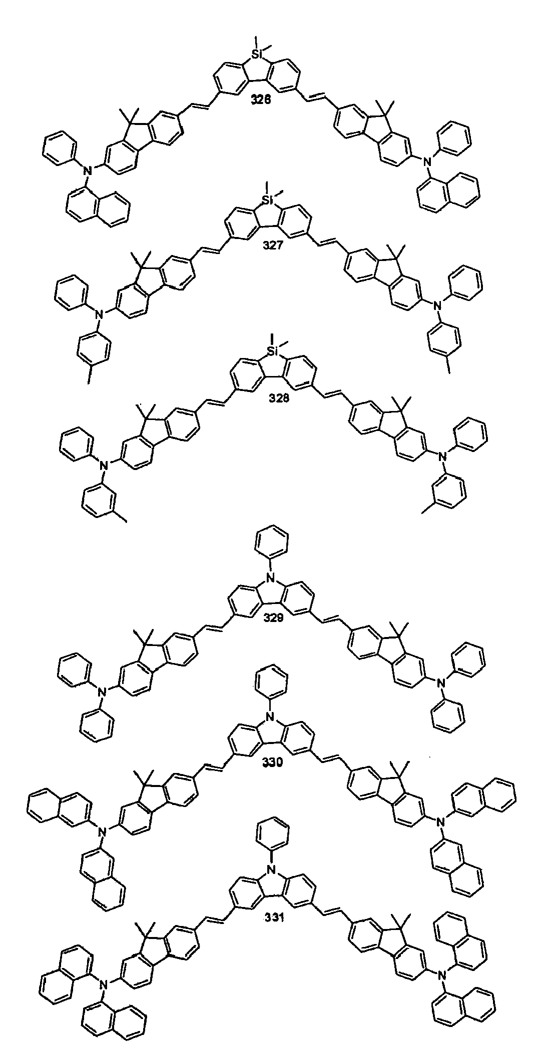

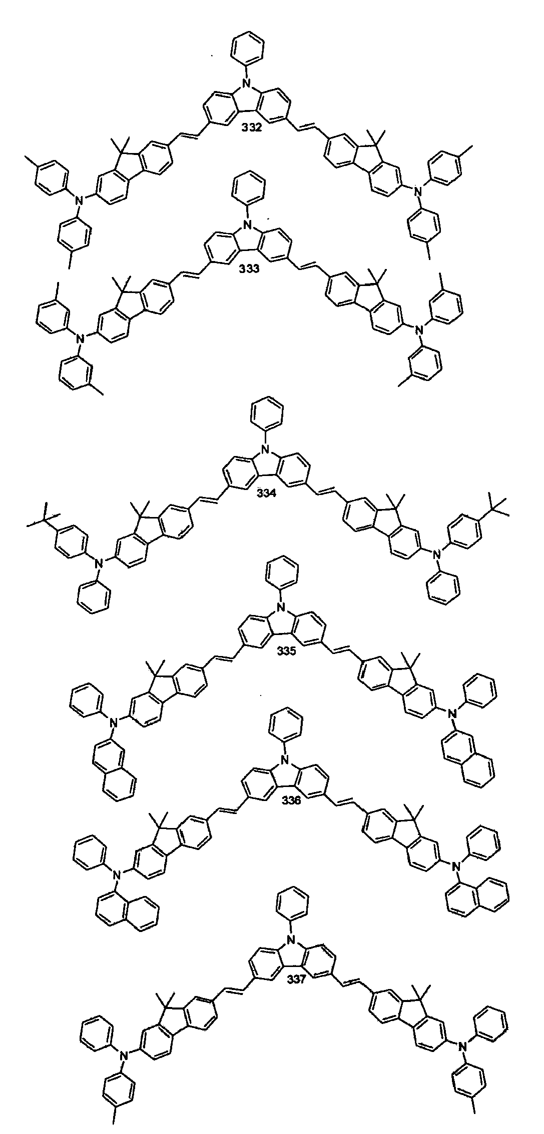

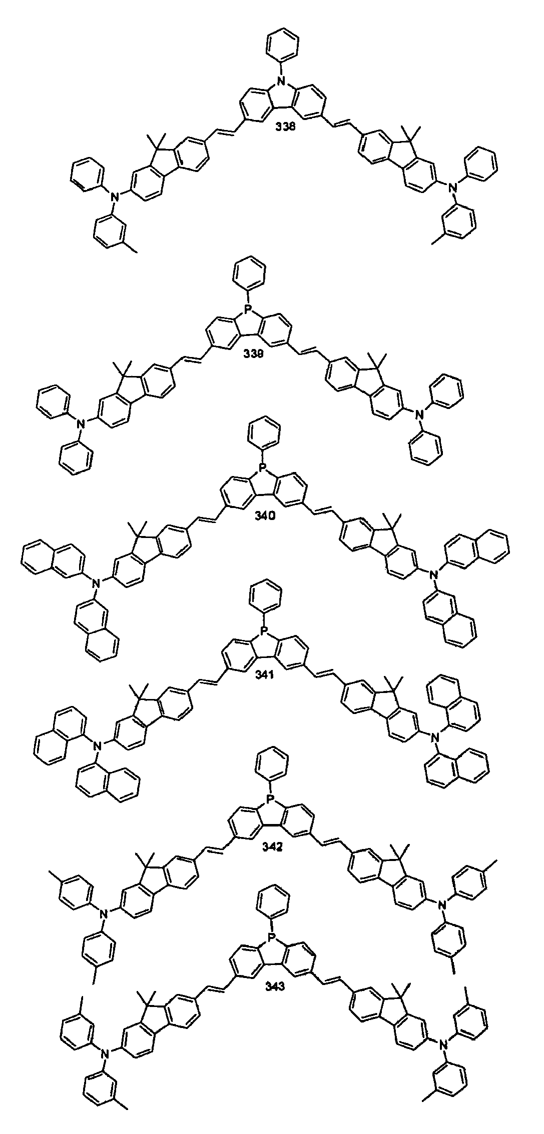

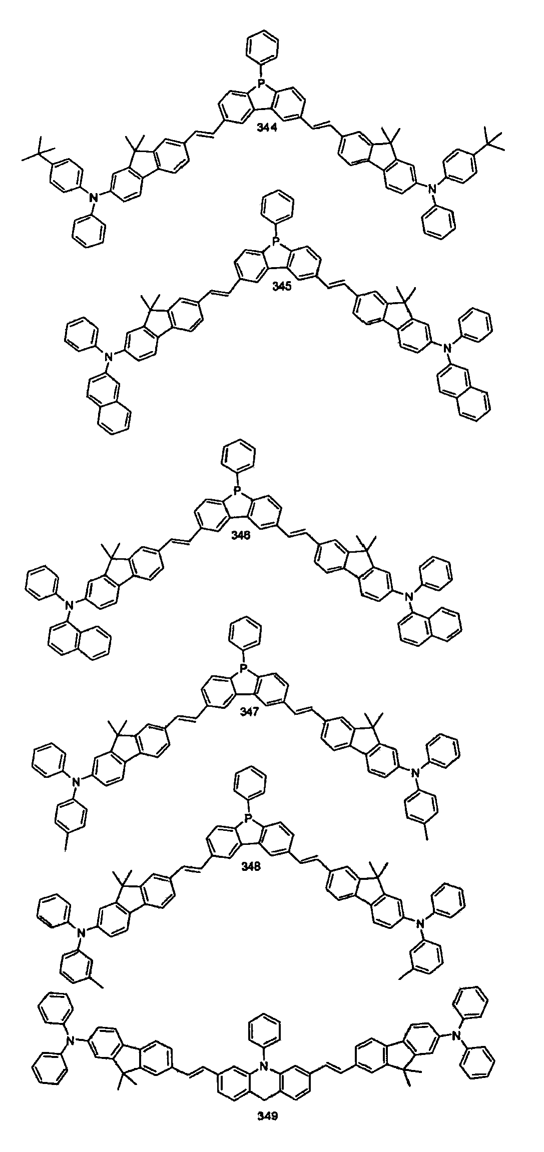

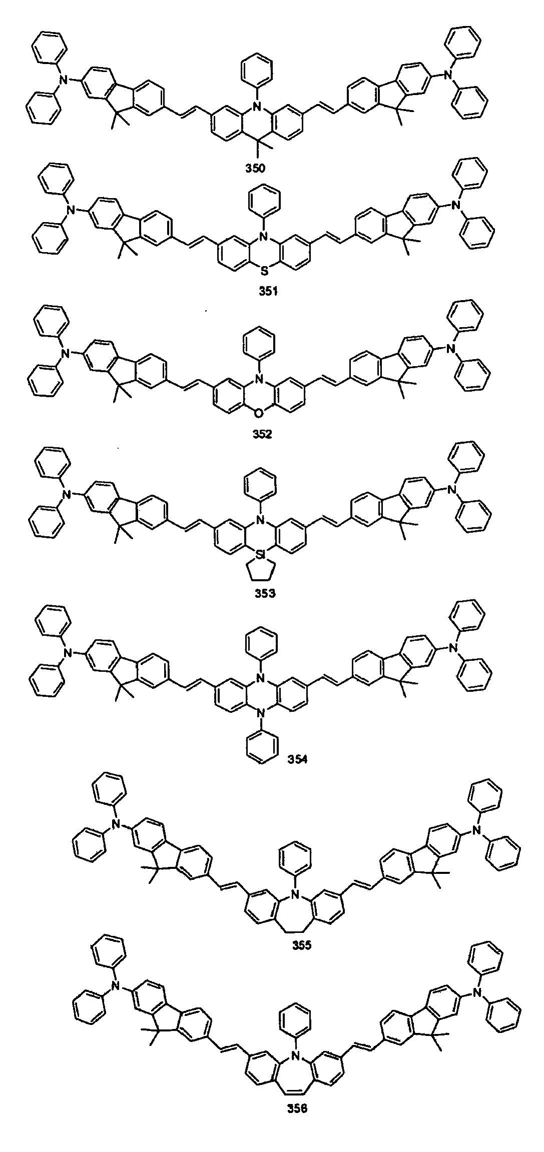

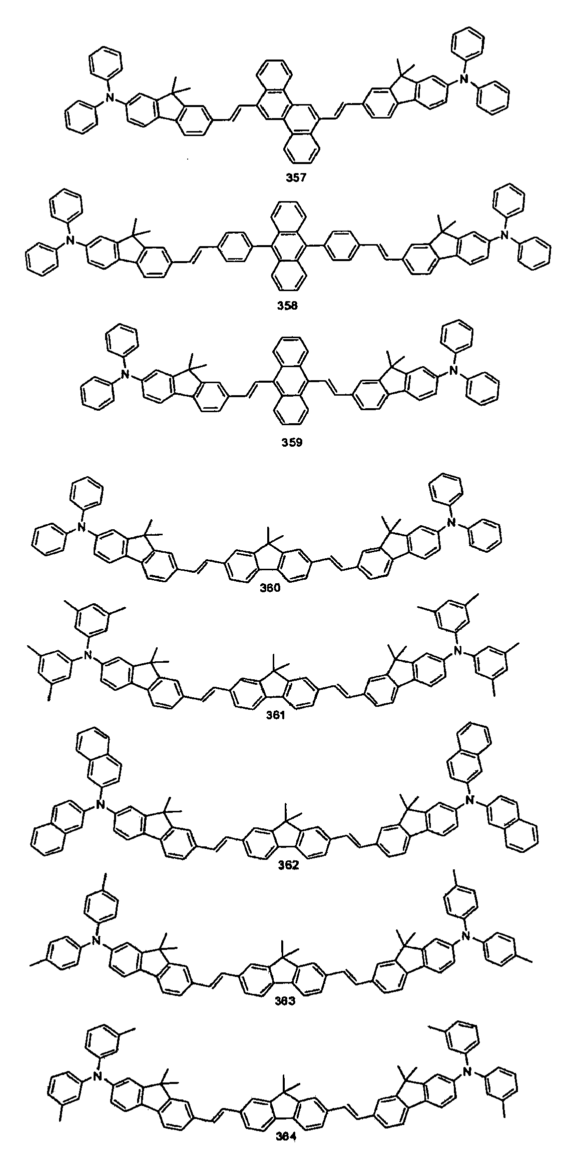

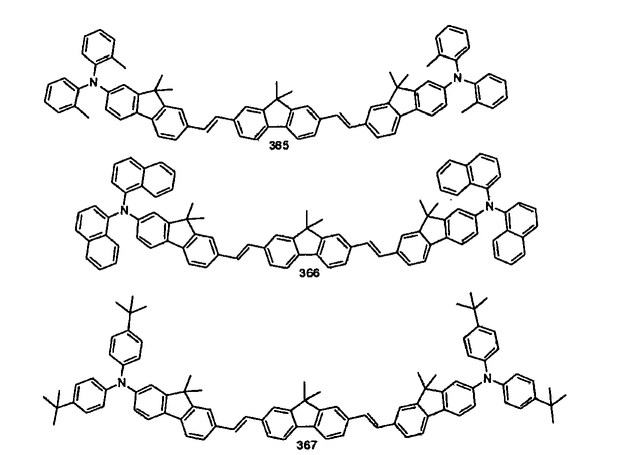

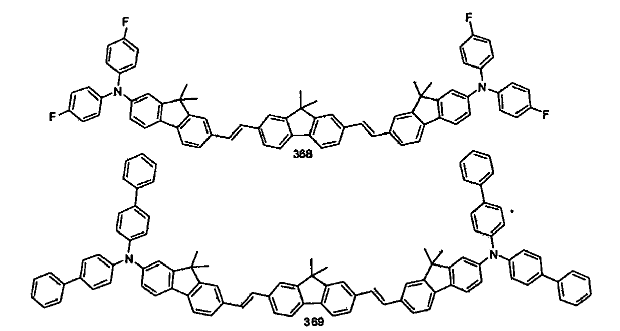

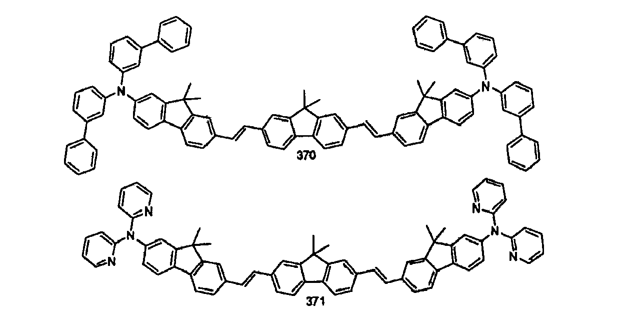

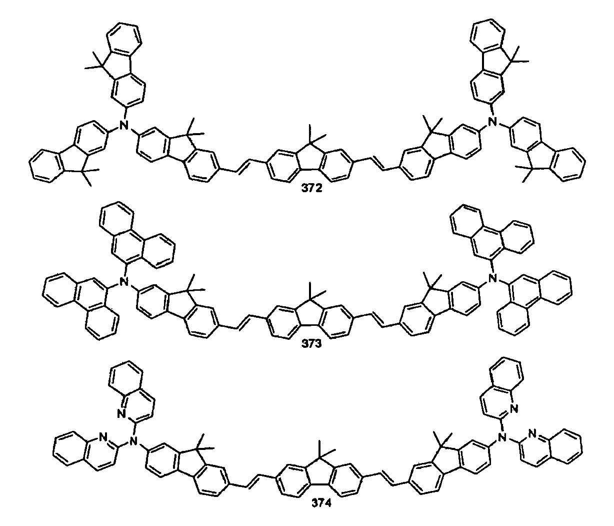

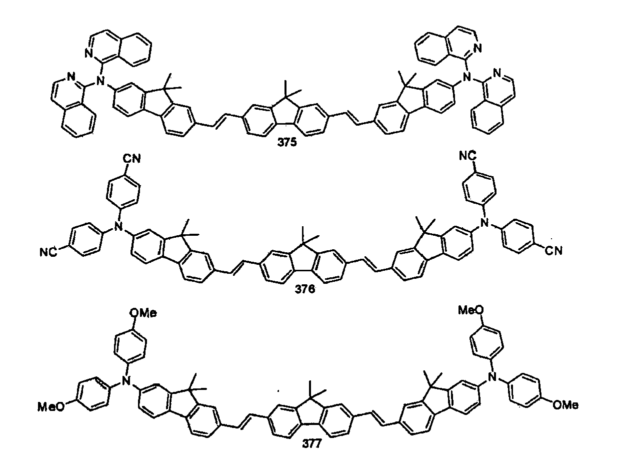

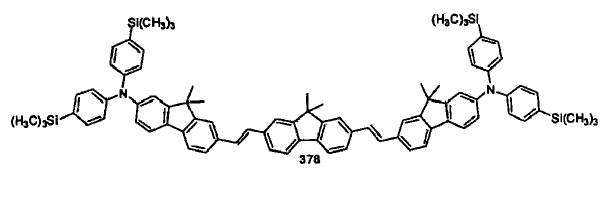

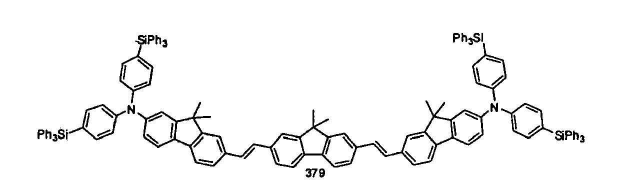

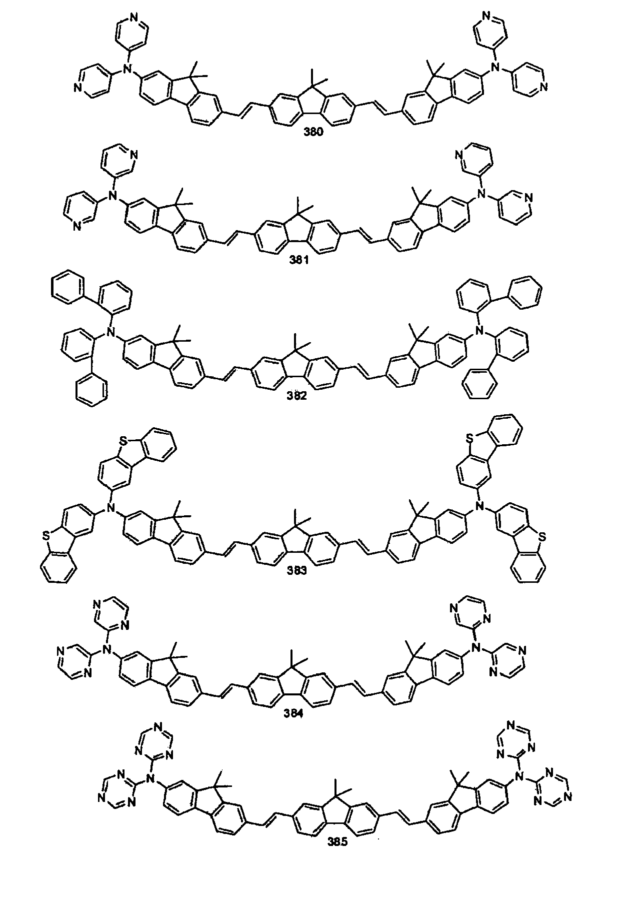

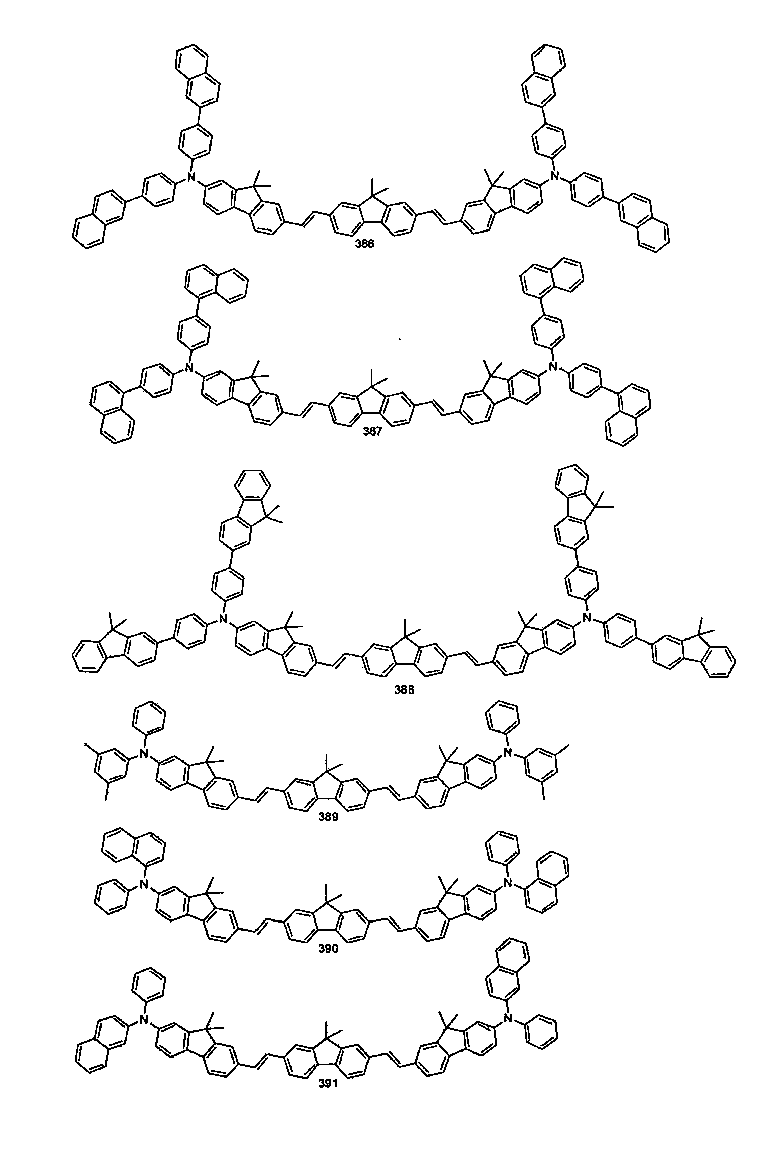

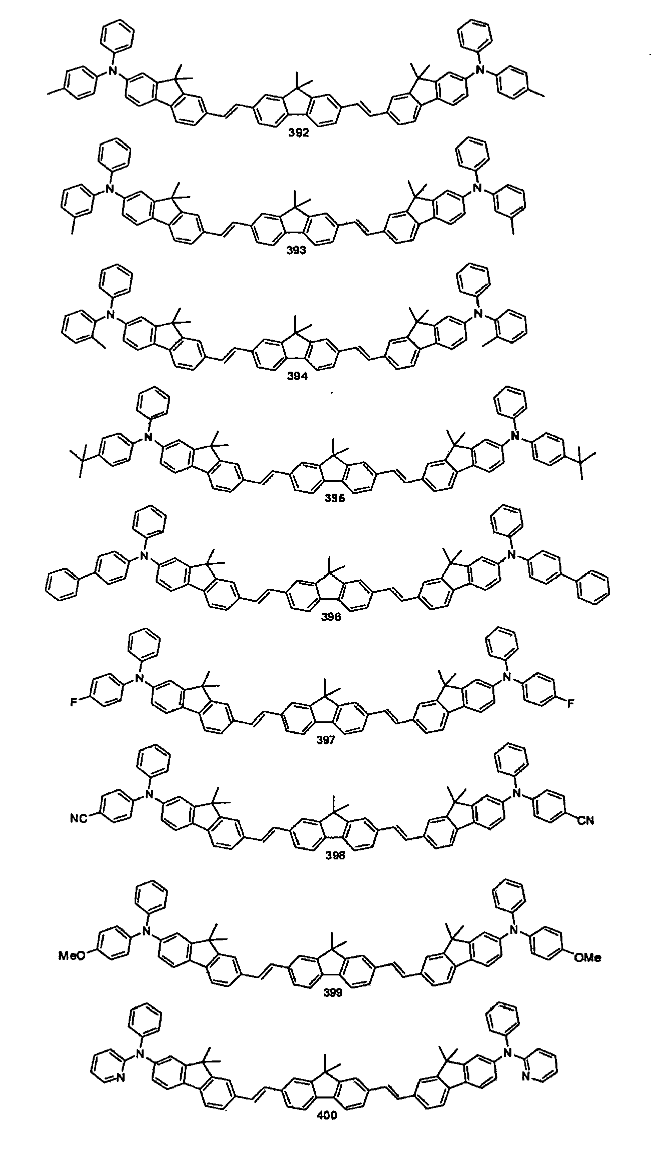

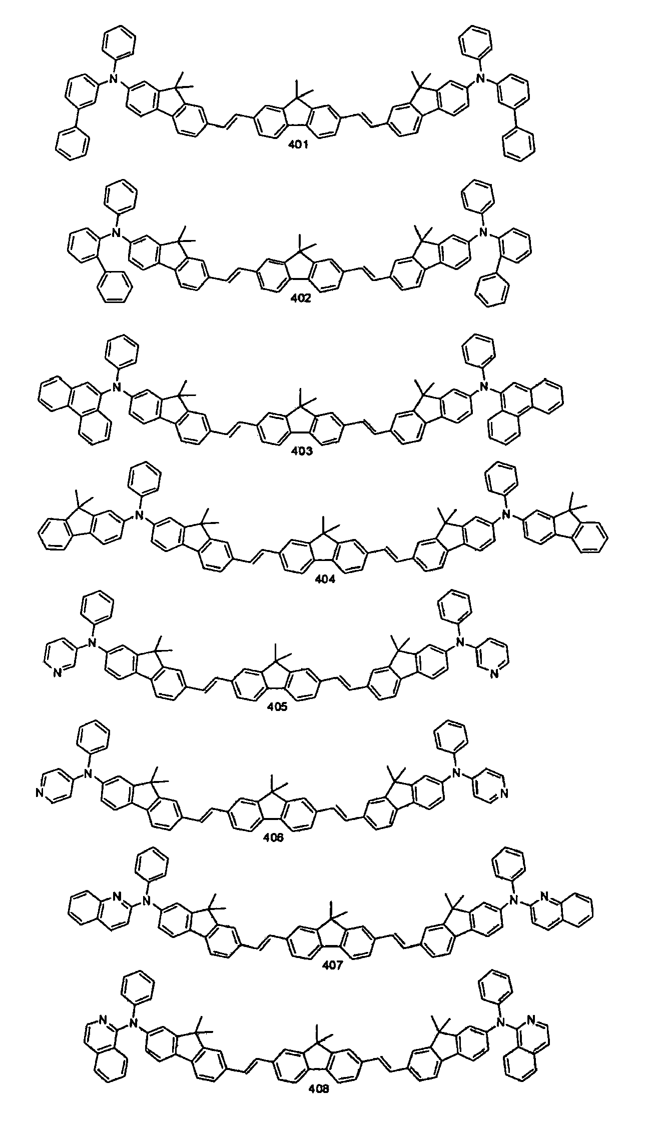

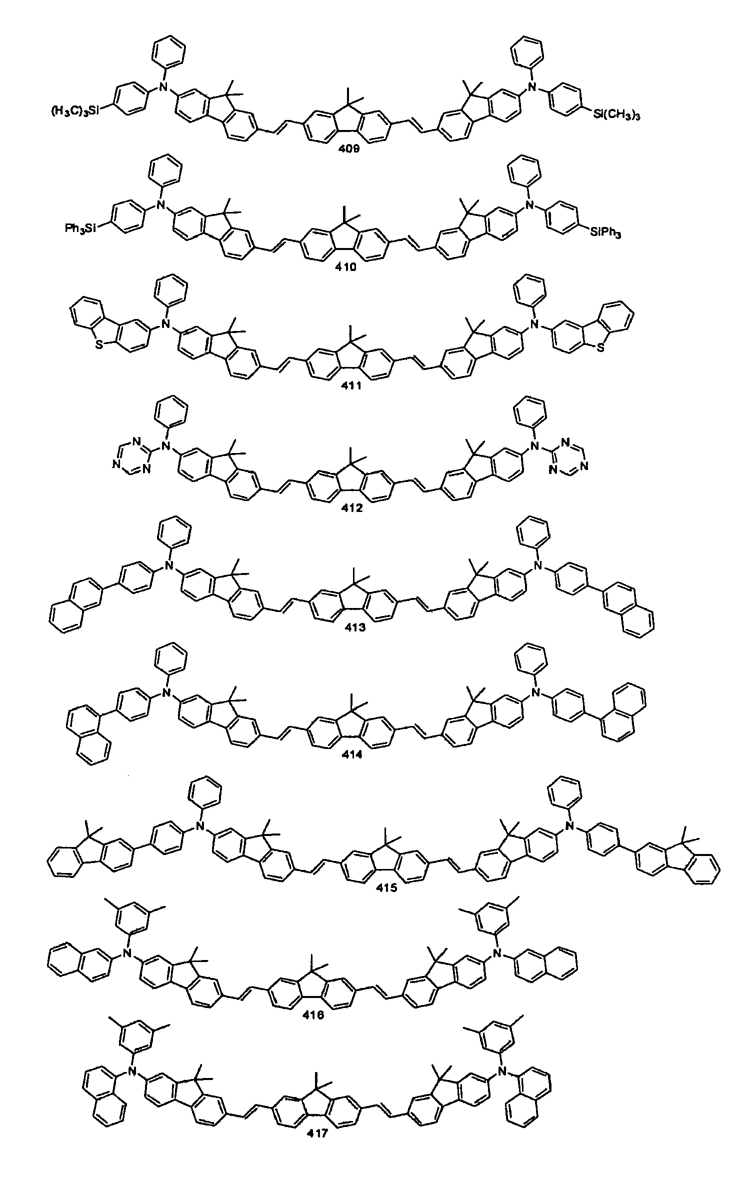

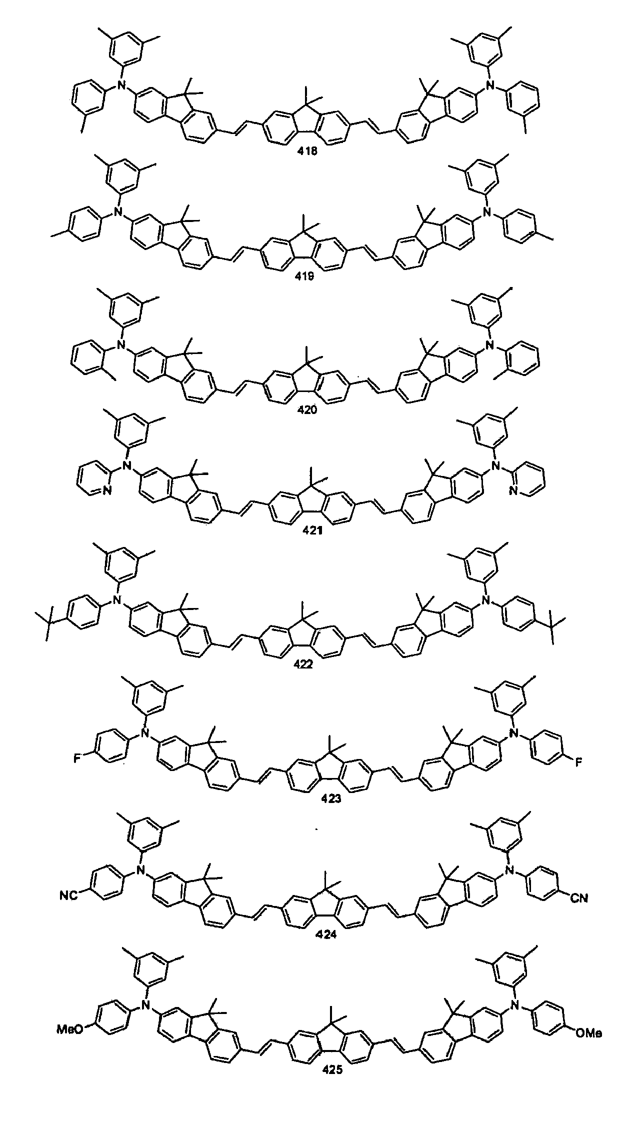

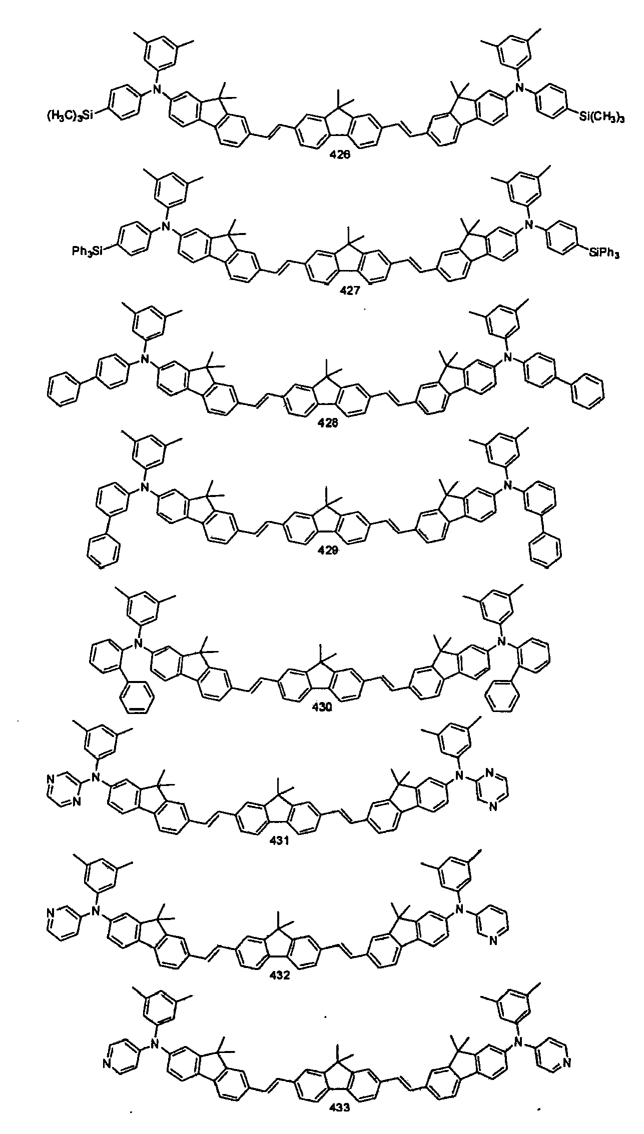

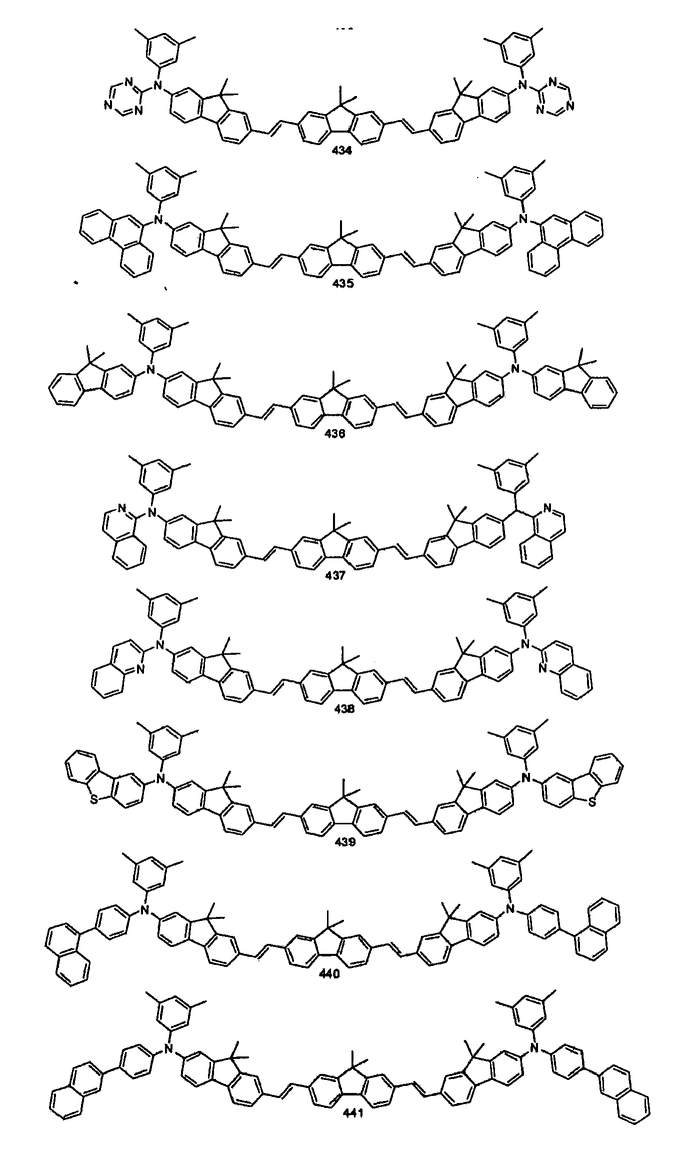

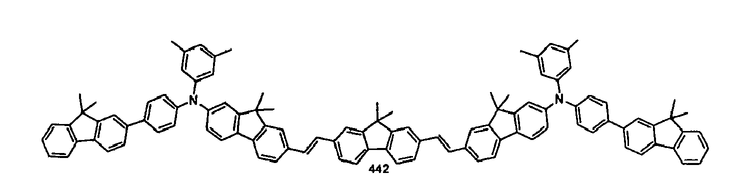

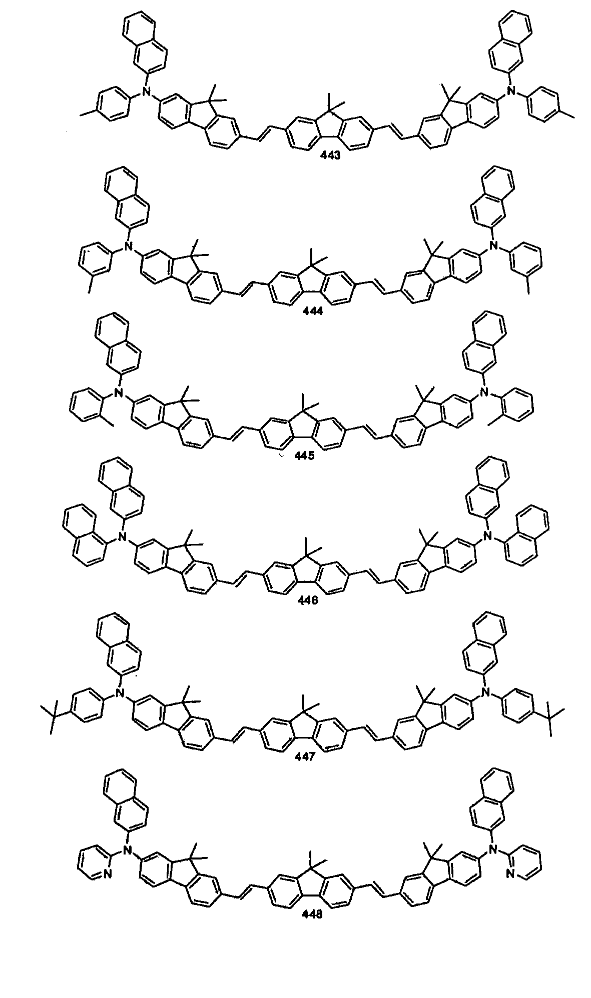

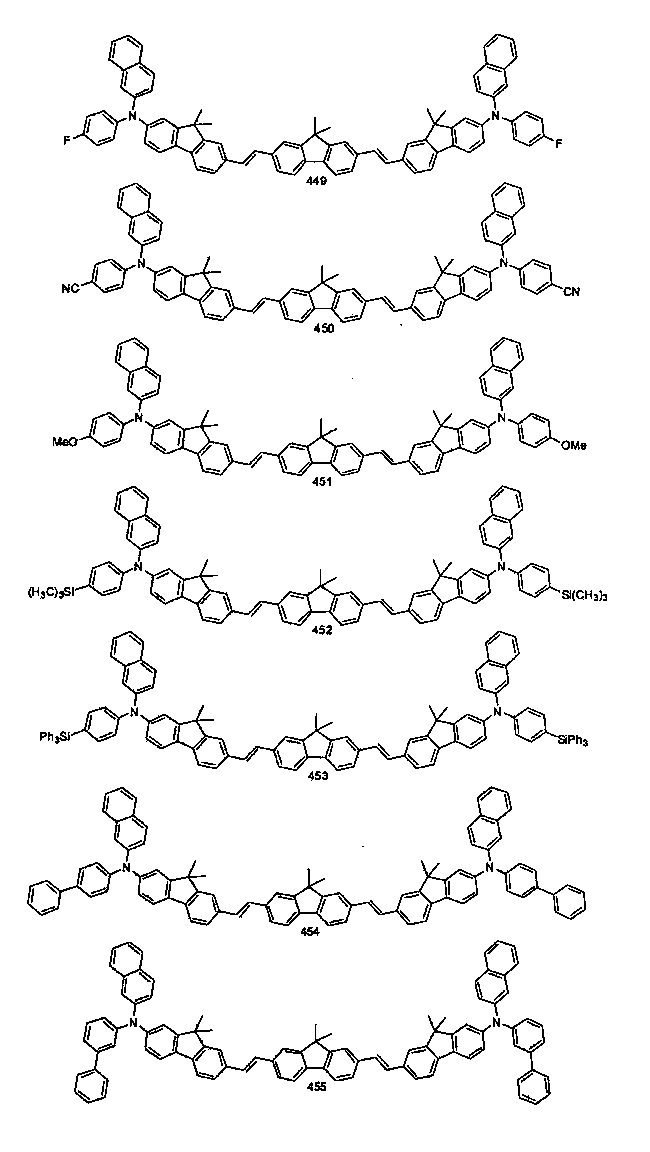

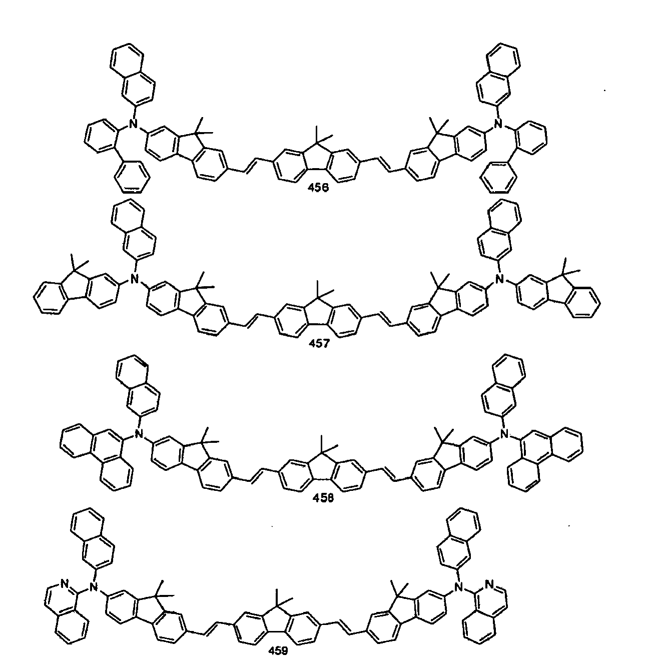

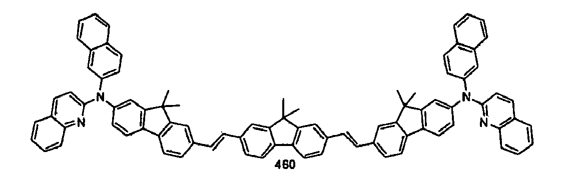

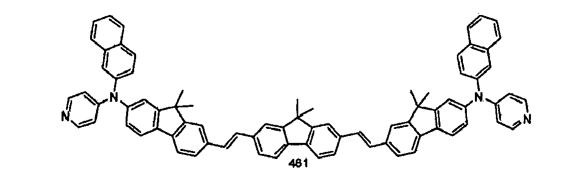

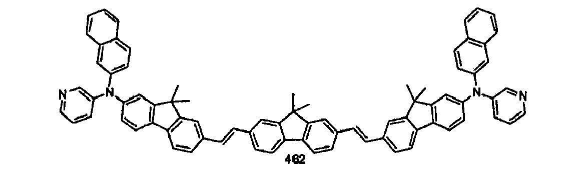

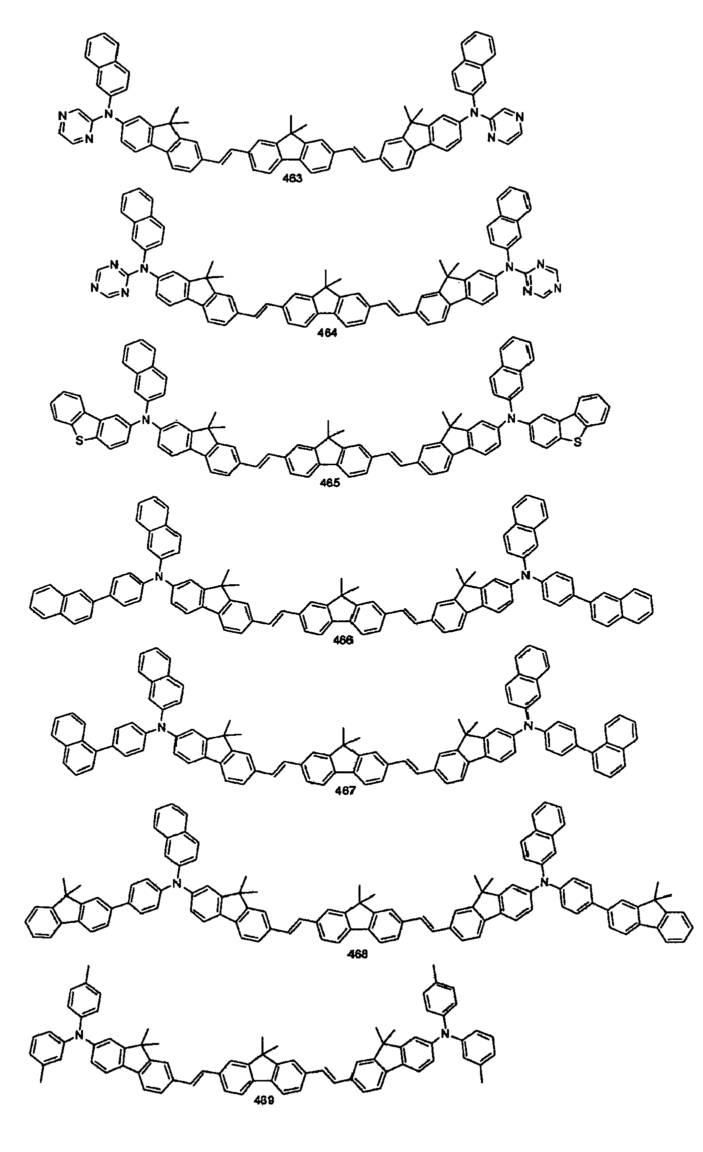

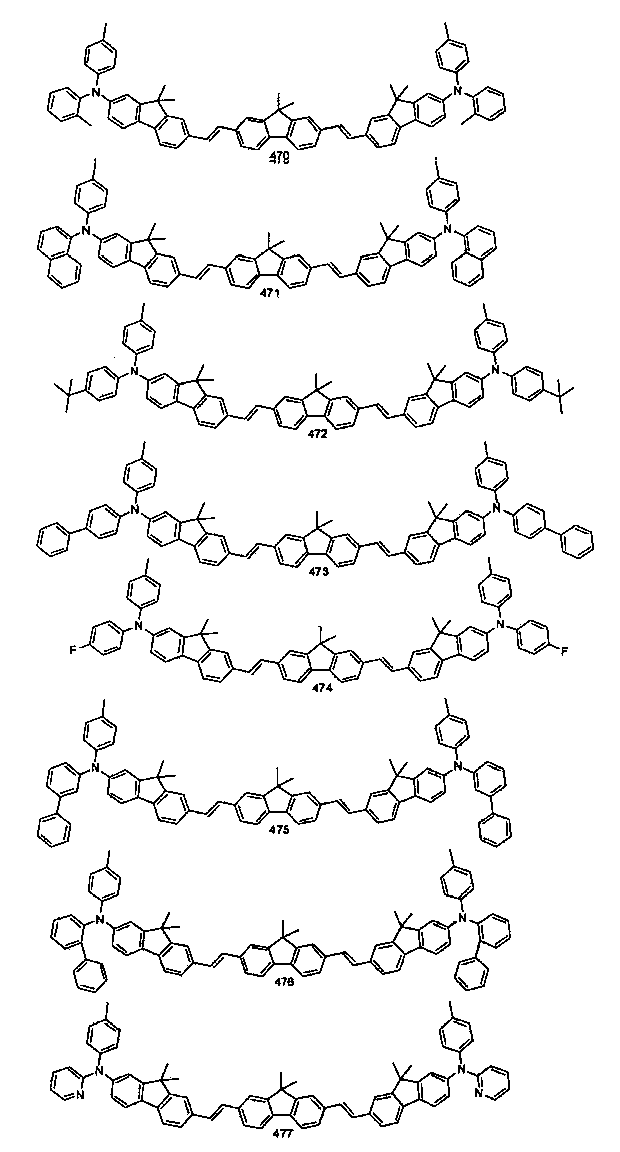

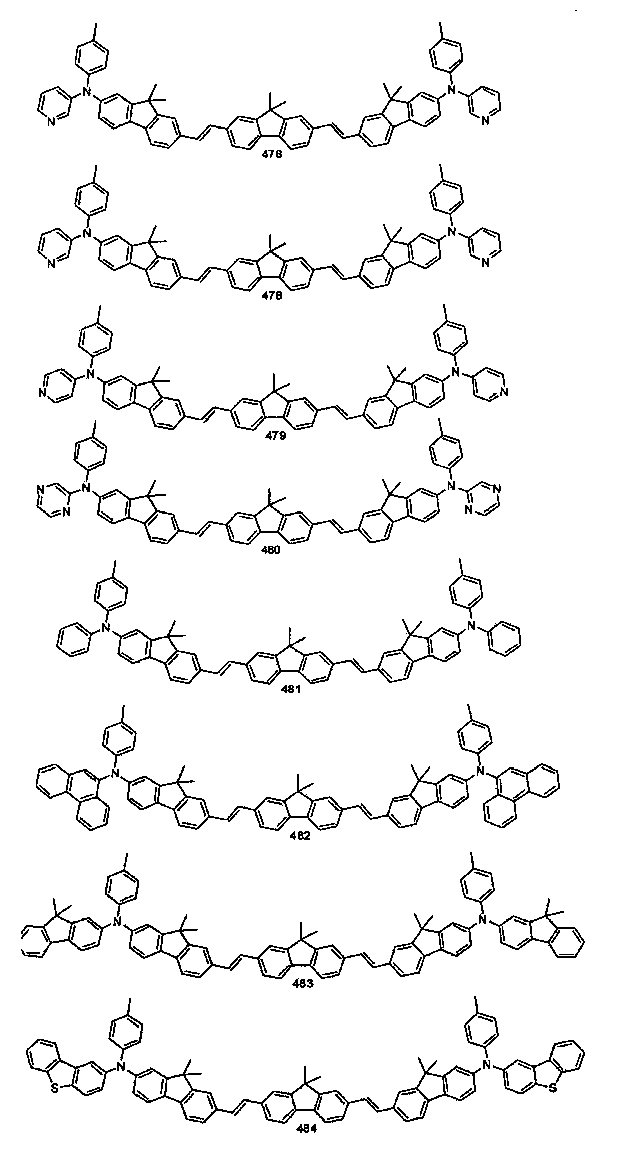

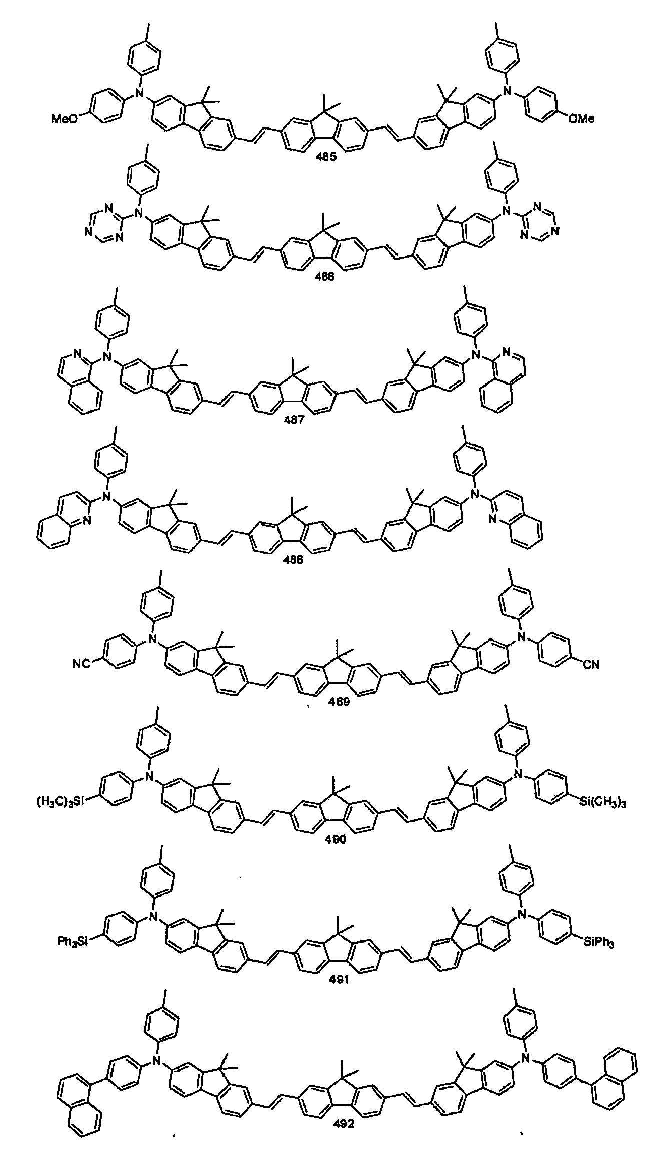

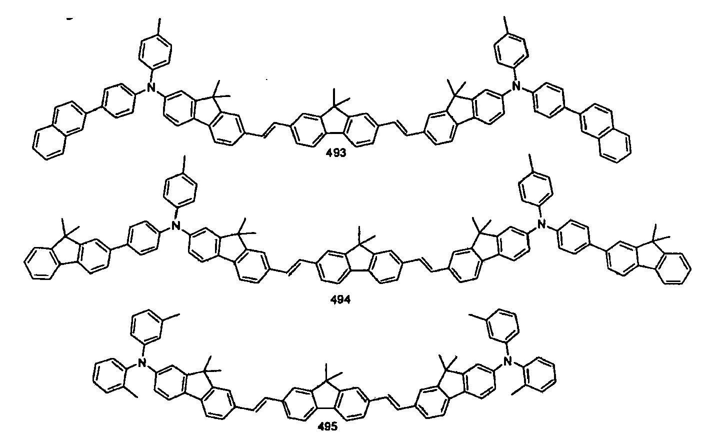

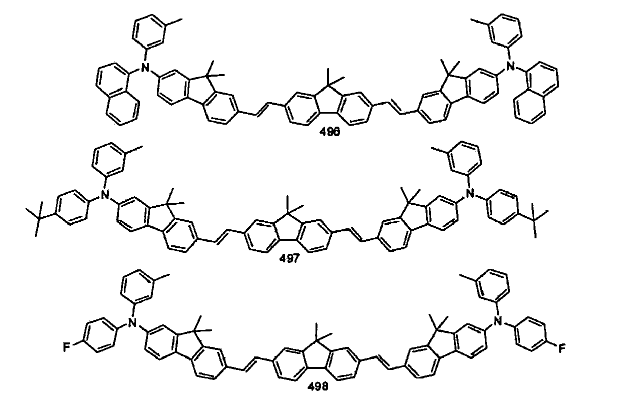

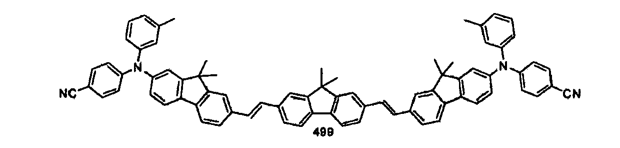

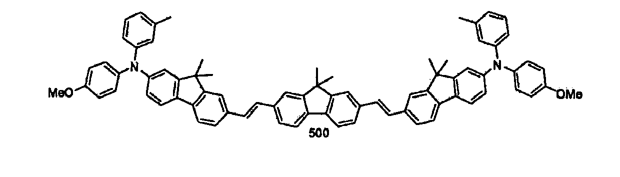

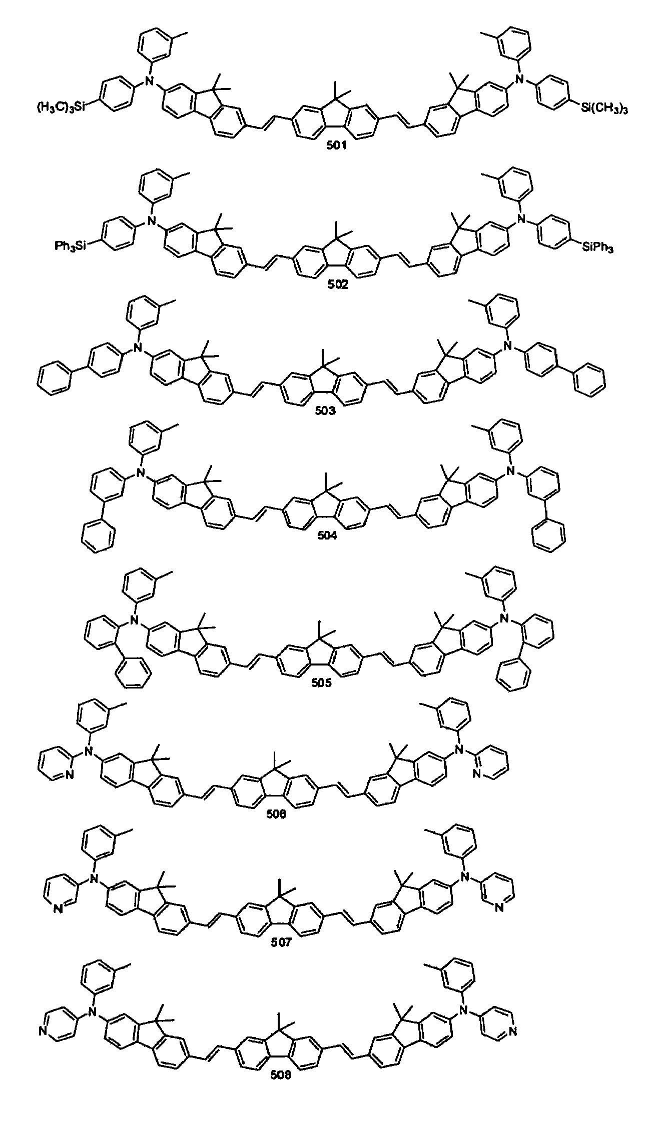

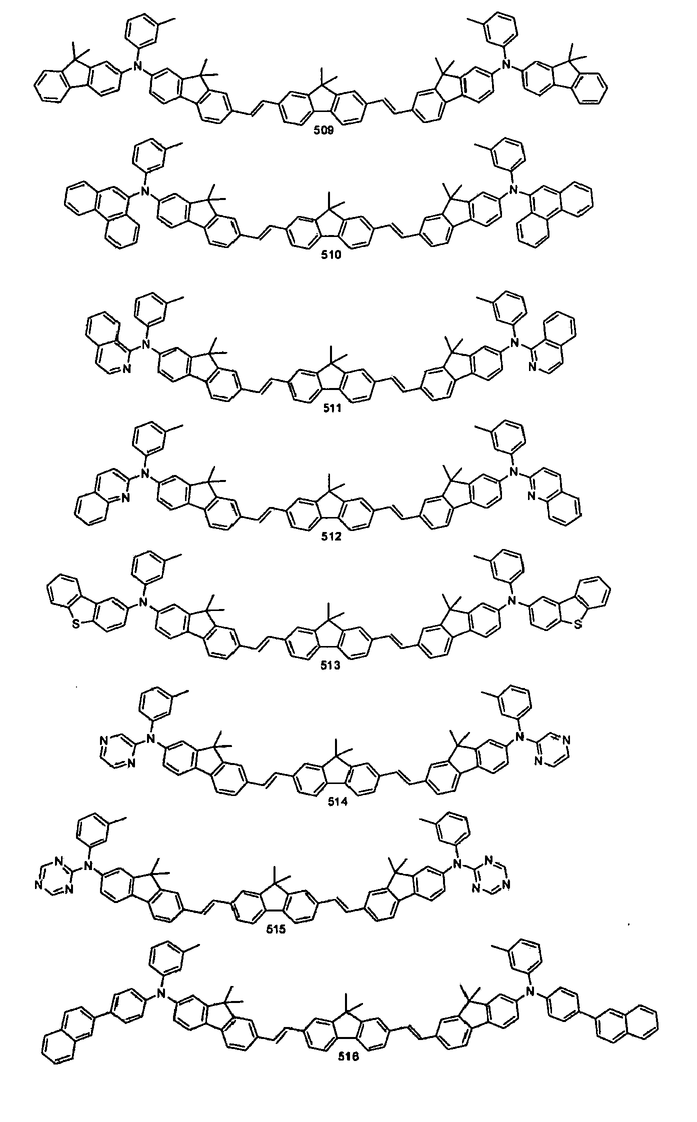

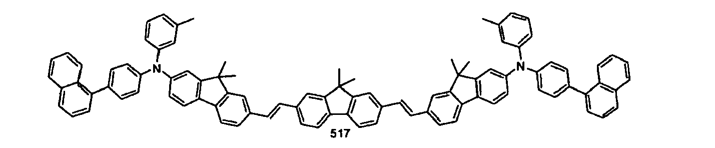

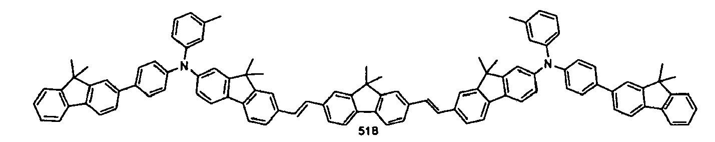

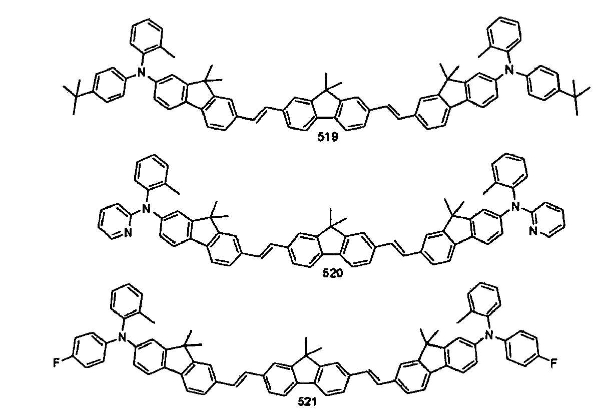

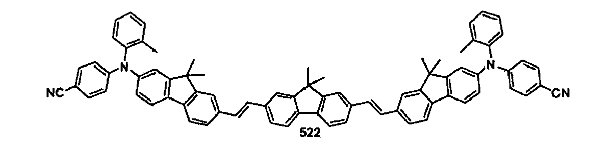

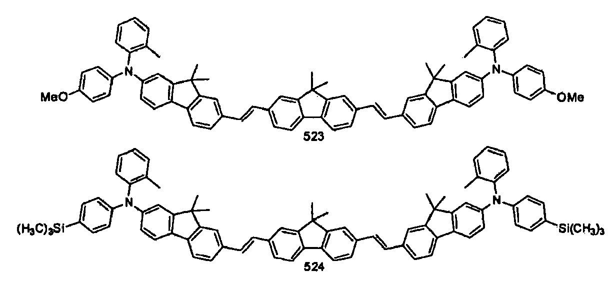

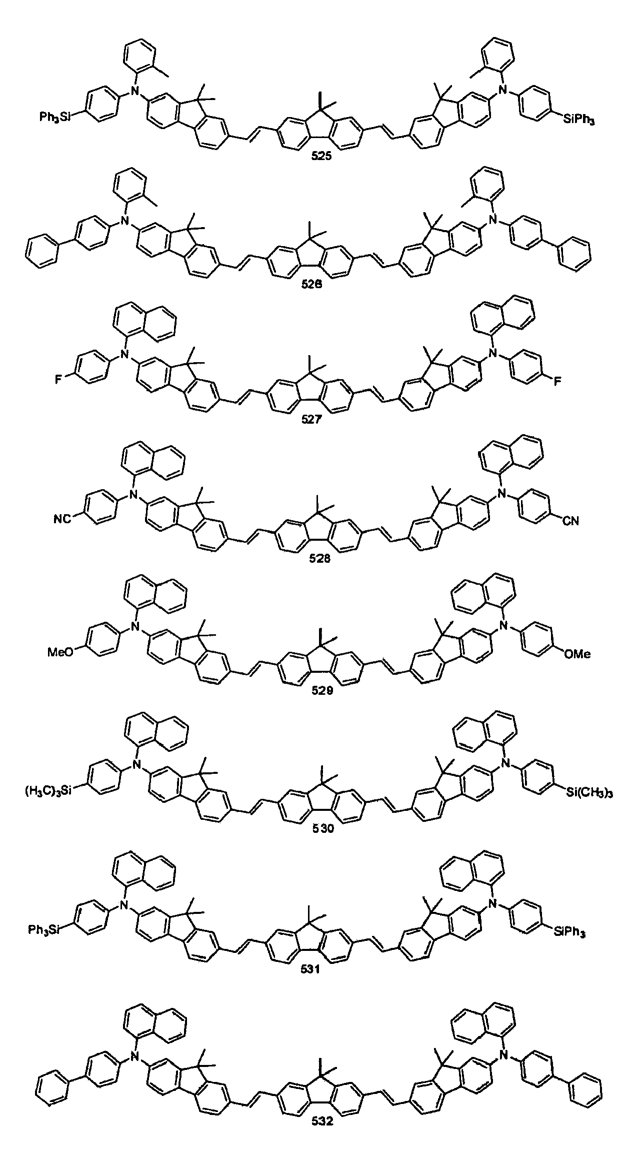

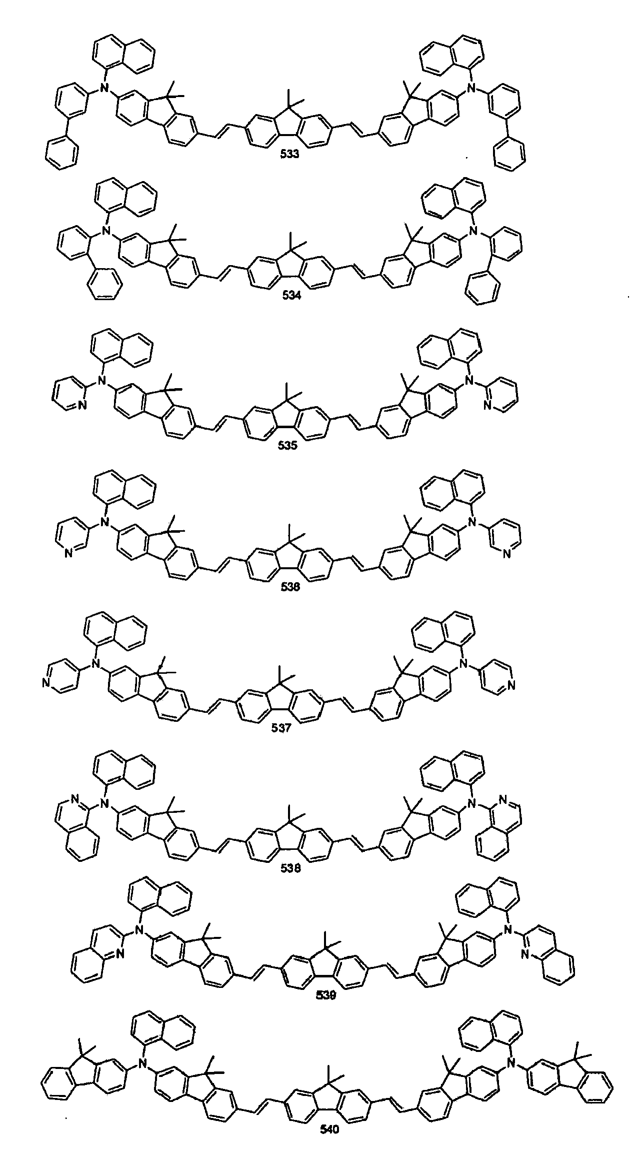

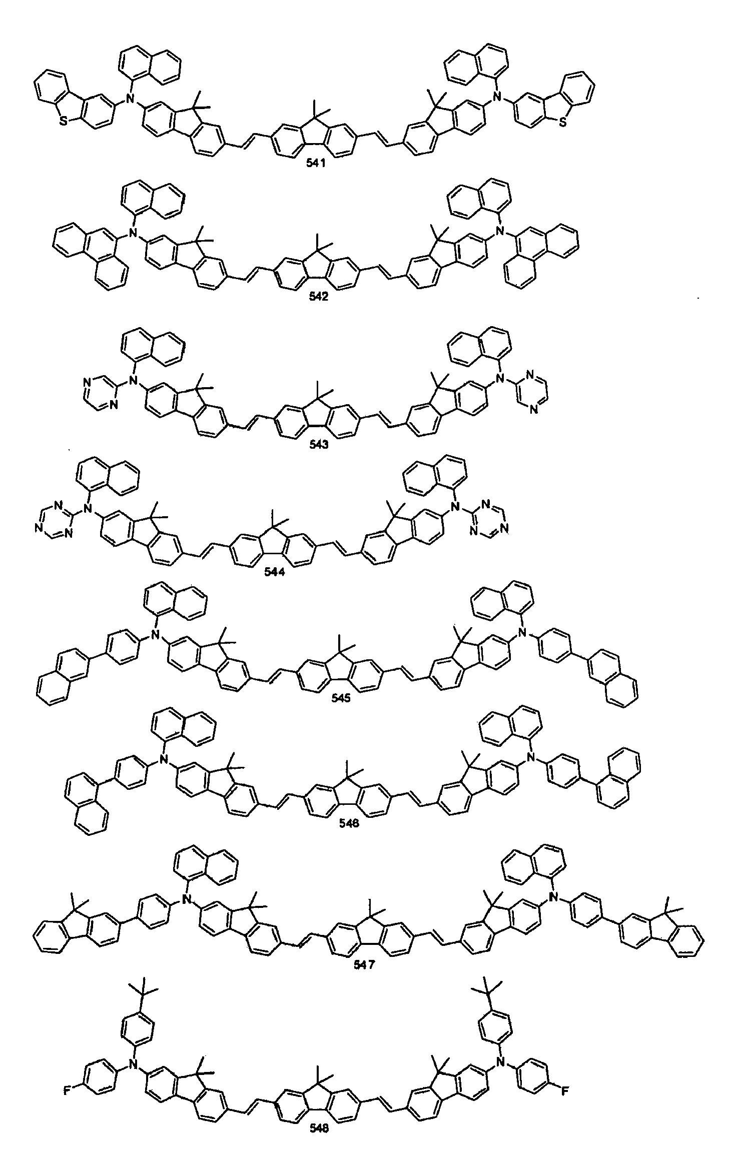

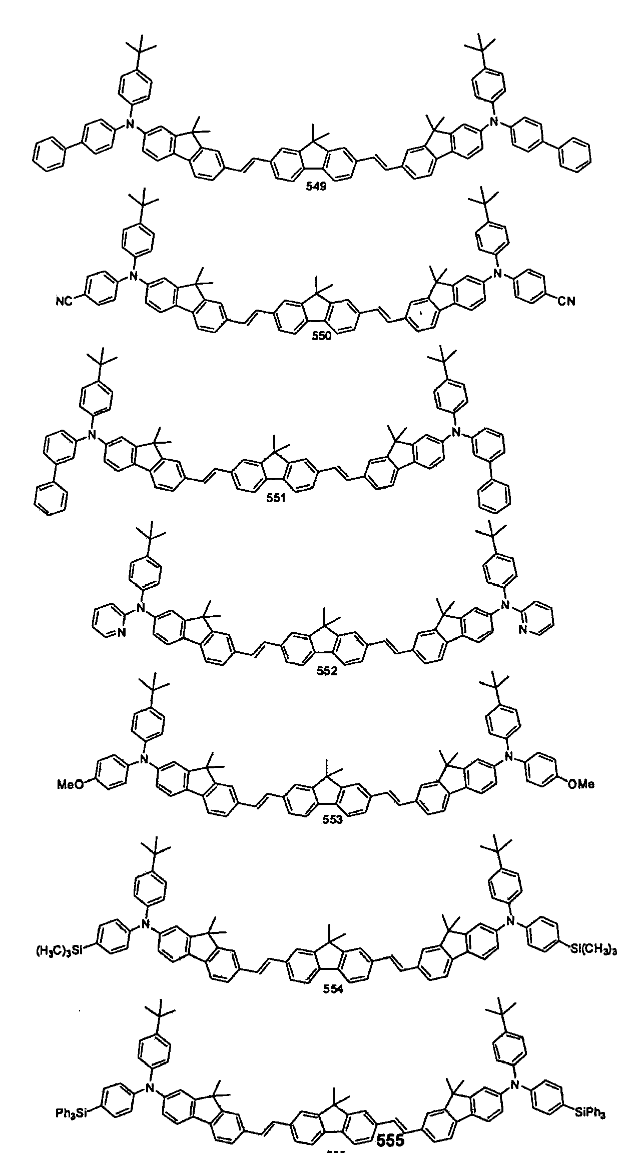

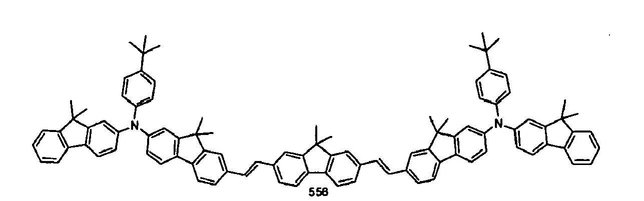

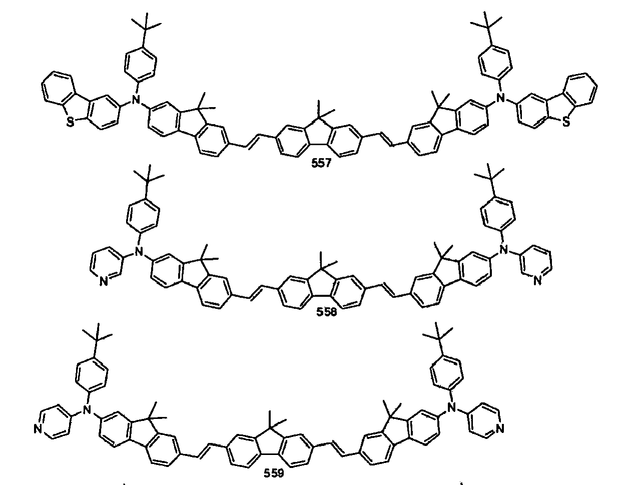

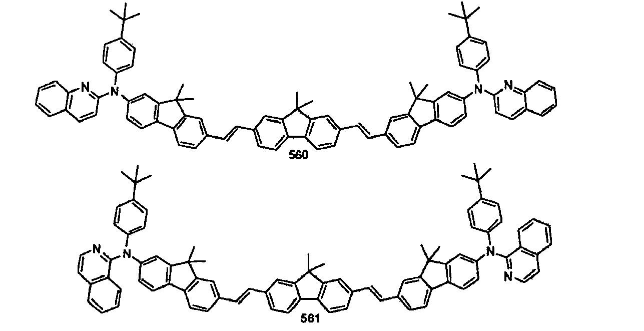

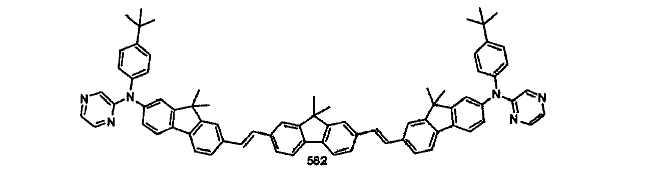

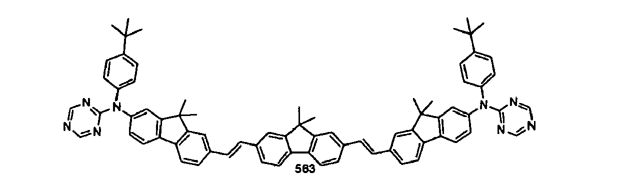

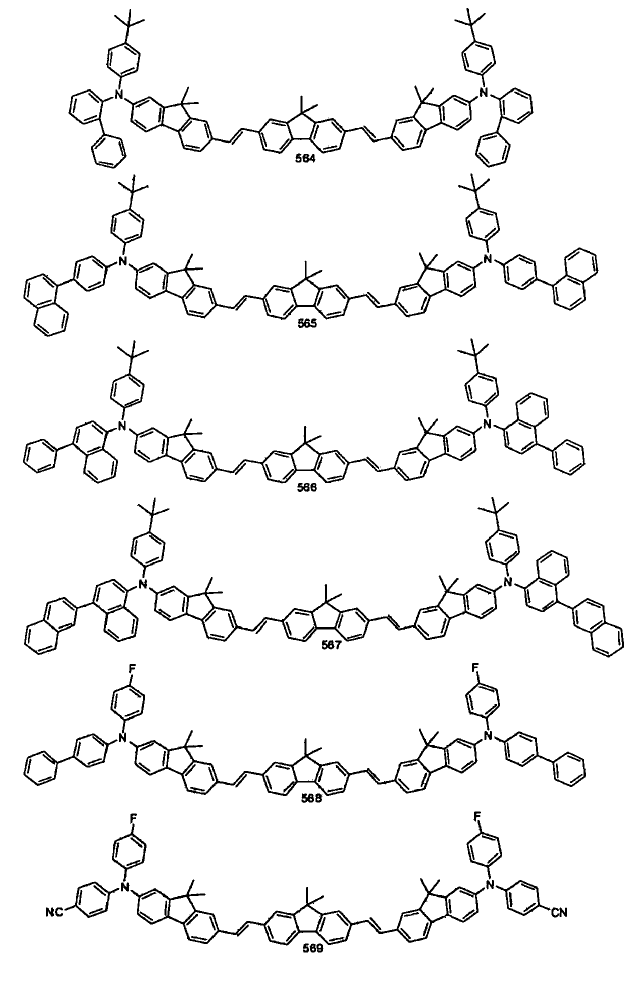

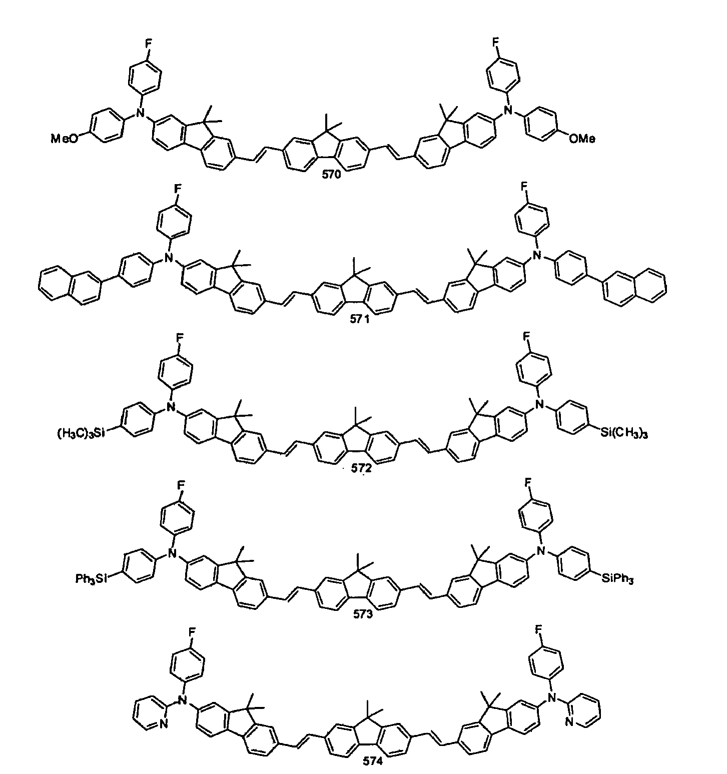

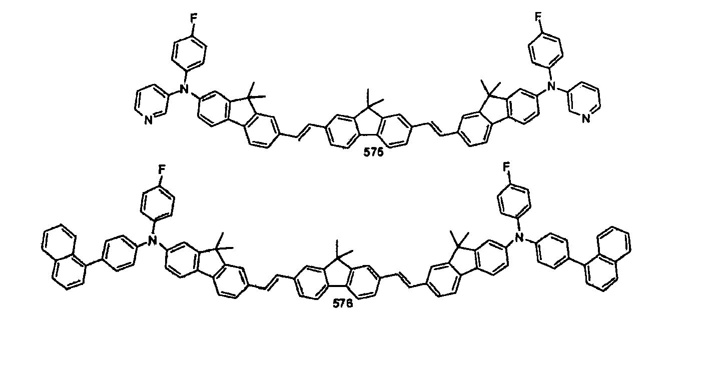

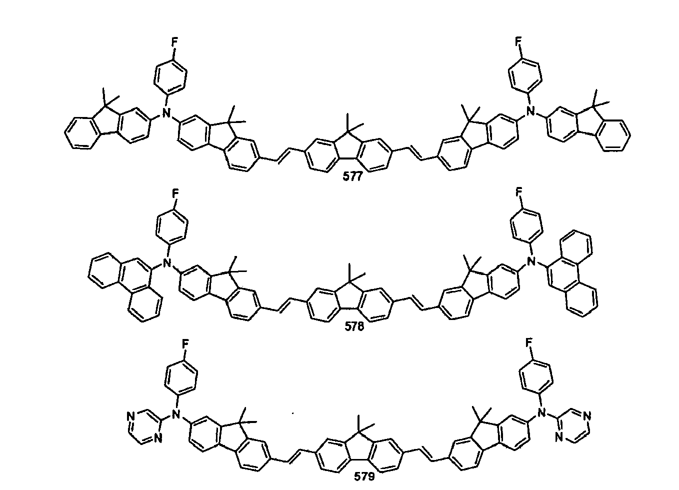

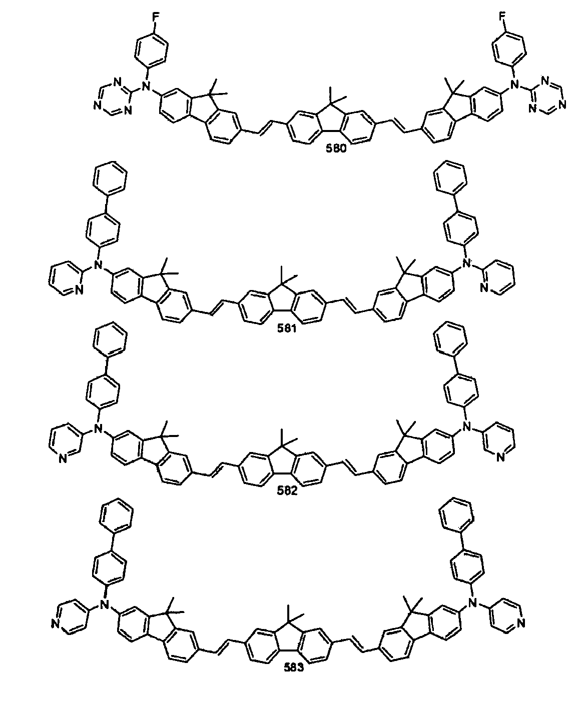

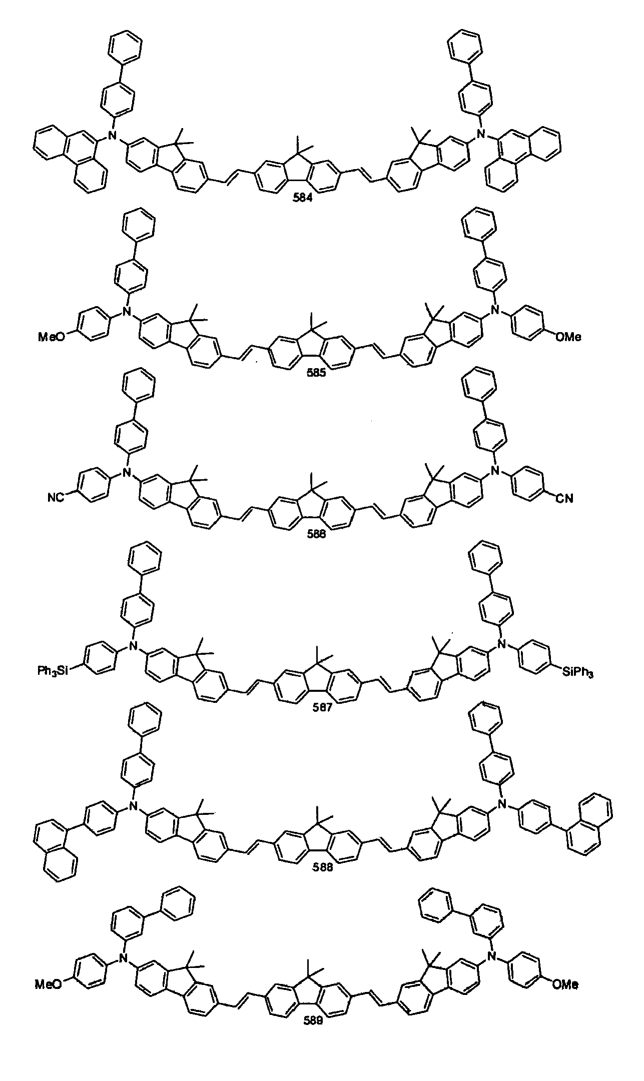

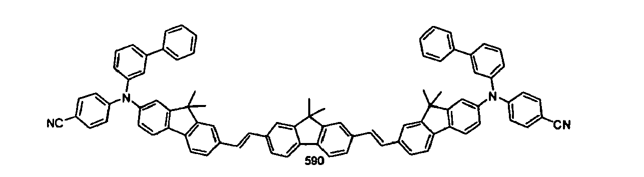

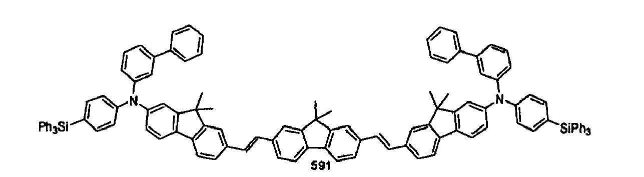

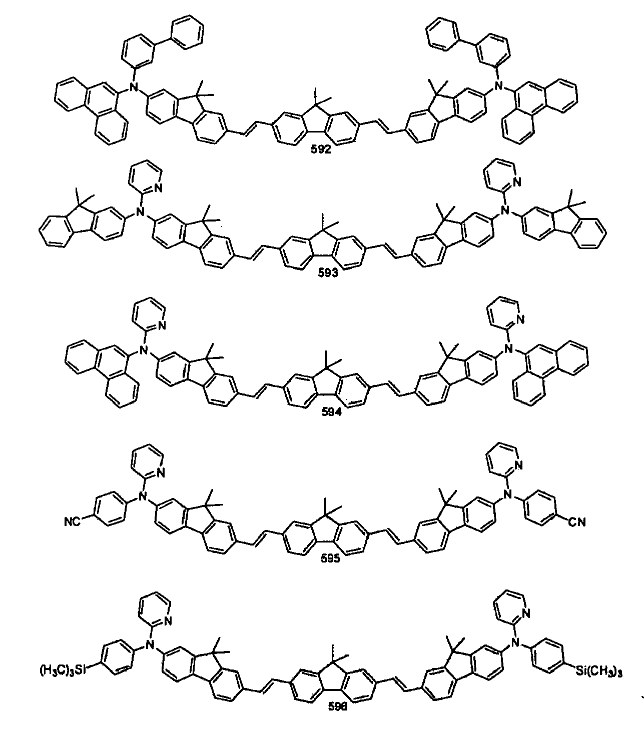

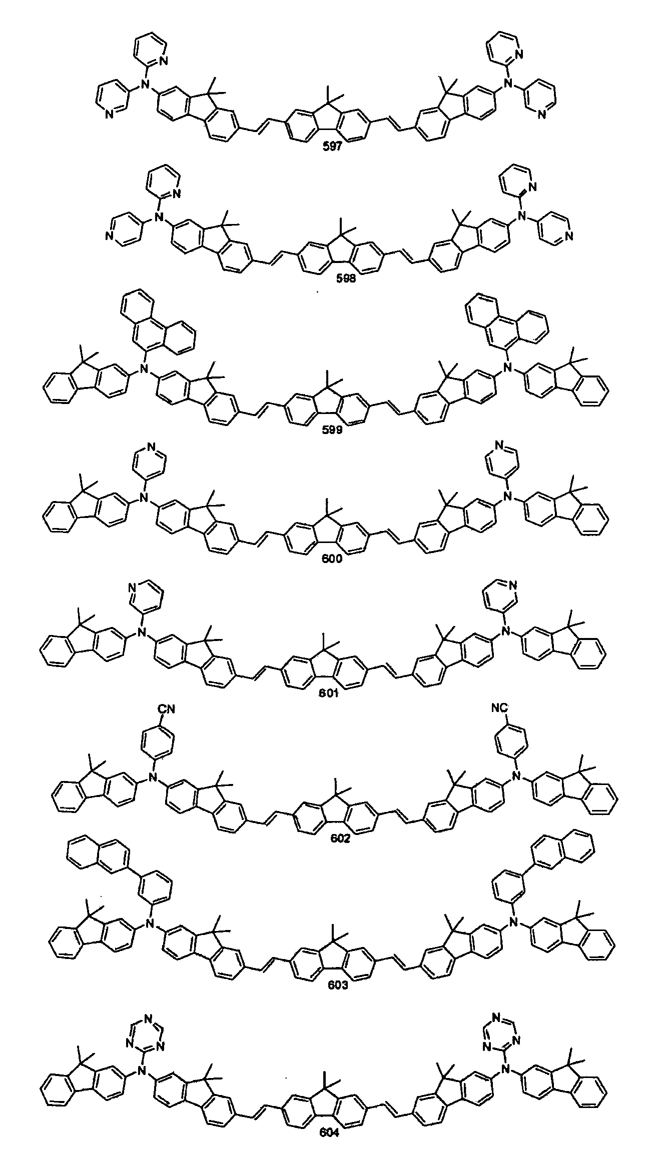

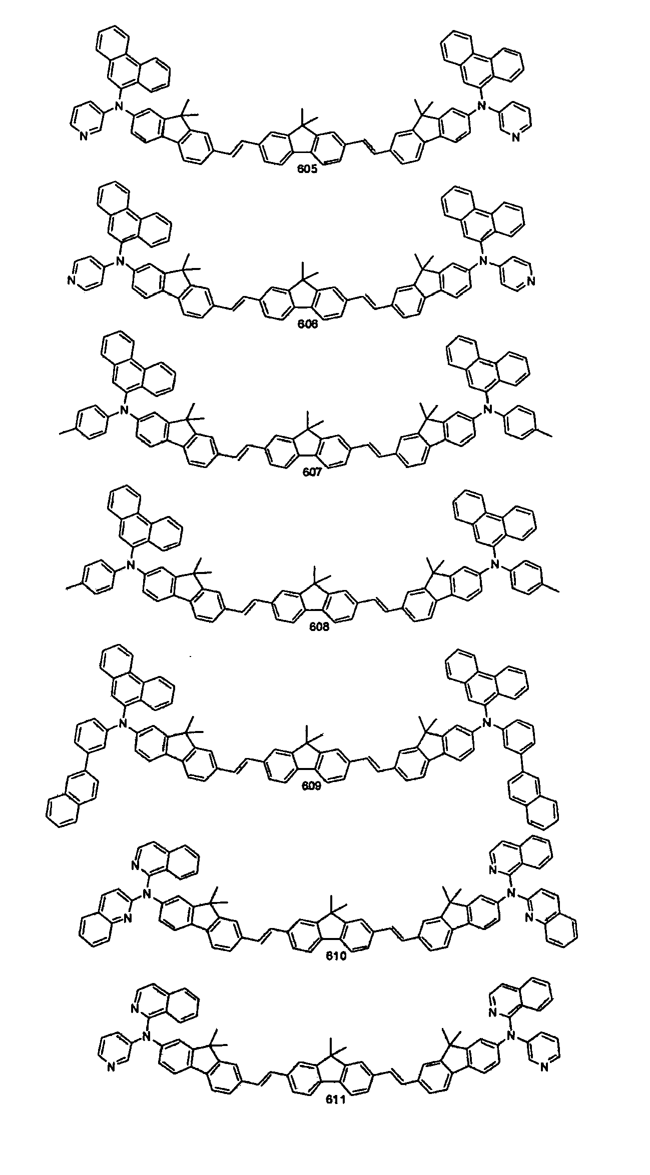

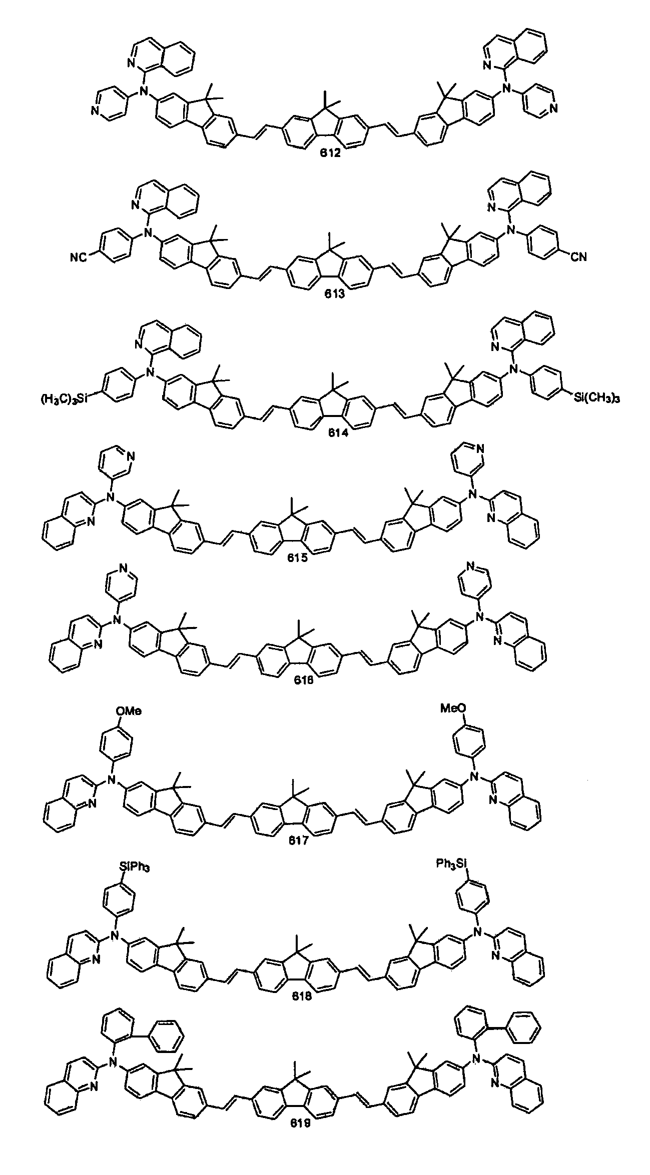

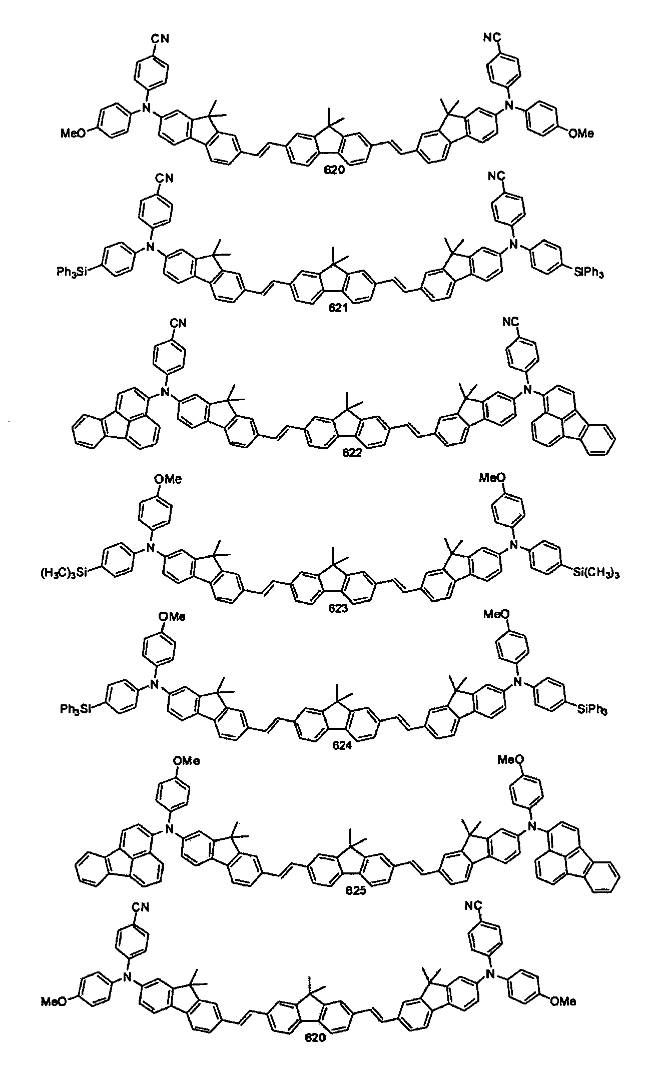

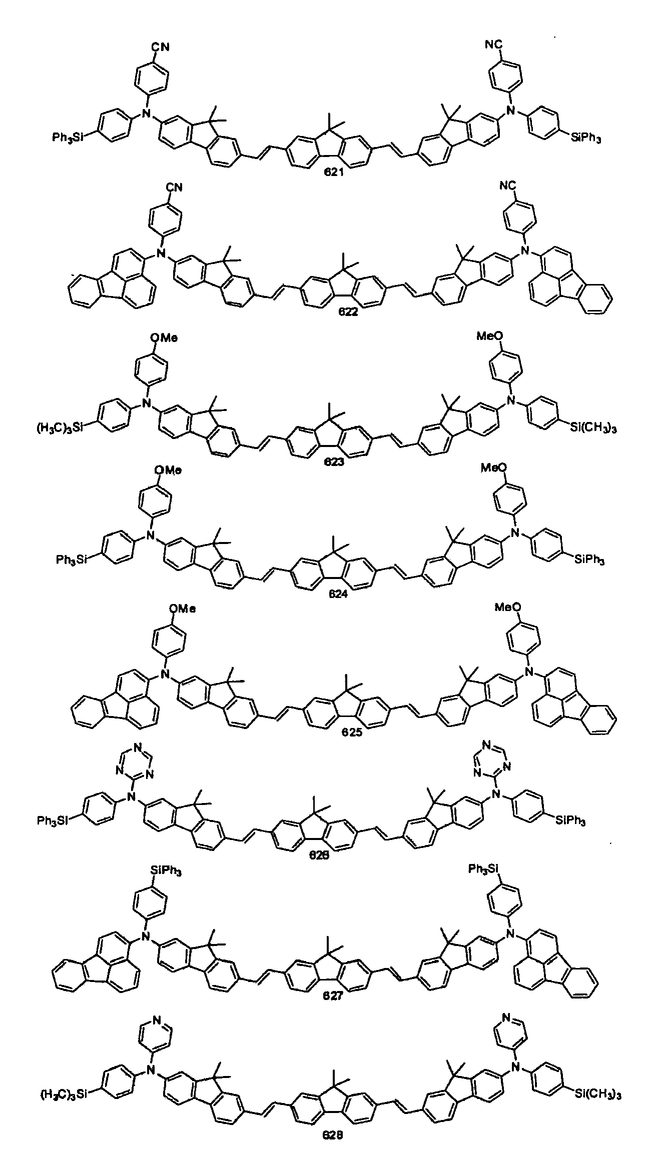

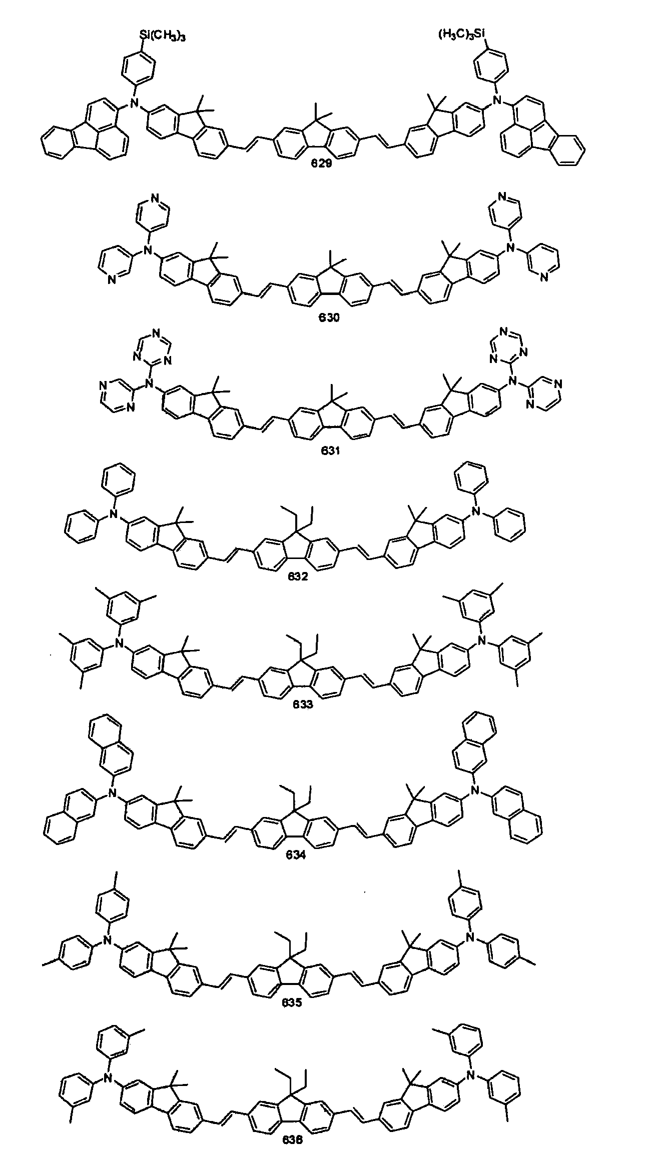

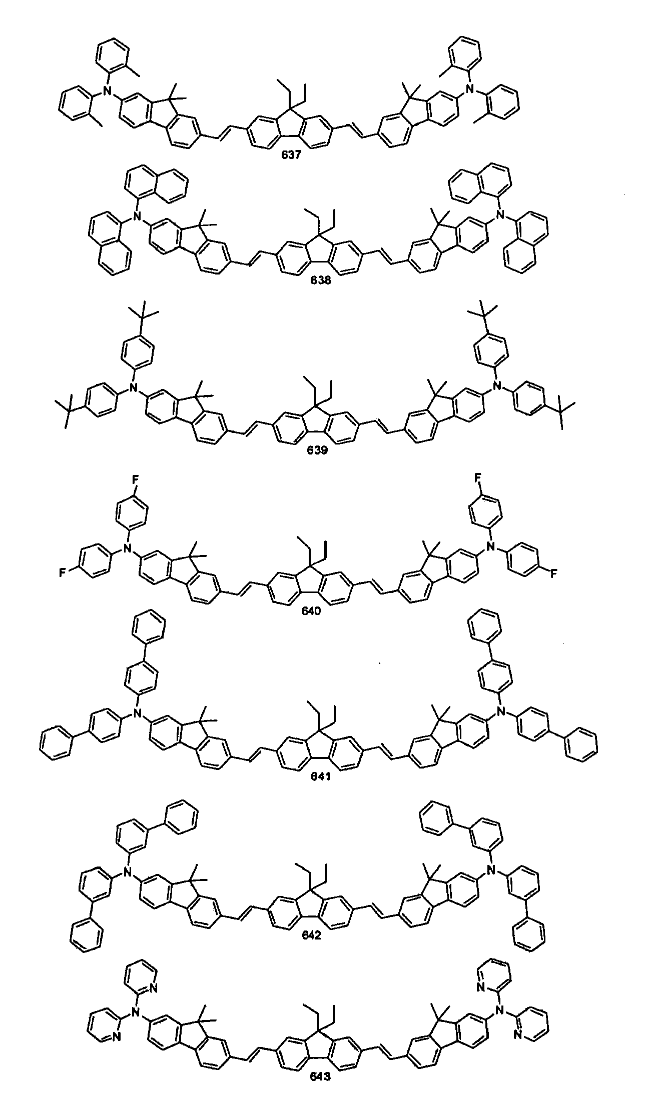

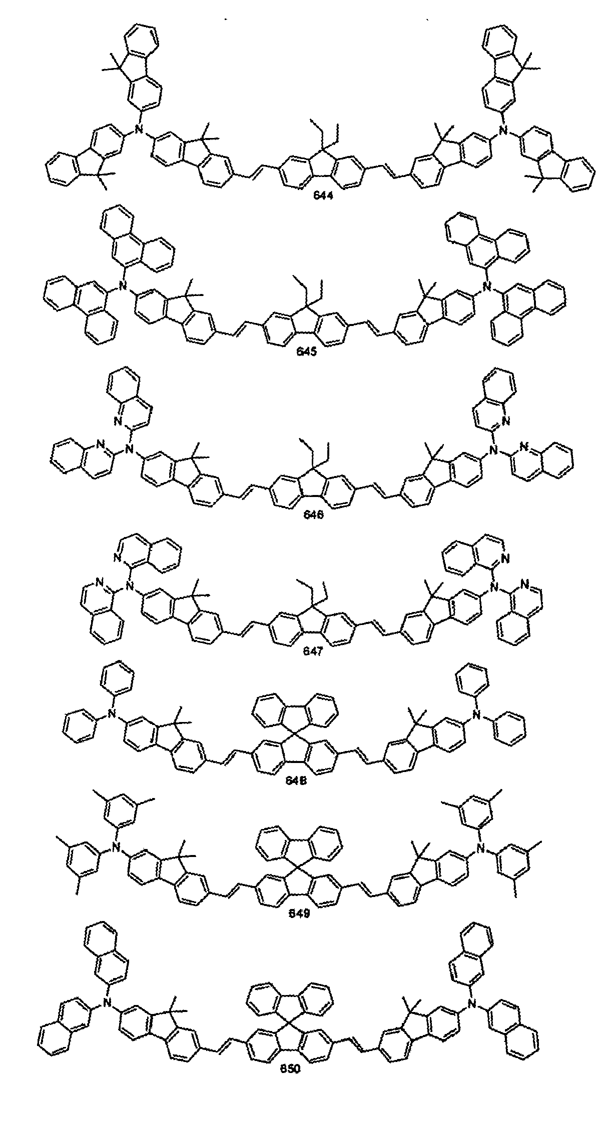

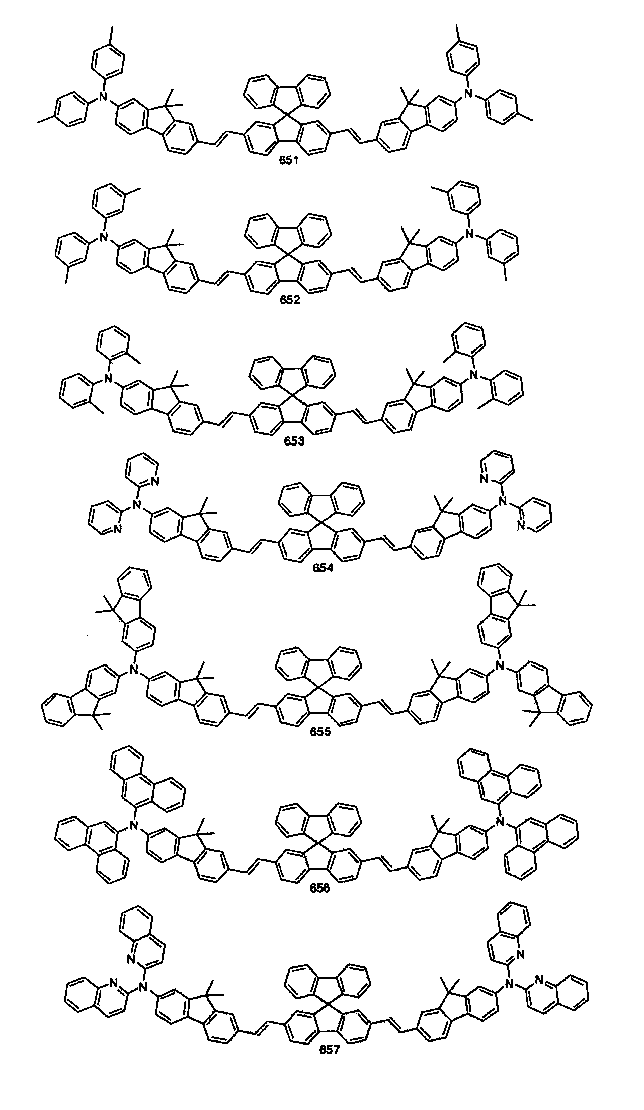

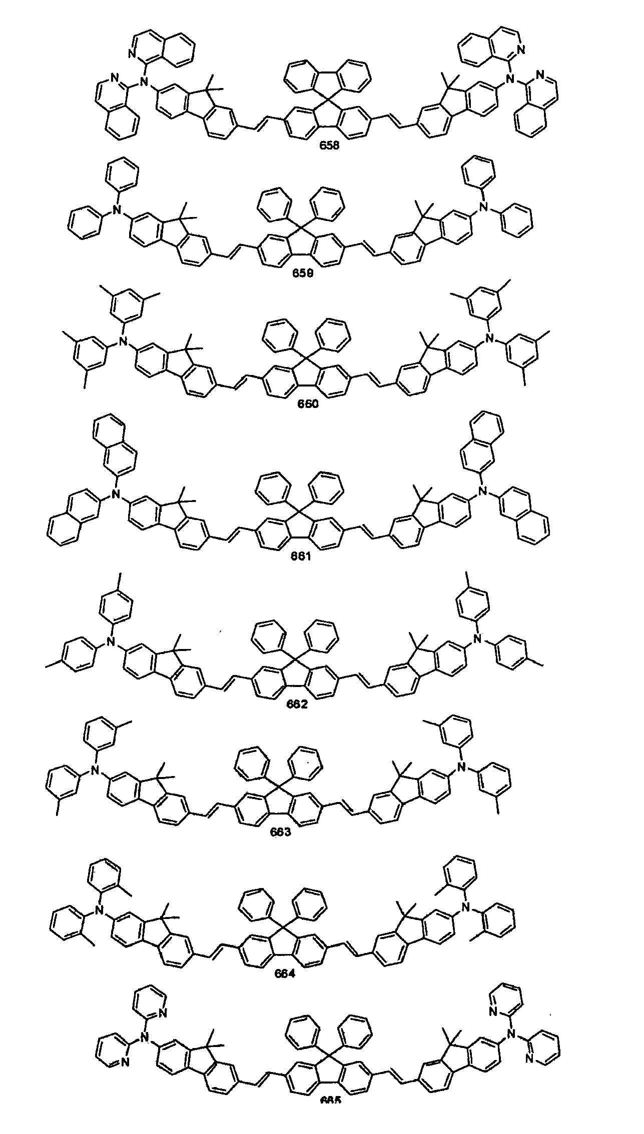

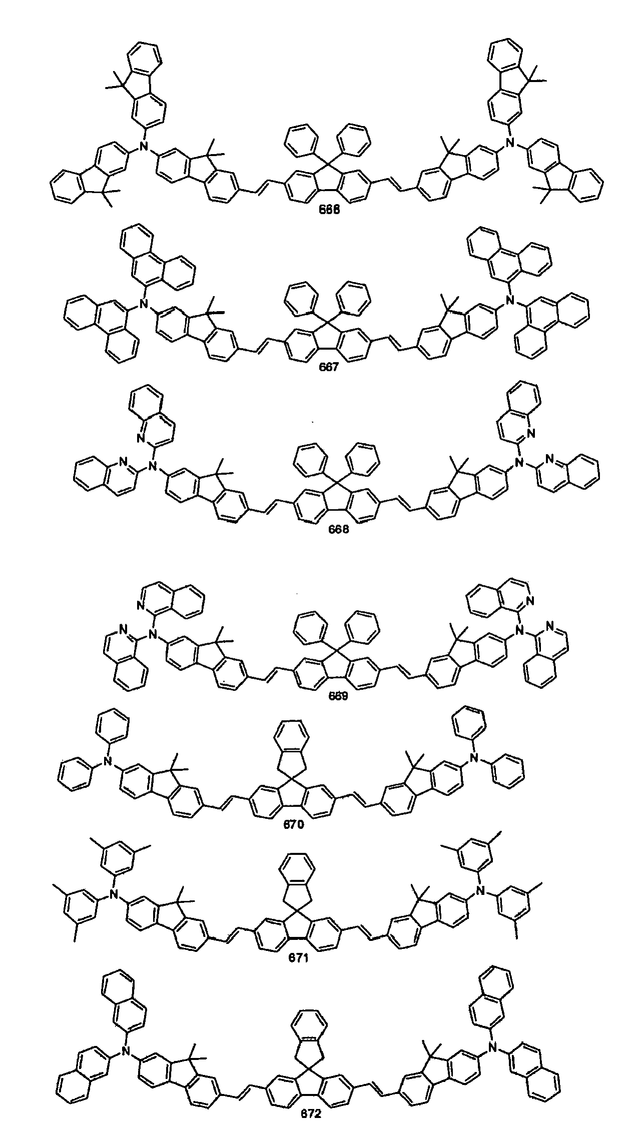

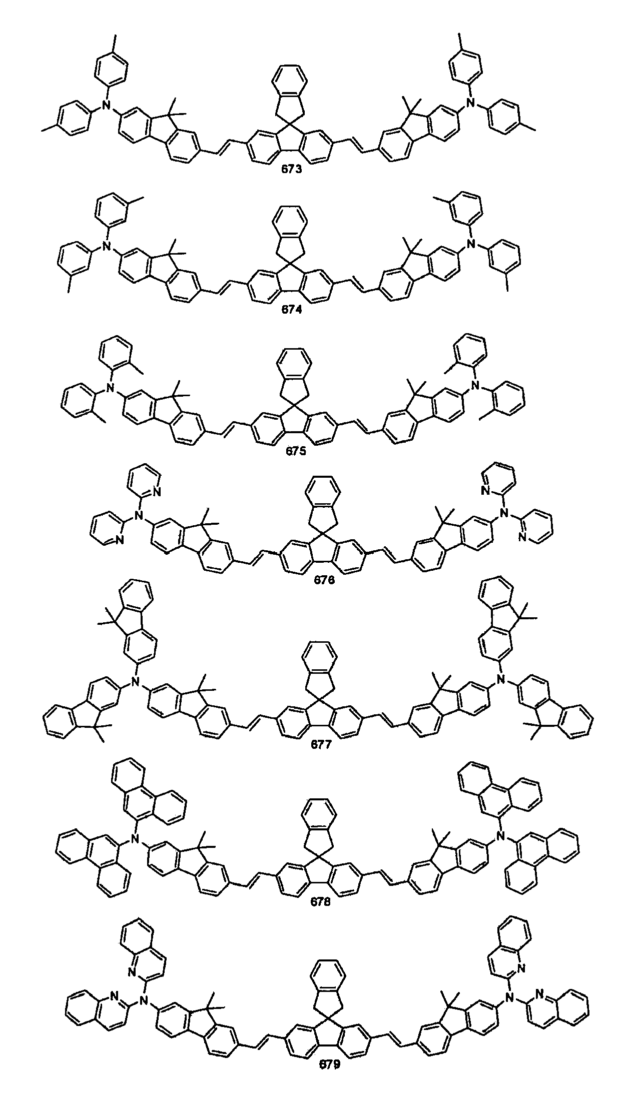

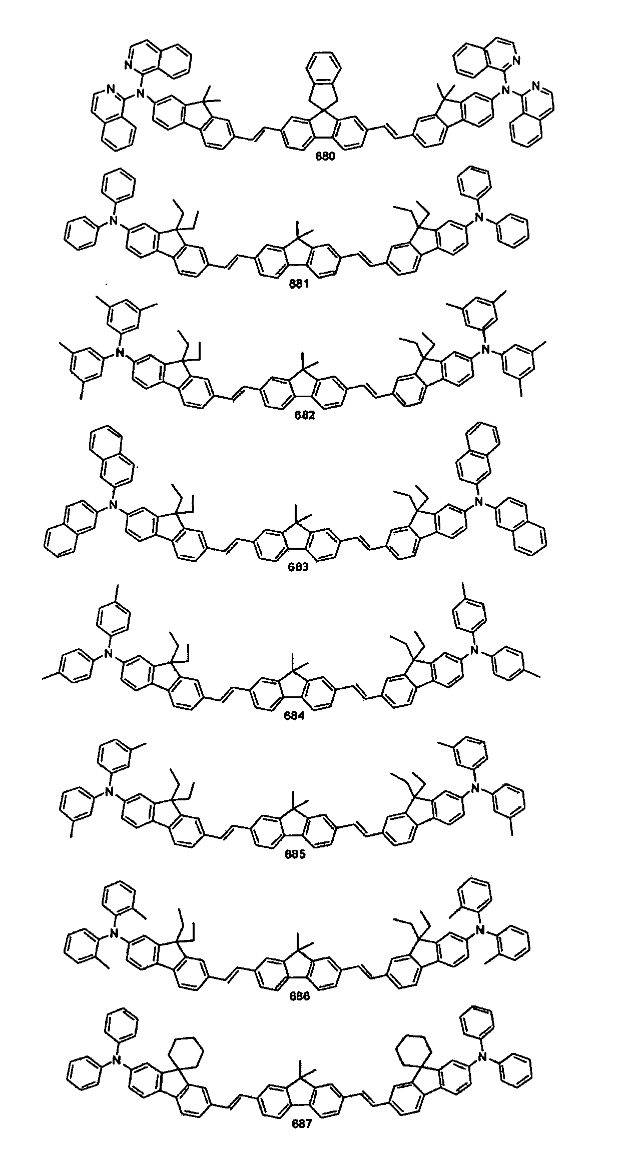

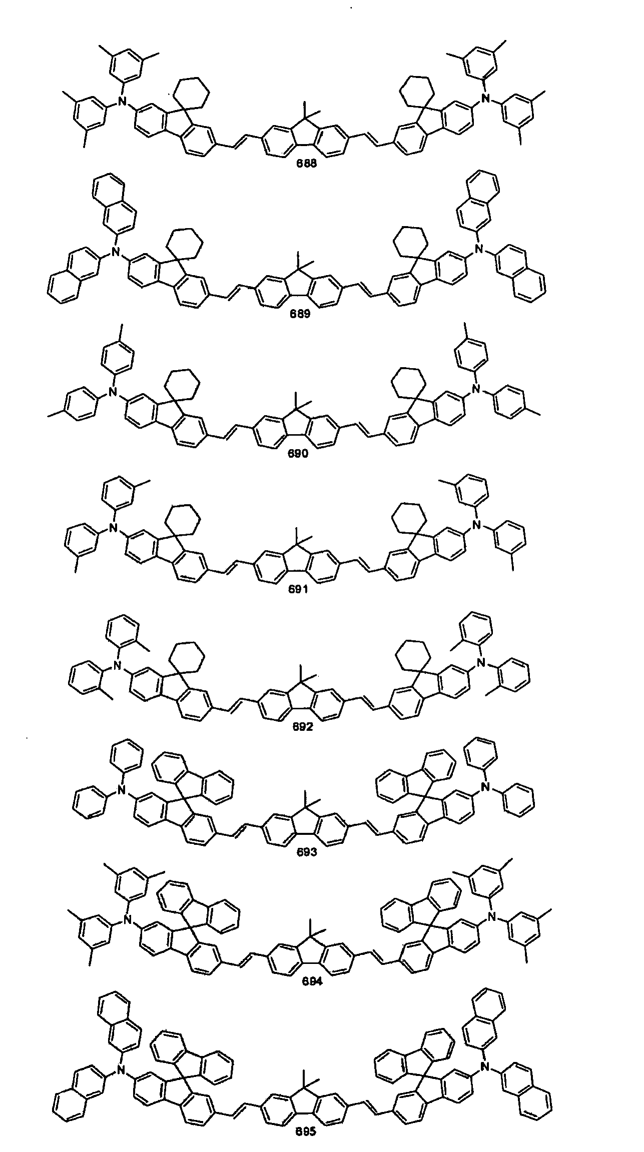

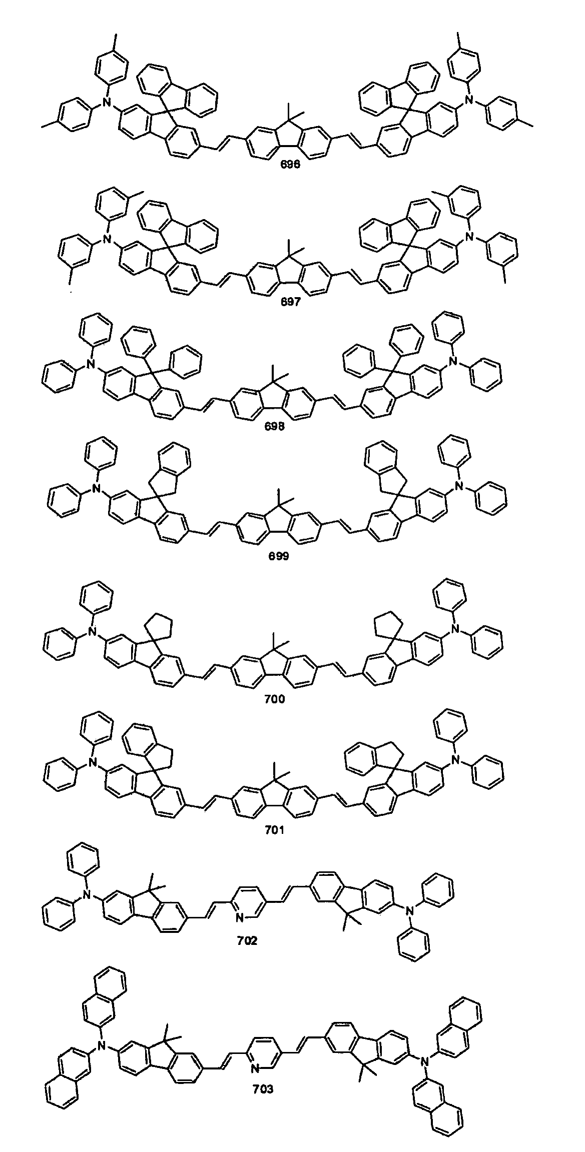

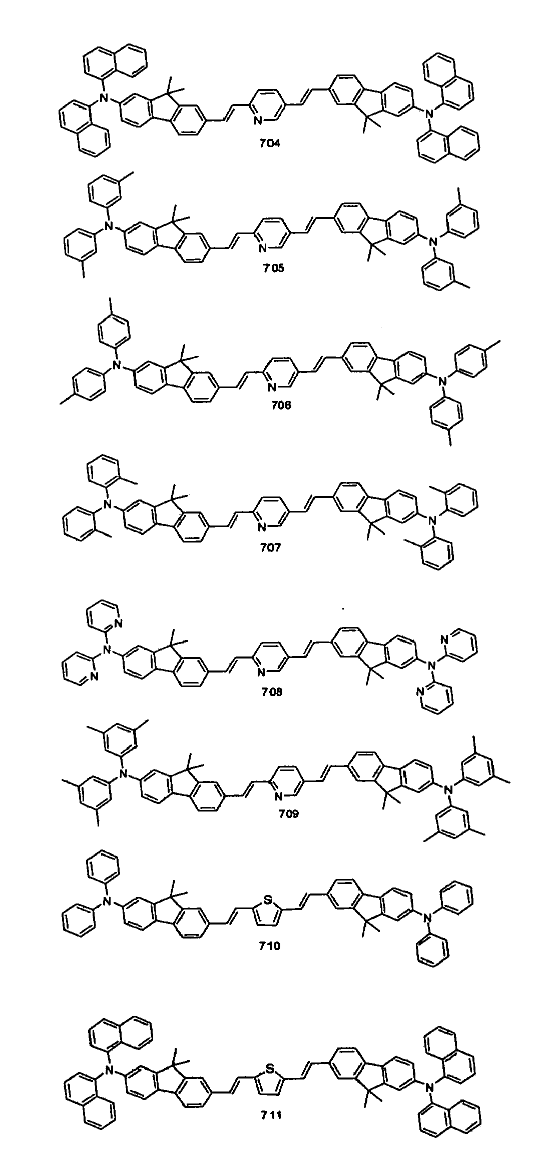

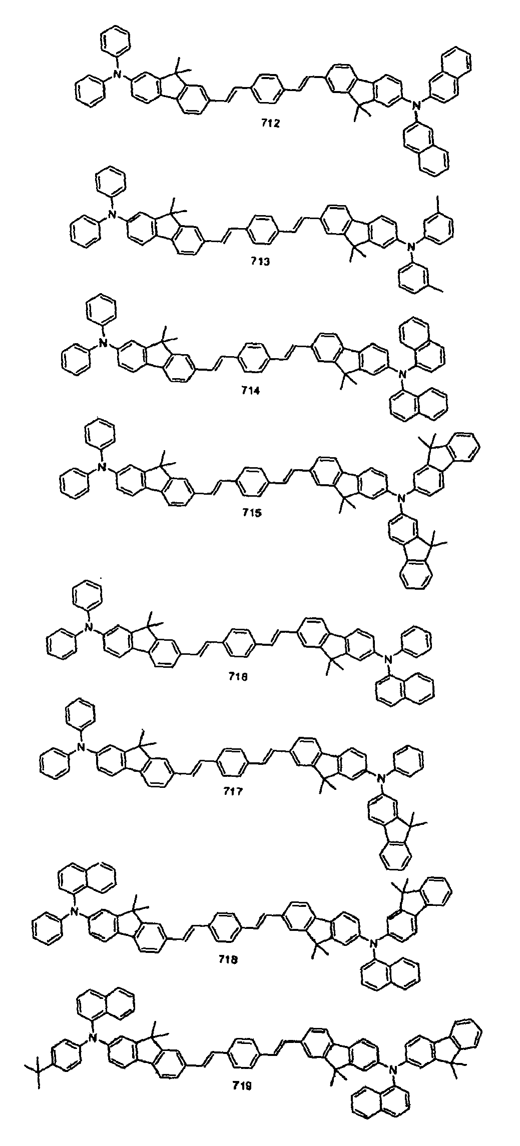

For example, the organic electroluminescent compound according to the present invention may be prepared according to Scheme 1, but not limited thereto:

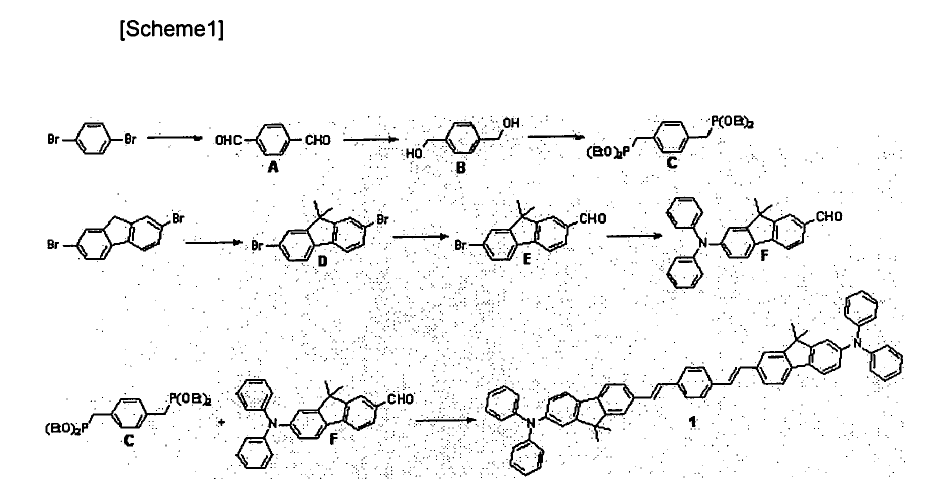

The present invention also provides an organic solar cell. The organic solar cell according to the present invention comprises one or more organic electroluminescent compound(s) represented by Chemical Formula 1.

The present invention further provides an organic electroluminescent device. The organic electroluminescent device according to the present invention comprises a first electrode; a second electrode; and one or more organic layer(s) interposed between the first electrode and the second electrode, wherein the organic layer comprises one or more organic electroluminescent compound(s) represented by Chemical Formula 1.

In the organic electroluminescent device according to the present invention, the organic layer comprises an electroluminescent layer. The electroluminescent layer comprises one or more organic electroluminescent compound(s) represented by Chemical Formula 1 as an electroluminescent dopant and one or more host(s). The host used in the organic electroluminescent device according to the present invention is not particularly limited, but may be selected from the compounds Chemical Formulas 2 and 3:

(Ar₁₁)ₐ-X-(Ar₁₂)_{b} (2)

(Ar₁₃)_{c}-Y-(Ar₁₄)_{d} (3)

wherein
X represents (C6-C60)arylene or (C4-C60)heteroarylene;
Y represents anthracenylene;
Ar₁₁ through Ar₁₄ independently represent hydrogen, deuterium, (C1-C60)alkyl, (C1-C60)alkoxy, halogen, (C4-C60)heteroaryl, (C5-C60)cycloalkyl or (C6-C60)aryl, and the cycloalkyl, aryl or heteroaryl of Ar₁₁ through Ar₁₄ may be further substituted by one or more substituent(s) selected from a group consisting of (C6-C60)aryl or (C4-C60)heteroaryl with or without one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl, (C1-C60)alkyl with or without halogen substituent(s), (C1-C60)alkooy, (C3-C60)cycloalkyl, deuterium, halogen, cyano, tri(C1-C60)alkylsilyl, di(C1-C60)alkyl(C6-C60)arylsilyl and tri(C6-C60)arylsilyl; and
a, b c and d independently represent an integer from 0 to 4.

The host represented by Chemical Formula 2 or 3 may be exemplified by the anthracene derivatives or benz[a]anthracene derivatives represented by Chemical Formulas 4 to 7: 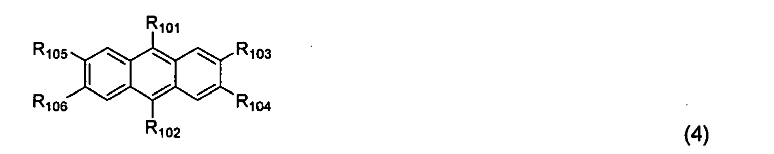 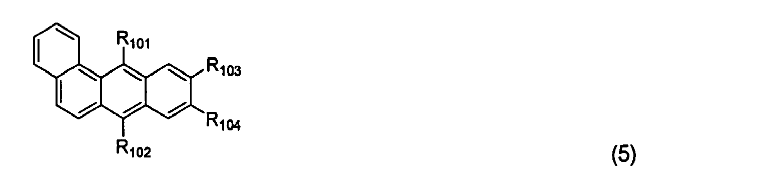 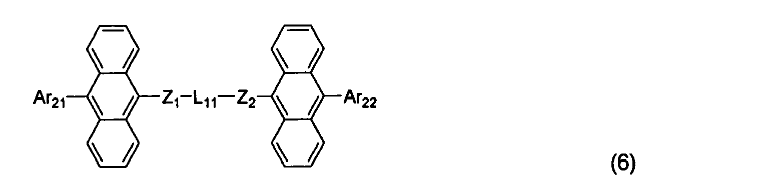 wherein
R₁₀₁ and R₁₀₂ independently represent hydrogen, deuterium, (C1-C60)alkyl, halogen, (C6-C60)aryl, (C4-C60)heteroaryl, 5- or 6-membered heterocycloalkyl containing one or more heteroatom(s) selected from N, O and S or (C3-C60)cycloalkyl, and the aryl or heteroaryl of R₁₀₁ and R₁₀₂ may be further substituted by one or more substituent(s) selected from a group consisting of deuterium, (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₁₀₃ through R₁₀₆ independently represent hydrogen, deuterium, (C1-C60)alkyl, (C1-C60)alkoxy, halogen, (C4-C60)heteroaryl, (C5-C60)cycloalkyl or (C6-C60)aryl, and the heteroaryl, cycloalkyl or aryl of R₁₀₃ through R₁₀₆ may be further substituted by one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
Z₁ and Z₂ independently represent a chemical bond or (C6-C60)arylene with or without one or more substituent(s) selected from (C1-C60)alkyl, (C1-C60)alkoxy, (C6-C60)aryl, (C4-C60)heteroaryl and halogen;
Ar₂₁ and Ar₂₂ independently represent aryl selected from the following structures or (C4-C60)heteroaryl: 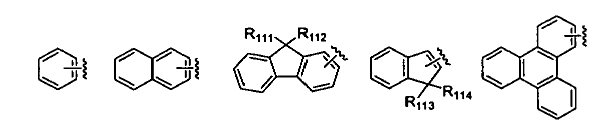 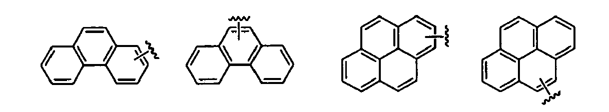 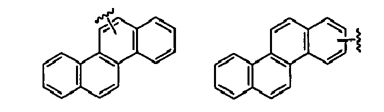
the aryl or heteroaryl of Ar₂₁ and Ar₂₂ may be substituted by one or more substituent(s) selected from (C1-C60)alkyl, (C1-C60)alkoxy, (C6-C60)aryl and (C4-C60)heteroaryl;
L₁₁ represents (C6-C60)arylene, (C4-C60)heteroarylene or a compound of the following formula: 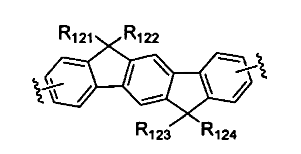
the arylene or heteroarylene of L₁₁ may be substituted by one or more substituent(s) selected from (C1-C60)alkyl, (C1-C60)alkoxy, (C6-C60)aryl, (C4-C60)heteroaryl and halogen;
R₁₁₁, R₁₁₂, R₁₁₃ and R₁₁₄ independently represent hydrogen, deuterium, (C1-C60)alkyl or (C6-C60)aryl, or each of them may be linked to an adjacent substituent via (C3-C60)alkylene or (C3-C60)alkenylene with or without a fused ring to form an alicyclic ring or a mono- or polycyclic aromatic ring;
R₁₂₁, R₁₂₂, R₁₂₃ and R₁₂₄ independently represent hydrogen, deuterium, (C1-C60)alkyl, (C1-C60)alkoxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring or a mono- or polycyclic aromatic ring; 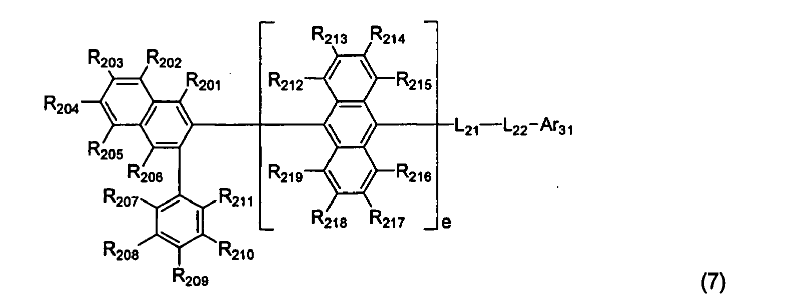
wherein
L₂₁ and L₂₂ independently represent a chemical bond, (C6-C60)arylene or (C3-C60)heteroarylene, and the arylene or heteroarylene of L₂₁ and L₂₂ may be further substituted by one or more substituent(s) selected from (C1-C60)alkyl, deuterium, halogen, cyano, (C1-C60)alkoxy, (C3-C60)cycloalkyl, (C6-C60)aryl, (C3-C60)heteroaryl, tri(C1-C30)alkylsilyl, di(C1-C30)alkyl(C6-C30)arylsilyl and tri(C6-C30)arylsilyl;
R₂₀₁ through R₂₁₉ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₀₁ through R₂₁₉ may be linked to an adjacent substituent via (C3-C60)alkylene or (C3-C60)alkenylene with or without a fused ring to form an alicyclic ring or a mono- or polycyclic aromatic ring;
Ar₃₁ represents (C6-C60)aryl, (C4-C60)heteroaryl, 5- or 6-membered heterocycloalkyl containing one or more heteroatom(s) selected from N, O and S, (C3-C60)cycloalkyl, adamantyl, (C7-C60)bicycloalkyl or a substituent selected from the following structures: 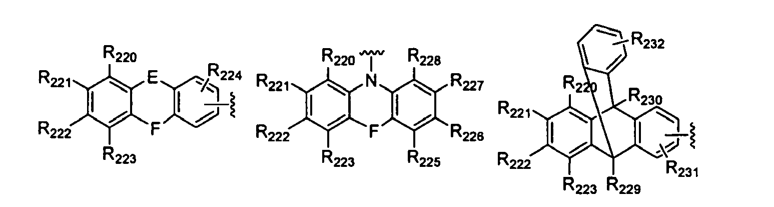
wherein
R₂₂₀ through R₂₃₂ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
E and F independently represent a chemical bond, -(CR₂₃₃R₂₃₄)ₙ-, -N(R₂₃₅)-, -S-, -O-, -Si(R₂₃₆)(R₂₃₇)-, -P(R₂₃₈)-, -C(=O)-, -B(R₂₃₉)-, -In(R₂₄₀)-, -Se-, -Ge(R₂₄₁)(R₂₄₂)-, -Sn(R₂₄₃)(R₂₄₄)-, -Ga(R₂₄₅)- or -(R₂₄₆)C=C(R₂₄₇)-;
R₂₃₃ through R₂₄₇ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₃₃ through R₂₄₇ may be linked to an adjacent substituent via (C3-C60)alkylene or (C3-C60)alkenylene with or without a fused ring to form an alicyclic ring or a mono- or polycyclic aromatic ring;
the aryl, heteroaryl, heterocycloalkyl, adamantyl or bicycloalkyl of Ar₃₁, or the alkyl, alkenyl, alkynyl, cycloalkyl, heterocycloalkyl, aryl, heteroaryl, arylsilyl, alkylsilyl, alkylamino or arylamino of R₂₀₁ through R₂₃₂may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
e represents an integer from 1 to 4; and
n represents an integer from 1 to 4.

The electroluminescent layer means a layer where electroluminescence occurs. It may be either a single layer or may comprise two or more layers. In case a dopant and a host are used together in accordance with the present invention, remarkable improvement in luminous efficiency may be attained. The doping concentration may be from 0.5 to 10% by weight. When compared with other existing host materials, the host provides excellent electron and hole conductivity, superior stability, good luminous efficiency, and significantly improved life property.

Therefore, when the compounds represented by Chemical Formulas 4 to 7 are used as an electroluminescent host, they may compensate for the electrical drawbacks of the organic electroluminescent compound represented by Chemical Formula 1.

The host compounds represented by Chemical Formulas 4 to 7 may be exemplified by the following compounds, but not limited thereto:

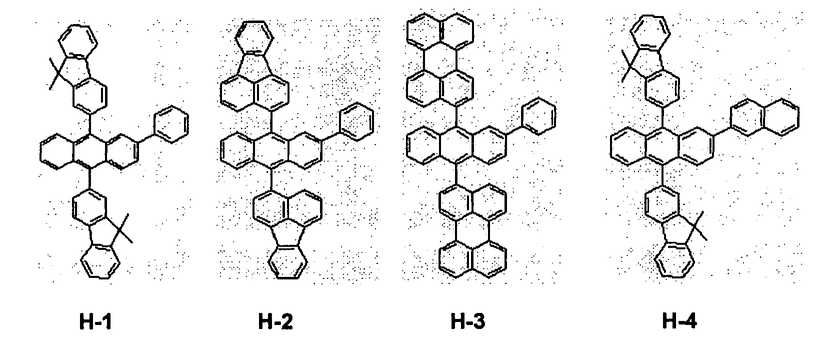

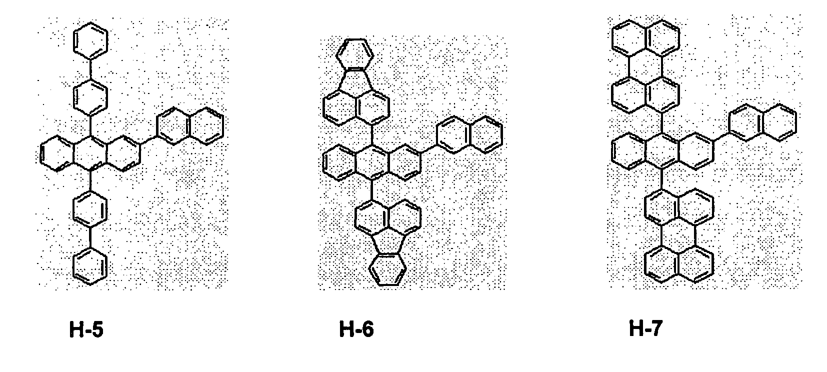

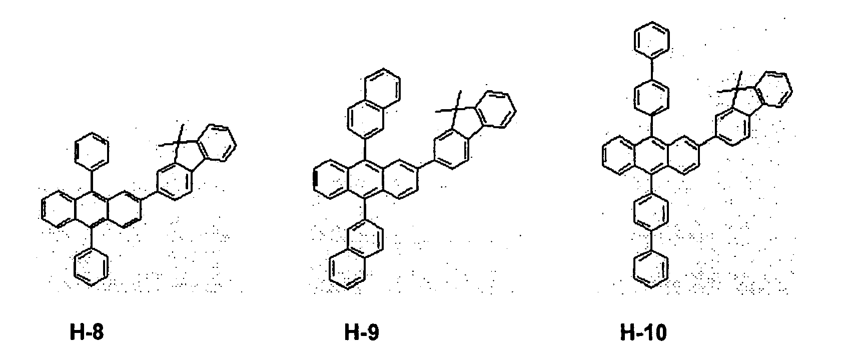

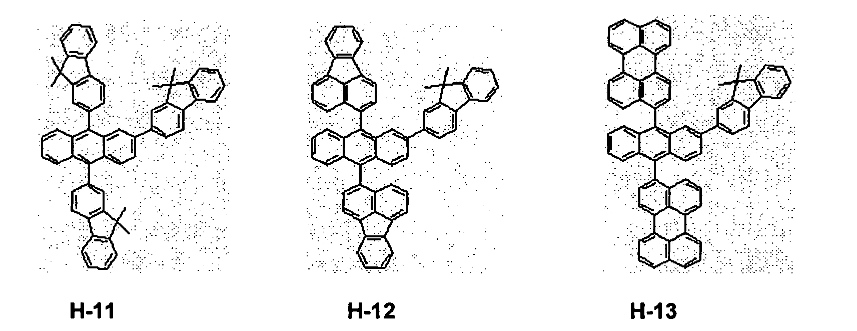

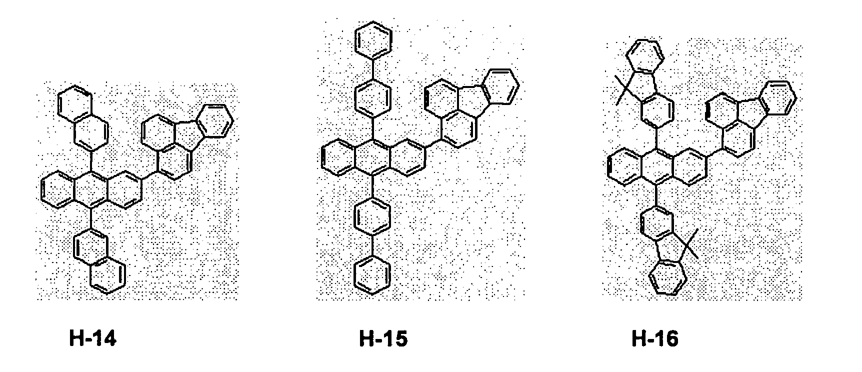

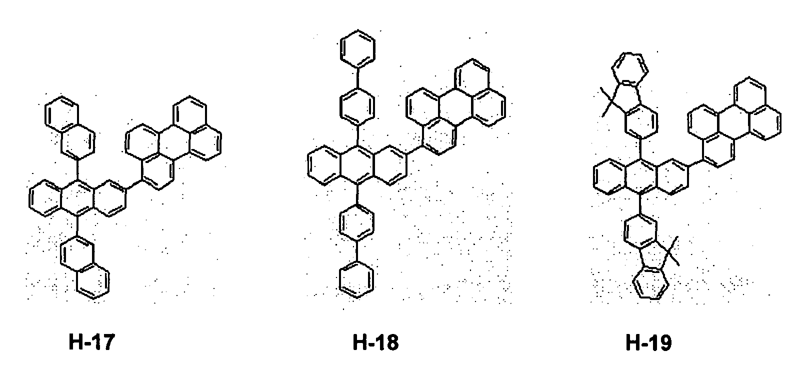

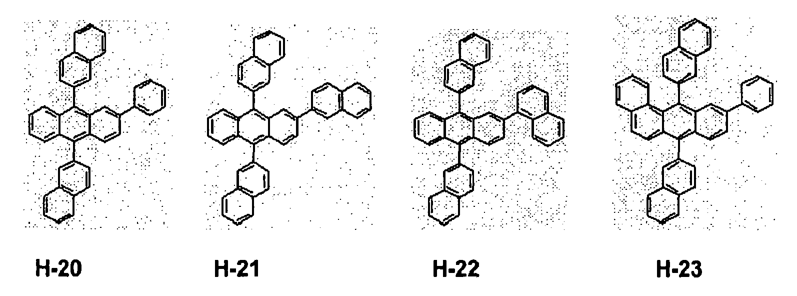

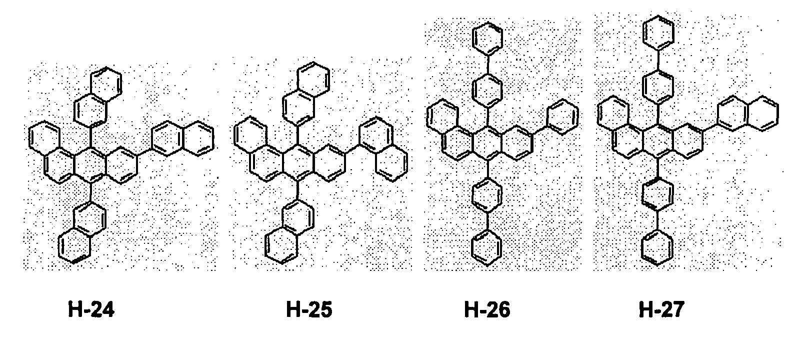

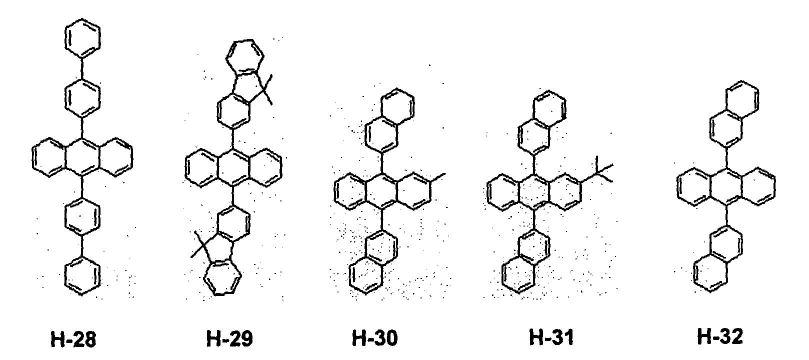

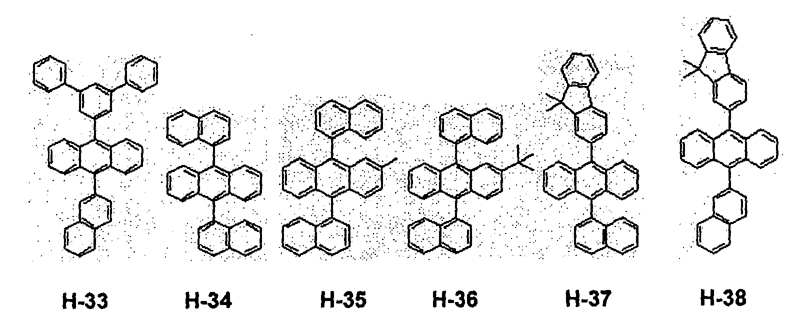

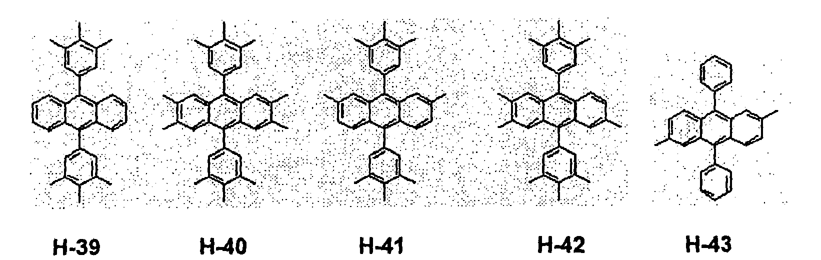

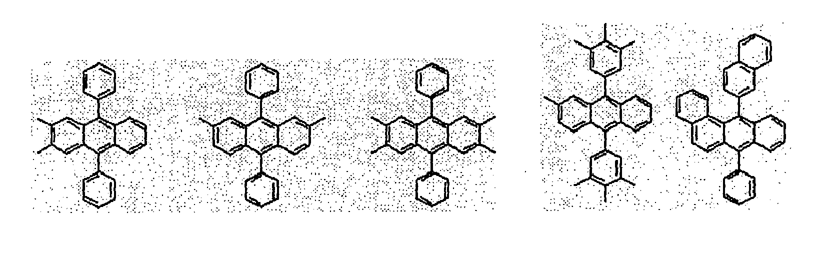

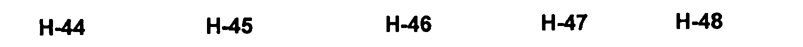

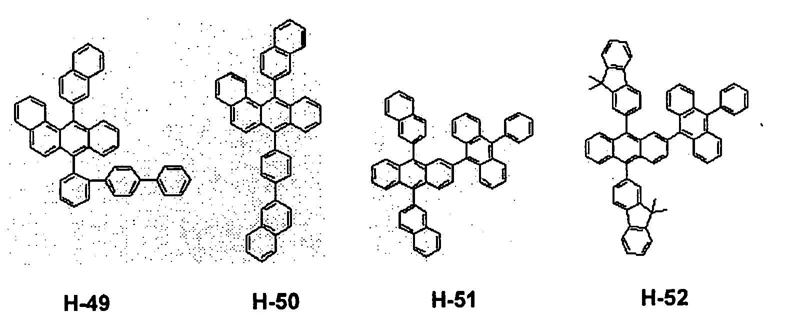

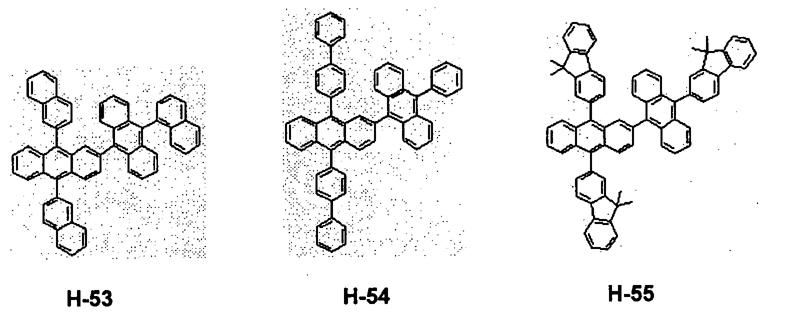

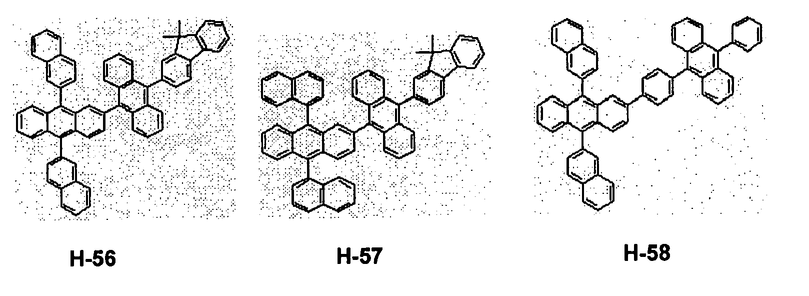

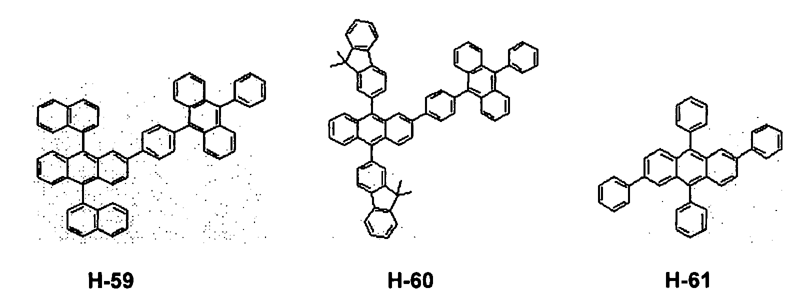

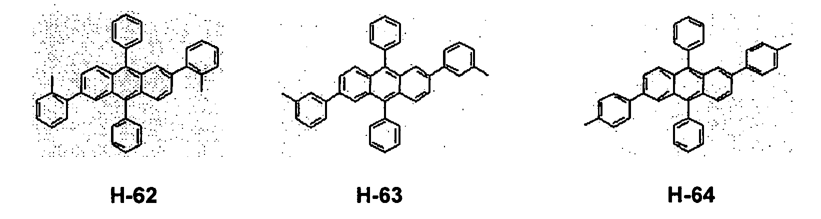

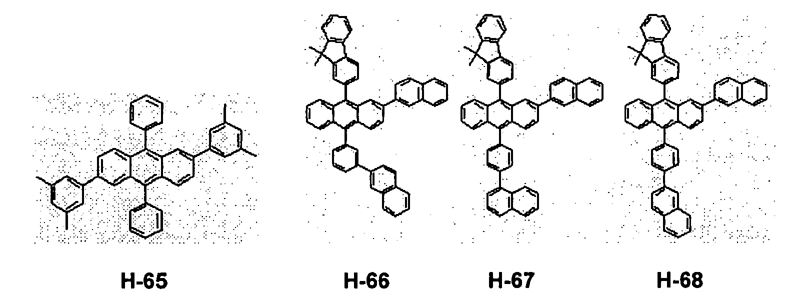

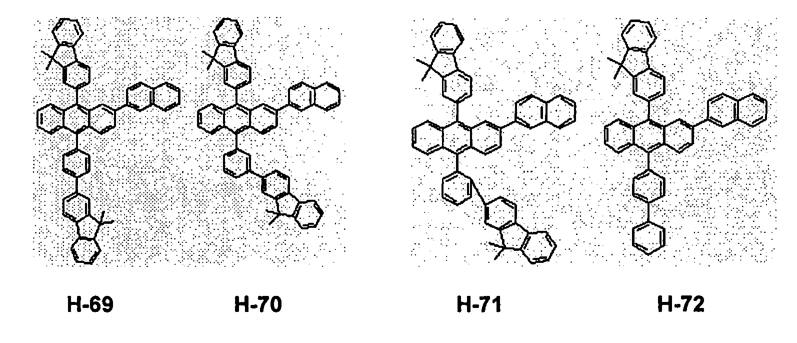

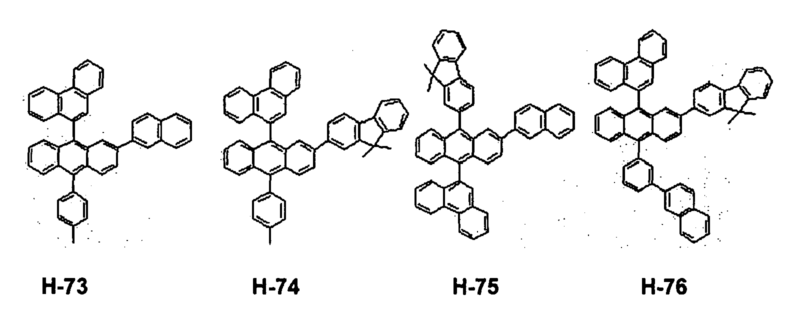

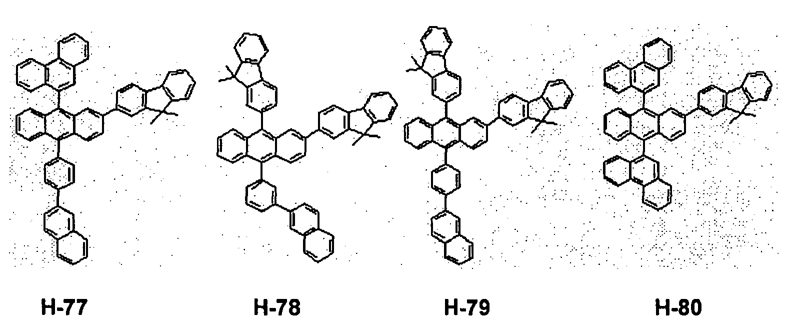

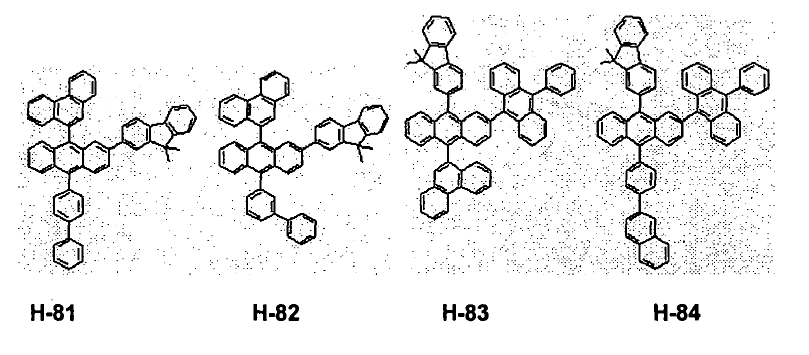

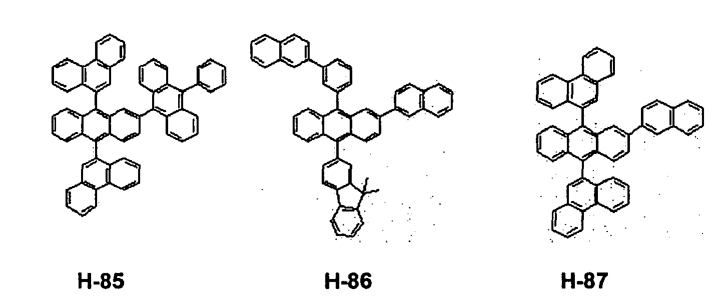

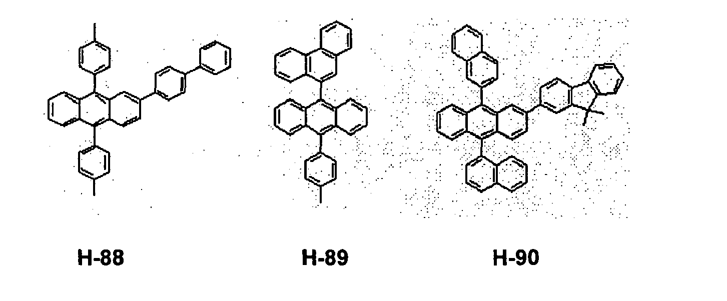

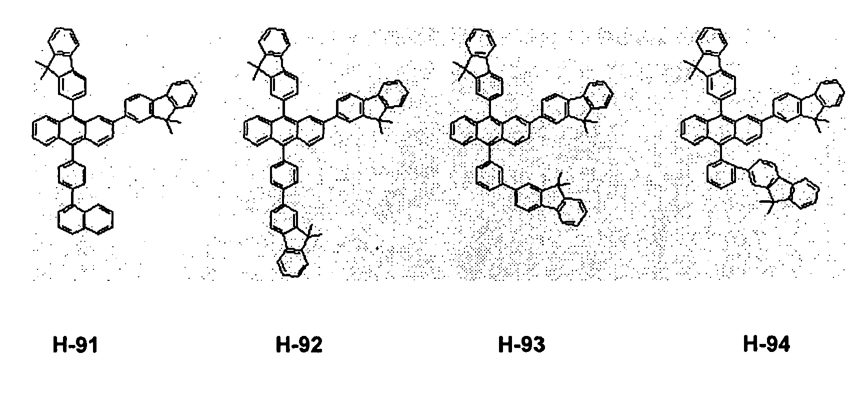

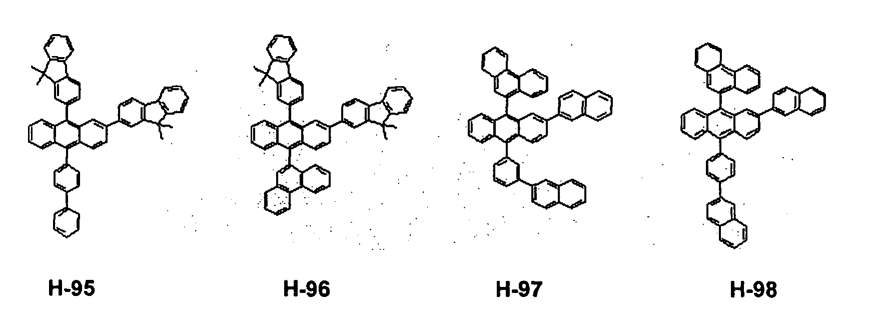

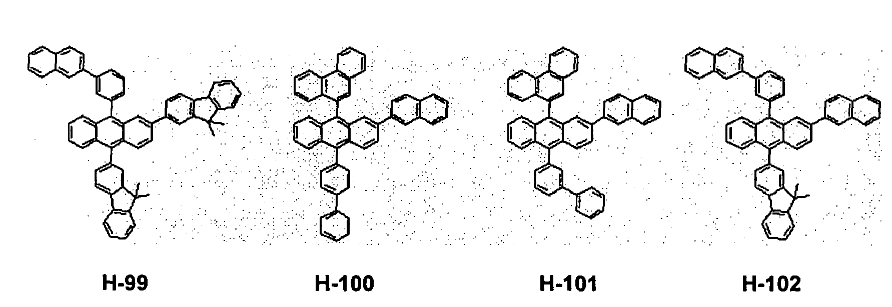

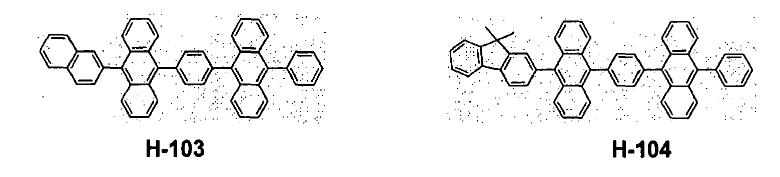

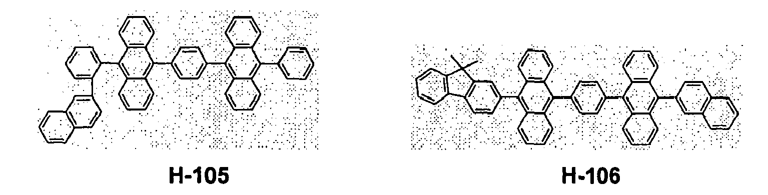

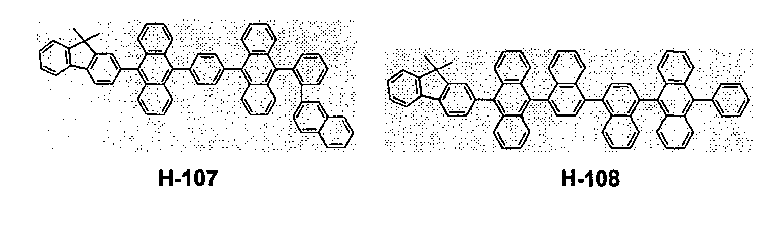

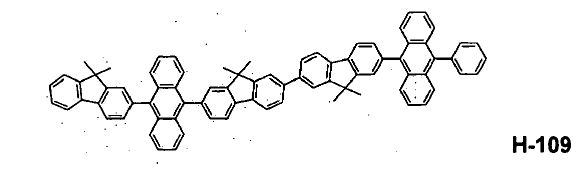

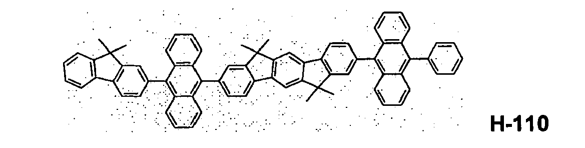

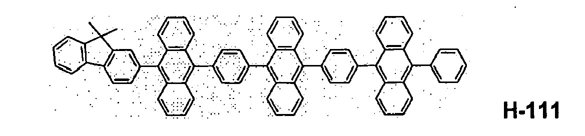

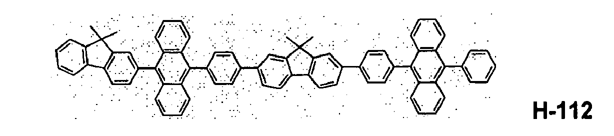

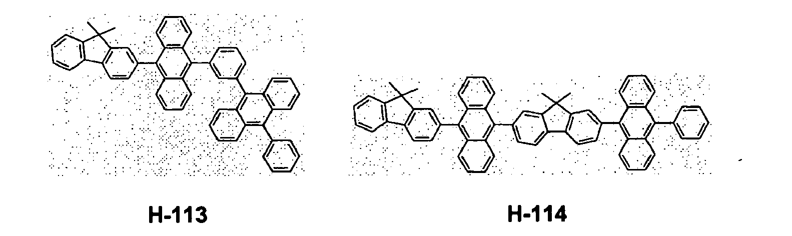

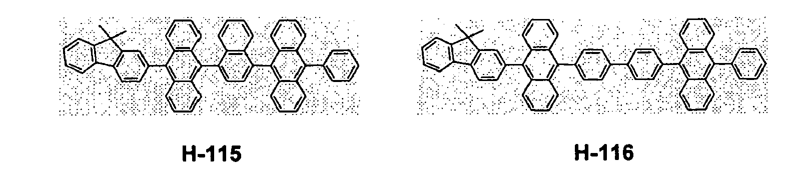

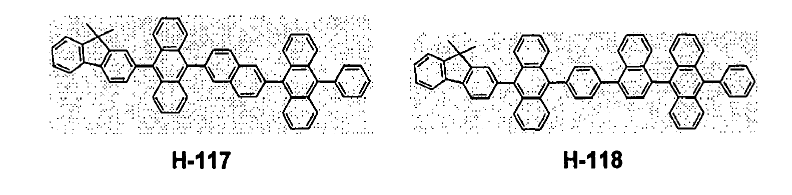

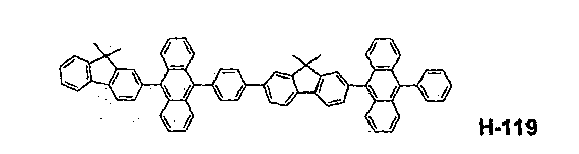

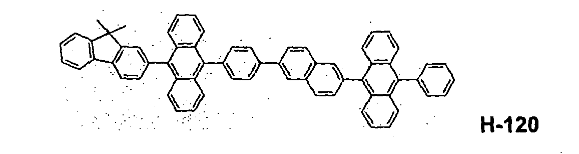

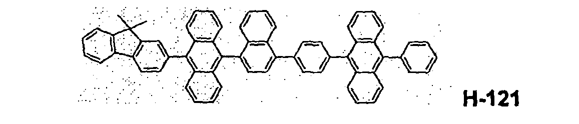

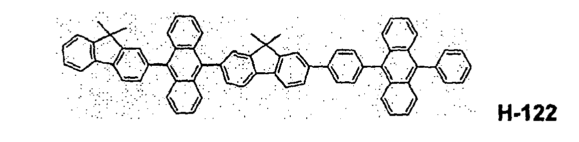

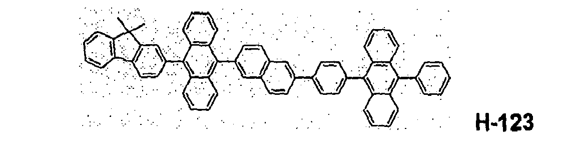

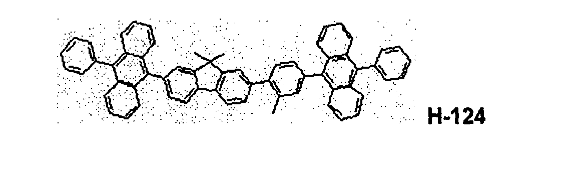

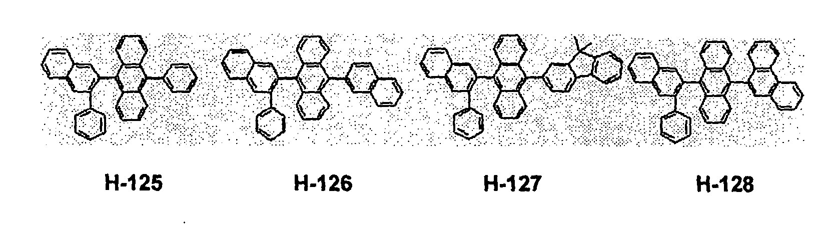

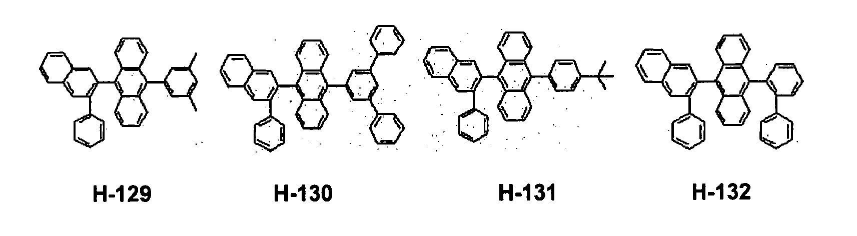

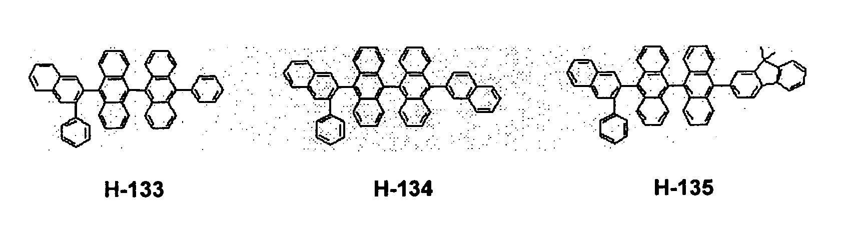

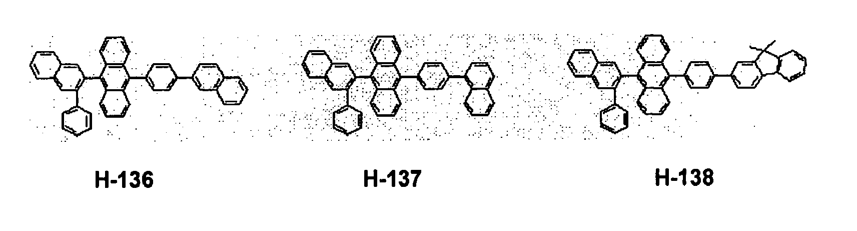

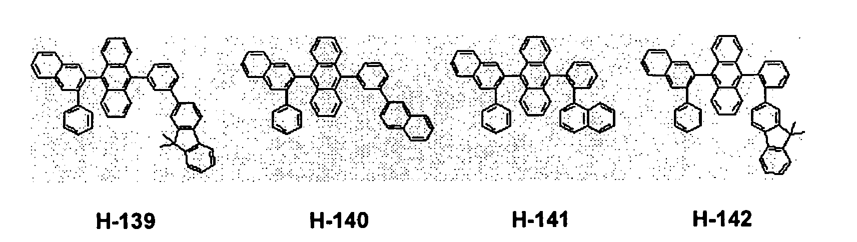

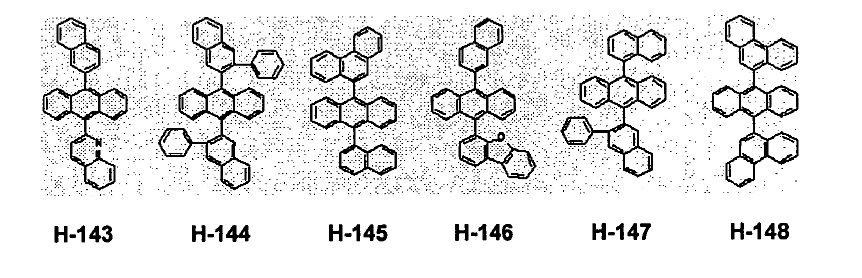

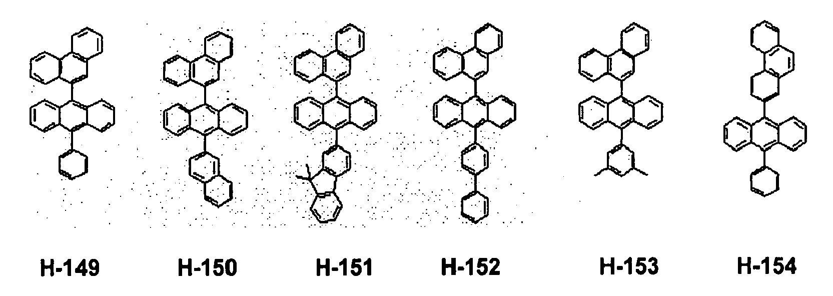

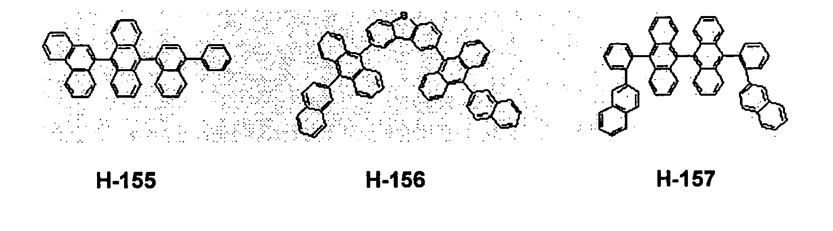

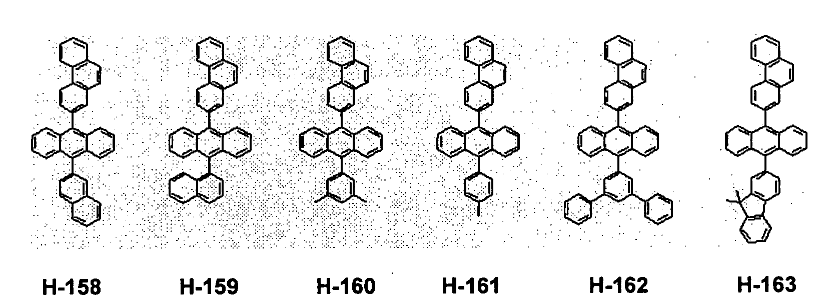

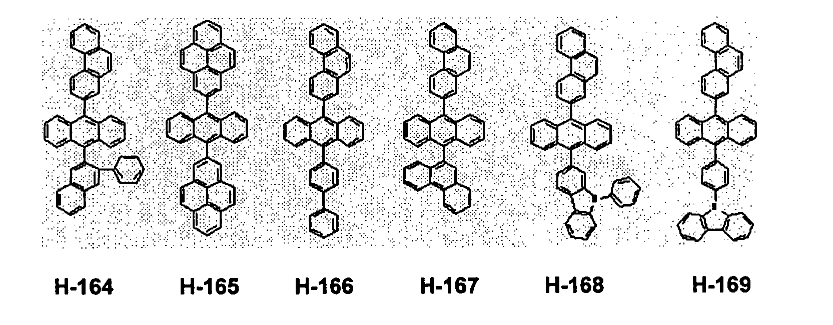

The electroluminescent device according to the present invention comprises the organic electroluminescent compound represented by Chemical Formula 1 and may further comprise one or more compound(s) selected from a group consisting of arylamine compounds and styrylarylamine compounds at the same time. For example, the arylamine compound or the styrylarylamine compound may be a compound represented by Chemical Formula 8, but not limited thereto: 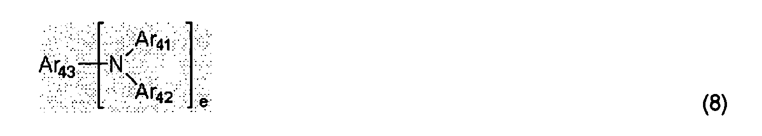 wherein
Ar₄₁ and Ar₄₂ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S or (C3-C60)cycloalkyl, and Ar₄₁ and Ar₄₂ may be linked via (C3-C60)alkylene or (C3-C60)alkenylene with or without a fused ring to form an alicyclic ring or a mono- or polycyclic aromatic ring;
Ar₄₃ represents (C6-C60)aryl, (C4-C60)heteroaryl or aryl having the following structure, when e is 1: 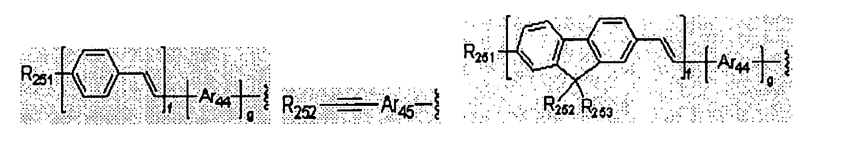
Ar₄₃ represents (C6-C60)arylene, (C4-C60)heteroarylene or arylene having the following structure, when e is 2: 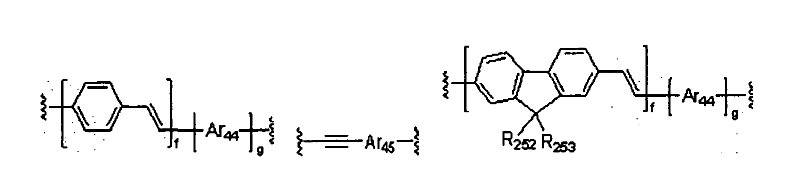
Ar₄₄ and Ar₄₅ independently represent (C6-C60)arylene or (C4-C60)heteroarylene;
R₂₅₁, R₂₅₂ and R₂₅₃ independently represent hydrogen, deuterium, (C1-C60)alkyl or (C6-C60)aryl;
f represents an integer from 1 to 4;
g represents an integer 0 or 1;
the alkyl, aryl, heteroaryl, arylamino, alkylamino, cycloalkyl or heterocycloalkyl of Ar₄₁ and Ar₄₂, the aryl, heteroaryl, arylene or heteroarylene of Ar₄₃, the arylene or heteroarylene of Ar₄₄ and Ar₄₅, or the alkyl or aryl of R₂₅₁ through R₂₅₃ may be further substituted by one or more substituent(s) selected from a group consisting of deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

More specifically, the arylamine compound or the styrylarylamine compound may be exemplified by the following compounds, but not limited thereto:

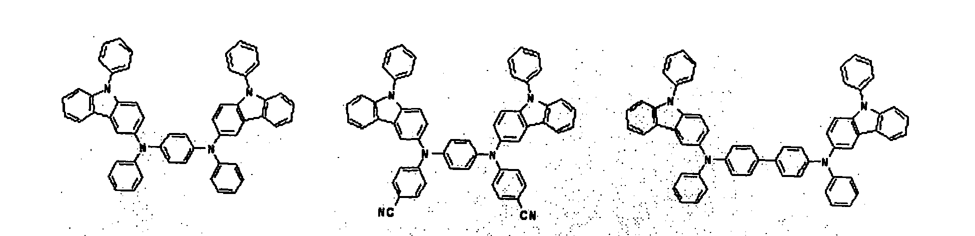

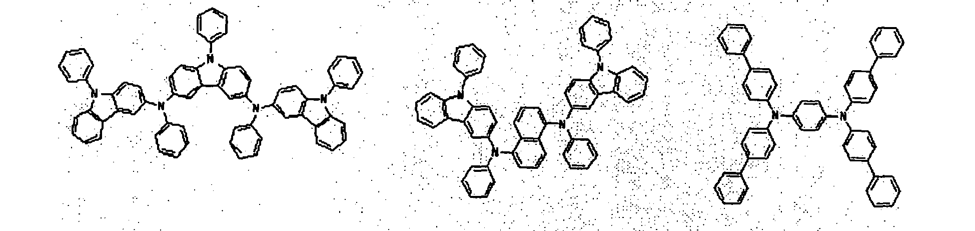

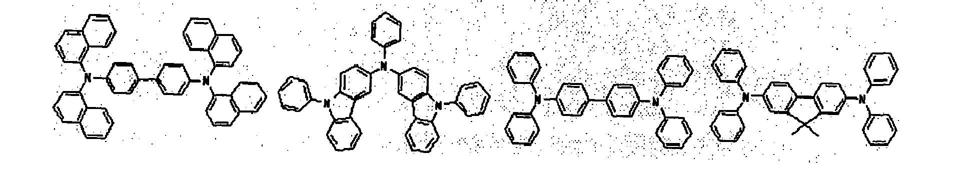

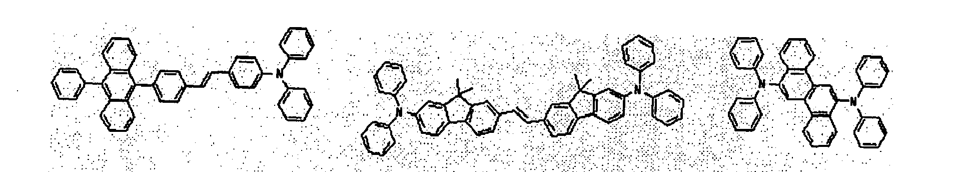

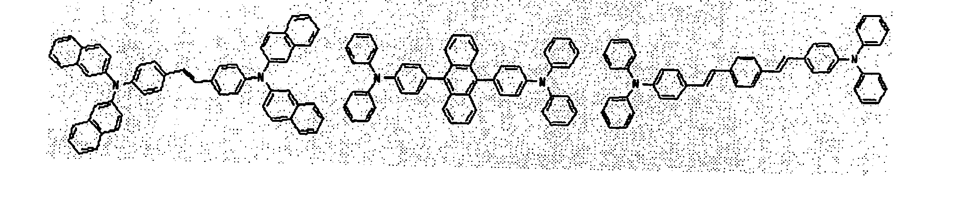

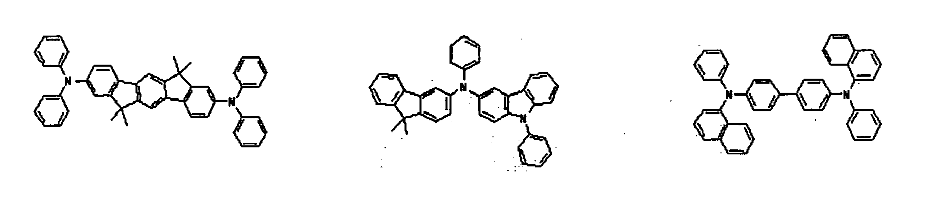

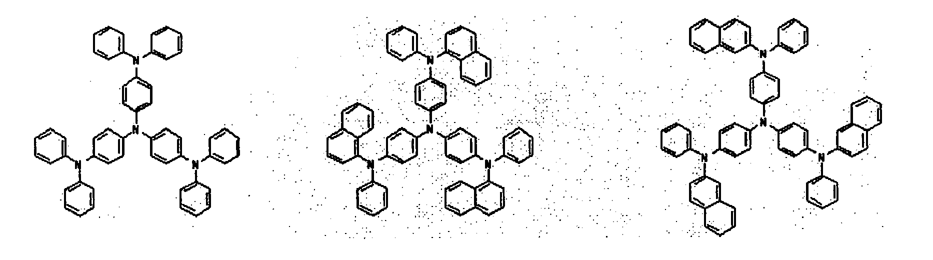

In the electroluminescent device according to the present invention, the organic layer may further comprise, in addition to the electroluminescent compound represented by Chemical Formula 1, one or more metal(s) selected from a group consisting of organic metals of Group 1, Group 2, 4th period and 5th period transition metals, lanthanide metals and d-transition elements. Also, the organic layer may comprise an electroluminescent layer and a charge generating layer at the same time.

An independent electroluminescence type electroluminescent device having a pixel structure in which the electroluminescent device according to the present invention comprising the electroluminescent compound represented by Chemical Formula 1 is used as a subpixel and one or more subpixel(s) comprising one or more metal compound(s) selected from a group consisting of Ir, Pt, Pd, Rh, Re, Os, Tl, Pb, Bi, In, Sn, Sb, Te, Au and Ag is patterned in parallel may be manufactured.

The organic layer may comprise a compound having an electroluminescence peak with wavelength of 500 to 560 nm and a compound having an electroluminescence peak with wavelength of not less than 560 nm at the same time. The compound having an electroluminescence peak with wavelength of 500 to 560 nm or the compound having an electroluminescence peak with wavelength of not less than 560 nm may be exemplified by the compounds represented by Chemical Formulas 9 to 15, but not limited thereto:

M¹L¹⁰¹L¹⁰²L¹⁰³ (9)

wherein
M¹ is a metal selected from a group consisting of Group 7, Group 8, Group 9, Group 10, Group 11, Group 13, Group 14, Group 15 and Group 16 metals;
ligands L¹⁰¹, L¹⁰² and L¹⁰³ are independently selected from the following structures: 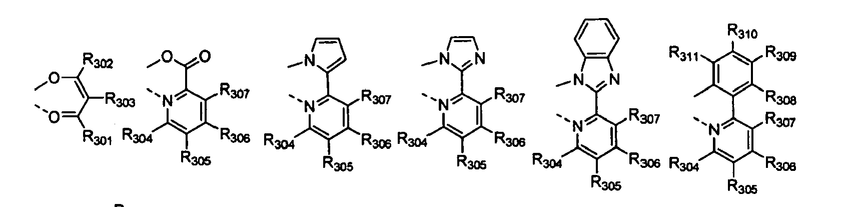 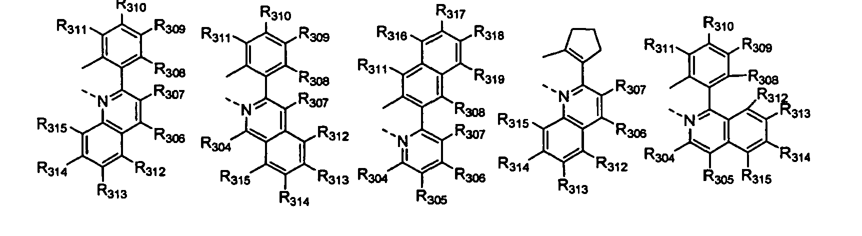 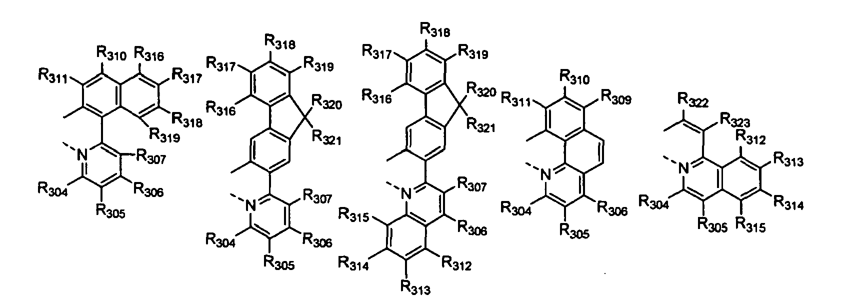 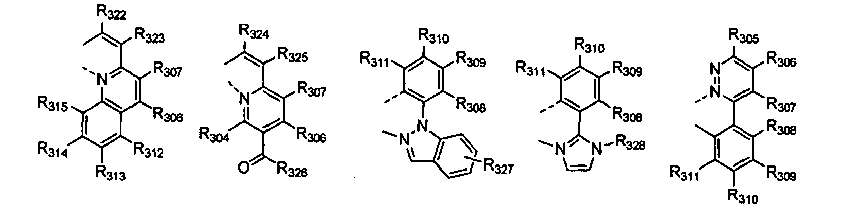 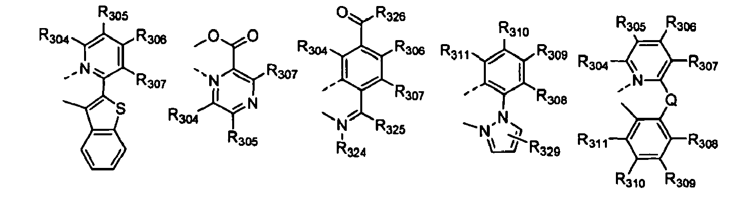
wherein
R₃₀₁ through R₃₀₃ independently represent hydrogen, deuterium, (C1-C60)alkyl with or without halogen substituent(s), (C6-C60)aryl with or without (C1-C60)alkyl substituent(s) or halogen;
R₃₀₄ through R₃₁₉ independently represent hydrogen, deuterium, (C1-C60)alkyl, (C1-C30)alkoxy, (C3-C60)cycloalkyl, (C2-C30)alkenyl, (C6-C60)aryl, mono- or di(C1-C30)alkylamino, mono- or di(C6-C30)arylamino, SF₅, tri(C1-C30)alkylsilyl, di(C1-C30)alkyl(C6-C30)arylsilyl, tri(C6-C30)arylsilyl, cyano or halogen, and the alkyl, cycloalkyl, alkenyl or aryl of R₃₀₄ through R₃₁₉ may be further substituted by one or more substituent(s) selected from deuterium, (C1-C60)alkyl, (C6-C60)aryl and halogen;
R₃₂₀ through R₃₂₃ independently represent hydrogen, deuterium, (C1-C60)alkyl with or without halogen substituent(s) or (C6-C60)aryl with or without (C1-C60)alkyl substituent(s);
R₃₂₄ and R₃₂₅ independently represent hydrogen, deuterium, (C1-C60)alkyl, (C6-C60)aryl or halogen, or R₃₂₄ and R₃₂₅ may be linked via (C3-C12)alkylene or (C3-C12)alkenylene with or without a fused ring to form an alicyclic ring or a mono- or polycyclic aromatic ring, and the alkyl or aryl of R₃₂₄ and R₃₂₅, or the alicyclic ring or the mono- or polycyclic aromatic ring formed as they are linked via (C3-C12)alkylene or (C3-C12)alkenylene with or without a fused ring may be further substituted by one or more substituent(s) selected from deuterium, (C1-C60)alkyl with or without halogen substituent(s), (C1-C30)alkoxy, halogen, tri(C1-C30)alkylsilyl, tri(C6-C30)arylsilyl and (C6-C60)aryl;
R₃₂₆ represents (C1-C60)alkyl, (C6-C60)aryl, (C5-C60)heteroaryl or halogen;
R₃₂₇ through R₃₂₉ independently represent hydrogen, deuterium, (C1-C60)alkyl, (C6-C60)aryl or halogen, and the alkyl or aryl of R₃₂₆ through R₃₂₉ may be further substituted by halogen or (C1-C60)alkyl;
Q represents 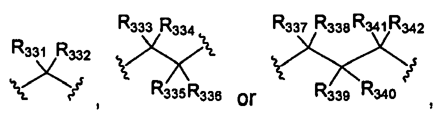 wherein R₃₃₁ through R₃₄₂ independently represent hydrogen, deuterium, (C1-C60)alkyl with or without halogen substituent(s), (C1-C30)alkoxy, halogen, (C6-C60)aryl, cyano or (C5-C60)cycloalkyl, or each of R₃₃₁ through R₃₄₂ may be linked to an adjacent substituent via alkylene or alkenylene to form a (C5-C7)spiro ring or a (C5-C9)fused ring, or may be linked to R₃₀₇ or R₃₀₈ via alkylene or alkenylene to form a (C5-C7)fused ring; 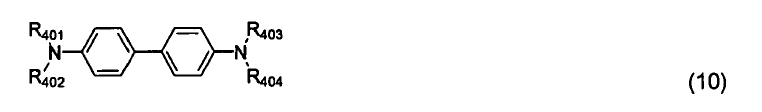
wherein
R₄₀₁ through R₄₀₄ independently represent (C1-C60)alkyl or (C6-C60)aryl, or each of them may be linked to an adjacent substituent via (C3-C60)alkylene or (C3-C60)alkenylene with or without a fused ring to form an alicyclic ring or a mono- or polycyclic aromatic ring;
the alkyl or aryl of R₄₀₁ through R₄₀₄, or the alicyclic ring or the mono- or polycyclic aromatic ring formed as they are linked via (C3-C60)alkylene or (C3-C60)alkenylene with or without a fused ring may be further substituted by one or more substituent(s) selected from deuterium, (C1-C60)alkyl with or without halogen substituent(s), (C1-C60)alkoxy, halogen, tri(C1-C60)alkylsilyl, tri(C6-C60)arylsilyl and (C6-C60)aryl;

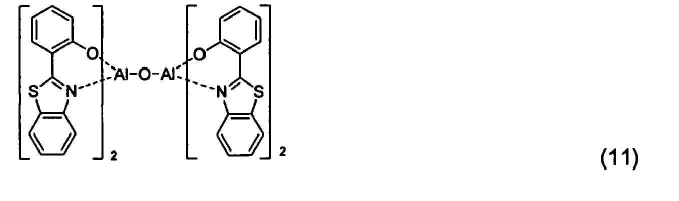

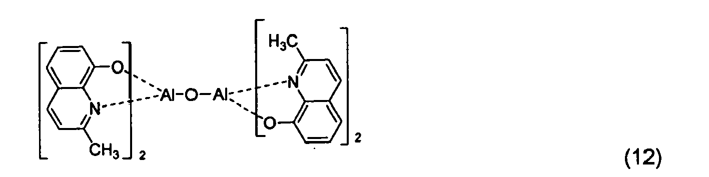

   L²⁰¹L²⁰²M2(T)ₕ (13)

   wherein
   ligands L²⁰¹ and L²⁰² are independently selected from the following structures: 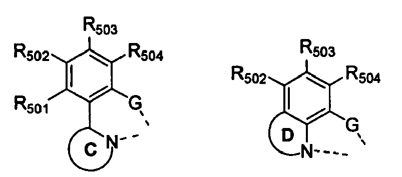
   M² represents a divalent or trivalent metal;
   h is 0 when M² is a divalent metal, and h is 1 when M² is a trivalent metal;
   T represents (C6-C60)aryloxy or tri(C6-C60)arylsilyl, and the aryloxy or triarylsilyl of T may be further substituted by (C1-C60)alkyl or (C6-C60)aryl;
   G represents O, S or Se;
   ring C represents oxazole, thiazole, imidazole, oxadiazole, thiadiazole, benzoxazole, benzothiazole, benzimidazole, pyridine or quinoline;
   ring D represents pyridine or quinoline, and the ring D may be further substituted by (C1-C60)alkyl, phenyl with or without (C1-C60)alkyl substituent(s) or naphthyl;
   R₅₀₁ through R₅₀₄ independently represent hydrogen, deuterium, (C1-C60)alkyl, halogen, tri(C1-C60)alkylsilyl, tri(C6-C60)arylsilyl or (C6-C60)aryl, or each of them may be linked to an adjacent substituent via (C3-C60)alkylene or (C3-C60)alkenylene to form a fused ring, and the pyridine or quinoline may be linked to R₅₀₁ via a chemical bond to form a fused ring;
   the aryl of ring C and R₄₀₁ through R₄₀₄ may be further substituted by deuterium, (C1-C60)alkyl, halogen, (C1-C60)alkyl with halogen substituent(s), phenyl, naphthyl, tri(C1-C60)alkylsilyl, tri(C6-C60)arylsilyl or amino; 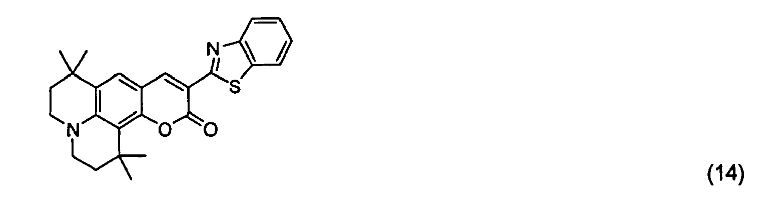 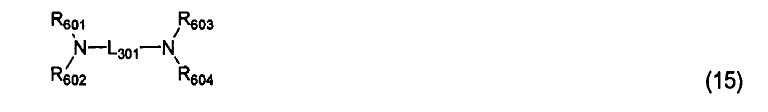
   wherein
   L₃₀₁ represents (C6-C60)arylene with or without one or more substituent(s) selected from a group consisting of deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl, and the alkyl, cycloalkyl, heterocycloalkyl, aryl, heteroaryl, arylsilyl, alkylsilyl, alkylamino or arylamino substituent substituted at the arylene may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
   R₅₀₁ through R₆₀₄ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S or (C3-C60)cycloalkyl, or each of R₆₀₁ through R₆₀₄ may be linked to an adjacent substituent via (C3-C60)alkylene or (C3-C60)alkenylene with or without a fused ring to form an alicyclic ring or a mono- or polycyclic aromatic ring; and
   the alkyl, aryl, heteroaryl, arylamino, alkylamino, cycloalkyl or heterocycloalkyl of R₆₀₁ through R₆₀₄ may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

The compound having an electroluminescence peak with wavelength of 500 to 560 nm or the compound having an electroluminescence peak with wavelength of not less than 560 nm may be exemplified by the compounds represented by the following compounds, but not limited thereto:

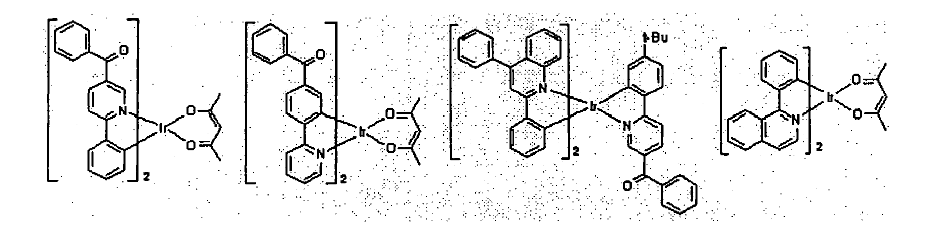

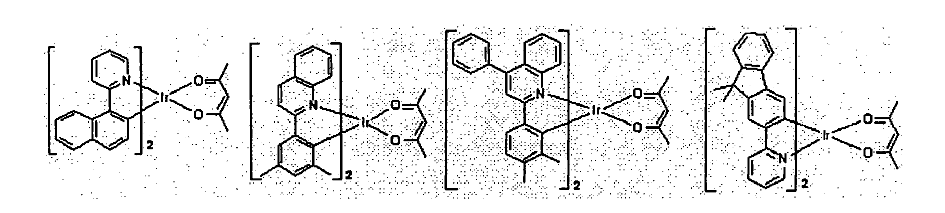

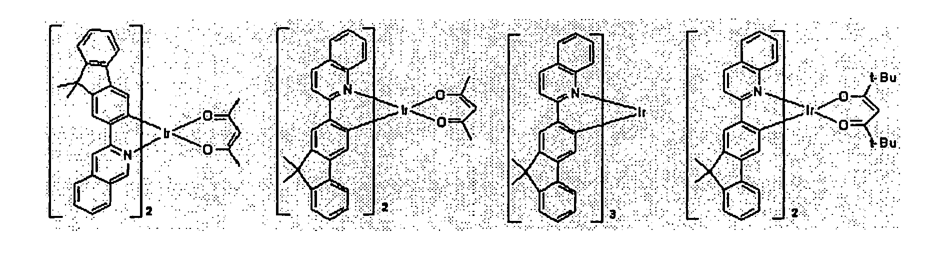

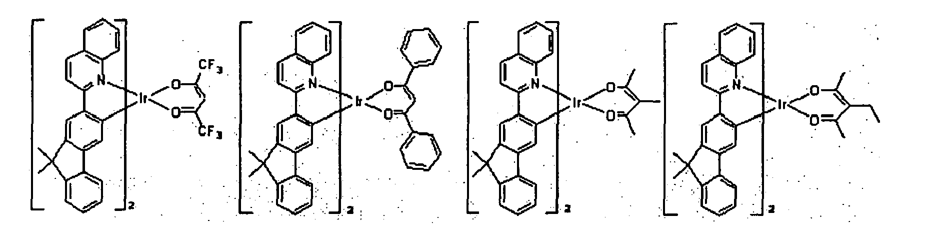

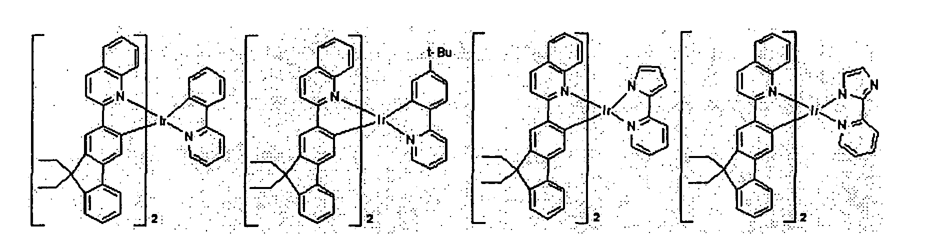

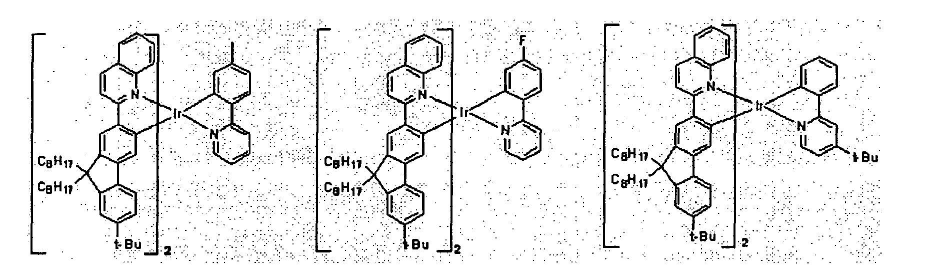

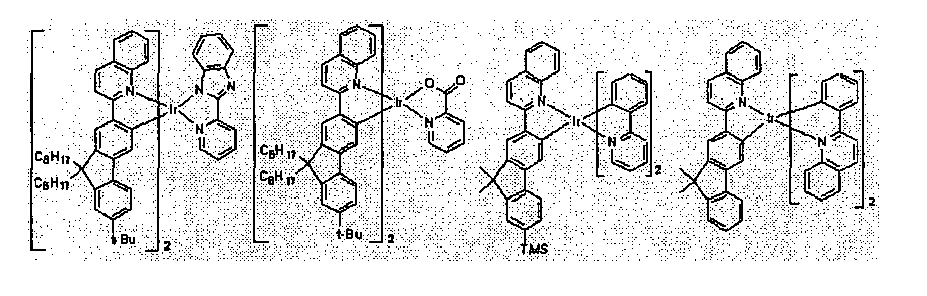

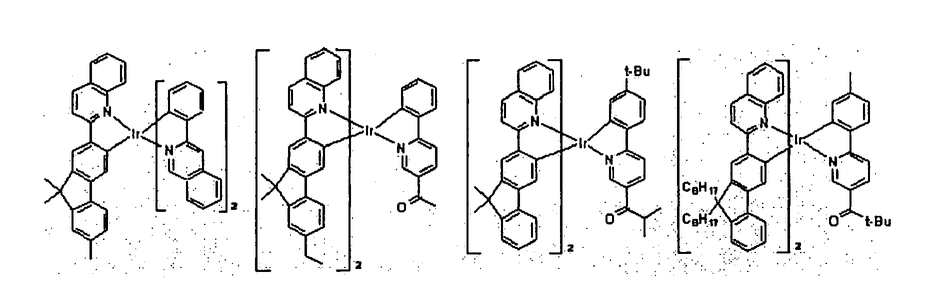

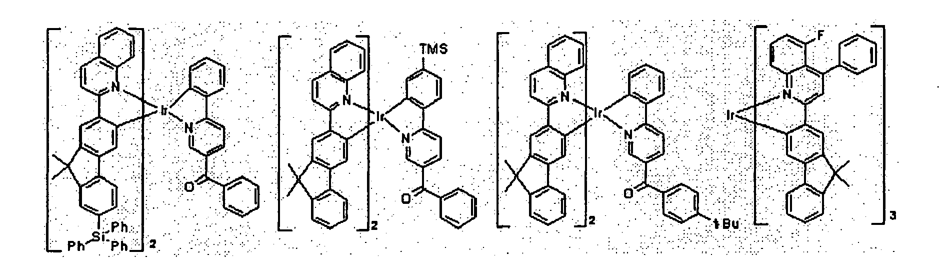

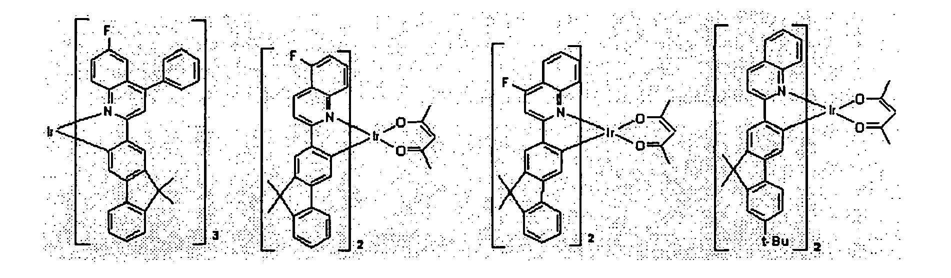

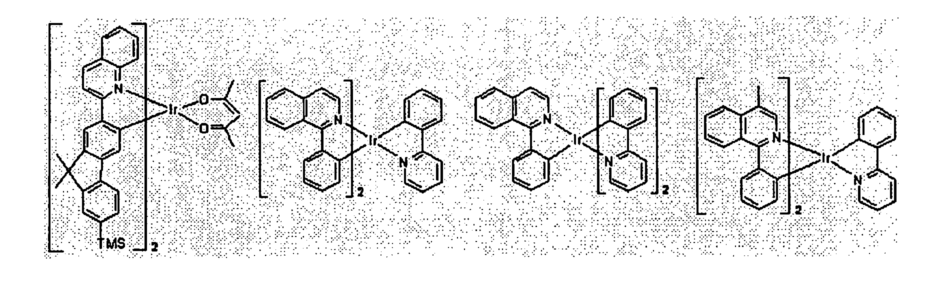

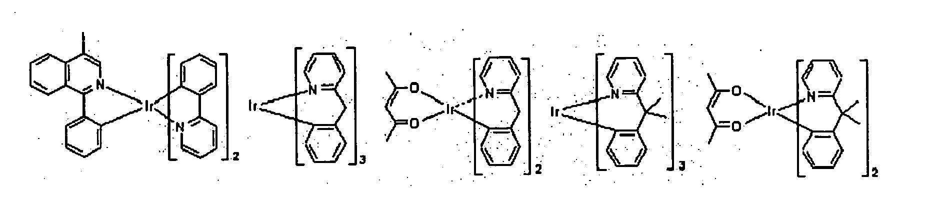

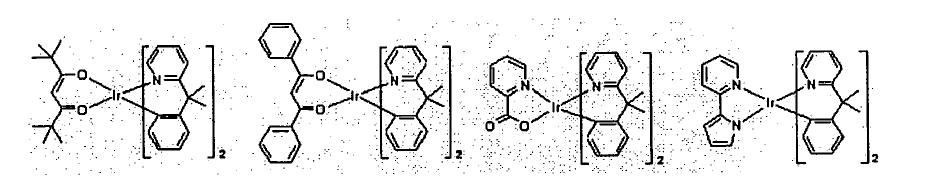

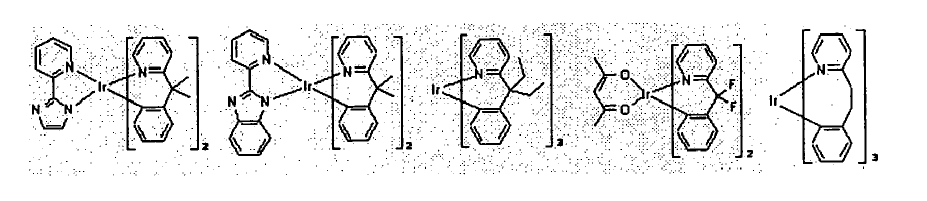

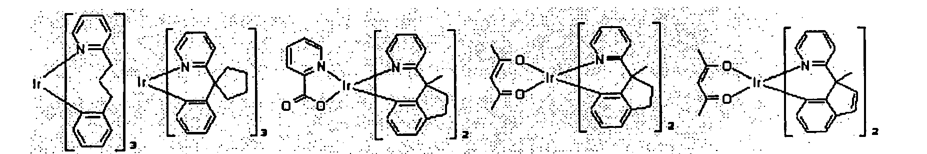

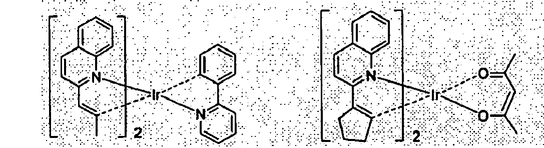

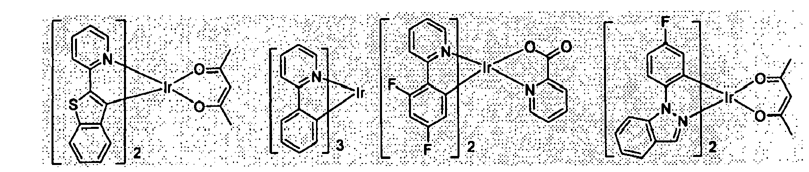

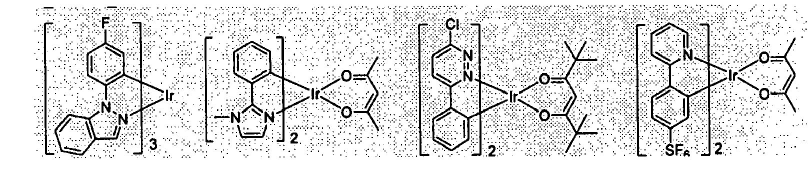

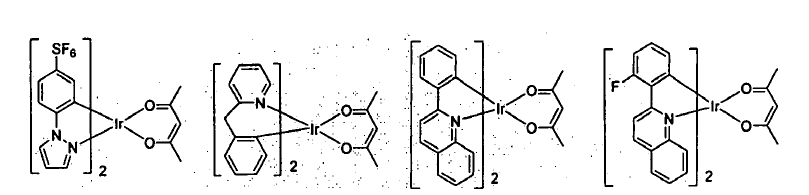

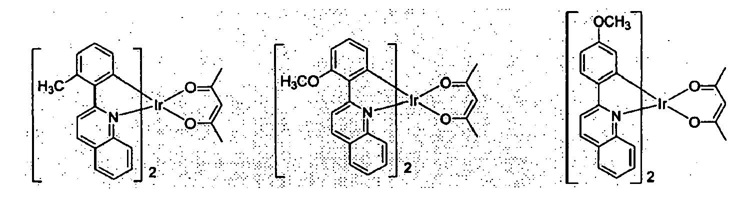

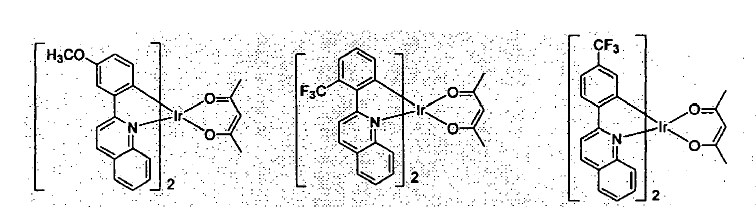

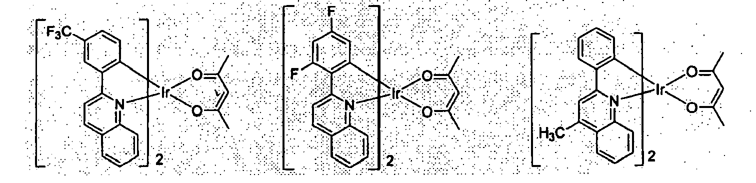

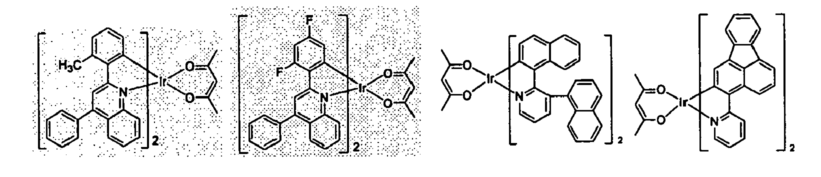

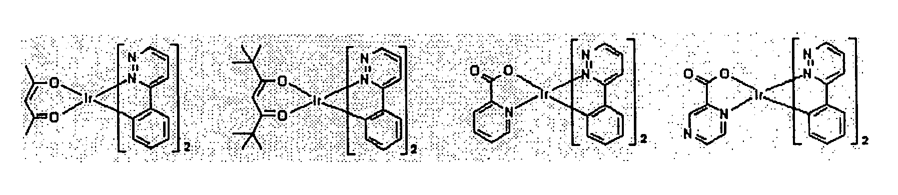

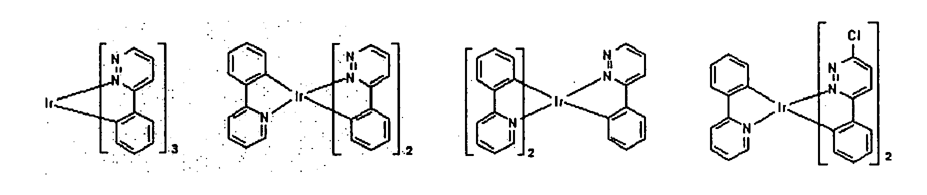

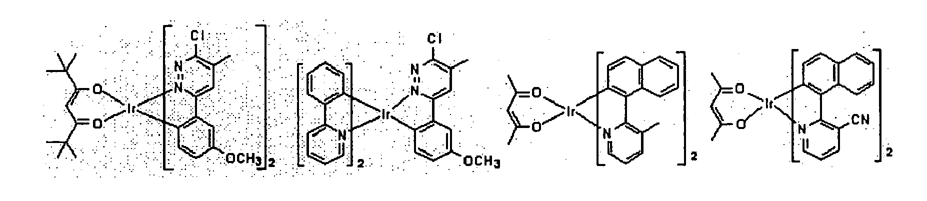

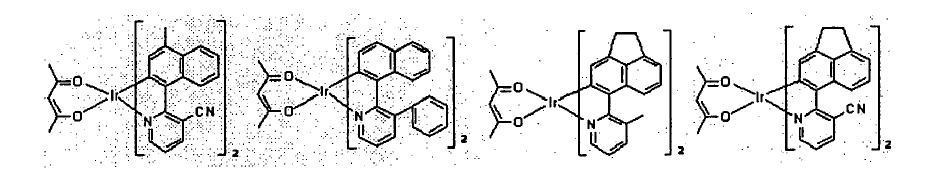

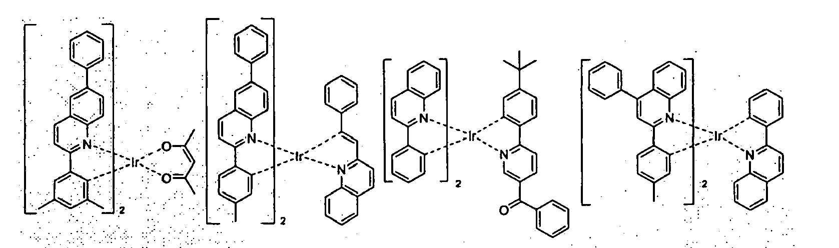

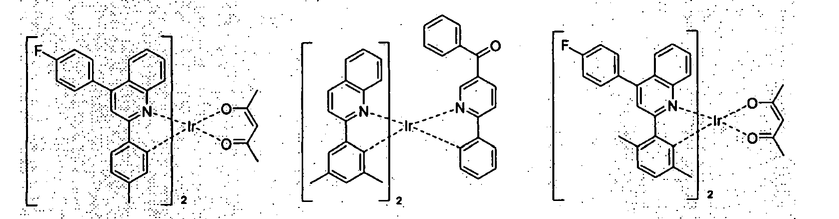

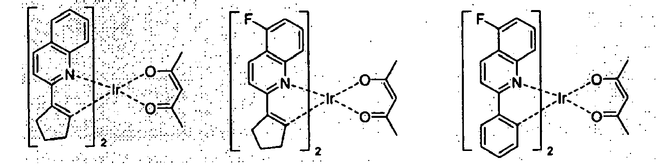

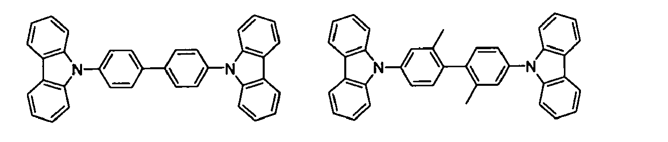

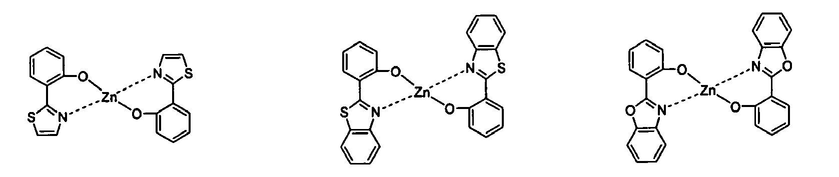

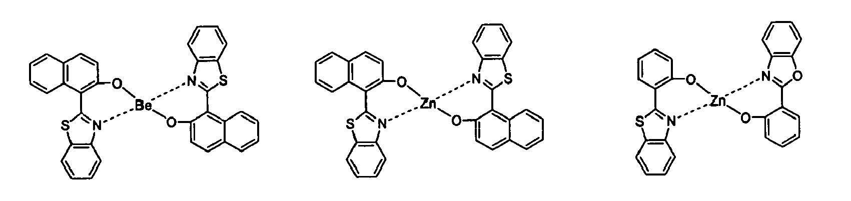

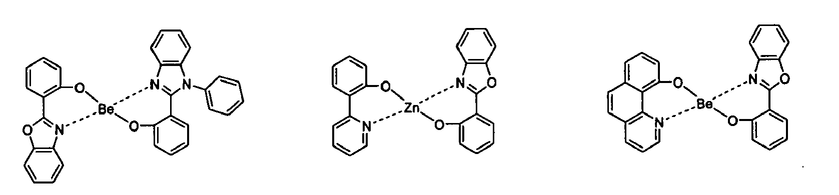

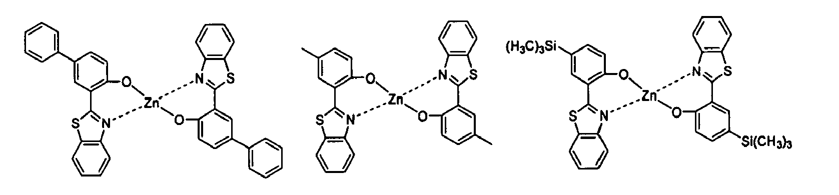

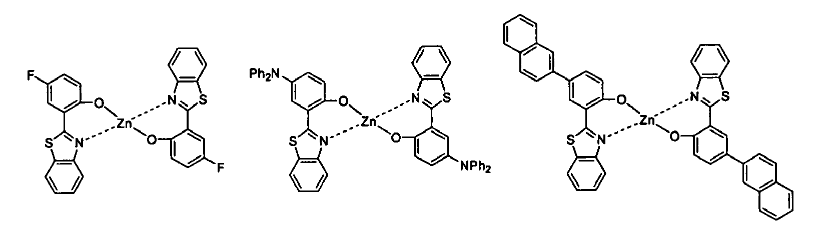

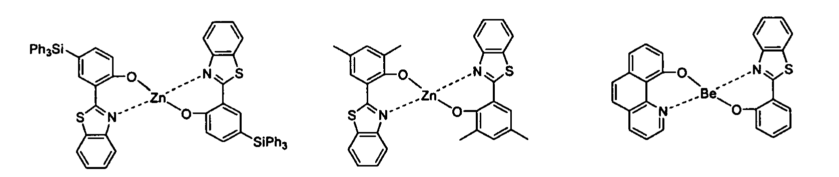

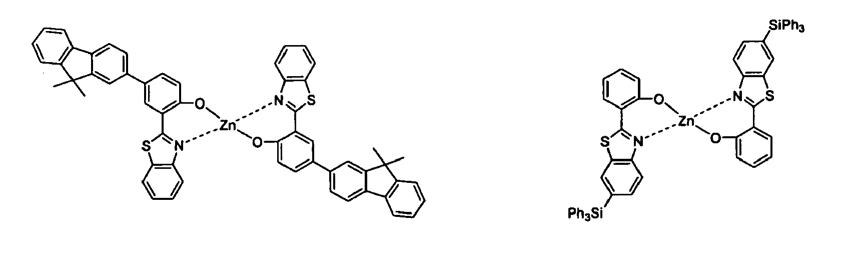

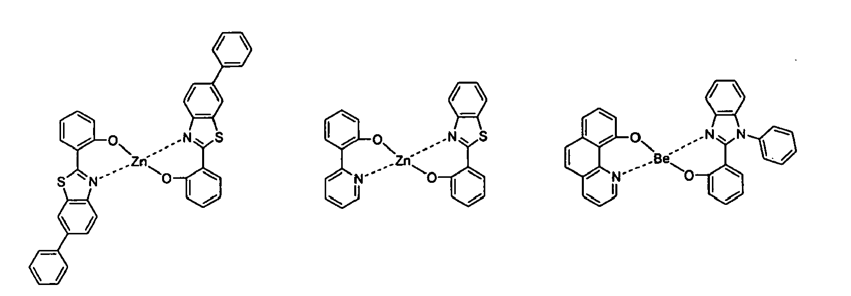

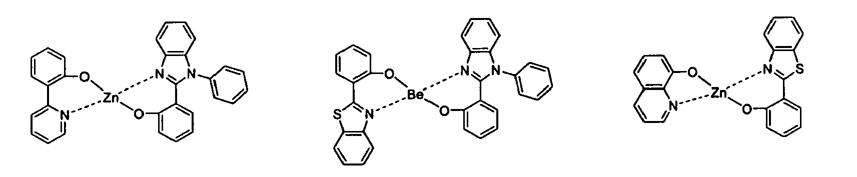

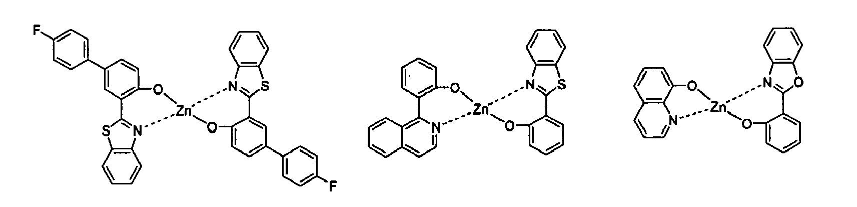

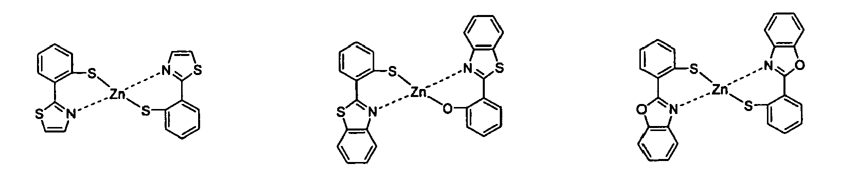

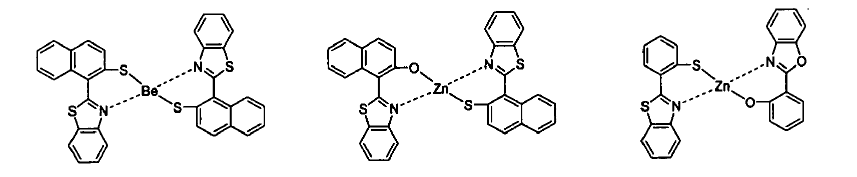

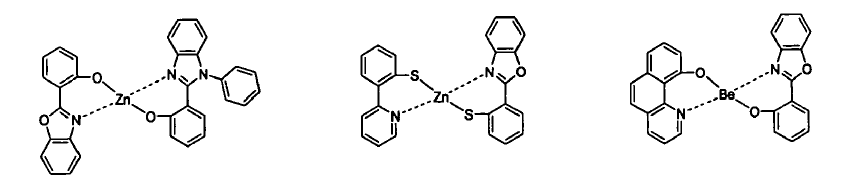

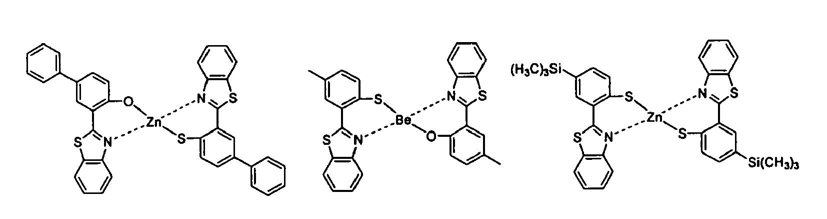

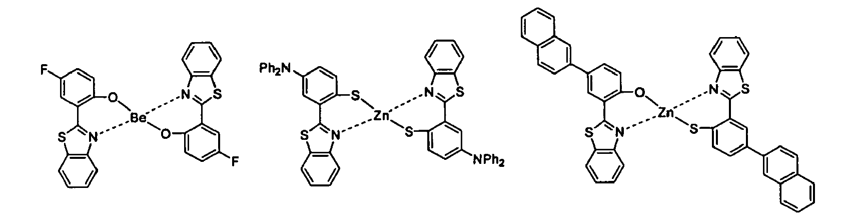

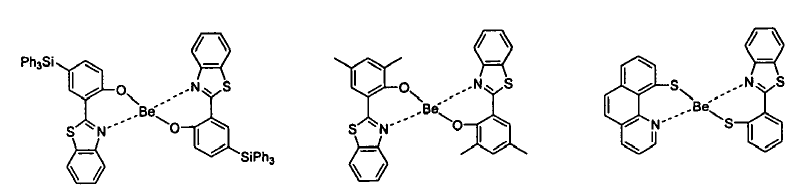

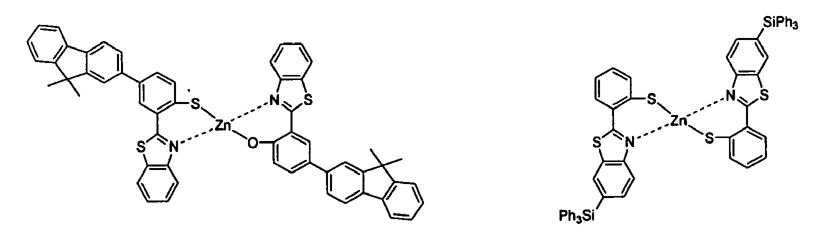

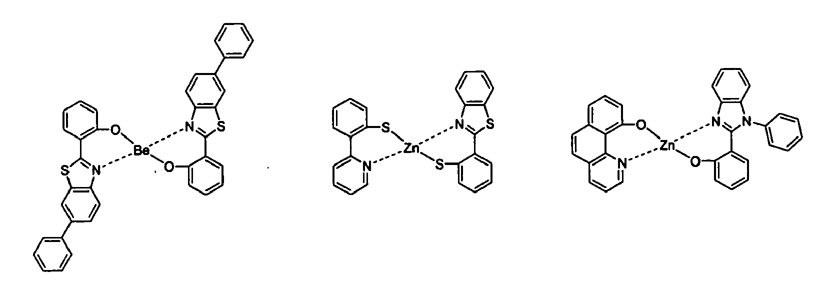

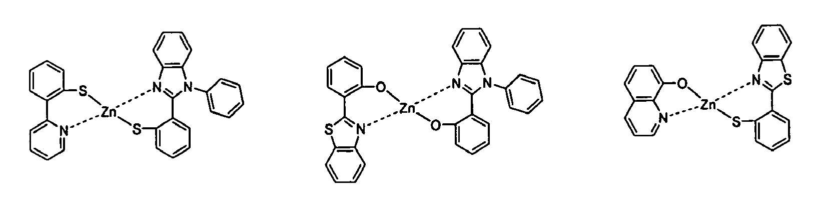

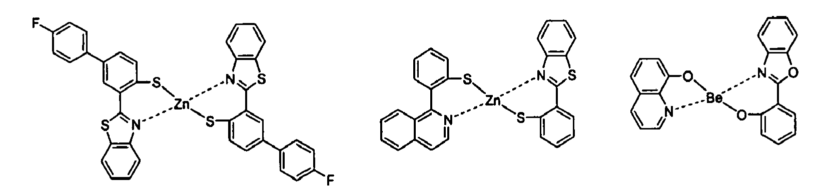

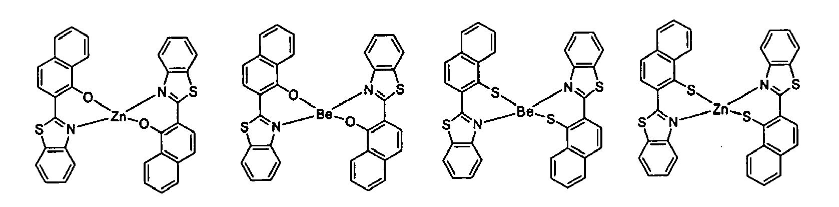

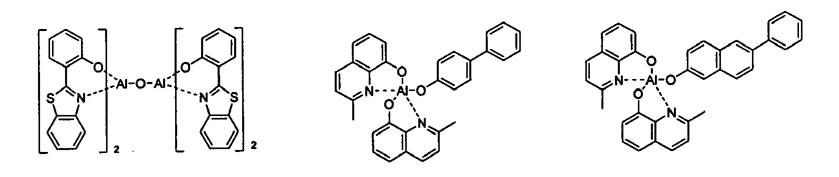

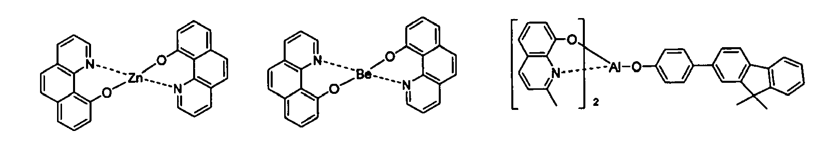

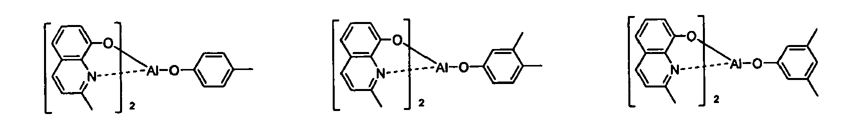

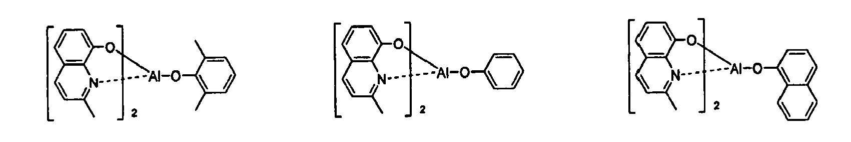

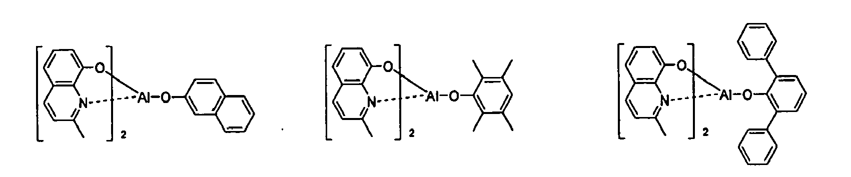

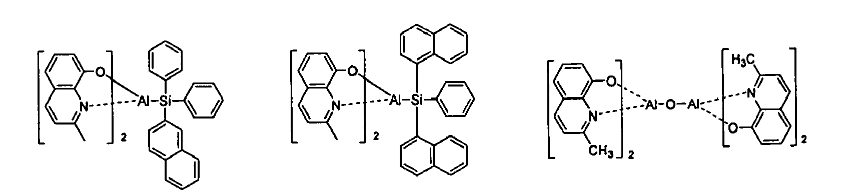

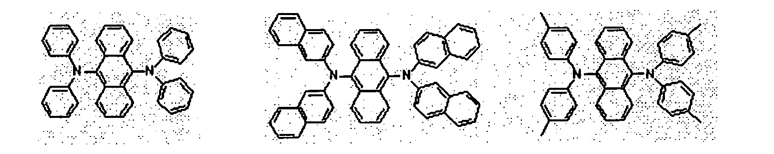

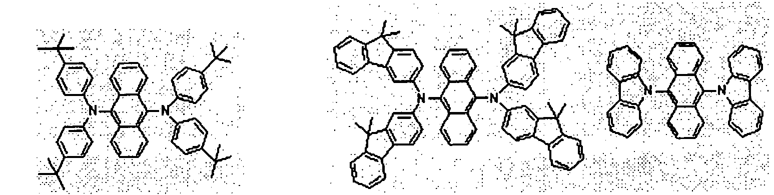

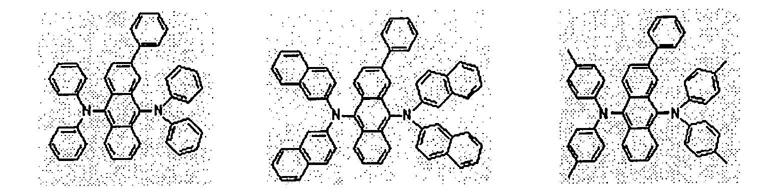

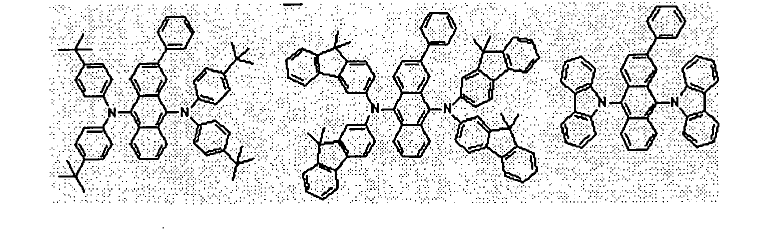

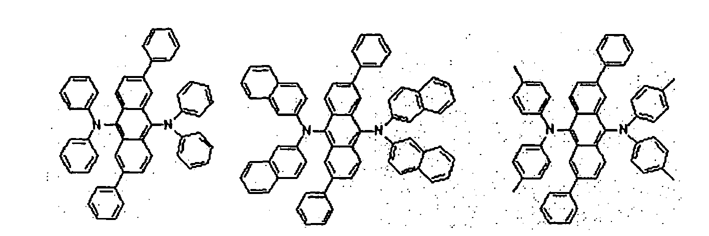

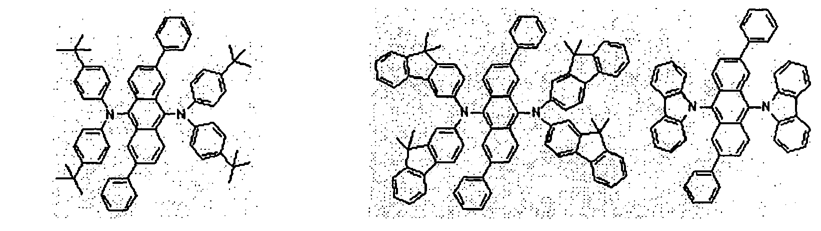

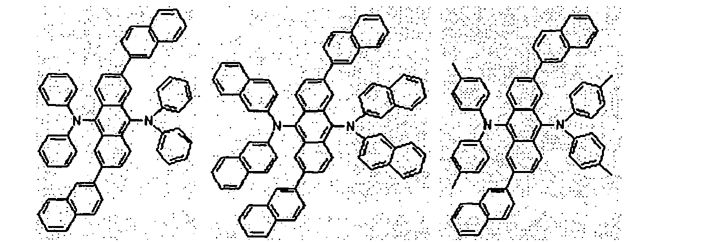

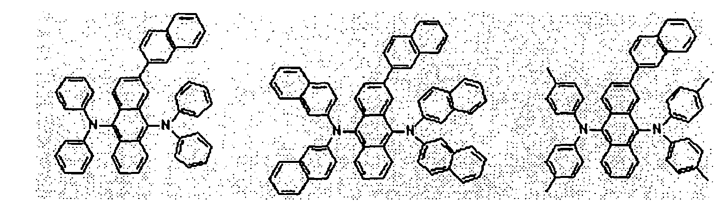

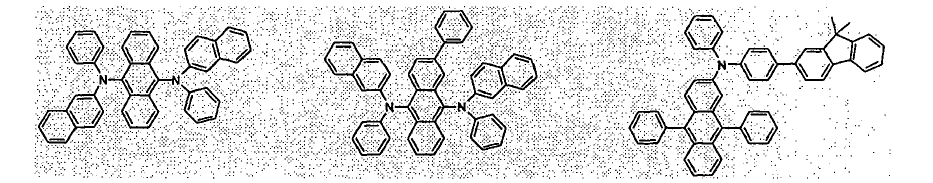

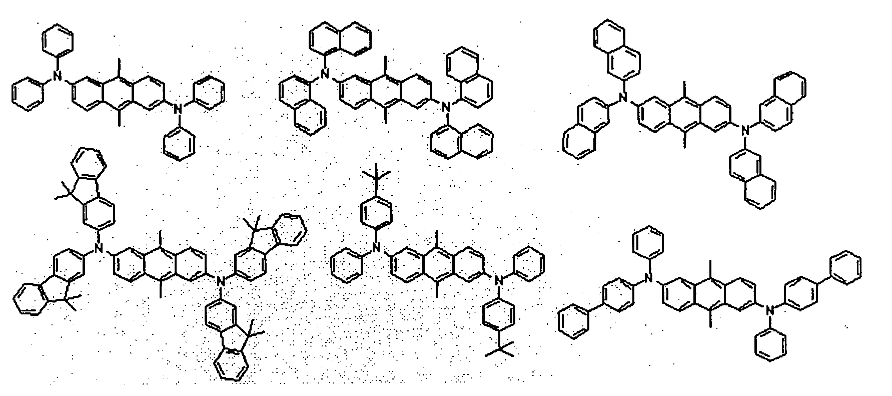

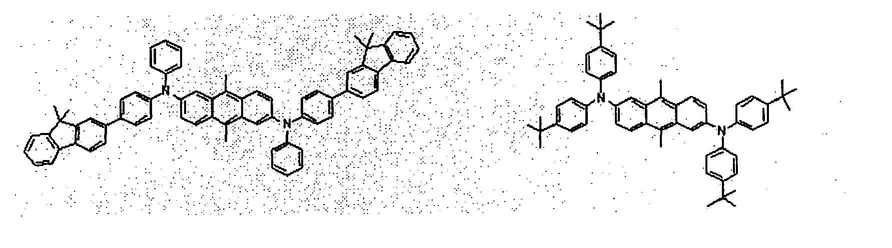

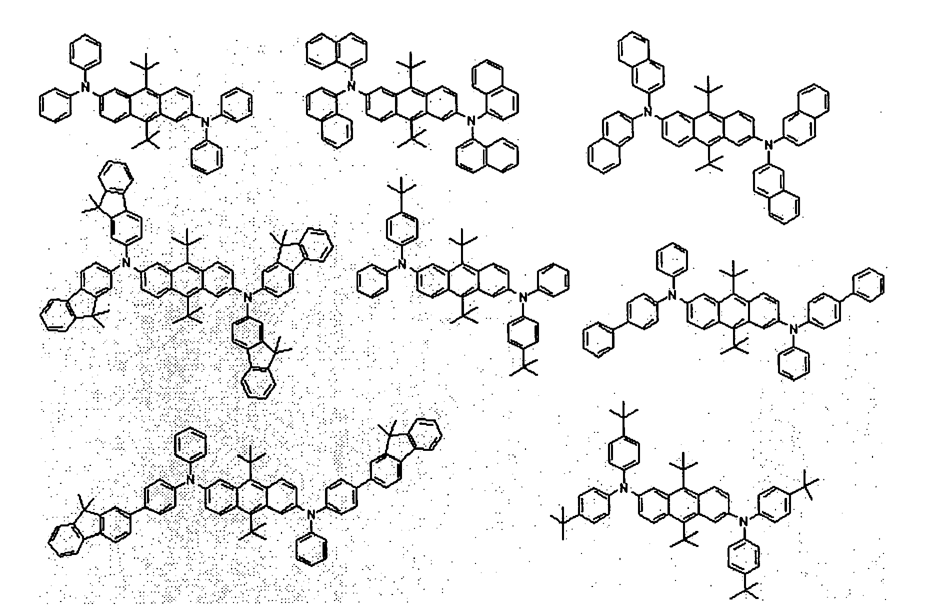

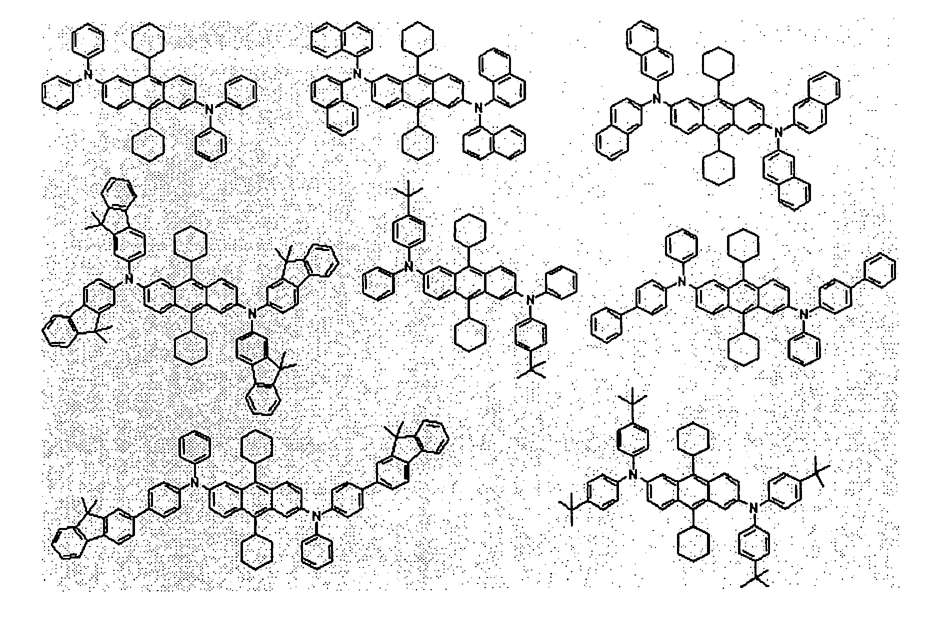

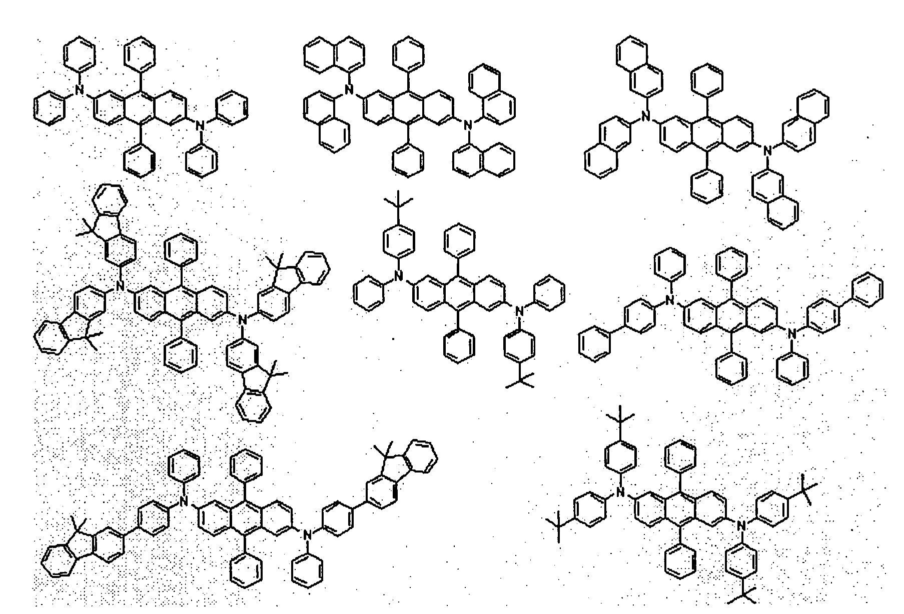

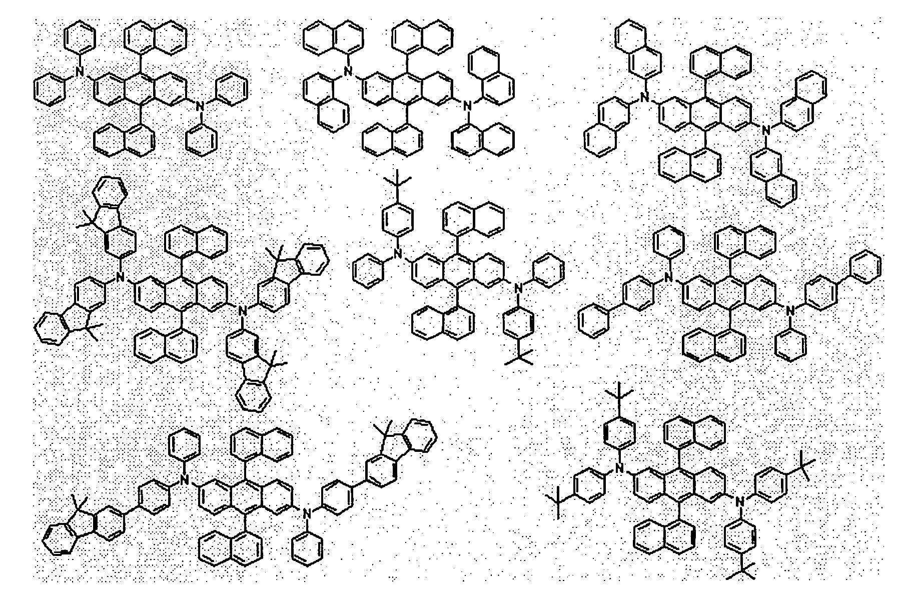

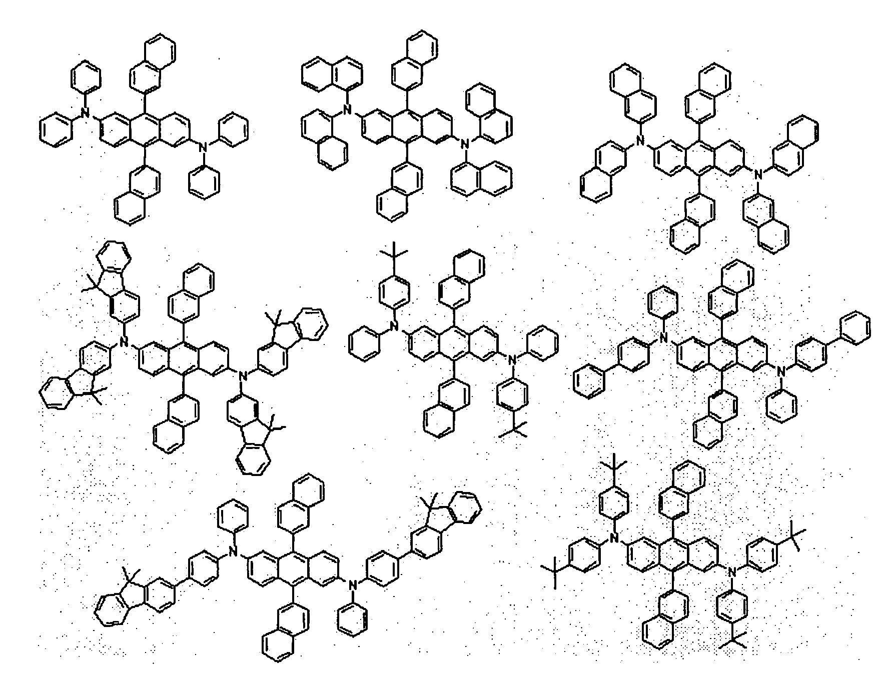

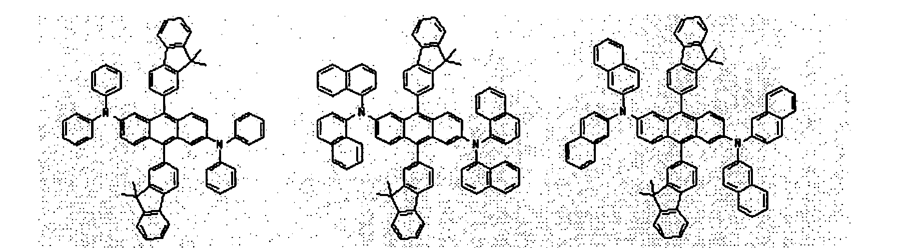

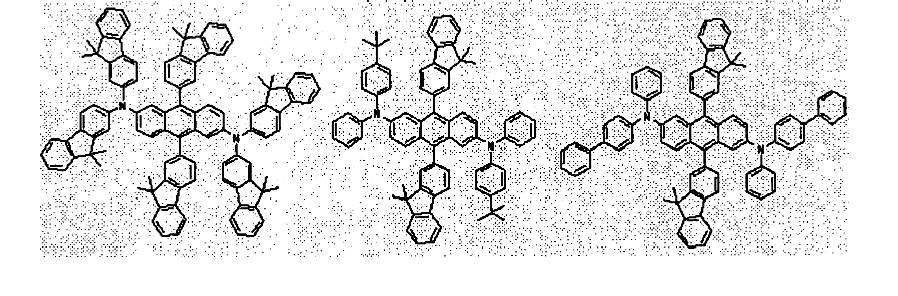

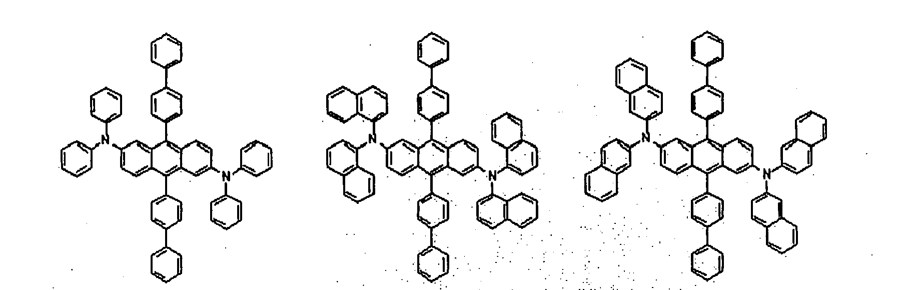

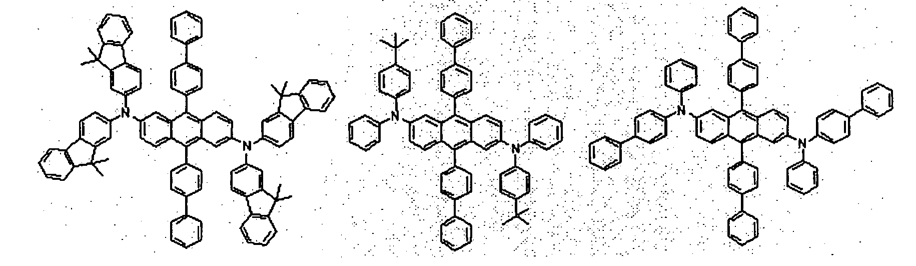

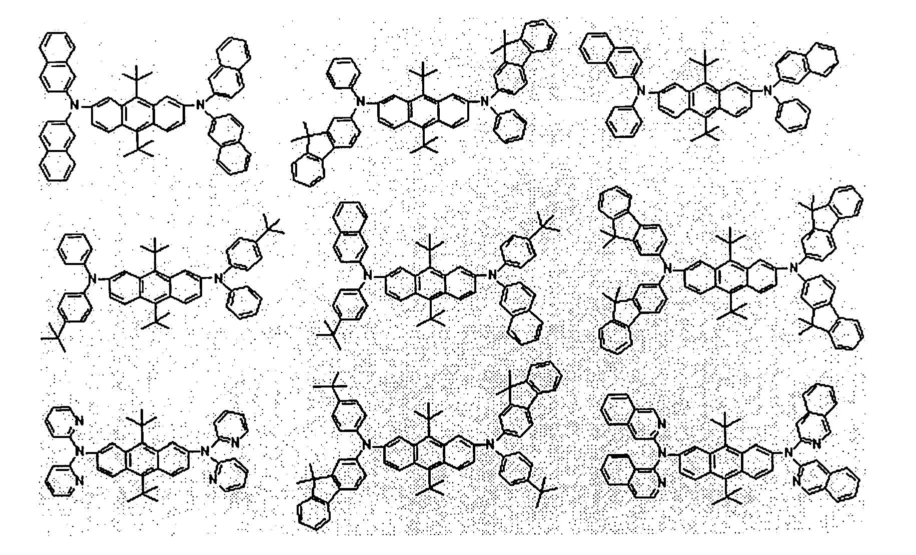

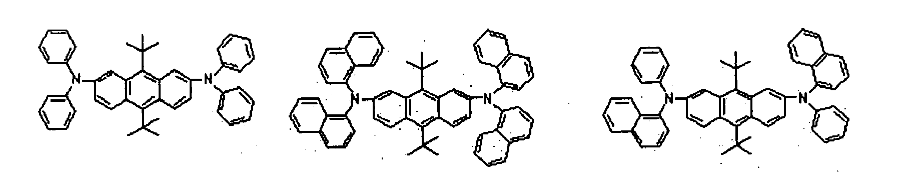

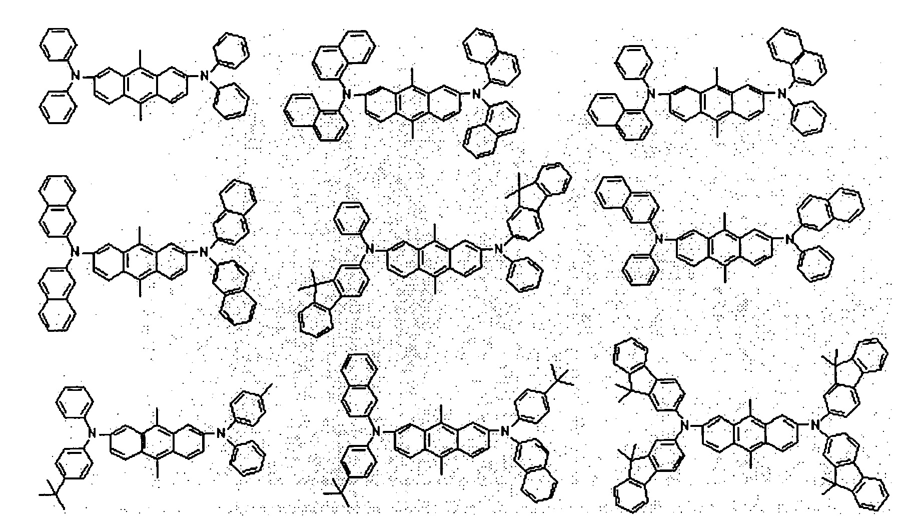

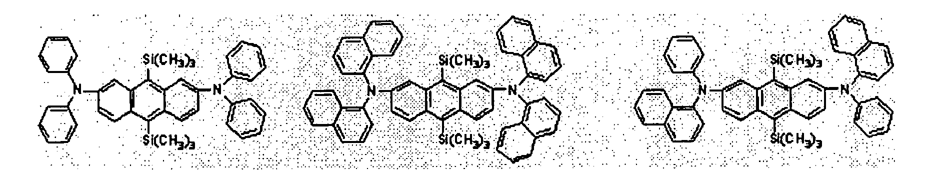

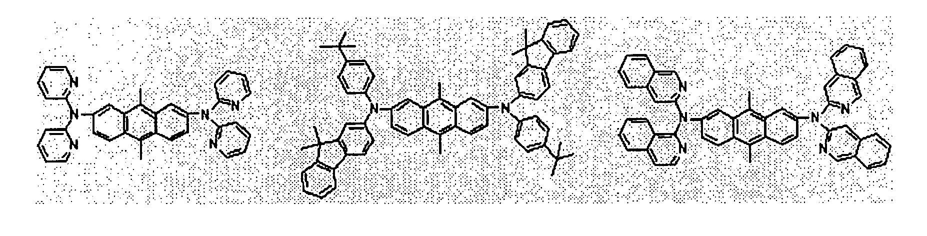

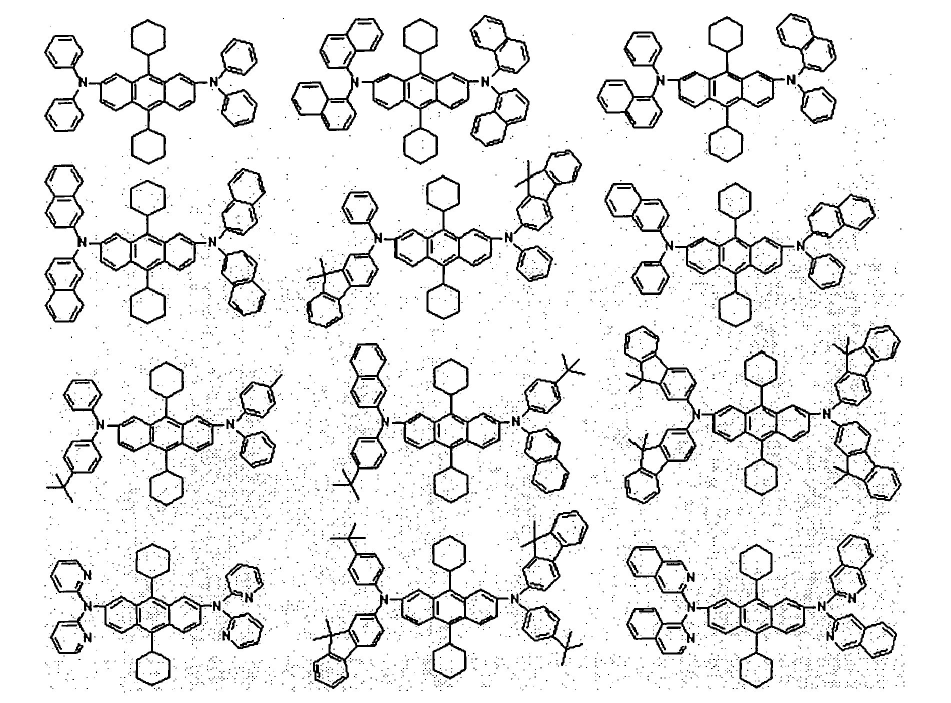

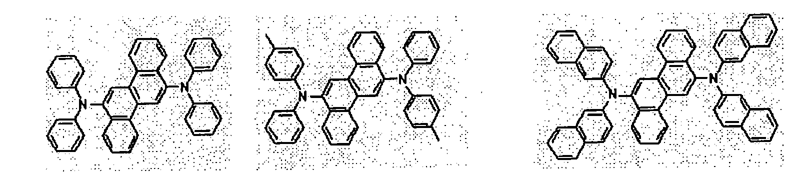

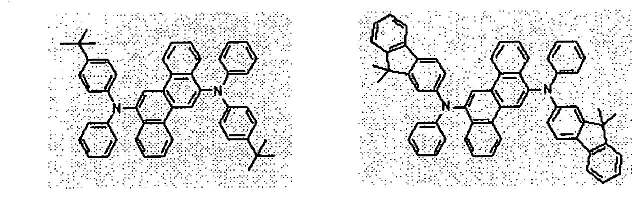

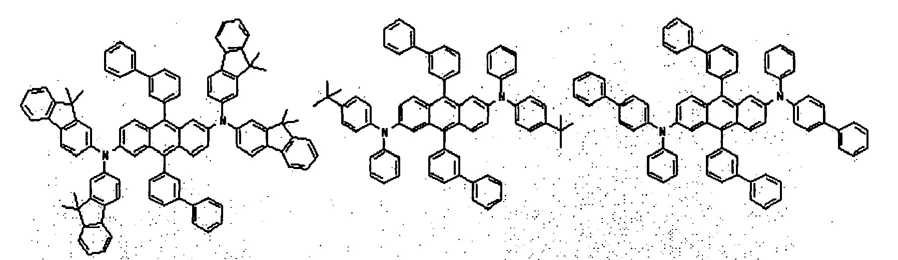

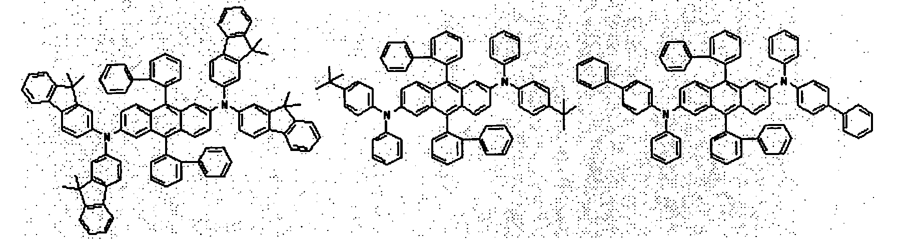

In the electroluminescent device according to the present invention, a layer (hereinafter referred to as "surface layer") selected from a chalcogenide layer, a metal halide layer and a metal oxide layer may be placed on the inner surface of one or both electrode(s) among the pair of electrodes. More specifically, a chalcogenide (including oxide) layer of silicon or aluminum may be placed on the anode surface of the electroluminescent layer, and a metal halide layer or metal oxide layer may be placed on the cathode surface of the electroluminescent layer. A driving stability may be attained therefrom.

The chalcogenide may be, for example, SiOₓ (1 ≤ x ≤ 2), AlOₓ (1 ≤ x ≤ 1.5), SiON, SiAlON, etc. The metal halide may be, for example, LiF, MgF₂, CaF₂, a rare earth metal fluoride, etc. The metal oxide may be, for example, Cs₂O, Li₂O, MgO, SrO, BaO, CaO, etc.

Further, in the electroluminescent device according to the present invention, a mixed region of an electron transport compound and a reductive dopant or a mixed region of a hole transport compound and an oxidative dopant may be placed on the inner surface of one or both electrode(s) among the pair of electrodes. In that case, transport of electrons from the mixed region to the electroluminescent layer becomes easier, because the electron transport compound is reduced to an anion. Further, transport of holes from the mixed region to the electroluminescent layer becomes easier, because the hole transport compound is oxidized to a cation. Preferred examples of the oxidative dopant include various Lewis acids and acceptor compounds. Preferred examples of the reductive dopant include alkali metals, alkali metal compounds, alkaline earth metals, rare earth metals and mixtures thereof.

Since the organic electroluminescent compound according to the present invention has superior luminous efficiency and excellent life property, it may be used to manufacture OLED devices having very good operation life.

### EXAMPLES

Hereinafter, the organic electroluminescent compounds according to the present invention, preparation methods thereof, and electroluminescence characteristics of devices will be described for the understanding of the present invention. However, the following examples are for illustrative purposes only and not intended to limit the scope of the present invention.

### [Preparation Examples]

### [Preparation Example 1] Preparation of Compound 1

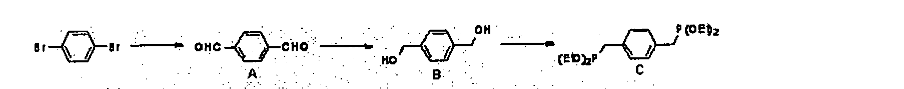

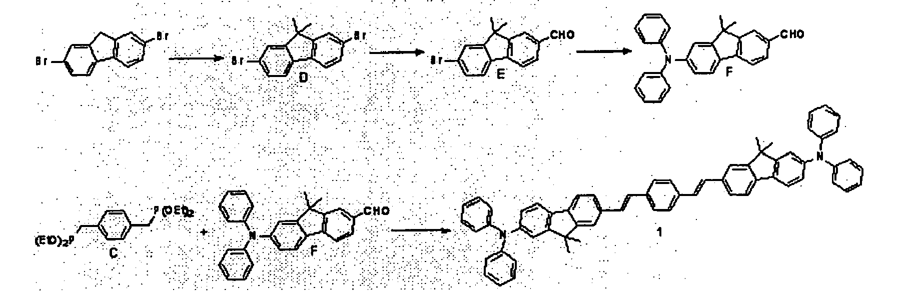

### Preparation of Compound A

1,4-Dibromobenzene (20.0 g, 56.81 mmol) was dissolved in tetrahydrofuran (1000 mL). After lowering temperature to -78 °C, 2.5 M butyllithium (n-BuLi) (56.81 mL, 142.02 mmol) was slowly added dropwise. After stirring for 30 minutes, N,N-dimethylformamide (DMF) (13.19 mL, 170.43 mmol) was added. After slowly heating, followed by stirring for 2 hours, aqueous NH₄Cl solution (800 mL) and distilled water (800 mL) were added to terminate the reaction. The organic layer was separated and the solvent was removed under reduced pressure. Compound **A** (11.09 g, 44.31 mmol) was obtained by recrystallization using methanol and hexane.

### Preparation of Compound B

Compound **A** (11.09 g, 44.31 mmol) and NaBH₄ (2.5g, 66.47 mmol) were added to a reactor. After removing air under reduced pressure, nitrogen gas was filled therein. After adding tetrahydrofuran (600 mL), methanol (350 mL) was slowly added dropwise while stirring the mixture. Upon completion of reaction, the product was washed with water (1000 mL) and extracted with ethyl acetate. Compound **B** (8.45 g, 33.12 mmol) was obtained by column chromatography using dichloromethane and hexane.

### Preparation of Compound C

Compound **B** (5.0 g, 19.66 mmol) was added to a reactor. After removing air under reduced pressure, nitrogen gas was filled therein, 5 mL of triethyl phosphite (of 10.15 mL, 58.98 mmol) was added and Compound **B** was dissolved. In another reactor, the remaining 5.15 mL of triethyl phosphite was added. While slowly adding iodine (I₂) (9.98 g, 39.32 mmol) with the lid open, the mixture was stirred at 0 °C for 30 minutes. The mixture of iodine and triethyl phosphite was added to the reactor containing Compound **B**. After heating to 150 °C, the mixture was stirred for 4 hours. Upon completion of reaction, the product was washed with water (50 mL) and extracted with ethyl acetate (50 mL). Compound **C** (13.59 g, 27.48 mmol) was obtained by recrystallization using methanol.

### Preparation of Compound D

2,7-Dibromofluorene (50 g, 154.26 mmol) and potassium hydroxide (KOH) (69.2 g, 1234 mmol) were dissolved in DMSO (700 mL) under nitrogen atmosphere. After cooling to 0 °C, followed by slowly adding distilled water (113 mL) dropwise, the mixture was stirred for an hour. Then, after slowly adding iodomethane (CH₃I) (38.49 mL, 617.04 mmol) dropwise, followed by slowly heating to room temperature, the mixture was stirred for 15 hours. Excess water was added to terminate the reaction and extraction was carried out using dichloromethane. The resultant organic layer was dried with magnesium sulfate, filtered, and concentrated under reduced pressure. Compound **D** (53.0 g, 150.50 mmol) was obtained by column separation.

### Preparation of Compound E

Compound D (53.0 g, 150.50 mmol) was dissolved in tetrahydrofuran (350 mL). After lowering temperature to -78 °C, 1.6 M *n*-BuLi (63.2 mL, 158.04 mmol) was slowly added dropwise. After stirring for 30 minutes, *N,N*-dimethyl formamide (16.32 mL, 210.7 mmol) was added. After stirring for 2 hours while slowly heating, aqueous NH₄Cl solution (80 mL) and distilled water (80 mL) were added to terminate the reaction. The organic layer was separated and the solvent was removed under reduced pressure. Compound **E** (20.9 g, 69.40 mmol) was obtained by column separation.

### Preparation of Compound F

Compound **E** (20.9 g, 69.40 mmol), diphenylamine (12.5 g, 104.10 mmol), cesium carbonate (24.1 g, 104.10 mmol) and Pd(OAc)₂ (332 mg, 2.08 mmol) were suspended in toluene (800 mL). P(*t*-Bu)₃ (599 mg, 4.16 mmol) was added and the mixture was stirred at 120 °C for 4 hours. After adding saturated aqueous NH₄Cl solution (500 mL), followed by extraction with ethyl acetate (500 mL), drying, filtration and concentration under reduced pressure, Compound **F** (15.2 g, 39.00 mmol) was obtained by recrystallization using methanol/n-hexane (300 mL, v/v = 1/1).

### Preparation of Compound 1

Compound **C** (5.1 g, 9.72 mmol) and Compound **F** (5.8 g, 21.37 mmol) were added to a reactor and dried under reduced pressure. Tetrahydrofuran (200 mL) was added thereto under nitrogen atmosphere and the mixture was stirred. Potassium *tert-*butoxide (t-BuOK) (4.14 g, 37.17 mmol) dissolved in tetrahydrofuran (30 mL) was slowly added dropwise at 0 °C or below, and the mixture was slowly heated to room temperature. After stirring for 4 hours, water (500 mL) was poured upon completion of reaction. The produced solid was filtered and washed with methanol (1 L). This procedure was repeated 3 times. Compound **1** (5.00 g, 6.6 mmol) was obtained by recrystallization using tetrahydrofuran and methanol, followed by washing with hexane.

Electroluminescent compounds (Compounds **1** to **719**) were prepared according to the method of Preparation Example 1. Table 1 shows ¹H NMR and MS/FAB data of the prepared electroluminescent compounds.

**[Table 1]**

| compound | ¹H NMR(CDCl₃, 200 MHz) | MS/FAB | |
|---|---|---|---|
| | | found | calculated |
| **1** | δ = 1.72(12H, s), 6.58∼6.63(10H, m), 6.75∼6.81(6H, m), 6.95(4H, m), 7.2(8H, m), 7.5∼7.54(6H, m), 7.62(2H, m), 7.71(2H, m), 7.87(2H, m) | 849.11 | 848.41 |
| **4** | δ = 1.72(12H, s), 2.34(12H, s), 6.44(4H, m), 6.55∼6.59(10H, m), 6.75(2H, m), 6.95(4H, m), 7.08(4H, m), 7.5∼7.54(6H, m), 7.62(2H, m), 7.71(2H, m), 7.87(2H, m) | 905.22 | 904.48 |
| **9** | δ = 1.35(36H, s), 1.72(12H, s), 6.55∼6.58(10H, m), 6.75(2H, m), 6.95∼7.01(12H, m), 7.5∼7.54(6H, m), 7.62(2H, m), 7.71(2H, m), 7.87(2H, m) | 1073.54 | 1072.66 |
| **10** | δ = 1.72(36H, s), 6.58(6H, m), 6.75(6H, m), 6.95(4H, m), 7.28(4H, m), 7.38(4H, m), 7.5∼7.55(10H, m), 7.62(6H, m), 7.71(2H, m), 7.87(6H, m) | 1313.75 | 1312.66 |
| **13** | δ = 1.72(12H, s), 6.95(4H, m), 7.17(2H, m), 7.25∼7.34(10H, m), 7.5(8H, m), 7.63(2H, m), 7.87(4H, m), 7.94(2H, m), 8.12(2H, m), 8.55(2H, m) | 845.08 | 844.38 |
| **15** | δ = 1.72(12H, s), 6.58∼6.63(6H, m), 6.75∼6.81(4H, m), 6.95∼6.98(6H, m), 7.2(4H, m), 7.38(2H, m), 7.5-7.62(14H, m), 7.71 (2H, m), 7.87(2H, m), 8.02∼8.07(4H, m) | 949.23 | 948.44 |
| **20** | δ = 1.72(24H, s), 6.58∼6.63(8H, m), 6.75∼6.81(6H, m), 6.95(4H, m), 7.2(4H, m), 7.28(2H, m), 7.38(2H, m), 7.5∼7.55(8H, m), 7.62(4H, m), 7.71(2H, m), 7.87(4H, m) | 1081.43 | 1080.54 |
| **28** | δ = 1.72(12H, s), 6.58∼6.63(6H, m), 6.75∼6.81(4H, m), 6.91∼6.95(6H, m), 7.2(4H, m), 7.5∼7.54(6H, m), 7.62(2H, m), 7.71(2H, m), 7.82∼7.88(10H, m), 8.12(4H, m), 8.93(4H, m) | 1049.35 | 1048.48 |
| **33** | δ = 1.35(18H, s), 1.72(12H, s), 6.55∼6.58(6H, m), 6.75(2H, m), 6.95∼7.01(10H, m), 7.38(2H, m), 7.5∼7.62(14H, m), 7.71(2H, m), 7.87(2H, m), 8.02∼8.07(4H, m) | 1061.44 | 1060.57 |
| **37** | δ = 1.72(24H, s), 6.58(4H, m), 6.75(4H, m), 6.95∼6.98(6H, m), 7.28(2H, m), 7.38(4H, m), 7.5∼7.62(18H, m), 7.71(2H, m), 7.87(4H, m), 8.02∼8.07(4H, m) | 1181.55 | 1180.57 |
| **46** | δ = 1.72(12H, s), 6.58(2H, m), 6.69∼6.75(6H, m), 6.95(4H, m), 7.36∼7.41(4H, m), 7.49∼7.54(22H, m), 7.62(2H, m), 7.71∼7.77(6H, m), 7.84∼7.88(6H, m) | 1101.42 | 1100.51 |
| **54** | δ = 1.72(24H, s), 2.34(12H, s), 6.36(4H, m), 6.58(4H, m), 6.71∼6.75(6H, m), 6.95(4H, m), 7.28(2H, m), 7.38(2H, m), 7.5∼7.55(8H, m), 7.62(4H, m), 7.71(2H, m), 7.87(4H, m) | 1137.54 | 1136.60 |
| **66** | δ = 1.35(18H, s), 1.72(12H, s), 6.55∼6.58(6H, m), 6.69∼6.75(6H, m), 6.95∼7.01(8H, m), 7.41(2H, m), 7.5∼7.54(18H, m), 7.62(2H, m), 7.71(2H, m), 7.87(2H, m) | 1113.52 | 1112.60 |
| **70** | δ = 1.72(12H, s), 2.34(18H, s), 6.36(4H, m), 6.51 (4H, m), 6.58(2H, m), 6.71∼6.75(4H, m), 6.95∼6.98(8H, m), 7.5∼7.54(6H, m), 7.62(2H, m), 7.71(2H, m), 7.87(2H, m) | 933.27 | 932.51 |
| **84** | δ = 1.72(12H, s), 2.34(12H, s), 6.36(4H, m), 6.58(2H, m), 6.71∼6.75(4H, m), 6.91∼6.95(6H, m), 7.5∼7.54(6H, m), 7.62(2H, m), 7.71(2H, m), 7.82∼7.88(10H, m), 8.12(4H, m), 8.93(4H, m) | 1105.45 | 1104.54 |
| **106** | δ = 1.72(12H, s), 6.58∼6.62(4H, m), 6.7∼6.75(4H, m), 6.95(4H, m), 7.27(2H, m), 7.36(2H, m), 7.5∼7.55(8H, m), 7.62(2H, m), 7.71(2H, m), 7.87(2H, m), 8.04∼8.09(6H, m) | 853.06 | 852.39 |
| **112** | δ = 1.72(12H, s), 6.58∼6.62(4H, m), 6.7∼6.75(4H, m), 6.95∼6.99(6H, m), 7.41∼7.62(16H, m), 7.71(2H, m), 7.87(2H, m), 8.07(2H, m), 8.17(2H, m), 8.28(2H, m) | 953.18 | 952.43 |
| **117** | δ = 1.72(12H, s), 6.58∼6.63(6H, m), 6.69∼6.81(8H, m), 6.95(4H, m), 7.2(4H, m), 7.5∼7.62(18H, m), 7.71∼7.73(4H, m), 7.87∼7.92(4H, m), 8(4H, m) | 1101.42 | 1100.51 |
| **120** | δ = 1.72(24H, s), 6.58∼6.63(6H, m), 6.69∼6.81(8H, m), 6.95(4H, m), 7.2(4H, m), 7.28(2H, m), 7.38(2H, m), 7.6∼7.55(12H, m), 7.62∼7.63(4H, m), 7.71∼7.77(4H, m), 7.87∼7.93(6H, m) | 1233.62 | 1232.60 |
| **123** | δ = 1.72(24H, s), 6.58(2H, m), 6.69∼6.75(6H, m), 6.95∼6.98(6H, m), 7.28(2H, m), 7.38(4H, m), 7.5∼7.63(22H, m), 7.71∼7.77(4H, m), 7.87∼7.93(6H, m), 8.02∼8.07(4H, m) | 1333.74 | 1332.63 |
| **130** | δ = 1.72(12H, s), 6.58(2H, m), 6.69∼6.75(10H, m), 6.95(4H, m), 7.5∼7.62(28H, m), 7.71∼7.73(6H, m), 7.87∼7.92(6H, m), 8(8H, m) | 1353.73 | 1352.60 |
| **135** | δ = 1.72(24H, s), 2.34(6H, s), 6.51(4H, m), 6.58(2H, m), 6.69∼6.75(6H, m), 6.95∼6.98(8H, m), 7.28(2H, m), 7.38(2H, m), 7.5∼7.55(12H, m), 7.62∼7.63(4H, m), 7.71∼7.77(4H, m), 7.87∼7.93(6H, m) | 1261.68 | 1260.63 |
| **142** | δ = 1.72(12H, s), 6.58∼6.63(10H, m), 6.75∼6.81(6H, m), 6.95(4H, m), 7.2(8H, m), 7.54∼7.56(6H, m), 7.62∼7.64(6H, m), 7.71(2H, m), 7.87(2H, m) | 925.21 | 924.44 |
| **145** | δ = 1.72(12H, s), 6.58(2H, m), 6.75(2H, m), 6.95(4H, m), 7.36(4H, m), 7.49∼7.56(14H, m), 7.62∼7.64(6H, m), 7.71∼7.77(10H, m), 7.84∼7.88(10H, m) | 1125.44 | 1124.51 |
| **154** | δ = 1.72(12H, s), 6.58∼6.63(6H, m), 6.75∼6.81(4H, m), 6.95∼6.98(6H, m), 7.2(4H, m), 7.38(2H, m), 7.53∼7.64(18H, m), 7.71(2H, m), 7.87(2H, m), 8.02∼8.07(4H, m) | 1025.32 | 1024.48 |
| **162** | δ = 1.72(24H, s), 6.58(4H, m), 6.75(4H, m), 6.95∼6.98(6H, m), 7.28(2H, m), 7.38(4H, m), 7.53∼7.64(22H, m), 7.71(2H, m), 7.87(4H, m), 8.02∼8.07(4H, m) | 1257.64 | 1256.60 |
| **166** | δ = 1.72(24H, s), 6.58(4H, m), 6.75(4H, m), 6.95(4H, m), 7.28(2H, m), 7.36∼7.38(4H, m), 7.49∼7.56(12H, m), 7.62∼7.64(8H, m), 7.71∼7.77(6H, m), 7.84∼7.88(8H, m) | 1257.64 | 1256.60 |
| **171** | δ = 1.72(24H, s), 6.58∼6.61(8H, m), 6.75(4H, m). 6.95∼6.99(8H, m), 7.28(2H, m), 7.38(2H, m), 7.54∼7.56(8H, m), 7.62∼7.64(8H, m), 7.71(2H, m), 7.87(4H, m) | 1193.51 | 1192.55 |
| **173** | δ = 1.72(12H, s), 6.58∼6.63(10H, m), 6.75∼6.81(6H, m), 6.95(4H, m), 7.2∼7.25(12H, m), 7.54∼7.56(6H, m), 7.62∼7.64(6H, m), 7.71 (2H, m), 7.87(2H, m) | 1001.30 | 1000.48 |
| **176** | δ = 1.72(12H, s), 2.34(24H, s), 6.36(8H, m), 6.58(2H, m), 6.71∼6.75(6H, m), 6.95(4H, m), 7.25(4H, m), 7.54∼7.56(6H, m), 7.62∼7.64(6H, m), 7.71(2H, m), 7.87(2H, m) | 1113.52 | 1112.60 |
| **181** | δ = 1.72(12H, s), 6.58∼6.63(6H, m), 6.75∼6.81(4H, m), 6.95(4H, m), 7.2∼7.26(8H, m), 7.36(2H, m), 7.49∼7.56(10H, m), 7.62∼7.64(6H, m), 7.71∼7.77(6H, m), 7.84∼7.88(6H, m) | 1101.42 | 1100.51 |
| **187** | δ = 1.35(18H, s), 1.72(24H, s), 6.55∼6.58(8H, m), 6.75(4H, m), 6.95∼7.01(8H, m), 7.25∼7.28(6H, m), 7.38(2H, m), 7.54∼7.56(8H, m), 7.62∼7.64(8H, m), 7.71(2H, m), 7.87(4H, m) | 1345.84 | 1344.73 |
| **194** | δ = 1.72(12H, s), 6.58∼6.63(10H, m), 6.75∼6.81(6H, m), 6.95(4H, m), 7.2(8H, m), 7.54∼7.55(4H, m), 7.62(2H, m), 7.71(2H, m), 7.87∼7.93(6H, m) | 899.17 | 898.43 |
| **198** | δ = 1.35(36H, s), 1.72(12H, s), 6.55∼6.58(10H, m), 6.75(2H, m), 6.95∼7.01(12H, m), 7.54∼7.55(4H, m), 7.62(2H, m), 7.71 (2H, m), 7.87∼7.93(6H, m) | 1123.60 | 1122.68 |
| **199** | δ = 1.72(12H, s), 6.58∼6.62(6H, m), 6.7∼6.75(6H, m), 6.95(4H, m), 7.54∼7.55(8H, m), 7.62(2H, m), 7.71(2H, m), 7.87∼7.93(6H, m), 8.07(4H, m) | 903.12 | 902.41 |
| **203** | δ = 1.72(24H, s), 6.58∼6.63(8H, m), 6.75∼6.81(6H, m), 6.95(4H, m), 7.2(4H, m), 7.28(2H, m), 7.38(2H, m), 7.54∼7.55(6H, m), 7.62(4H, m), 7.71(2H, m), 7.87∼7.93(8H, m) | 1131.49 | 1130.55 |
| **212** | δ = 1.72(12H, s), 2.34(24H, s), 6.36(8H, m), 6.58(2H, m), 6.71∼6.75(6H, m), 6.95(4H, m), 7.54∼7.55(4H, m), 7.62(2H, m), 7.71(2H, m), 7.78(2H, m), 7.87∼7.9(4H, m) | 1011.38 | 1010.55 |
| **220** | δ = 1.72(12H, s), 6.58∼6.63(6H, m), 6.69∼6.81(8H, m), 6.95(4H, m), 7.2(4H, m), 7.41(2H, m), 7.51∼7.55(16H, m), 7.62(2H, m), 7.71(2H, m), 7.78(2H, m), 7.87∼7.9(4H, m) | 1051.36 | 1050.49 |
| **223** | δ = 1.72(12H, s), 6.58∼6.63(10H, m), 6.75∼6.81(6H, m), 6.95(4H, m), 7.2(8H, m), 7.54∼7.62(6H, m), 7.71(2H, m), 7.8(2H, m), 7.87(2H, m), 7.95(2H, m) | 899.17 | 898.43 |
| **231** | δ = 1.35(18H, s), 1.72(12H, s), 6.55∼6.63(10H, m), 6.75∼6.81(4H, m), 6.95∼7.01(8H, m), 7.2(4H, m), 7.54∼7.62(6H, m), 7.71(2H, m), 7.8(2H, m), 7.87(2H, m), 7.95(2H, m) | 1011.38 | 1010.55 |
| **237** | δ = 1.35(18H, s), 1.72(12H, s), 6.55∼6.58(6H, m), 6.75(2H, m), 6.95∼7.01(10H, m), 7.38(2H, m), 7.53∼7.62(12H, m), 7.71(2H, m), 7.8(2H, m), 7.87(2H, m), 7.95(2H, m), 8.02∼8.07(4H, m) | 1111.50 | 1110.59 |
| **238** | δ = 1.72(12H, s), 6.58∼6.63(10H, m), 6.75∼6.81(6H, m), 6.95(4H, m), 7.2(8H, m), 7.54(2H, m), 7.62(2H, m), 7.71(4H, m), 7.87(2H, m), 8.04(2H, m), 8.28(2H, m), 8.93(2H, m) | 949.23 | 948.44 |
| **242** | δ = 1.72(12H, s), 2.34(12H, s), 6.44(4H, m), 6.55∼6.59(10H, m), 6.75(2H, m), 6.95(4H, m), 7.08(4H, m), 7.54(2H, m), 7.62(2H, m), 7.71(4H, m), 7.87(2H, m), 8.04(2H, m), 8.28(2H, m), 8.93(2H, m) | 1005.33 | 1004.51 |
| **251** | δ = 1.35(18H, s), 1.72(12H, s), 6.55∼6.63(10H, m), 6.75∼6.81(4H, m), 6.95∼7.01(8H, m), 7.2(4H, m), 7.54(2H, m), 7.62(2H, m), 7.71(4H, m), 7.87(2H, m), 8.04(2H, m), 8.28(2H, m), 8.93(2H, m) | 1061.44 | 1060.57 |
| **256** | δ = 1.72(12H, s), 6.58(2H, m), 6.75(2H, m), 6.95∼6.98(8H, m), 7.38(4H, m), 7.53∼7.62(16H, m), 7.71(6H, m), 7.87(2H, m), 7.98∼8.07(12H, m) | 1173.48 | 1172.51 |
| **265** | δ = 1.72(12H, s), 6.58∼6.63(10H, m), 6.75∼6.81(6H, m), 6.95(4H, m), 7.2(8H, m), 7.39(4H, m), 7.54∼7.55(4H, m), 7.62(2H, m), 7.71 (2H, m), 7.87∼7.91(6H, m) | 1023.31 | 1022.46 |
| **270** | δ = 1.72(36H, s), 6.58(6H, m), 6.75(6H, m), 6.95(4H, m), 7.28(4H, m), 7.38∼7.39(8H, m), 7.54∼7.55(8H, m), 7.62(6H, m), 7.71(2H, m), 7.87∼7.91(10H, m) | 1487.95 | 1486.71 |
| **275** | δ = 1.72(12H, s), 6.58(2H, m), 6.69∼6.75(6H, m), 6.95(4H, m), 7.36∼7.41(8H, m), 7.49∼7.55(20H, m), 7.62(2H, m), 7.71∼7.77(6H, m), 7.84∼7.91 (10H, m) | 1275.62 | 1274.55 |
| **280** | δ = 1.72(12H, s), 6.58∼6.63(10H, m), 6.75∼6.81(6H, m), 6.95(4H, m), 7.2(8H, m), 7.54(2H, m), 7.62(2H, m), 7.71(2H, m), 7.85∼7.87(4H, m), 8.04(2H, m), 8.28(2H, m), 8.52(2H, m), 8.93(2H, m) | 999.29 | 998.46 |
| **289** | δ = 1.72(12H, s), 6.58(2H, m), 6.69∼6.75(10H, m), 6.95(4H, m), 7.41(4H, m), 7.51∼7.54(26H, m), 7.62(2H, m), 7.71 (2H, m), 7.85∼7.87(4H, m), 8.04(2H, m), 8.28(2H, m), 8.52(2H, m), 8.93(2H, m) | 1303.67 | 1302.59 |
| **296** | δ = 1.72(12H, s), 2.34(6H, s), 6.44(2H, m), 6.55∼6.63(10H, m), 6.75∼6.81(4H, m), 6.95(4H, m), 7.08(2H, m), 7.2(4H, m), 7.54(2H, m), 7.62(2H, m), 7.71(2H, m), 7.85∼7.87(4H, m), 8.04(2H, m), 8.28(2H, m), 8.52(2H, m), 8.93(2H, m) | 1027.34 | 1026.49 |
| **299** | δ = 1.35(18H, s), 1.72(12H, s), 6.55∼6.63(10H, m), 6.75∼6.81(4H, m), 6.95∼7.01(8H, m), 7.2(4H, m), 7.54(2H, m), 7.62(2H, m), 7.71(2H, m), 7.85∼7.87(4H, m), 8.04(2H, m), 8.28(2H, m), 8.52(2H, m), 8.93(2H, m) | 1111.50 | 1110.59 |
| **305** | δ = 1.72(12H, s), 6.58∼6.63(10H, m), 6.75∼6.81(6H, m), 6.95(4H, m), 7.2(8H, m), 7.43(2H, m), 7.54(2H, m), 7.62(2H, m), 7.71(2H, m), 7.87(2H, m), 7.98∼7.99(4H, m) | 955.26 | 954.40 |
| **309** | δ = 1.72(12H, s), 2.34(12H, s), 6.44(4H, m), 6.55∼6.59(10H, m), 6.75(2H, m), 6.95(4H, m), 7.08(4H, m), 7.43(2H, m), 7.54(2H, m), 7.62(2H, m), 7.71(2H, m), 7.87(2H, m), 7.98∼7.99(4H, m) | 1011.36 | 1010.46 |
| **315** | δ = 1.35(36H, s), 1.72(12H, s), 6.55∼6.58(10H, m), 6.75(2H, m), 6.95∼7.01(12H, m), 7.43(2H, m), 7.54(2H, m), 7.62(2H, m), 7.71(2H, m), 7.87(2H, m), 7.98∼7.99(4H, m) | 1179.68 | 1178.65 |
| **320** | δ = 0.66(6H, s), 1.72(12H, s), 6.58(2H, m), 6.75(2H, m), 6.95(4H, m), 7.36(4H, m), 7.49∼7.56(14H, m), 7.62(2H, m), 7.71∼7.77(10H, m), 7.84∼7.88(10H, m), 8.17(2H, m) | 1181.58 | 1180.52 |
| **326** | δ = 0.66(6H, s), 1.72(12H, s), 6.58∼6.63(6H, m), 6.75∼6.81(4H, m), 6.95∼6.98(6H, m), 7.2(4H, m), 7.38(2H, m), 7.52∼7.62(14H, m), 7.71(2H, m), 7.87(2H, m), 8.02∼8.07(4H, m), 8.17(2H, m) | 1081.46 | 1080.48 |
| **330** | δ = 1.72(12H, s), 6.58(2H, m), 6.75(2H, m), 6.95(4H, m), 7.08(1H, m), 7.36(4H, m), 7.45∼7.63(18H, m), 7.7∼7.77(11H, m), 7.84∼7.88(10H, m), 7.94(1H. m). 8.46(1H, m), 8.59(1H, m) | 1214.54 | 1213.53 |
| **337** | δ = 1.72(12H, s), 2.34(6H, s), 6.51(4H, m), 6.58∼6.63(6H, m), 6.75∼6.81(4H, m), 6.95∼6.98(8H, m), 7.08(1H, m), 7.2(4H, m), 7.45∼7.63(10H, m), 7.7∼7.71(3H, m), 7.87(2H, m), 7.94(1H, m), 8.46(1H, m), 8.59(1H, m) | 1042.35 | 1041.50 |
| **340** | δ = 1.72(12H, s), 6.58(2H, m), 6.75(2H, m), 6.95(4H, m), 7.36∼7.42(8H, m), 7.49∼7.54(10H, m), 7.62(2H, m), 7.71∼7.78(25H, m), 8.4(2H, m) | 1231.50 | 1230.50 |
| **345** | δ = 1.72(12H, s), 6.58∼6.63(6H, m), 6.75∼6.81(4H, m), 6.95(4H, m), 7.2(4H, m), 7.36∼7.42(6H, m), 7.49∼7.54(6H, m), 7.62(2H, m), 7.71∼7.78(17H, m), 8.4(2H, m) | 1131.39 | 1130.47 |
| **349** | δ = 1.72(12H, s), 3.81 (2H, s), 6.44(2H, m), 6.58∼6.63(12H, m), 6.75∼6.81(7H, m), 6.95∼6.98(6H, m), 7.07(2H, m), 7.2(10H, m), 7.54(2H, m), 7.62(2H, m), 7.71(2H, m), 7.87(2H, m) | 1028.33 | 1027.49 |
| **350** | δ = 1.72(18H, s), 6.48(2H, m), 6.58∼6.63(12H, m), 6.75∼6.81(7H, m), 6.95(4H, m), 7.05(2H, m), 7.11(2H, m), 7.2(10H, m), 7.54(2H, m), 7.62(2H, m), 7.71(2H, m), 7.87(2H, m) | 1056.38 | 1055.52 |
| **351** | δ = 1.72(12H, s), 6.58∼6.63(12H, m), 6.75∼6.84(9H, m), 6.91∼6.95(6H, m), 7.16∼7.2(12H, m), 7.54(2H, m), 7.62(2H, m), 7.71 (2H, m), 7.87(2H, m) | 1046.37 | 1045.44 |
| **352** | δ = 1.72(12H, s), 6.52(2H, m), 6.58∼6.63(12H, m), 6.75∼6.81(7H. m), 6.89(2H, m), 6.95(4H, m), 7.17∼7.2(12H, m), 7.54(2H, m), 7.62(2H, m), 7.71(2H, m), 7.87(2H, m) | 1030.30 | 1029.47 |
| **353** | δ = 1.3(4H, m), 1.45(4H, m), 1.72(12H, s), 6.58∼6.66(14H, m), 6.75∼6.81(7H, m), 6.95∼6.96(6H, m), 7.2∼7.21(12H, m), 7.54(2H, m), 7.62(2H, m), 7.71(2H, m), 7.87(2H, m) | 1098.49 | 1097.51 |
| **354** | δ = 1.72(12H, s), 6.31(2H, m), 6.38(2H, m), 6.58∼6.63(14H, m), 6.75∼6.81(8H, m), 6.95(4H, m), 7.13(2H, m), 7.2(12H, m), 7.54(2H, m), 7.62(2H, m), 7.71 (2H, m), 7.87(2H, m) | 1105.41 | 1104.51 |
| **355** | δ = 1.72(12H, s), 2.88(4H, m), 6.51(2H, m), 6.58∼6.63(12H, m), 6.75∼6.81(7H, m), 6.95(4H, m), 7.04(2H, m), 7.14(2H, m), 7.2(10H, m), 7.54(2H, m), 7.62(2H, m), 7.71 (2H, m), 7.87(2H, m) | 1042.35 | 1041.50 |
| **356** | δ = 1.72(12H, s), 6.56∼6.63(14H, m), 6.75∼6.86(9H, m), 6.95∼6.99(6H, m), 7.2∼7.25(12H, m), 7.54(2H, m), 7.62(2H, m), 7.71(2H, m), 7.87(2H, m) | 1040.34 | 1039.49 |
| **357** | δ = 1.72(12H, s), 6.58∼6.63(10H, m), 6.75∼6.81 (6H, m), 6.95(4H, m), 7.2(8H, m), 7.54(2H, m), 7.62(2H, m), 7.71(2H, m), 7.82∼7.88(6H, m), 8.12(2H, m), 8.48(2H, s), 8.93(2H, m) | 999.29 | 998.46 |
| **358** | δ = 1.72(12H, s), 6.58∼6.63(10H, m), 6.75∼6.81(6H, m), 6.95(4H, m), 7.2(8H, m), 7.39(4H, m), 7.54∼7.56(6H, m), 7.62∼7.64(6H, m), 7.71(2H, m), 7.87∼7.91(6H, m) | 1101.42 | 1100.51 |
| **359** | δ = 1.72(12H, s), 6.58∼6.63(10H, m), 6.75∼6.81(6H, m), 6.95(4H, m), 7.2(8H, m), 7.39(4H, m), 7.54(2H, m), 7.62(2H, m), 7.71 (2H, m), 7.87∼7.91(6H, m) | 649.23 | 648.44 |
| **360** | δ = 1.72(18H, s), 6.58∼6.63(10H, m), 6.75∼6.81(6H, m), 6.95(4H, m), 7.2(8H, m), 7.54(4H, m), 7.62(2H, m), 7.71(4H, m), 7.87(4H, m) | 965.27 | 964.48 |
| **364** | δ = 1.72(18H, s), 2.34(12H, s), 6.44(4H, m), 6.55∼6.59(10H, m), 6.75(2H, m), 6.95(4H, m), 7.08(4H, m), 7.54(4H, m), 7.62(2H, m), 7.71(4H, m), 7.87(4H, m) | 1021.38 | 1020.54 |
| **368** | δ = 1.72(18H, s), 6.58∼6.61(10H, m), 6.75(2H, m), 6.95∼6.99(12H, m), 7.54(4H, m), 7.62(2H, m), 7.71(4H, m), 7.87(4H, m) | 1037.23 | 1036.44 |
| **373** | δ = 1.72(18H, s), 6.58(2H, m), 6.75(2H, m), 6.91∼6.95(8H, m), 7.54(4H, m), 7.62(2H, m), 7.71(4H, m), 7.82∼7.88(20H, m), 8.12(8H, m), 8.93(8H, m) | 1365.74 | 1364.60 |
| **378** | δ = 0.25(36H, s), 1.72(18H, s), 6.58∼6.61(10H, m), 6.75(2H, m), 6.95(4H, m), 7.15(8H, m), 7.54(4H, m), 7.62(2H, m), 7.71(4H, m), 7.87(4H, m) | 1254.00 | 1252.63 |
| **381** | δ = 1.72(18H, s), 6.58(2H, m), 6.75(2H, m), 6.95(4H, m), 7.27(4H, m), 7.36(4H, m), 7.54(4H, m), 7.62(2H, m), 7.71(4H, m), 7.87(4H, m), 8.04∼8.09(8H, m) | 969.22 | 968.46 |
| **389** | δ = 1.72(18H, s), 2.34(12H, s), 6.36(4H, m), 6.58∼6.63(6H, m), 6.71∼6.81(6H, m), 6.95(4H, m), 7.2(4H, m), 7.54(4H, m), 7.62(2H, m), 7.71(4H, m), 7.87(4H, m) | 1021.38 | 1020.54 |
| **395** | δ = 1.35(18H, s), 1.72(18H, s), 6.55∼6.63(10H, m), 6.75∼6.81(4H, m), 6.95∼7.01(8H, m), 7.2(4H, m), 7.54(4H, m), 7.62(2H, m), 7.71 (4H, m), 7.87(4H, m) | 1077.48 | 1076.60 |
| **404** | δ = 1.72(30H, s), 6.58∼6.63(8H, m), 6.75∼6.81(6H, m), 6.95(4H, m), 7.2(4H, m), 7.28(2H, m), 7.38(2H, m), 7.54∼7.55(6H, m), 7.62(4H, m), 7.71(4H, m), 7.87(6H, m) | 1197.59 | 1196.60 |
| **411** | δ = 1.72(18H, s), 6.58∼6.63(6H, m), 6.75∼6.86(6H, m), 6.95(4H, m), 7.2(4H, m), 7.4(2H, m), 7.5∼7.54(8H, m), 7.62(2H, m), 7.71∼7.73(6H, m), 7.87(4H, m), 7.98(2H, m), 8.45(2H, m) | 1177.56 | 1176.45 |
| **415** | δ = 1.72(30H, s), 6.58∼6.63(6H, m), 6.69∼6.81(8H, m), 6.95(4H, m), 7.2(4H, m), 7.28(2H, m), 7.38(2H, m), 7.54∼7.55(10H, m), 7.62∼7.63(4H, m), 7.71∼7.77(6H, m), 7.87∼7.93(8H, m) | 1349.78 | 1348.66 |
| **422** | δ = 1.35(18H, s), 1.72(18H, s), 2.34(12H, s), 6.36(4H, m), 6.55∼6.58(6H, m), 6.71∼6.75(4H, m), 6.95∼7.01(8H, m), 7.54(4H, m), 7.62(2H, m), 7.71 (4H, m), 7.87(4H, m) | 1133.59 | 1132.66 |
| **428** | δ = 1.72(18H, s), 2.34(12H, s), 6.36(4H, m), 6.58(2H, m), 6.69∼6.75(8H, m), 6.95(4H, m), 7.41(2H, m), 7.51∼7.54(16H, m), 7.62(2H, m), 7.71(4H, m), 7.87(4H, m) | 1173.57 | 1172.60 |
| **435** | δ = 1.72(18H, s), 2.34(12H, s), 6.36(4H, m), 6.58(2H, m), 6.71∼6.75(4H, m), 6.91∼6.95(6H, m), 7.54(4H, m), 7.62(2H, m), 7.71(4H, m), 7.82∼7.88(12H, m), 8.12(4H, m), 8.93(4H, m) | 1221.61 | 1220.60 |
| **447** | δ = 1.35(18H, s), 1.72(18H, s), 6.55∼6.58(6H, m), 6.75(2H, m), 6.95∼7.01(8H, m), 7.36(2H, m), 7.49∼7.54(8H, m), 7.62(2H, m), 7.71∼7.77(8H, m), 7.84∼7.88(8H, m) | 1177.60 | 1176.63 |
| **454** | δ = 1.72(18H, s), 6.58(2H, m), 6.69∼6.75(6H, m), 6.95(4H, m), 7.36∼7.41(4H, m), 7.49∼7.54(20H, m), 7.62(2H, m), 7.71∼7.77(8H, m), 7.84∼7.88(8H, m) | 1217.58 | 1216.57 |
| **457** | δ = 1.72(30H, s), 6.58(4H, m), 6.75(4H, m), 6.95(4H, m), 7.28(2H, m), 7.36∼7.38(4H, m), 7.49∼7.55(10H, m), 7.62(4H, m), 7.71∼7.77(8H, m), 7.84∼7.88(10H, m) | 1297.71 | 1296.63 |
| **458** | δ = 1.72(18H, s), 6.58(2H, m), 6.75(2H, m), 6.91∼6.95(6H, m), 7.36(2H, m), 7.49∼7.54(8H, m), 7.62(2H, m), 7.71∼7.88(24H, m), 8.12(4H, m), 8.93(4H, m) | 1265.62 | 1264.57 |
| **466** | δ = 1.72(18H, s), 6.58(2H, m), 6.69∼6.75(6H, m), 6.95(4H, m), 7.36(2H, m), 7.49∼7.62(20H, m), 7.71∼7.77(10H, m), 7.84∼7.92(10H, m), 8(4H, m) | 1317.70 | 1316.60 |
| **472** | δ = 1.35(18H, s), 1.72(18H, s), 2.34(6H, s), 6.51∼6.58(10H, m), 6.75(2H, m), 6.95∼7.01(12H, m), 7.54(4H, m), 7.62(2H, m), 7.71(4H, m), 7.87(4H, m) | 1105.54 | 1104.60 |
| **483** | δ = 1.72(30H, s), 2.34(6H, s), 6.51(4H, m), 6.58(4H, m), 6.75(4H, m), 6.95∼6.98(8H, m), 7.28(2H, m), 7.38(2H, m), 7.54∼7.55(6H, m), 7.62(4H, m), 7.71(4H, m), 7.87(6H, m) | 1225.64 | 1224.63 |
| **497** | δ = 1.35(18H, s), 1.72(18H, s), 2.34(6H, s), 6.44(2H, m), 6.55∼6.59(10H, m), 6.75(2H, m), 6.95∼7.01(8H, m), 7.08(2H, m), 7.54(4H, m), 7.62(2H, m), 7.71(4H, m), 7.87(4H, m) | 1105.54 | 1104.63 |
| **510** | δ = 1.72(18H, s), 2.34(6H, s), 6.44(2H, m), 6.55∼6.59(6H, m), 6.75(2H, m), 6.91∼6.95(6H, m), 7.08(2H, m), 7.54(4H, m), 7.62(2H, m), 7.71(4H, m), 7.82∼7.88(12H, m), 8.12(4H, m), 8.93(4H, m) | 1193.56 | 1192.57 |
| **521** | δ = 1.72(18H, s), 2.12(6H, s), 6.51(2H, m), 6.58∼6.61(6H, m), 6.69∼6.75(4H, m), 6.95∼7.01(10H, m), 7.15(2H, m), 7.54(4H, m), 7.62(2H, m), 7.71(4H, m), 7.87(4H, m) | 1029.30 | 1028.49 |
| **527** | δ 1.72(18H, s), 6.58∼6.61(6H, m), 6.75(2H, m), 6.95∼6.99(10H, m), 7.38(2H, m), 7.53∼7.62(12H, m), 7.71(4H, m), 7.87(4H, m), 8.02∼8.07(4H, m) | 1101.37 | 1100.49 |
| **537** | δ = 1.72(18H, s), 6.58(2H, m), 6.75(2H, m), 6.95∼6.99(10H, m), 7.38(2H, m), 7.53∼7.62(12H, m), 7.71 (4H, m), 7.87(4H, m), 8.02∼8.07(4H, m), 8.46(4H, m) | 1067.36 | 1066.50 |
| **540** | δ = 1.72(30H, s), 6.58(4H, m), 6.75(4H, m), 6.95∼6.98(6H, m), 7.28(2H, m), 7.38(4H, m), 7.53∼7.62(16H, m), 7.71(4H, m), 7.87(6H, m), 8.02∼8.07(4H, m) | 1297.71 | 1296.63 |
| **542** | δ = 1.72(18H, s), 6.58(2H, m), 6.75(2H, m), 6.91∼6.98(8H, m), 7.38(2H, m), 7.53∼7.62(12H, m), 7.71(4H, m), 7.82∼7.88(12H, m), 8.02∼8.12(8H, m), 8.93(4H, m) | 1265.62 | 1264.57 |
| **547** | δ = 1.72(30H. s), 6.58(2H, m), 6.69∼6.75(6H, m), 6.95∼6.98(6H, m), 7.28(2H, m). 7.38(4H, m), 7.53∼7.63(20H, m), 7.71∼7.77(6H, m), 7.87∼7.93(8H, m), 8.02∼8.07(4H, m) | 1449.90 | 1448.69 |
| **549** | δ = 1.35(18H. s), 1.72(18H, s), 6.55∼6.58(6H, m), 6.69∼6.75(6H, m), 6.95∼7.01(8H, m), 7.41(2H, m), 7.51∼7.54(16H, m), 7.62(2H, m), 7.71(4H, m), 7.87(4H, m) | 1229.68 | 1228.66 |
| **556** | δ = 1.35(18H, s), 1.72(30H. s), 6.55∼6.58(8H, m), 6.75(4H, m), 6.95∼7.01(8H, m), 7.28(2H, m), 7.38(2H, m), 7.54∼7.55(6H, m), 7.62(4H, m), 7.71(4H, m), 7.87(6H, m) | 1309.80 | 1308.73 |
| **567** | δ = 1.35(18H, s), 1.72(18H, s), 6.55∼6.58(6H, m), 6.75(2H, m), 6.95∼7.04(10H, m), 7.53∼7.62(16H, m), 7.71∼7.78(8H, m), 7.87∼7.92(6H, m), 8(4H, m), 8.07(2H, m), 8.49(2H, m) | 1429.91 | 1428.73 |
| **577** | δ = 1.72(30H, s), 6.58~6.61(8H, m), 6.75(4H, m), 6.95∼6.99(8H, m), 7.28(2H, m), 7.38(2H, m), 7.54∼7.55(6H, m), 7.62(4H, m), 7.71(4H, m), 7.87(6H, m) | 1233.57 | 1232.58 |
| **584** | δ 1.72(18H, s), 6.58(2H, m), 6.69∼6.75(6H, m), 6.91∼6.95(6H, m), 7.41(2H, m), 7.51∼7.54(16H, m), 7.62(2H, m), 7.71(4H, m), 7.82∼7.88(12H, m), 8.12(4H, m), 8.93(4H, m) | 1317.70 | 1316.60 |
| **588** | δ = 1.72(18H, s), 6.58(2H, m), 6.69∼6.75(10H, m), 6.95(4H, m), 7.41(2H, m), 7.51∼7.55(24H, m), 7.61∼7.62(4H, m), 7.71(4H, m), 7.87(4H, m), 8.04∼8.08(4H, m), 8.42(2H, m), 8.55(2H, m) | 1369.77 | 1368.63 |
| **598** | δ = 1.72(18H, s), 6.58∼6.62(4H, m), 6.7∼6.75(4H, m), 6.95∼6.99(8H, m), 7.54∼7.55(6H, m), 7.62(2H, m), 7.71(4H, m), 7.87(4H, m), 8.07(2H, m), 8.46(4H, m) | 969.22 | 968.46 |
| **599** | δ = 1.72(30H, s), 6.58(4H, m), 6.75(4H, m), 6.91∼6.95(6H, m), 7.28(2H, m), 7.38(2H, m), 7.54∼7.55(6H, m), 7.62(4H, m), 7.71(4H, m), 7.82∼7.88(14H, m), 8.12(4H, m), 8.93(4H, m) | 1397.83 | 1396.66 |
| **607** | δ = 1.72(18H, s), 2.34(6H, s), 6.51(4H, m), 6.58(2H, m), 6.75(2H, m), 6.91∼6.98(10H, m), 7.54(4H, m), 7.62(2H, m), 7.71(4H, m), 7.82∼7.88(12H, m), 8.12(4H, m), 8.93(4H, m) | 1193.56 | 1192.57 |
| **612** | δ = 1.72(18H, s), 6.58(2H, m), 6.75(2H, m), 6.95∼6.99(10H, m), 7.41∼7.47(4H, m), 7.54m), 7.62(8H, m), 7.71 (4H, m), 7.87(4H, m), 8.17(2H, m), 8.28(2H, m), 8.46(4H, m) | 1069.34 | 1068.49 |
| **620** | δ = 1.72(18H, s), 3.83(6H, s), 6.52(4H, m), 6.58(2H, m), 6.74∼6.81(10H, m), 6.95(4H, m), 7.39(4H, m), 7.54(4H, m), 7.62(2H, m), 7.71 (4H, m), 7.87(4H, m) | 1075.34 | 1074.49 |
| **625** | δ = 1.72(18H, s), 3.83(6H, s), 6.52(4H, m), 6.58(2H, m), 6.74∼6.81(8H, m), 6.95(4H, m), 7.54∼7.62(8H, m), 7.71∼7.78(8H, m), 7.87∼7.88(6H, m), 8.1(4H, m), 8.42(4H, m) | 1273.60 | 1272.56 |
| **632** | δ = 0.9(6H, m), 1.72(12H, s), 1.91(4H, m), 6.58∼6.63(10H, m), 6.75∼6.81(6H, m), 6.95(4H, m), 7.2(8H, m), 7.54(4H, m), 7.62(2H, m), 7.71(4H, m), 7.87(4H, m) | 993.32 | 992.51 |
| **635** | δ = 0.9(6H, m), 1.72(12H, s), 1.91 (4H, m), 2.34(12H, s), 6.51(8H, m), 6.58(2H, m), 6.75(2H, m), 6.95∼6.98(12H, m), 7.54(4H, m), 7.62(2H, m), 7.71(4H, m), 7.87(4H, m) | 1049.43 | 1048.57 |
| **643** | δ = 0.9(6H, m), 1.72(12H, s), 1.91(4H, m), 6.58∼6.62(6H, m), 6.7∼6.75(6H, m), 6.95(4H, m), 7.54∼7.55(8H, m), 7.62(2H, m), 7.71(4H, m), 7.87(4H, m), 8.07(4H, m) | 997.28 | 996.46 |
| **648** | δ = 1.72(12H, s), 6.58∼6.63(10H, m), 6.75∼6.81(6H, m), 6.95(4H, m), 7.16∼7.2(12H, m), 7.35(2H, m), 7.54(4H, m), 7.62(2H, m), 7.71∼7.75(6H, m), 7.87(4H, m) | 1087.39 | 1086.49 |
| **659** | δ = 1.72(12H, s), 6.58∼6.63(10H, m), 6.75∼6.81(6H, m), 6.95(4H, m), 7.11(4H, m), 7.2∼7.26(10H, m), 7.33(4H, m), 7.54(4H, m), 7.62(2H, m), 7.71(4H, m), 7.87(4H, m) | 1089.41 | 1088.51 |
| **663** | δ = 1.72(12H, s), 2.34(12H, s), 6.44(4H, m), 6.55∼6.59(10H, m), 6.75(2H, m), 6.95(4H, m), 7.08∼7.11(8H, m), 7.26(2H, m), 7.33(4H, m), 7.54(4H, m), 7.62(2H, m), 7.71(4H, m), 7.87(4H, m) | 1145.52 | 1144.57 |
| **670** | δ = 1.72(12H, s), 3.49(4H, s), 6.58∼6.63(10H, m), 6.75∼6.81(6H, m), 6.95(4H, m), 7.2(12H, m), 7.54(4H, m), 7.62(2H, m), 7.71(4H, m), 7.87(4H, m) | 1039.35 | 1038.49 |
| **680** | δ = 1.72(12H, s), 3.49(4H, s), 6.58(2H, m), 6.75(2H, m), 6.95~6.99(8H, m), 7.2(4H, m), 7.41∼7.47(8H, m), 7.54∼7.62(10H, m), 7.71(4H, m), 7.87(4H, m), 8.17(4H, m), 8.28(4H, m) | 1243.54 | 1242.53 |
| **681** | δ = 0.9(12H, m), 1.72(6H, s), 1.91(8H, m), 6.58∼6.63(10H, m), 6.75∼6.81(6H, m), 6.95(4H, m), 7.2(8H, m), 7.54(4H, m), 7.62(2H, m), 7.71(4H, m), 7.87(4H, m) | 1021.38 | 1020.54 |
| **686** | δ = 0.9(12H, m), 1.72(6H, s), 1.91(8H, m), 2.12(12H, s), 6.51(4H, m), 6.58(2H, m), 6.69∼6.75(6H, m), 6.95∼7.01(8H, m), 7.15(4H, m), 7.54(4H, m), 7.62(2H, m), 7.71(4H, m), 7.87(4H, m) | 1077.48 | 1076.60 |
| **698** | δ = 1.72(6H, s), 6.58∼6.63(10H, m), 6.75∼6.81(6H, m), 6.95(4H, m), 7.11(8H, m), 7.2∼7.26(12H, m), 7.33(8H, m), 7.54(4H, m), 7.62(2H, m), 7.71(4H, m), 7.87(4H, m) | 1213.55 | 1212.54 |
| **699** | δ 1.72(6H, s), 3.49(8H, s), 6.58∼6.63(10H, m), 6.75∼6.81(6H, m), 6.95(4H, m), 7.2(16H, m), 7.54(4H, m), 7.62(2H, m), 7.71(4H, m), 7.87(4H, m) | 1113.43 | 1112.51 |
| **700** | δ = 1.51(8H, m), 1.72(6H, s), 2.09(8H, m), 6.58∼6.63(10H, m), 6.75∼6.81(6H, m), 6.95(4H, m), 7.2(8H, m), 7.54(4H, m), 7.62(2H, m), 7.71(4H, m), 7.87(4H, m) | 1017.35 | 1016.51 |
| **701** | δ = 1.72(6H, s), 2.62(4H, m), 2.91(4H, m), 6.58∼6.63(10H, m), 6.75∼6.81(6H, m), 6.95(4H, m), 7.02∼7.06(4H, m), 7.15∼7.2(12H, m), 7.54(4H, m), 7.62(2H, m), 7.71(4H, m), 7.87(4H, m) | 1113.43 | 1112.51 |
| **702** | δ = 1.72(12H, s), 6.58∼6.63(10H, m), 6.75∼6.81(6H, m), 6.95(2H, m), 7.2(9H, m), 7.42∼7.43(3H, m), 7.56∼7.63(6H, m), 7.82(2H, m), 8.4(1H, m) | 850.10 | 849.41 |
| **710** | δ = 1.72(12H, s), 6.58∼6.63(10H, m), 6.75∼6.81(6H, m), 6.95∼6.99(4H, m), 7.08(2H, m), 7.2(8H, m), 7.42(2H, m), 7.59∼7.62(4H, m), 7.82(2H, m) | 855.14 | 854.37 |
| **712** | δ = 1.72(12H, s), 6.58∼6.63(6H, m), 6.75∼6.81(4H, m), 6.95(4H, m), 7.2(4H, m), 7.36(2H, m), 7.49∼7.54(10H, m), 7.62(2H, m), 7.71∼7.77(6H, m), 7.84∼7.88(6H, m) | 949.23 | 948.44 |
| **716** | δ = 1.72(12H, s), 6.58∼6.63(8H, m), 6.75∼6.81(5H, m), 6.95∼6.98(5H, m), 7.2(6H, m), 7.38(1H, m), 7.5∼7.62(11H, m), 7.71(2H, m), 7.87(2H, m), 8.02∼8.07(2H, m) | 899.17 | 898.43 |
| **717** | δ = 1.72(18H, s), 6.58∼6.63(9H, m), 6.75∼6.81(6H, m), 6.95(4H, m), 7.2(6H, m), 7.28(1H, m), 7.38(1H, m), 7.5∼7.55(7H, m), 7.62(3H, m), 7.71(2H, m), 7.87(3H, m) | 965.27 | 964.48 |
| **719** | δ 1.35(9H, s), 1.72(18H, s), 6.55∼6.58(5H, m), 6.75(3H, m), 6.95∼7.01(8H, m), 7.28(1H, m), 7.38(3H, m), 7.5∼7.62(16H, m), 7.71(2H, m), 7.87(3H, m), 8.02∼8.07(4H, m) | 1121.49 | 1120.57 |

### [Example 1] Manufacture of OLED device using organic electroluminescent compound according to the present invention

An OLED device was manufactured using the organic electroluminescent compound according to the present invention.

First, a transparent electrode ITO film (15 Ω/□) 2 prepared from a glass substrate for an OLED (Samsung Corning) 1 was subjected to ultrasonic washing sequentially using trichloroethylene, acetone, ethanol and distilled water, and stored in isopropanol for later use.

Next, the ITO substrate was mounted on a substrate holder of a vacuum deposition apparatus. After filling 4,4',4"-tris(N,N-(2-naphthyl)-phenylamino)triphenylamine (2-TNATA) of the following structure in a cell of the vacuum deposition apparatus, the pressure inside the chamber was reduced to 10⁻⁶ torr. Then, 2-TNATA was evaporated by applying electrical current to the cell. A hole injection layer 3 having a thickness of 60 nm was formed on the ITO substrate.

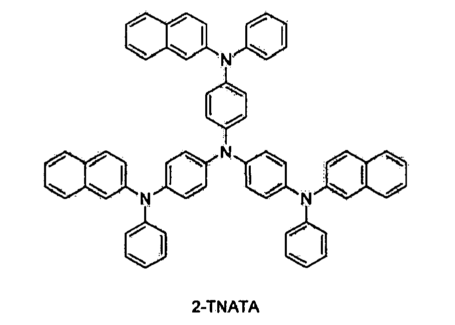

Subsequently, after filling *N,N*'-bis(a-naphthyl)-*N,N*'-diphenyl-4,4'-diamine (NPB) of the following structure in another cell of the vacuum deposition apparatus, NPB was evaporated by applying electrical current. A hole transport layer 4 having a thickness of 20 nm was formed on the hole injection layer.

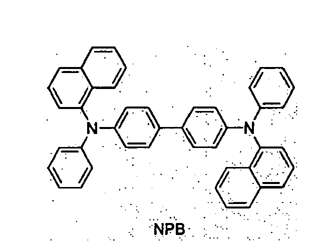

After forming the hole injection layer and the hole transport layer, an electroluminescent layer was formed thereon as follows. Compound H-32 of the following structure was filled in a cell of a vacuum deposition apparatus as a host, and Compound 1 was filled in another cell as a dopant. The two materials were evaporated at different speed, so that an electroluminescent layer 5 having a thickness of 30 nm was formed on the hole transport layer at 2 to 5 wt% based on the host.

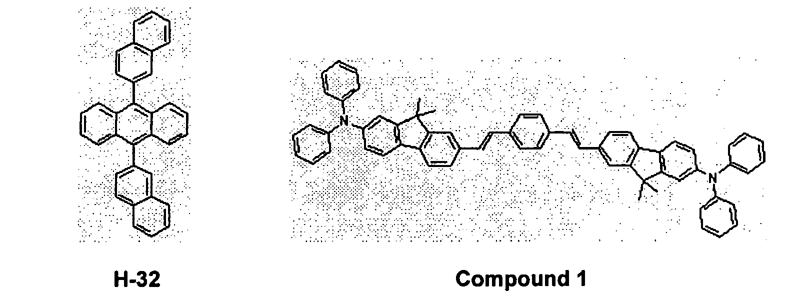

Thereafter, tris(8-hydroxyquinoline)-aluminum(lil) (Alq) of the following structure was deposited with a thickness of 20 nm as an electron transport layer 6. Then, lithium quinolate (Liq) of the following structure was deposited with a thickness of 1 to 2 nm as an electron injection layer 7. Then, an Al cathode 8 having a thickness of 150 nm was formed using another vacuum deposition apparatus to manufacture an OLED.

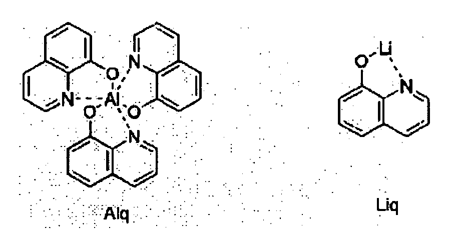

Each OLED electroluminescent material was purified by vacuum sublimation at 10⁻⁶ torr.

### [Comparative Example 1] Manufacture of OLED device using existing electroluminescent material

A hole injection layer and a hole transport layer were formed as in Example 1. Dinaphthylanthracene (DNA) was filled in a cell of a vacuum deposition apparatus as an electroluminescent host material, and Compound A of the following structure was filled in another cell as a blue-emitting electroluminescent material. An electroluminescent layer having a thickness of 30 nm was formed on the hole transport layer by setting the deposition speed at 100:1.

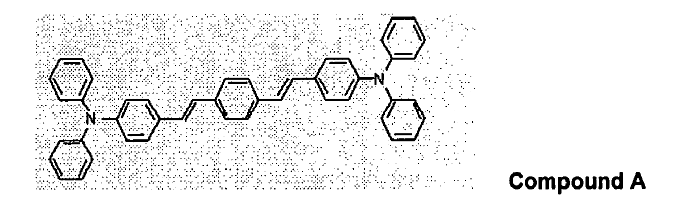

Then, an electron transport layer and an electron injection layer were formed as in Example 1, and an Al cathode having a thickness of 150 nm was formed using another vacuum deposition apparatus to manufacture an OLED.

### [Example 2] Electroluminescence characteristics of manufactured OLED devices

Luminous efficiency of the OLED devices manufactured in Example 1 and Comparative Example 1, which comprise the organic electroluminescent compounds according to the present invention and the existing electroluminescent compound respectively, was measured at 1,000 cd/m². The result is given in Table 2.

**[Table 2]**

| No. | Host | Dopant | Doping concentration (wt%) | Luminous efficiency (cd/A) | Color |
|---|---|---|---|---|---|
| | | | | @ 1000 cd/m² | |
| 1 | H-28 | 5 | 3.0 | 12.8 | Light blue |
| 2 | H-29 | 42 | 3.0 | 12.9 | Light blue |
| 3 | H-30 | 84 | 3.0 | 12.5 | Light blue |
| 4 | H-32 | 1 | 3.0 | 12.6 | Light blue |
| 5 | H-48 | 148 | 3.0 | 13.0 | Light blue |
| 6 | H-49 | 178 | 3.0 | 11.5 | Blue |
| 7 | H-89 | 212 | 3.0 | 11.8 | Blue |
| 8 | H-104 | 255 | 3.0 | 12.6 | Light blue |
| 9 | H-116 | 274 | 3.0 | 12.8 | Light blue |
| 10 | H-124 | 289 | 3.0 | 13.2 | Light blue |
| 11 | H-127 | 315 | 3.0 | 13.0 | Light blue |
| 12 | H-134 | 360 | 3.0 | 12.9 | Light blue |
| 13 | H-136 | 404 | 3.0 | 12.7 | Light blue |
| 14 | H-139 | 542 | 3.0 | 12.6 | Light blue |
| Comp. Ex. 1 | DNA | Compound A | 3.0 | 12.4 | Jade green |

As seen in Table 2, when the compounds according to the present invention were used to manufacture blue-emitting electroluminescent devices, color purity was significantly improved as light blue to blue, as compared to jade green, while the luminous efficiency was maintained comparable or better.

While the exemplary embodiments have been shown and described, it will be understood by those skilled in the art that various changes in form and details may be made thereto without departing from the spirit and scope of this disclosure as defined by the appended claims.

In addition, many modifications can be made to adapt a particular situation or material to the teachings of this disclosure without departing from the essential scope thereof. Therefore, it is intended that this disclosure not be limited to the particular exemplary embodiments disclosed as the best mode contemplated for carrying out this disclosure, but that this disclosure will include all embodiments falling within the scope of the appended claims.

## Claims

1. An organic electroluminescent compound represented by Chemical Formula 1: 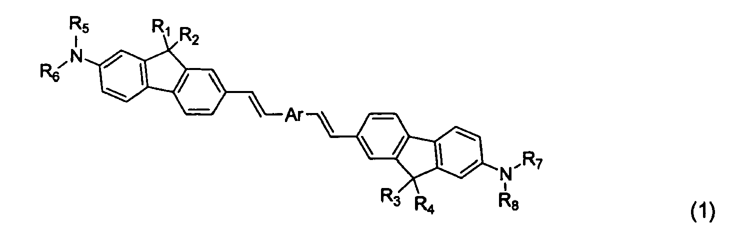 In Chemical Formula 1,
Ar represents (C6-C60)arylene, (C3-C60)heteroarylene, 5- or 6-membered heterocycloalkylene containing one or more heteroatom(s) selected from N, O and S, (C3-C60)cycloalkylene, adamantylene, (C7-C60)bicycloalkylene, (C2-C60)alkenylene, (C2-C60)alkynylene, (C1-C60)alkylenethio, (C1-C60)alkyleneoxy, (C6-C60)aryleneoxy, (C6-C60)arylenethio or 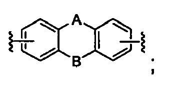
A and B independently represent a chemical bond, -(CR₁₁)(R₁₂)ₘ)-, -N(R₁₃)-, -S-, -O-, -Si(R₁₄)(R₁₅)-, -P(R₁₆)-, -C(=O)-, -B(R₁₇)-, -In(R₁₈)-, -Se-, -Ge(R₁₉)(R₂₀)-, -Sn(R₂₁)(R₂₂)-, -Ga(R₂₃)-or -(R₂₄)C=C(R₂₅)-, except for the case where A and B are chemical bonds at the same time;
R₁ through R₈ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁ and R₂, R₃ and R₄, R₅ and R₆, and R₇ and R₈ may be linked via (C3-C60)alkylene or (C3-C60)alkenylene with or without a fused ring to form an alicyclic ring or a mono- or polycyclic aromatic ring;
R₁₁ through R₂₅ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(Cl-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁₁ and R₁₂, R₁₄ and R₁₅, R₁₉ and R₂₀, R₂₁ and R₂₂, and R₂₄ and R₂₅ may be linked via (C3-C60)alkylene or (C3-C60)alkenylene with or without a fused ring to form an alicyclic ring or a mono- or polycyclic aromatic ring;
the arylene, heteroarylene, heterocycloalkylene, cycloalkylene, adamantylene, bicycloalkylene, alkenylene, alkylenethio, alkyleneoxy, aryleneoxy or arylenethio of Ar, and the alkyl, aryl, heteroaryl, heterocycloalkyl, cycloalkyl, trialkylsilyl, dialkylarylsilyl, triarylsilyl, adamantyl, bicycloalkyl, alkenyl, aralkyl, alkyloxy, alkylthio, aryloxy, arylthio, alkylamino, arylamino, alkoxycarbonyl, alkylcarbonyl or arylcarbonyl of R₁ through R₈ and R₁₁ through R₂₅ may be further substituted by one or more substituent(s) selected from a group consisting of deuterium, halogen, (C1-C60)alkyl with or without halogen substituent(s), (C6-C60)aryl, (C3-C60)heteroaryl containing one or more heteroatom(s) selected from N, O and S with or without (C6-C60)aryl substituent(s), morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl; and
m represents an integer from 1 to 3.

2. The organic electroluminescent compound according to claim 1, wherein Ar is selected from the following structures: 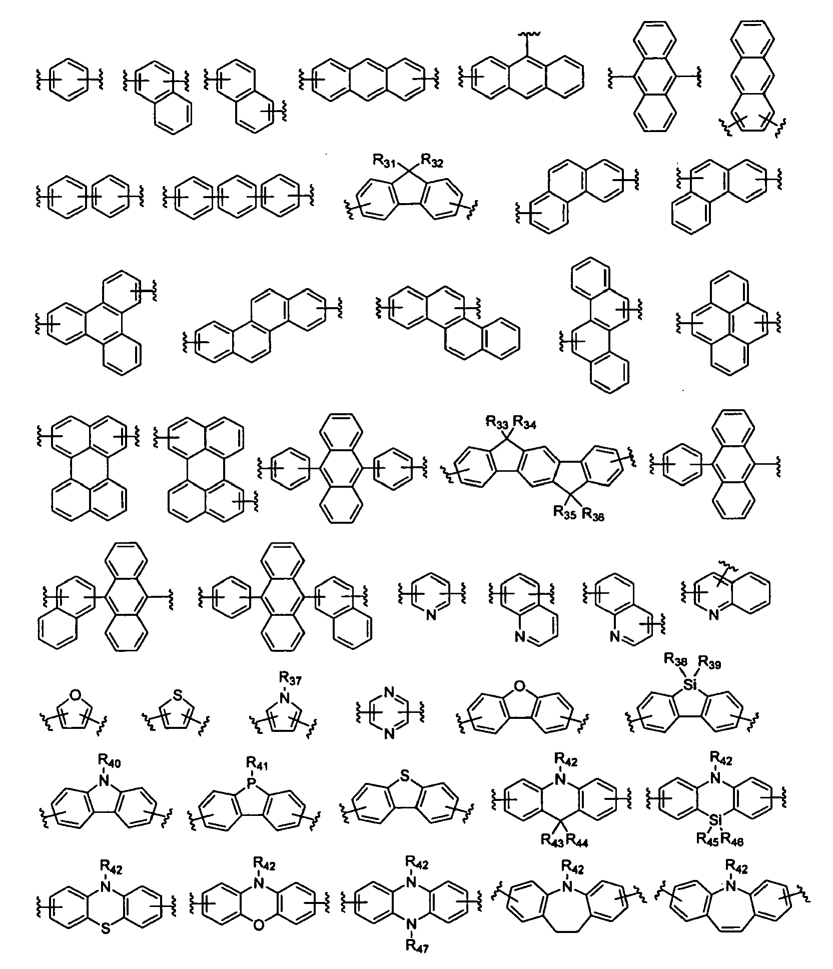 wherein
R₃₁ through R₄₂ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₃₁ and R₃₂, R₃₃ and R₃₄, R₃₅ and R₃₆, R₃₈ and R₃₉, R₄₃ and R₄₄, and R₄₅ and R₄₆ may be linked via (C3-C60)alkylene or (C3-C60)alkenylene with or without a fused ring to form an alicyclic ring or a mono- or polycyclic aromatic ring; and
the alkyl, aryl, heteroaryl, heterocycloalkyl, cycloalkyl, trialkylsilyl, dialkylarylsilyl, triarylsilyl, adamantyl, bicycloalkyl, alkenyl, aralkyl, alkyloxy, alkylthio, aryloxy, arylthio, alkylamino, arylamino, alkoxycarbonyl, alkylcarbonyl or arylcarbonyl of R₃₁ through R₄₂ may be further substituted by one or more substituent(s) selected from a group consisting of deuterium, halogen, (C1-C60)alkyl with or without halogen substituent(s), (C6-C60)aryl, (C3-C60)heteroaryl containing one or more heteroatom(s) selected from N, O and S with or without (C6-C60)aryl substituent(s), morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyi, adamantyl, (C7-C60)bicycloalkyl, (C2-C60)alkenyl, (C2-C60)alkynyl, cyano, carbazolyl, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl.

3. The organic electroluminescent device which comprises a first electrode; a second electrode; and one or more organic layer(s) interposed between the first electrode and the second electrode, wherein the organic layer comprises an organic electroluminescent compound represented by Chemical Formula 1: 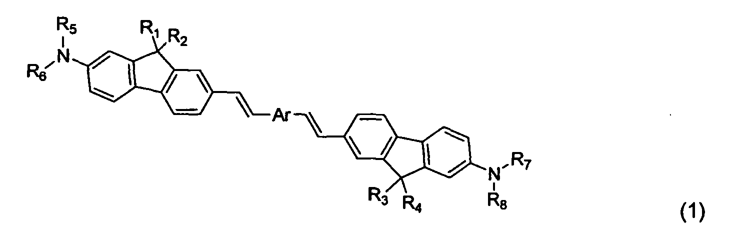 In Chemical Formula 1,
Ar represents (C6-C60)arylene, (C3-C60)heteroarylene, 5- or 6-membered heterocycloalkylene containing one or more heteroatom(s) selected from N, O and S, (C3-C60)cycloalkylene, adamantylene, (C7-C60)bicycloalkylene, (C2-C60)alkenylene, (C2-C60)alkynylene, (C1-C60)alkylenethio, (C1-C60)alkyleneoxy, (C6-C60)aryleneoxy, (C6-C60)arylenethio or 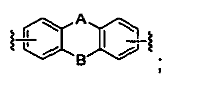
A and B independently represent a chemical bond, -(CR₁₁)(R₁₂)ₘ-, -N(R₁₃)-, -S-, **-**O**-,** -Si(R₁₄)(R₁₅)-, -P(R₁₆)-, -C(=O)-, -B(R₁₇)-, -In(R₁₈)-, -Se-, -Ge(R₁₉)(R₂₀)-, -Sn(R₂₁)(R₂₂)-, -Ga(R₂₃)-or -(R₂₄)C=C(R₂₅)-, except for the case where A and B are chemical bonds at the same time;
R₁ through R₈ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁ and R₂, R₃ and R₄, R₅ and R₆, and R₇ and R₈ may be linked via (C3-C60)alkylene or (C3-C60)alkenylene with or without a fused ring to form an alicyclic ring or a mono- or polycyclic aromatic ring;
R₁₁ through R₂₅ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁₁ and R₁₂, R₁₄ and R₁₅, R₁₉ and R₂₀, R₂₁ and R₂₂, and R₂₄ and R₂₅ may be linked via (C3-C60)alkylene or (C3-C60)alkenylene with or without a fused ring to form an alicyclic ring or a mono- or polycyclic aromatic ring;
the arylene, heteroarylene, heterocycloalkylene, cycloalkylene, adamantylene, bicycloalkylene, alkenylene, alkylenethio, alkyleneoxy, aryleneoxy or arylenethio of Ar, and the alkyl, aryl, heteroaryl, heterocycloalkyl, cycloalkyl, trialkylsilyl, dialkylarylsilyl, triarylsilyl, adamantyl, bicycloalkyl, alkenyl, aralkyl, alkyloxy, alkylthio, aryloxy, arylthio, alkylamino, arylamino, alkoxycarbonyl, alkylcarbonyl or arylcarbonyl of R₁ through R₈ and R₁₁ through R₂₅ may be further substituted by one or more substituent(s) selected from a group consisting of deuterium, halogen, (C1-C60)alkyl with or without halogen substituent(s), (C6-C60)aryl, (C3-C60)heteroaryl containing one or more heteroatom(s) selected from N, O and S with or without (C6-C60)aryl substituent(s), morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl; and
m represents an integer from 1 to 3 and one or more host(s) selected from the compounds represented by Chemical Formula 2 or 3:
(Ar₁₁)ₐ-X-(Ar₁₂)_{b} (2)
(Ar₁₃)_{c}-Y-(Ar₁₄)_{d} (3)
wherein
X represents (C6-C60)arylene or (C4-C60)heteroarylene;
Y represents anthracenylene;
Ar₁₁ through Ar₁₄ independently represent hydrogen, deuterium, (C1-C60)alkyl, (C1-C60)alkoxy, halogen, (C4-C60)heteroaryl containing one or more heteroatom(s) selected from N, O and S, (C5-C60)cycloalkyl or (C6-C60)aryl, and the cycloalkyl, aryl or heteroaryl of Ar₁₁ through Ar₁₄ may be further substituted by one or more substituent(s) selected from a group consisting of (C6-C60)aryl with or without one or more substituent(s) selected from a group consisting of (C1-C60)alkyl with or without halogen substituent(s), (C1-C60)alkoxy, (C3-C60)cycloalkyl, deuterium, halogen, cyano, tri(C1-C60)alkylsilyl, di(C1-C60)alkyl(C6-C60)arylsilyl and tri(C6-C60)arylsilyl, (C4-C60)heteroaryl containing one or more heteroatom(s) selected from N, O and S, (C1-C60)alkyl with or without halogen substituent(s), (C1-C60)alkoxy, (C3-C60)cycloalkyl, deuterium, halogen, cyano, tri(C1-C60)alkylsilyl, di(C1-C60)alkyl(C6-C60)arylsilyl and tri(C6-C60)arylsilyi; and
a, b c and d independently represent an integer from 0 to 4.

4. The organic electroluminescent device according to claim 3, wherein the organic layer comprises one or more compound(s) selected from a group consisting of arylamine compounds and styrylarylamine compounds.

5. The organic electroluminescent device according to claim 3, wherein the organic layer further comprises one or more metal(s) selected from a group consisting of organic metals of Group 1, Group 2, 4th period and 5th period transition metals, lanthanide metals and d-transition elements.

6. The organic electroluminescent device according to claim 3, wherein the organic layer comprises an electroluminescent layer and a charge generating layer.

7. The organic electroluminescent device according to claim 3, wherein a mixed region of a reductive dopant and an organic substance or a mixed region of an oxidative dopant and an organic substance is placed on the inner surface of one or both electrode(s) among the pair to f electrodes.

8. An organic solar cell comprising the organic electroluminescent compound represented by Chemical Formula 1: 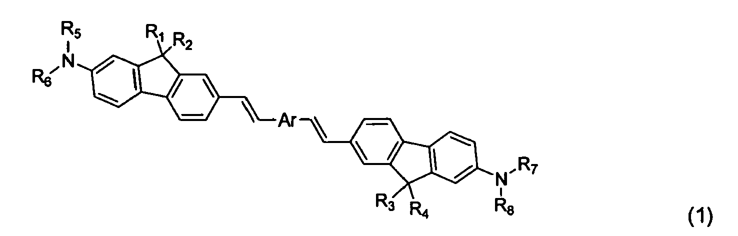 In Chemical Formula 1,
Ar represents (C6-C60)arylene, (C3-C60)heteroarylene, 5- or 6-membered heterocycloalkylene containing one or more heteroatom(s) selected from N, O and S, (C3-C60)cycloalkylene, adamantylene, (C7-C60)bicycloalkylene, (C2-C60)alkenylene, (C2-C60)alkynylene, (C1-C60)alkylenethio, (C1-C60)alkyleneoxy, (C6-C60)aryleneoxy, (C6-C60)arylenethio or 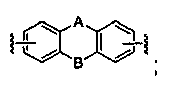
A and B independently represent a chemical bond, -(CR₁₁)(R₁₂)ₘ-, -N(R₁₃)-, -S-, -O-, -Si(R₁₄)(R₁₅)-, -P(R₁₆)-, -C(=O)-, -B(R₁₇)-, -In(R₁₈)-, -Se-, -Ge(R₁₉)(R₂₀)-, -Sn(R₂₁)(R₂₂)-, -Ga(R₂₃)-or -(R₂₄)C=C(R₂₅)-, except for the case where A and B are chemical bonds at the same time;
R₁ through R₈ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁ and R₂, R₃ and R₄, R₅ and R₆, and R₇ and R₈ may be linked via (C3-C60)alkylene or (C3-C60)alkenylene with or without a fused ring to form an alicyclic ring or a mono- or polycyclic aromatic ring;
R₁₁ through R₂₅ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁₁ and R₁₂, R₁₄ and R₁₅, R₁₉ and R₂₀, R₂₁ and R₂₂, and R₂₄ and R₂₅ may be linked via (C3-C60)alkylene or (C3-C60)alkenylene with or without a fused ring to form an alicyclic ring or a mono- or polycyclic aromatic ring;
the arylene, heteroarylene, heterocycloalkylene, cycloalkylene, adamantylene, bicycloalkylene, alkenylene, alkylenethio, alkyleneoxy, aryleneoxy or arylenethio of Ar, and the alkyl, aryl, heteroaryl, heterocycloalkyl, cycloalkyl, trialkylsilyl, dialkylarylsilyl, triarylsilyl, adamantyl, bicycloalkyl, alkenyl, aralkyl, alkyloxy, alkylthio, aryloxy, arylthio, alkylamino, arylamino, alkoxycarbonyl, alkylcarbonyl or arylcarbonyl of R₁ through R₈ and R₁₁ through R₂₅ may be further substituted by one or more substituent(s) selected from a group consisting of deuterium, halogen, (C1-C60)alkyl with or without halogen substituent(s), (C6-C60)aryl, (C3-C60)heteroaryl containing one or more heteroatom(s) selected from N, O and S with or without (C6-C60)aryl substituent(s), morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, (C1-C60)alkylamino, (C6-C60)arylamino, (C6-C60)ar(C1-C60)alkyl, (C1-C60)alkyl(C6-C60)aryl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl; and
m represents an integer from 1 to 3.
